# EUROPEAN PATENT SPECIFICATION

(11) **EP 2 319 916 B1**
(45) Date of publication and mention of the grant of the patent: **20.04.2016**
(21) Application number: 09811491.1
(22) Date of filing: 01.09.2009
(51) Int. Cl.: C12N 5/10, A61K 45/00, A61P 31/14, C12N 7/00, C12N 15/09, C12Q 1/02, G01N 33/15, G01N 33/50

(54) **CELL CAPABLE OF REPLICATING NOVEL HCV REPLICON, CELL CAPABLE OF REPLICATING FULL-LENGTH HCV RNA, AND USE OF THOSE CELLS**
ZUR REPLIKATION EINES NEUEN HCV-REPLIKONS FÄHIGE ZELLE, ZUR REPLIKATION VON HCV-RNA MIT VOLLER LÄNGE FÄHIGE ZELLE UND VERWENDUNG SOLCHER ZELLEN
CELLULE CAPABLE DE RÉPLIQUER UN NOUVEAU RÉPLICON DU VIRUS DE L'HÉPATITE C (HCV), CELLULE CAPABLE DE RÉPLIQUER UN ARN DU HCV COMPLET, ET UTILISATION DE CES CELLULES

(30) Priority: 02.09.2008 JP 2008225323
(43) Date of publication of application: 11.05.2011
(73) Proprietor: National University Corporation Okayama University, Kita-ku Okayama-shi Okayama 700-8530 (JP)
(72) Inventor: KATO, Nobuyuki, Okayama-shi, Okayama 700-8558 (JP); IKEDA, Masanori, Okayama-shi, Okayama 700-8558 (JP)
(74) Representative: Vossius & Partner Patentanwälte Rechtsanwälte mbB
(86) International application number: PCT/JP2009/065263
(87) International publication number: WO 2010/026965

(56) References cited:
- JP-A- 2006 325 582
- KATO NOBUYUKI ET AL: "Efficient replication systems for hepatitis C virus using a new human hepatoma cell line", VIRUS RESEARCH, AMSTERDAM, NL, vol. 146, no. 1-2, 29 August 2009 (2009-08-29), pages 41-50, XP009156385, ISSN: 0168-1702
- KATO N ET AL: "Susceptibility of human T-lymphotropic virus type I infected cell line MT-2 to hepatitis C virus infection", BIOCHEMICAL AND BIOPHYSICAL RESEARCH COMMUNICATIONS, ACADEMIC PRESS INC. ORLANDO, FL, US, vol. 206, no. 3, 26 January 1995 (1995-01-26), pages 863-869, XP002120166, ISSN: 0006-291X, DOI: 10.1006/BBRC.1995.1123
- ABE ET AL: "Cell culture-adaptive NS3 mutations required for the robust replication of genome-length hepatitis C virus RNA", VIRUS RESEARCH, AMSTERDAM, NL, vol. 125, no. 1, 19 March 2007 (2007-03-19), pages 88-97, XP005932557, ISSN: 0168-1702, DOI: 10.1016/J.VIRUSRES.2006.12.011
- IKEDA M ET AL: "Efficient replication of a full-length hepatitis C virus genome, strain O, in cell culture, and development of a luciferase reporter system", BIOCHEMICAL AND BIOPHYSICAL RESEARCH COMMUNICATIONS, ACADEMIC PRESS INC. ORLANDO, FL, US, vol. 329, no. 4, 22 April 2005 (2005-04-22), pages 1350-1359, XP004784881, ISSN: 0006-291X, DOI: 10.1016/J.BBRC.2005.02.138
- BARTENSCHALER R ET AL: "NOVEL CELL CULTURE SYSTEMS FOR THE HEPATITIS C VIRUS", ANTIVIRAL RESEARCH, ELSEVIER BV, NL, vol. 52, 1 October 2001 (2001-10-01), pages 1-17, XP001029117, ISSN: 0166-3542, DOI: 10.1016/S0166-3542(01)00164-4
- KATO N ET AL: "REPLICATION OF HEPATITIS C VIRUS IN CULTURED NON-NEOPLASTIC HUMAN HEPATOCYTES", JAPANESE JOURNAL OF CANCER RESEARCH, JAPANESE CANCER ASSOCIATION, TOKYO, JP, vol. 87, no. 8, 1 August 1996 (1996-08-01) , pages 787-792, XP001022326, ISSN: 0910-5050
- LOHMANN V ET AL: "REPLICATION OF SUBGENOMIC HEPATITIS C VIRUS RNAS IN A HEPATOMA CELLLINE", SCIENCE, AMERICAN ASSOCIATION FOR THE ADVANCEMENT OF SCIENCE, WASHINGTON, DC; US, vol. 285, 2 July 1999 (1999-07-02), pages 110-113, XP000960693, ISSN: 0036-8075, DOI: 10.1126/SCIENCE.285.5424.110
- KATO N. ET AL.: 'Establishment of a hepatitis C virus subgenomic replicon derived from human hepatocytes infected in vitro' BIOCHEM. BIOPHYS. RES. COMMUN. vol. 306, 2003, pages 756 - 766, XP008142769
- ABE K. ET AL.: 'Cell culture-adaptive NS3 mutations required for the robust replication of genome-length hepatitis C virus RNA' VIRUS RES. vol. 125, 2007, pages 88 - 97, XP005932557
- IKEDA M. ET AL.: 'Selectable subgenomic and genome-length dicistronic RNAs derived from an infectious molecular clone of the HCV-N strain of hepatitis C virus replicate efficiently in cultured Huh7 cells' J. VIROL. vol. 76, 2002, pages 2997 - 3006, XP002967510
- IKEDA M. ET AL.: 'Efficient replication of a full-length hepatitis C virus genome, strain O, in cell culture, and development of a luciferase reporter system' BIOCHEM. BIOPHYS. RES. COMMUN. vol. 329, 2005, pages 1350 - 1359, XP004784881
- NOBUYUKI KATO ET AL.: 'Atarashii Human Hepatoma Cell Lines Li23 o Mochiita HCV Seikatsukan Saigen System' DAI 56 KAI THE JAPANESE SOCIETY OF VIROLOGY GAKUJUTSU SHUKAI PROGRAM SHOROKU- SHU 01 October 2008, page 165, XP008142775
- KATO N. ET AL.: 'Establishment of a hepatitis C virus subgenomic replicon derived from human hepatocytes infected in vitro' BIOCHEM. BIOPHYS. RES. COMMUN. vol. 306, 2003, pages 756 - 766, XP008142769
- ABE K. ET AL.: 'Cell culture-adaptive NS3 mutations required for the robust replication of genome-length hepatitis C virus RNA' VIRUS RES. vol. 125, 2007, pages 88 - 97, XP005932557

### SEQUENCE LISTING

<110> National University Corporation Okayama University
<120> A novel cell replicating HCV replicon and a novel cell replicating full-length HCV RNA, and use thereof
<130> T1117 EP S3
<140> EP 09 81 1491.1
   <141> 2009-09-01
<150> JP 2008-225323
   <151> 2008-09-02
<160> 27
<170> PatentIn Ver. 2.1
<210> 1
   <211> 9587
   <212> DNA
   <213> Hepatitis C virus
<400> 1
   gccagccccc gattgggggc gacactccac catagatcac tcccctgtga ggaactactg 60
   tcttcacgca gaaagcgtct agccatggcg ttagtatgag tgtcgtgcag cctccaggac 120
   cccccctccc gggagagcca tagtggtctg cggaaccggt gagtacaccg gaattgccag 180
   gacgaccggg tcctttcttg gattaacccg ctcaatgcct ggagatttgg gcgtgccccc 240
   gcaagactgc tagccgagta gtgttgggtc gcgaaaggcc ttgtggtact gcctgatagg 300
   gtgcttgcga gtgccccggg aggtctcgta gaccgtgcac catgagcacg aatcctaaac 360
   ctcaaagaaa aaccaaacgt aacaccaacc gccgcccaca ggacgtcaag ttcccgggcg 420
   gtggtcagat cgttggtgga gtttacctgt tgccgcgcag gggccccagg ttgggtgtgc 480
   gcgcgactag gaagacttcc gagcggtcgc agcctcgtgg aaggcgacaa cctatcccca 540
   aggctcgcca gcccgagggc agggcctggg ctcagcccgg atacccttgg cccctctacg 600
   gcaatgaggg tctggggtgg gcaggatggc tcctgtcacc ccgtggctct cggcctagtt 660
   ggggccctac ggacccccgg cgtaggtcgc gtaatttggg taaggtcatc gataccctca 720
   catgcggctt cgccgacctc atggggtaca tcccgctcgt cggcgccccc ctaggaggcg 780
   ctgccagggc cctggcgcat ggcgtccggg ttctggagga cggcgtgaac tatgcaacag 840
   ggaatttgcc cggttgctct ttctctatct tcctcttggc tttgctgtcc tgtttgacca 900
   tcccagcttc cgcttacgaa gtgcgcaacg tatccggggt gtaccatgtc acgaacgact 960
   gctccaactc aagtattgtg tatgaggcag cggacatgat catgcacacc cccgggtgcg 1020
   tgccctgcgt tcgggaggac aattcctccc gttgctgggt agcgctcact cccacgcttg 1080
   cggccaggaa cagcagcgtc cccaccacga caatacgacg ccacgtcgac ttgctcgttg 1140
   gggcagctgc tttctgttcc gctatgtacg tgggagacct ctgcggatct gttttcctca 1200
   tttcccagct gttcaccttc tcacctcgcc ggtatgagac ggtgcaagac tgcaactgct 1260
   cgctctatcc cggccacgta tcaggtcacc gcatggcttg ggatatgatg atgaactggt 1320
   cacctacaac agccctggtg gtgtcgcagt tgctccggat cccacaagcc gtcgtggaca 1380
   tggtggcggg ggcccactgg ggagtcctgg cgggccttgc ctactattcc atggtgggga 1440
   actgggctaa ggtcttgatt gtgatgctac tttttgccgg cgtcgacggg aacactcgtg 1500
   tgtcaggggg ggaagcagcc aagaacacca tgggctttgc atccctcttt gtgtctggcc 1560
   cgtcgcagaa aatccaactt attaacacca atggcagctg gcacatcaac aggactgctc 1620
   tgaattgcga tgactccctc cacaccgggt tccttgctgc gctgttttac gcgcacaagt 1680
   tcaactcgtc cggatgctcg gggcgcatgg ccagctgccg ccccattgac gagtttgccc 1740
   aggggtgggg tcccatcact cacggtgtgc ctgacaacct ggaccagagg ccttactgct 1800
   ggcactacgc gcctcggccg tgcggtatcg tacccgcgtc gcaggtgtgt ggcccagtgt 1860
   attgcttcac cccgagccct gtcgtggtgg ggacgaccga tcgtttcggc gcccccacgt 1920
   acagctgggg ggagaatgag acggacgtgc tactccttaa caacacgcga ccgccacaag 1980
   gcaactggtt cggttgcaca tggatgaaca gcaccgggtt caccaagacg tgcgggggcc 2040
   ccccgtgcaa cattggaggg gtcggcaaca acaccttgac ctgtcctacg gactgcttcc 2100
   ggaagcaccc cgaggccact tacaccaaat gcggctcggg cccttggttg acacctaggt 2160
   gcatggttga ctacccatac agactctggc actacccttg cactgtcaat tttaccatct 2220
   tcaaggtcag gatgtatgta gggggtgtgg agcacaggct cgacgccgcg tgcaattgga 2280
   cccgaggaga gcgttgcaat gtggaggaca gggatagatc agagcttagc ccactgctac 2340
   tgtccacaac agagtggcag atactgccct gttccttcac caccctaccg gctctgtcca 2400
   ctggtttgat ccacctccac cagaacatcg tggacgtgca atacctgtac ggtgtggggt 2460
   cagtggttgt ctccgttgta atcagatggg agtatgtcgt gctgctcttc cttctcctgg 2520
   cggacgcacg cgtctgcgcc tgcttgtgga tgatgctgct gatagctcag gctgaggccg 2580
   ccttagagaa cttggtggtc ctcaatgcgg catctgtagc tggagcgcat ggcactctct 2640
   ccttccttgt gttcttctgt gccgcctggt acatcaaggg caagctggtc cctggagcgg 2700
   catatgcttt ctatggcgtg tggccgctgc tcctgctcct gctggcatta ccaccacgag 2760
   catacgccat ggaccgggag atggctgcat catgcggagg cgcggtcttc ataggtctgg 2820
   cactcctgac cttgtcacca cactataaag cgttcctcgc taggctcatt tggtggttgc 2880
   agtatcttat caccagggtc gaggcgctac tgcaagtgtg ggtcccccct cttaacgttc 2940
   gggggggccg cgatgccatc atcctcctca cgtgcgcggt ccatccagag ctaatctttg 3000
   aaatcaccaa aatcttgctc gccataattg gtccgctcat ggtgctccag gctggcttaa 3060
   ccagagtgcc gtacttcgtg cgcgctcagg ggctcatccg tgtgtgcatg ttggtgcgga 3120
   aagtcgctgg gggtcactac gtccagatgg ctctcatgag gctggccgcg ctgacgggca 3180
   cgtacgttta caaccatctt actccgctgc gggactgggc ccacgcgggc ttgcgggacc 3240
   ttgtggtggc agttgagccc gtcgtcttct ctgacatgga gaccaagatc atcacctggg 3300
   gggcagacac cgcggcgtgt ggggacatca tcttgggtct acccgtctcc gcccgaaggg 3360
   ggagggagat acttttggga ccggccgaca gttatgaagg gcagggatgg cggcttcttg 3420
   cgcctatcac ggcctattcc caacagacgc ggggcctact cggttgcatc atcactagtc 3480
   tcacaggccg ggacaggaac caggtcgagg gggaggttca ggtggtttcc accgcaacgc 3540
   agtccttctt ggcgacctgc atcaatggtg tgtgttggac tgtctaccat ggtgccggct 3600
   caaagaccct agccggccca aagggtccga tcacccaaat gtacaccaat gtagaccagg 3660
   acctcgtcgg ctggcaggcg ccccccgggg cgcgttcctt aacaccatgc acctgcggca 3720
   gctcggacct ctacttggtc acgaggcatg ctgatgtcat tccggtgcgc cggcggggcg 3780
   ataacagggg cagcctgctt tcccccagac ctgtctctta cttaaagggc tcctcgggtg 3840
   gtccactgct ctgcccctca gggcacgctg tgggcatctt ccgggccgcc gtgtgcacca 3900
   ggggggttgc gaaggcggtg gactttgtgc ccgttgagtc catggaaact accatgcggt 3960
   ctccggtctt cacggacaat tcatctcccc cggccgtacc gcagacattc caagtggccc 4020
   atctacacgc tcccactggc agcggtaaga gcactaaggt gccggctgct tatgcagccc 4080
   aagggtacaa ggtactcgtc ctgaacccat ccgttgccgc caccttaggt tttggggcgt 4140
   atatgtctaa agcacatggt gttgacccca acatcagaac tggggtaagg accatcacca 4200
   caggcgctcc cattacgtat tccacttatg gcaagttcct tgccgacggt ggttgctctg 4260
   ggggcgccta tgacatcata atatgtgatg agtgccactc aactgactcg acttccatct 4320
   tgggcatcgg cactgtcttg gaccaagcgg agacggctgg agcgcggctc gtcgtgctcg 4380
   ccaccgctac acctccggga tcagtcaccg tgccacatcc caatatcgag gaggtggctt 4440
   tgtccaacac tggagagatc cccttttatg gtaaggcctt tcccatcgag gccatcaagg 4500
   gggggaggca tctcattttc tgccactcca agaagaaatg tgacgagctc gccacaaagc 4560
   tgtcggccct tggaatcaac gctgtagcgt actaccgggg tcttgatgtg tccgtcatac 4620
   cgacaagcgg agatgtcgtt gtcgtggcaa cagacgctct gatgacgggc tacactggcg 4680
   actttgactc agtgatcgac tgtaacacat gtgtcaccca gacagtcgac ttcagcttgg 4740
   accctacctt caccattgag acgacgaccg tgccccaaga cgcggtatcg cgctcgcaac 4800
   ggcgaggcag gactggtagg ggcagggggg gcatatacag gtttgtgact ccaggggaac 4860
   ggccctcggg catgttcgat tcttcggtcc tgtgcgagtg ctatgacgcg ggctgtgctt 4920
   ggtacgagct cacgcccgcc gagacctcgg ttaggttgcg ggcttaccta aatacaccag 4980
   ggttgcccgt ctgccaggac catctggagt tctgggagag cgtcttcaca ggcctcaccc 5040
   acatagatgc ccatttctta tcccagacca agcaggcagg agacaacttc ccctacctgg 5100
   tagcgtacca ggctacggtg tgcgccaggg cccaggcccc accaccatcg tgggaccaaa 5160
   tgtggaagtg tctcatacgg ctaaagccta cactgcacgg gccaacaccc ctgctgtaca 5220
   ggctaggagc cgtccaaaac gaagtcactc tcacacaccc cataaccaaa ttcatcatgg 5280
   catgcatgtc ggctgaccta gaggtcgtca cgagcacctg ggtgctagta ggcggggtcc 5340
   ttgcagctct ggccgcgtac tgcctgacaa cgggcagcgt ggtcattgtg ggtaggatca 5400
   tcttgtctgg gaagccggct gtcattcccg atagggaagt gctctaccag gagttcgatg 5460
   aaatggaaga gtgcgcctcg cacctccctt acattgaaca gggaatgcag ctcgccgagc 5520
   aattcaagca gaaggcgctc gggttgctgc aagcagccac caagcaagcg gaggctgctg 5580
   ctcccgtggt ggagtccaag tggcaagccc ttgaggcctt ttgggcgaag cacatgtgga 5640
   atttcatcag cgggatacag tacttagcag gcttgtccac tctgcctggg aaccccccga 5700
   tagcttcact gatggcattc acagcctcta tcaccagccc gctcaccacc cagcataccc 5760
   tcttgtttaa catcttgggg gggtgggtgg cagcccaact cgcccccccc agcgctgctt 5820
   cagctttcgt gggcgccggg atcgctggcg cggctgttgg cagcataggc cttgggaagg 5880
   tgcttgtgga catcctggcg ggctatgggg cgggggtggc aggcgcgctc gtggccttta 5940
   aggtcatgag cggcgaaatg ccctccaccg aggacctagt caacttgctc cctgccatcc 6000
   tctcccccgg tgccctggtt gtcggggtcg tgtgcgcggc aatactgcgc cggcacgtgg 6060
   gcccggggga gggggctgtg cagtggatga accggctgat agcgttcgct tcgcggggta 6120
   accatgtttc ccccacgcac tatgtgcctg agagcgacgc ggccgcgcgt gtcactcaga 6180
   tcctctccag ccttaccatc acccagctgt tgaagagact ccaccagtgg atcaatgagg 6240
   actgctccac gccatgctcc ggctcgtggc tcagggatgt ttgggactgg atatgcacgg 6300
   tgttgactga cttcaagacc tggctccagt ccaagctcct gccacggttg ccgggggtcc 6360
   ctttcctttc atgccaacgt ggatataagg gagtctggcg aggagatggc gtcatgcaca 6420
   ccacttgccc atgtggcgca caaatcaccg gacatgtcaa aaacggttcc atgaggatcg 6480
   ttgggcctaa aacctgtagc aacacgtggc acggaacatt ccccatcaac gcgtacacca 6540
   cgggttcctg cacaccctcc ccggcgccaa actattccag ggcgttgtgg cgggtagctg 6600
   ctgaggagta cgtggaggtc acgcgggtgg gggatttcca ctacgtgacg ggcatgacca 6660
   ctgacaacat aaaatgccca tgccaggttc cggcccccga atttttcaca gagttggacg 6720
   gggtgcggct gcacaggtac gctccggcgt gcaaacctct cttacgggat gaggtcacat 6780
   tccaggtcgg gctcaaccaa tacgtggttg ggtcacagct tccatgcgag cccgaaccgg 6840
   atgtaacagt gctcacttcc atgctcaccg acccctccca cattacagca gagacggcta 6900
   agcgtaggct ggccaggggg tctcccccct ccttggccag ctcttcagct agtcagttgt 6960
   ctgcgccttc tctgaaggcg acatgcacca cccatcatga ctccccagac gccgacctca 7020
   tcgaggccaa cctcctgtgg cggcaggaga tgggcgggaa catcacccgc gtggagtcag 7080
   agaacaaagt agtgattctg gactctttcg acccgcttcg agcggaggag gatgagaggg 7140
   aagtatccgt tccggcggag atcctgcgga aaaccaggaa attcccctca gcgttgccca 7200
   tatgggcacg cccggactac aacccaccac tgttagagtc ttggagagac ccggactatg 7260
   tccctccggt ggtacacggg tgcccgttac cgcccaccaa ggcccctcca ataccacctc 7320
   cacggagaaa gagaacagtt atcctgacag aatccactgt gtcttctgcc ttggcggagc 7380
   ttgctacaaa gaccttcggc agctccggat cgtcggccgt cgacagcggc acagcgactg 7440
   cccctcctga tgggccctcc gacgacggtg atgcaggatc ggacgctgag tcatactcct 7500
   ccatgccccc ccttgagggg gagccggggg atcccgatct cagcgacggg tcttggtcca 7560
   ctgtgagcga ggaggccagt gaggacgtcg tctgctgctc aatgtcctac acatggacag 7620
   gtgccttaat cacgccatgc gccgcggagg agagcaagct gcccatcaat gcgttgagca 7680
   actctttgct gcgccaccac aacatggtct atgctacaac atcccgcagc gcaagccagc 7740
   ggcagaaaaa ggtcaccttt gacagactgc aagtcctgga cgaccactac cgggacgtgc 7800
   tcaaggagat gaaggcgaag gcgtccacag ttaaggccaa acttctatcc gtagaagagg 7860
   cctgcaagct gacgccccca cattcagcca gatctaagtt tggctatggg gcaaaggacg 7920
   tccggaacct atccagtaag gccgttaacc acatccactc cgtgtggaag gacttgctgg 7980
   aagacactga gacaccaatt gataccaccg tcatggcaaa aaatgaggtc ttctgtgtcc 8040
   aaccagagaa aggaggccgc aagccagctc gccttatcgt gttcccagac ctgggggtgc 8100
   gtgtgtgcga gaaaatggcc ctttatgatg tggtctccac tcttcctcag gccgtgatgg 8160
   gctcctcata cggattccaa tactctcctg gacagcgggt cgagttcctg gtgaatgcct 8220
   ggaaatcaaa gaagacccct atgggcttcg catatgacac ccgctgtttt gactcaacag 8280
   tcactgagaa tgacatccgt gttgaggagt caatttacca atgttgtgac ttggcccccg 8340
   aggccagaca ggccataagg tcgctcacag agcggcttta tatcgggggc cccctgacta 8400
   attcaaaagg gcagaactgc ggttatcgcc ggtgccgcgc cagcggcgtg ctgacgacta 8460
   gctgcggtaa caccctcaca tgttacttga aggcctctgc agcctgtcga gctgcaaagc 8520
   tccaggactg cacgatgctc gtgtgcggag acgacctcgt cgttatctgt gagagcgcgg 8580
   ggacccagga ggacgcggcg agcctgcgag tcttcacgga ggctatgacc aggtactctg 8640
   ccccccccgg ggacccgccc caaccggaat acgacttgga gctgataaca tcatgctcct 8700
   ccaacgtgtc ggtcgcgcac gatgcatctg gcaagcgggt gtactatctc acccgcgacc 8760
   ccaccacccc tcttgctcgg gctgcgtggg agacagctag acacactcca gtcaactcct 8820
   ggctaggcaa catcatcatg tatgcgccca ccttatgggc aaggatgatt ctgatgaccc 8880
   acttcttctc aatccttcta gcccaggagc aacttgagaa agccctagat tgccagatct 8940
   acggggccac ttactccatt gagccacttg acctacctca gatcattcaa cgactccatg 9000
   gtcttagcgc attttcactc catagttact ctccaggtga gatcaatagg gtggcttcat 9060
   gcctcaggaa acttggggta ccgcccttgc gagtctggag acatcgggcc agaagtgtcc 9120
   gcgctaagct actgtcccag ggggggaggg ccgccacgtg tggcaagtac ctcttcaact 9180
   gggccgtacg gaccaagctc aaactcactc caattccggc tgcgtcccgg ttggacttgt 9240
   ccggctggtt cgtcgccggt tacagcgggg gagacatata tcacagcctg tctcgcgccc 9300
   gaccccgctg gttcatgtgg tgcctactcc tactttccgt aggggtaggc atctacctgc 9360
   tccccaaccg gtgaacgggg agctaaacac tccaggccaa taggccattc cttttttttt 9420
   tttttttttt ttttttcttt ttttttcctt tttttttttt tttttttttt ttccttttct 9480
   ttcttctttg gtggctccat cttagcccta gtcacggcta gctgtgaaag gtccgtgagc 9540
   cgcatgactg cagagagtgc tgatactggc ctctctgcag atcatgt 9587
<210> 2
   <211> 3010
   <212> PRT
   <213> Hepatitis C virus
<400> 2
   Met Ser Thr Asn Pro Lys Pro Gln Arg Lys Thr Lys Arg Asn Thr Asn
    1               5                  10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Gln Pro Glu Gly Arg Ala Trp Ala Gln Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Ile Pro Ala Ser Ala Tyr
               180                 185                 190
   Glu Val Arg Asn Val Ser Gly Val Tyr His Val Thr Asn Asp Cys Ser
           195                 200                 205
   Asn Ser Ser Ile Val Tyr Glu Ala Ala Asp Met Ile Met His Thr Pro
       210                 215                 220
   Gly Cys Val Pro Cys Val Arg Glu Asp Asn Ser Ser Arg Cys Trp Val
   225                 230                 235                 240
   Ala Leu Thr Pro Thr Leu Ala Ala Arg Asn Ser Ser Val Pro Thr Thr
                   245                 250                 255
   Thr Ile Arg Arg His Val Asp Leu Leu Val Gly Ala Ala Ala Phe Cys
               260                 265                 270
   Ser Ala Met Tyr Val Gly Asp Leu Cys Gly Ser Val Phe Leu Ile Ser
           275                 280                 285
   Gln Leu Phe Thr Phe Ser Pro Arg Arg Tyr Glu Thr Val Gln Asp Cys
       290                 295                 300
   Asn Cys Ser Leu Tyr Pro Gly His Val Ser Gly His Arg Met Ala Trp
   305                 310                 315                 320
   Asp Met Met Met Asn Trp Ser Pro Thr Thr Ala Leu Val Val Ser Gln
                   325                 330                 335
   Leu Leu Arg Ile Pro Gln Ala Val Val Asp Met Val Ala Gly Ala His
               340                 345                 350
   Trp Gly Val Leu Ala Gly Leu Ala Tyr Tyr Ser Met Val Gly Asn Trp
           355                 360                 365
   Ala Lys Val Leu Ile Val Met Leu Leu Phe Ala Gly Val Asp Gly Asn
       370                 375                 380
   Thr Arg Val Ser Gly Gly Glu Ala Ala Lys Asn Thr Met Gly Phe Ala
   385                 390                 395                 400
   Ser Leu Phe Val Ser Gly Pro Ser Gln Lys Ile Gln Leu Ile Asn Thr
                   405                 410                 415
   Asn Gly Ser Trp His Ile Asn Arg Thr Ala Leu Asn Cys Asp Asp Ser
               420                 425                 430
   Leu His Thr Gly Phe Leu Ala Ala Leu Phe Tyr Ala His Lys Phe Asn
           435                 440                 445
   Ser Ser Gly Cys Ser Gly Arg Met Ala Ser Cys Arg Pro Ile Asp Glu
       450                 455                 460
   Phe Ala Gln Gly Trp Gly Pro Ile Thr His Gly Val Pro Asp Asn Leu
   465                 470                 475                 480
   Asp Gln Arg Pro Tyr Cys Trp His Tyr Ala Pro Arg Pro Cys Gly Ile
                   485                 490                 495
   Val Pro Ala Ser Gln Val Cys Gly Pro Val Tyr Cys Phe Thr Pro Ser
               500                 505                 510
   Pro Val Val Val Gly Thr Thr Asp Arg Phe Gly Ala Pro Thr Tyr Ser
           515                 520                 525
   Trp Gly Glu Asn Glu Thr Asp Val Leu Leu Leu Asn Asn Thr Arg Pro
       530                 535                 540
   Pro Gln Gly Asn Trp Phe Gly Cys Thr Trp Met Asn Ser Thr Gly Phe
   545                 550                 555                 560
   Thr Lys Thr Cys Gly Gly Pro Pro Cys Asn Ile Gly Gly Val Gly Asn
                   565                 570                 575
   Asn Thr Leu Thr Cys Pro Thr Asp Cys Phe Arg Lys His Pro Glu Ala
               580                 585                 590
   Thr Tyr Thr Lys Cys Gly Ser Gly Pro Trp Leu Thr Pro Arg Cys Met
           595                 600                 605
   Val Asp Tyr Pro Tyr Arg Leu Trp His Tyr Pro Cys Thr Val Asn Phe
       610                 615                 620
   Thr Ile Phe Lys Val Arg Met Tyr Val Gly Gly Val Glu His Arg Leu
   625                 630                 635                 640
   Asp Ala Ala Cys Asn Trp Thr Arg Gly Glu Arg Cys Asn Val Glu Asp
                   645                 650                 655
   Arg Asp Arg Ser Glu Leu Ser Pro Leu Leu Leu Ser Thr Thr Glu Trp
   660 665 670
   Gln Ile Leu Pro Cys Ser Phe Thr Thr Leu Pro Ala Leu Ser Thr Gly
   675 680 685
   Leu Ile His Leu His Gln Asn Ile Val Asp Val Gln Tyr Leu Tyr Gly
   690 695 700
   Val Gly Ser Val Val Val Ser Val Val Ile Arg Trp Glu Tyr Val Val
   705 710 715 720
   Leu Leu Phe Leu Leu Leu Ala Asp Ala Arg Val Cys Ala Cys Leu Trp
   725 730 735
   Met Met Leu Leu Ile Ala Gln Ala Glu Ala Ala Leu Glu Asn Leu Val
   740 745 750
   Val Leu Asn Ala Ala Ser Val Ala Gly Ala His Gly Thr Leu Ser Phe
   755 760 765
   Leu Val Phe Phe Cys Ala Ala Trp Tyr Ile Lys Gly Lys Leu Val Pro
   770 775 780
   Gly Ala Ala Tyr Ala Phe Tyr Gly Val Trp Pro Leu Leu Leu Leu Leu
   785 790 795 800
   Leu Ala Leu Pro Pro Arg Ala Tyr Ala Met Asp Arg Glu Met Ala Ala
   805 810 815
   Ser Cys Gly Gly Ala Val Phe Ile Gly Leu Ala Leu Leu Thr Leu Ser
   820 825 830
   Pro His Tyr Lys Ala Phe Leu Ala Arg Leu Ile Trp Trp Leu Gln Tyr
   835 840 845
   Leu Ile Thr Arg Val Glu Ala Leu Leu Gln Val Trp Val Pro Pro Leu
   850 855 860
   Asn Val Arg Gly Gly Arg Asp Ala Ile Ile Leu Leu Thr Cys Ala Val
   865 870 875 880
   His Pro Glu Leu Ile Phe Glu Ile Thr Lys Ile Leu Leu Ala Ile Ile
   885 890 895
   Gly Pro Leu Met Val Leu Gln Ala Gly Leu Thr Arg Val Pro Tyr Phe
   900 905 910
   Val Arg Ala Gln Gly Leu Ile Arg Val Cys Met Leu Val Arg Lys Val
   915 920 925
   Ala Gly Gly His Tyr Val Gln Met Ala Leu Met Arg Leu Ala Ala Leu
   930 935 940
   Thr Gly Thr Tyr Val Tyr Asn His Leu Thr Pro Leu Arg Asp Trp Ala
   945 950 955 960
   His Ala Gly Leu Arg Asp Leu Val Val Ala Val Glu Pro Val Val Phe
   965 970 975
   Ser Asp Met Glu Thr Lys Ile Ile Thr Trp Gly Ala Asp Thr Ala Ala
   980 985 990
   Cys Gly Asp Ile Ile Leu Gly Leu Pro Val Ser Ala Arg Arg Gly Arg
   995 1000 1005
   Glu Ile Leu Leu Gly Pro Ala Asp Ser Tyr Glu Gly Gln Gly Trp Arg
   1010 1015 1020
   Leu Leu Ala Pro Ile Thr Ala Tyr Ser Gln Gln Thr Arg Gly Leu Leu
   1025 1030 1035 1040
   Gly Cys Ile Ile Thr Ser Leu Thr Gly Arg Asp Arg Asn Gln Val Glu
   1045 1050 1055
   Gly Glu Val Gln Val Val Ser Thr Ala Thr Gln Ser Phe Leu Ala Thr
   1060 1065 1070
   Cys Ile Asn Gly Val Cys Trp Thr Val Tyr His Gly Ala Gly Ser Lys
   1075 1080 1085
   Thr Leu Ala Gly Pro Lys Gly Pro Ile Thr Gln Met Tyr Thr Asn Val
   1090 1095 1100
   Asp Gln Asp Leu Val Gly Trp Gln Ala Pro Pro Gly Ala Arg Ser Leu
   1105 1110 1115 1120
   Thr Pro Cys Thr Cys Gly Ser Ser Asp Leu Tyr Leu Val Thr Arg His
   1125 1130 1135
   Ala Asp Val Ile Pro Val Arg Arg Arg Gly Asp Asn Arg Gly Ser Leu
   1140 1145 1150
   Leu Ser Pro Arg Pro Val Ser Tyr Leu Lys Gly Ser Ser Gly Gly Pro
   1155 1160 1165
   Leu Leu Cys Pro Ser Gly His Ala Val Gly Ile Phe Arg Ala Ala Val
   1170 1175 1180
   Cys Thr Arg Gly Val Ala Lys Ala Val Asp Phe Val Pro Val Glu Ser
   1185 1190 1195 1200
   Met Glu Thr Thr Met Arg Ser Pro Val Phe Thr Asp Asn Ser Ser Pro
   1205 1210 1215
   Pro Ala Val Pro Gln Thr Phe Gln Val Ala His Leu His Ala Pro Thr
   1220 1225 1230
   Gly Ser Gly Lys Ser Thr Lys Val Pro Ala Ala Tyr Ala Ala Gln Gly
   1235 1240 1245
   Tyr Lys Val Leu Val Leu Asn Pro Ser Val Ala Ala Thr Leu Gly Phe
   1250 1255 1260
   Gly Ala Tyr Met Ser Lys Ala His Gly Val Asp Pro Asn Ile Arg Thr
   1265 1270 1275 1280
   Gly Val Arg Thr Ile Thr Thr Gly Ala Pro Ile Thr Tyr Ser Thr Tyr
   1285 1290 1295
   Gly Lys Phe Leu Ala Asp Gly Gly Cys Ser Gly Gly Ala Tyr Asp Ile
   1300 1305 1310
   Ile Ile Cys Asp Glu Cys His Ser Thr Asp Ser Thr Ser Ile Leu Gly
   1315 1320 1325
   Ile Gly Thr Val Leu Asp Gln Ala Glu Thr Ala Gly Ala Arg Leu Val
      1330                1335                1340
   Val Leu Ala Thr Ala Thr Pro Pro Gly Ser Val Thr Val Pro His Pro
   1345                1350                1355                1360
   Asn Ile Glu Glu Val Ala Leu Ser Asn Thr Gly Glu Ile Pro Phe Tyr
                   1365                1370                1375
   Gly Lys Ala Phe Pro Ile Glu Ala Ile Lys Gly Gly Arg His Leu Ile
               1380                1385                1390
   Phe Cys His Ser Lys Lys Lys Cys Asp Glu Leu Ala Thr Lys Leu Ser
           1395                1400                1405
   Ala Leu Gly Ile Asn Ala Val Ala Tyr Tyr Arg Gly Leu Asp Val Ser
      1410                1415                1420
   Val Ile Pro Thr Ser Gly Asp Val Val Val Val Ala Thr Asp Ala Leu
   1425                1430                1435                1440
   Met Thr Gly Tyr Thr Gly Asp Phe Asp Ser Val Ile Asp Cys Asn Thr
                   1445                1450                1455
   Cys Val Thr Gln Thr Val Asp Phe Ser Leu Asp Pro Thr Phe Thr Ile
               1460                1465                1470
   Glu Thr Thr Thr Val Pro Gln Asp Ala Val Ser Arg Ser Gln Arg Arg
           1475                1480                1485
   Gly Arg Thr Gly Arg Gly Arg Gly Gly Ile Tyr Arg Phe Val Thr Pro
      1490                1495                1500
   Gly Glu Arg Pro Ser Gly Met Phe Asp Ser Ser Val Leu Cys Glu Cys
   1505                1510                1515                1520
   Tyr Asp Ala Gly Cys Ala Trp Tyr Glu Leu Thr Pro Ala Glu Thr Ser
                   1525                1530                1535
   Val Arg Leu Arg Ala Tyr Leu Asn Thr Pro Gly Leu Pro Val Cys Gln
               1540                1545                1550
   Asp His Leu Glu Phe Trp Glu Ser Val Phe Thr Gly Leu Thr His Ile
           1555                1560                1565
   Asp Ala His Phe Leu Ser Gln Thr Lys Gln Ala Gly Asp Asn Phe Pro
      1570                1575                1580
   Tyr Leu Val Ala Tyr Gln Ala Thr Val Cys Ala Arg Ala Gln Ala Pro
   1585                1590                1595                1600
   Pro Pro Ser Trp Asp Gln Met Trp Lys Cys Leu Ile Arg Leu Lys Pro
                   1605                1610                1615
   Thr Leu His Gly Pro Thr Pro Leu Leu Tyr Arg Leu Gly Ala Val Gln
               1620                1625                1630
   Asn Glu Val Thr Leu Thr His Pro Ile Thr Lys Phe Ile Met Ala Cys
           1635                1640                1645
   Met Ser Ala Asp Leu Glu Val Val Thr Ser Thr Trp Val Leu Val Gly
      1650                1655                1660
   Gly Val Leu Ala Ala Leu Ala Ala Tyr Cys Leu Thr Thr Gly Ser Val
   1665                1670                1675                1680
   Val Ile Val Gly Arg Ile Ile Leu Ser Gly Lys Pro Ala Val Ile Pro
                   1685                1690                1695
   Asp Arg Glu Val Leu Tyr Gln Glu Phe Asp Glu Met Glu Glu Cys Ala
               1700                1705                1710
   Ser His Leu Pro Tyr Ile Glu Gln Gly Met Gln Leu Ala Glu Gln Phe
           1715                1720                1725
   Lys Gln Lys Ala Leu Gly Leu Leu Gln Ala Ala Thr Lys Gln Ala Glu
       1730                1735                1740
   Ala Ala Ala Pro Val Val Glu Ser Lys Trp Gln Ala Leu Glu Ala Phe
   1745                1750                1755                1760
   Trp Ala Lys His Met Trp Asn Phe Ile Ser Gly Ile Gln Tyr Leu Ala
                   1765                1770                1775
   Gly Leu Ser Thr Leu Pro Gly Asn Pro Pro Ile Ala Ser Leu Met Ala
               1780                1785                1790
   Phe Thr Ala Ser Ile Thr Ser Pro Leu Thr Thr Gln His Thr Leu Leu
           1795                1800                1805
   Phe Asn Ile Leu Gly Gly Trp Val Ala Ala Gln Leu Ala Pro Pro Ser
       1810                1815                1820
   Ala Ala Ser Ala Phe Val Gly Ala Gly Ile Ala Gly Ala Ala Val Gly
   1825                1830                1835                1840
   Ser Ile Gly Leu Gly Lys Val Leu Val Asp Ile Leu Ala Gly Tyr Gly
                   1845                1850                1855
   Ala Gly Val Ala Gly Ala Leu Val Ala Phe Lys Val Met Ser Gly Glu
               1860                1865                1870
   Met Pro Ser Thr Glu Asp Leu Val Asn Leu Leu Pro Ala Ile Leu Ser
           1875                1880                1885
   Pro Gly Ala Leu Val Val Gly Val Val Cys Ala Ala Ile Leu Arg Arg
       1890                1895                1900
   His Val Gly Pro Gly Glu Gly Ala Val Gln Trp Met Asn Arg Leu Ile
   1905                1910                1915                1920
   Ala Phe Ala Ser Arg Gly Asn His Val Ser Pro Thr His Tyr Val Pro
                   1925                1930                1935
   Glu Ser Asp Ala Ala Ala Arg Val Thr Gln Ile Leu Ser Ser Leu Thr
               1940                1945                1950
   Ile Thr Gln Leu Leu Lys Arg Leu His Gln Trp Ile Asn Glu Asp Cys
           1955                1960                1965
   Ser Thr Pro Cys Ser Gly Ser Trp Leu Arg Asp Val Trp Asp Trp Ile
       1970                1975                1980
   Cys Thr Val Leu Thr Asp Phe Lys Thr Trp Leu Gln Ser Lys Leu Leu
   1985                1990                1995                2000
   Pro Arg Leu Pro Gly Val Pro Phe Leu Ser Cys Gln Arg Gly Tyr Lys
                   2005                2010                2015
   Gly Val Trp Arg Gly Asp Gly Val Met His Thr Thr Cys Pro Cys Gly
               2020                2025                2030
   Ala Gln Ile Thr Gly His Val Lys Asn Gly Ser Met Arg Ile Val Gly
           2035                2040                2045
   Pro Lys Thr Cys Ser Asn Thr Trp His Gly Thr Phe Pro Ile Asn Ala
       2050                2055                2060
   Tyr Thr Thr Gly Ser Cys Thr Pro Ser Pro Ala Pro Asn Tyr Ser Arg
   2065                2070                2075                2080
   Ala Leu Trp Arg Val Ala Ala Glu Glu Tyr Val Glu Val Thr Arg Val
                   2085                2090                2095
   Gly Asp Phe His Tyr Val Thr Gly Met Thr Thr Asp Asn Ile Lys Cys
               2100                2105                2110
   Pro Cys Gln Val Pro Ala Pro Glu Phe Phe Thr Glu Leu Asp Gly Val
           2115                2120                2125
   Arg Leu His Arg Tyr Ala Pro Ala Cys Lys Pro Leu Leu Arg Asp Glu
       2130                2135                2140
   Val Thr Phe Gln Val Gly Leu Asn Gln Tyr Val Val Gly Ser Gln Leu
   2145                2150                2155                2160
   Pro Cys Glu Pro Glu Pro Asp Val Thr Val Leu Thr Ser Met Leu Thr
                   2165                2170                2175
   Asp Pro Ser His Ile Thr Ala Glu Thr Ala Lys Arg Arg Leu Ala Arg
               2180                2185                2190
   Gly Ser Pro Pro Ser Leu Ala Ser Ser Ser Ala Ser Gln Leu Ser Ala
           2195                2200                2205
   Pro Ser Leu Lys Ala Thr Cys Thr Thr His His Asp Ser Pro Asp Ala
       2210                2215                2220
   Asp Leu Ile Glu Ala Asn Leu Leu Trp Arg Gln Glu Met Gly Gly Asn
   2225                2230                2235                2240
   Ile Thr Arg Val Glu Ser Glu Asn Lys Val Val Ile Leu Asp Ser Phe
                   2245                2250                2255
   Asp Pro Leu Arg Ala Glu Glu Asp Glu Arg Glu Val Ser Val Pro Ala
               2260                2265                2270
   Glu Ile Leu Arg Lys Thr Arg Lys Phe Pro Ser Ala Leu Pro Ile Trp
           2275                2280                2285
   Ala Arg Pro Asp Tyr Asn Pro Pro Leu Leu Glu Ser Trp Arg Asp Pro
       2290                2295                2300
   Asp Tyr Val Pro Pro Val Val His Gly Cys Pro Leu Pro Pro Thr Lys
   2305                2310                2315                2320
   Ala Pro Pro Ile Pro Pro Pro Arg Arg Lys Arg Thr Val Ile Leu Thr
                   2325                2330                2335
   Glu Ser Thr Val Ser Ser Ala Leu Ala Glu Leu Ala Thr Lys Thr Phe
               2340                2345                2350
   Gly Ser Ser Gly Ser Ser Ala Val Asp Ser Gly Thr Ala Thr Ala Pro
           2355                2360                2365
   Pro Asp Gly Pro Ser Asp Asp Gly Asp Ala Gly Ser Asp Ala Glu Ser
       2370                2375                2380
   Tyr Ser Ser Met Pro Pro Leu Glu Gly Glu Pro Gly Asp Pro Asp Leu
   2385                2390                2395                2400
   Ser Asp Gly Ser Trp Ser Thr Val Ser Glu Glu Ala Ser Glu Asp Val
                   2405                2410                2415
   Val Cys Cys Ser Met Ser Tyr Thr Trp Thr Gly Ala Leu Ile Thr Pro
               2420                2425                2430
   Cys Ala Ala Glu Glu Ser Lys Leu Pro Ile Asn Ala Leu Ser Asn Ser
           2435                2440                2445
   Leu Leu Arg His His Asn Met Val Tyr Ala Thr Thr Ser Arg Ser Ala
       2450                2455                2460
   Ser Gln Arg Gln Lys Lys Val Thr Phe Asp Arg Leu Gln Val Leu Asp
   2465                2470                2475                2480
   Asp His Tyr Arg Asp Val Leu Lys Glu Met Lys Ala Lys Ala Ser Thr
                   2485                2490                2495
   Val Lys Ala Lys Leu Leu Ser Val Glu Glu Ala Cys Lys Leu Thr Pro
               2500                2505                2510
   Pro His Ser Ala Arg Ser Lys Phe Gly Tyr Gly Ala Lys Asp Val Arg
           2515                2520                2525
   Asn Leu Ser Ser Lys Ala Val Asn His Ile His Ser Val Trp Lys Asp
       2530                2535                2540
   Leu Leu Glu Asp Thr Glu Thr Pro Ile Asp Thr Thr Val Met Ala Lys
   2545                2550                2555                2560
   Asn Glu Val Phe Cys Val Gln Pro Glu Lys Gly Gly Arg Lys Pro Ala
                   2565                2570                2575
   Arg Leu Ile Val Phe Pro Asp Leu Gly Val Arg Val Cys Glu Lys Met
               2580                2585                2590
   Ala Leu Tyr Asp Val Val Ser Thr Leu Pro Gln Ala Val Met Gly Ser
           2595                2600                2605
   Ser Tyr Gly Phe Gln Tyr Ser Pro Gly Gln Arg Val Glu Phe Leu Val
       2610                2615                2620
   Asn Ala Trp Lys Ser Lys Lys Thr Pro Met Gly Phe Ala Tyr Asp Thr
   2625                2630                2635                2640
   Arg Cys Phe Asp Ser Thr Val Thr Glu Asn Asp Ile Arg Val Glu Glu
                   2645                2650                2655
   Ser Ile Tyr Gln Cys Cys Asp Leu Ala Pro Glu Ala Arg Gln Ala Ile
               2660                2665                2670
   Arg Ser Leu Thr Glu Arg Leu Tyr Ile Gly Gly Pro Leu Thr Asn Ser
           2675                2680                2685
   Lys Gly Gln Asn Cys Gly Tyr Arg Arg Cys Arg Ala Ser Gly Val Leu
       2690                2695                2700
   Thr Thr Ser Cys Gly Asn Thr Leu Thr Cys Tyr Leu Lys Ala Ser Ala
   2705                2710                2715                2720
   Ala Cys Arg Ala Ala Lys Leu Gln Asp Cys Thr Met Leu Val Cys Gly
                   2725                2730                2735
   Asp Asp Leu Val Val Ile Cys Glu Ser Ala Gly Thr Gln Glu Asp Ala
               2740                2745                2750
   Ala Ser Leu Arg Val Phe Thr Glu Ala Met Thr Arg Tyr Ser Ala Pro
           2755                2760                2765
   Pro Gly Asp Pro Pro Gln Pro Glu Tyr Asp Leu Glu Leu Ile Thr Ser
       2770                2775                2780
   Cys Ser Ser Asn Val Ser Val Ala His Asp Ala Ser Gly Lys Arg Val
   2785                2790                2795                2800
   Tyr Tyr Leu Thr Arg Asp Pro Thr Thr Pro Leu Ala Arg Ala Ala Trp
                   2805                2810                2815
   Glu Thr Ala Arg His Thr Pro Val Asn Ser Trp Leu Gly Asn Ile Ile
               2820                2825                2830
   Met Tyr Ala Pro Thr Leu Trp Ala Arg Met Ile Leu Met Thr His Phe
           2835                2840                2845
   Phe Ser Ile Leu Leu Ala Gln Glu Gln Leu Glu Lys Ala Leu Asp Cys
       2850                2855                2860
   Gln Ile Tyr Gly Ala Thr Tyr Ser Ile Glu Pro Leu Asp Leu Pro Gln
   2865                2870                2875                2880
   Ile Ile Gln Arg Leu His Gly Leu Ser Ala Phe Ser Leu His Ser Tyr
                   2885                2890                2895
   Ser Pro Gly Glu Ile Asn Arg Val Ala Ser Cys Leu Arg Lys Leu Gly
               2900                2905                2910
   Val Pro Pro Leu Arg Val Trp Arg His Arg Ala Arg Ser Val Arg Ala
           2915                2920                2925
   Lys Leu Leu Ser Gln Gly Gly Arg Ala Ala Thr Cys Gly Lys Tyr Leu
       2930                2935                2940
   Phe Asn Trp Ala Val Arg Thr Lys Leu Lys Leu Thr Pro Ile Pro Ala
   2945                2950                2955                2960
   Ala Ser Arg Leu Asp Leu Ser Gly Trp Phe Val Ala Gly Tyr Ser Gly
                   2965                2970                2975
   Gly Asp Ile Tyr His Ser Leu Ser Arg Ala Arg Pro Arg Trp Phe Met
               2980                2985                2990
   Trp Cys Leu Leu Leu Leu Ser Val Gly Val Gly Ile Tyr Leu Leu Pro
           2995                3000                3005
   Asn Arg
      3010
<210> 3
   <211> 6168
   <212> DNA
   <213> Hepatitis C virus
<400> 3
   gcgcccatca cggcttactc ccaacagacg cggggcctac tcggttgcat catcactagt 60
   ctcacaggcc gggacaggaa ccaggtcgag ggggaggttc aggtggtttc caccgcaacg 120
   cagtccttct tggcgacctg catcaatggt gtgtgttgga ctgtctacca tggtgccggc 180
   tcaaagaccc tagccggccc aaagggtccg atcacccaaa tgtacaccaa tgtagaccag 240
   gacctcgtcg gctggcgggc gccccccggg gcgcgttcct taacaccatg cacctgcggc 300
   agctcggacc tctacttggt cacgaggcat gctgatgtca ttccggtgcg ccggcggggc 360
   gataacaggg gcagcctgct ttcccccaga cctgtctctt acttaaaggg ctcctcgggt 420
   ggtccactgc tctgcccctc agggcacgct gtgggcatct tccgggccgc cgtgtgcacc 480
   aggggggttg cgaaggcggt ggactttgtg cccgttgagt ccatggaaac taccatgcgg 540
   tctccggtct tcacggacaa ttcatctccc ccggccgtac cgcagacatt ccaagtggcc 600
   catctacacg ctcccactgg cagcggtaag agcactaagg tgccggctgc ttatgcagcc 660
   caagggtaca aggtactcgt cctgaaccca tccgttgccg ccaccttagg ttttggggcg 720
   tatatgtcta aagcacatgg tgttgacccc aacatcagaa ctggggtaag gaccatcacc 780
   acaggcgctc ccattacgta ttccacttat ggcaagttcc ttgccgacgg tggttgctct 840
   gggggcgcct atgacatcat aatatgtgat gagtgccact caactgactc gacttccatc 900
   ttgggcatcg gcactgtctt ggaccaagcg gagacggctg gagcgcggct cgtcgtgctc 960
   gccaccgcta cacctccggg atcagtcacc gtgccacatc ccaatatcga ggaggtggct 1020
   ttgtccaaca ctggagagat ccccttttat ggtaaggcct ttcccatcga ggccatcaag 1080
   ggggggaggc atctcatttt ctgccactcc aagaagaaat gtgacgagct cgccacaaag 1140
   ctgtcggccc ttggaatcaa cgctgtagcg tactaccggg gtcttgatgt gtccgtcata 1200
   ccgacaagcg gagatgtcgt tgtcgtggca acagacgctc tgatgacggg ctacactggc 1260
   gactttgact cagtgatcga ctgtaacaca tgtgtcaccc agacagtcga cttcagcttg 1320
   gaccctacct tcaccattga gacgacgacc gtgccccaag acgcggtatc gcgctcgcaa 1380
   cggcgaggca ggactggtag ggcagggggg ggcatataca ggtttgtgac tccaggggaa 1440
   cggccctcgg gcatgttcga ttcttcggtc ctgtgcgagt gctatgacgc gggctgtgct 1500
   tggtacgagc tcacgcccgc cgagacctcg gttaggttgc gggcttacct aaatacacca 1560
   gggttgcccg tctgccagga ccatctggag ttctgggaga gcgtcttcac aggcctcacc 1620
   cacatagatg cccatttctt atcccagacc aagcaggcag gagacaactt cccctacctg 1680
   gtagcgtacc aggctacggt gtgcgccagg gcccaggccc caccaccatc gtgggaccaa 1740
   atgtgggagt gtctcatacg gctaaagcct acactgcacg ggccaacacc cctgctgtac 1800
   aggctaggag ccgtccaaaa cgaagtcact ctcacacacc ccataaccaa attcatcatg 1860
   gcatgcatgt cggctgacct agaggtcgtc acgagcacct gggtgctagt aggcggggtc 1920
   cttgcagctc tggccgcgta ctgcctgaca acgggcagcg tggtcattgt gggtaggatc 1980
   atcttgtctg ggaagccggc tgtcattccc gatagggaag tgctctacca ggagttcgat 2040
   gaaatggaag agtgcgcctc gcacctccct tacattgaac agggaatgca gctcgccgag 2100
   caattcaagc agaaggcgct cgggttgctg caagcagcca ccaagcaagc ggaggctgct 2160
   gctcccgtgg tggagtccaa gtggcaagcc cttgaggcct tttgggcgaa gcacatgtgg 2220
   aatttcatca gcgggataca gtacttagca ggcttgtcca ctctgcctgg gaaccccccg 2280
   atagcttcac tgatggcatt cacagcctct atcaccagcc cgctcaccac ccagcatacc 2340
   ctcttgttta acatcttggg ggggtgggtg gcagcccaac tcgccccccc cagcgctgct 2400
   tcagctttcg tgggcgccgg gatcgctggc gcggctgttg gcagcatagg ccttgggaag 2460
   gtgcttgtgg acatcctggc gggctatggg gcgggggtgg caggcgcgct cgtggccttt 2520
   aaggtcatga gcggcgaaat gccctccacc gaggacctag tcaacttgct ccctgccatc 2580
   ctctcccccg gtgccctggt tgtcggggtc gtgtgcgcgg caatactgcg ccggcacgtg 2640
   ggcccggggg agggggctgt gcagtggatg aaccggctga tagcgttcgc ttcgcggggt 2700
   aaccatgttt cccccacgca ctatgtgcct gagagcgacg cggccgcgcg tgtcactcag 2760
   atcctctcca gccttaccat cacccagctg ttgaagagac tccaccagtg gatcaatgag 2820
   gactgctcca cgccatgctc cggctcgtgg ctcagggatg tttgggactg gatatgcacg 2880
   gtgttgactg acttcaagac ctggctccag tccaagctcc tgccacggtt gccgggggtc 2940
   cctttccttt catgccaacg tggatataag ggagtctggc gaggagatgg cgtcatgcac 3000
   accacttgcc catgtggcgc acaaatcacc ggacatgtca aaaacggttc catgaggatc 3060
   gttgggccta aaacctgtag caacacgtgg cacggaacat tccccatcaa cgcgtacacc 3120
   acgggttcct gcacaccctc cccggcgcca aactattcca gggcgttgtg gcgggtagct 3180
   gctgaggagt acgtggaggt cacgcgggtg ggggatttcc actacgtgac gggcatgacc 3240
   actgacaaca taaaatgccc atgccaggtt ccggcccccg aatttttcac agagttggac 3300
   ggggtgcggc tgcacaggta cgctccggcg tgcaaacctc tcttacggga tgaggtcaca 3360
   ttccaggtcg ggctcaacca atacgtggtt gggtcacagc ttccatgcga gcccgaaccg 3420
   gatgtaacag tgctcacttc catgctcacc gacccctccc acattacagc agagacggct 3480
   aagcgtaggc tggccagggg gtctccccct tccttggccc gctcttcagc tagtcagttg 3540
   tctgcgcctt ctctgaaggc gacatgcacc acccatcatg actccccaga cgccgacctc 3600
   atcgaggcca acctcctgtg gcggcaggag atgggcggga acatcacccg cgtggagtca 3660
   gagaacaaag tagtgattct ggactctttc gacccgcttc gagcggagga ggatgagagg 3720
   gaagtatccg ttccggcgga gatcctgcgg aaaaccagga aattcccctc agcgttgccc 3780
   atatgggcac gcccggacta caacccacca ctgttagagt cttggagaga cccggactat 3840
   gtccctccgg tggtacacgg gtgcccgtta ccgcccacca aggcccctcc aataccacct 3900
   ccacggagaa agagaacagt tatcctgaca gaatccactg tgtcttctgc cttggcggag 3960
   cttgctacaa agaccttcgg cagctccgga tcgtcggccg tcgacagcgg cacagcgact 4020
   gcccctcctg atgggccctc cgacgacggt gatgcaggat cggacgctga gtcatactcc 4080
   tccatgcccc cccttgaggg ggagccgggg gatcccgatc tcagcgacgg gtcttggtcc 4140
   actgtgagcg aggaggccag tgaggacgtc gtctgctgct caatgtccta cacatggaca 4200
   ggtgccttaa tcacgccatg cgccgcggag gagagcaagc tgcccatcaa tgcgttgagc 4260
   aactctttgc tgcgccacca caacatggtc tatgctacaa catcccgcag cgcaagccag 4320
   cggcagaaaa aggtcacctt tgacagactg caagtcctgg acgaccacta ccgggacgtg 4380
   ctcaaggaga tgaaggcgaa ggcgtccaca gttaaggcca aacttctatc cgtagaagag 4440
   gcctgcaagc tgacgccccc acattcagcc agatctaagt ttggctatgg ggcaaaggac 4500
   gtccggaacc tatccagtaa ggccgttaac cacatccact ccgtgtggaa ggacttgctg 4560
   gaagacactg agacaccaat tgataccacc gtcatggcaa aaaatgaggt cttctgtgtc 4620
   caaccagaga aaggaggccg caagccagct cgccttatcg tgttcccaga cctgggggtg 4680
   cgtgtgtgcg agaaaatggc cctttatgat gtggtctcca ctcttcctca ggccgtgatg 4740
   ggctcctcat acggattcca atactctcct ggacagcggg tcgagttcct ggtgaatgcc 4800
   tggaaatcaa agaagacccc tatgggcttc gcatatgaca cccgctgttt tgactcaaca 4860
   gtcactgaga atgacatccg tgttgaggag tcaatttacc aatgttgtga cttggccccc 4920
   gaggccagac aggccataag gtcgctcaca gagcggcttt atatcggggg ccccctgact 4980
   aattcaaaag ggcagaactg cggttatcgc cggtgccgcg ccagcggcgt gctgacgact 5040
   agctgcggta acaccctcac atgttacttg aaggcctctg cagcctgtcg agctgcaaag 5100
   ctccaggact gcacgatgct cgtgtgcgga gacgacctcg tcgttatctg tgagagcgcg 5160
   gggacccagg aggacgcggc gagcctgcga gtcttcacgg aggctatgac caggtactct 5220
   gccccccccg gggacccgcc ccaaccggaa tacgacttgg agctgataac atcatgctcc 5280
   tccaacgtgt cggtcgcgca cgatgcatct ggcaagcggg tgtactatct cacccgcgac 5340
   cccaccaccc ctcttgctcg ggctgcgtgg gagacagcta gacacactcc agtcaactcc 5400
   tggctaggca acatcatcat gtatgcgccc accttatggg caaggatgat tctgatgacc 5460
   cacttcttct caatccttct agcccaggag caacttgaga aagccctaga ttgccagatc 5520
   tacggggcca cttactccat tgagccactt gacctacctc agatcattca acgactccat 5580
   ggtcttagcg cattttcact ccatagttac tctccaggtg agatcaatag ggtggcttca 5640
   tgcctcagga aacttggggt accgcccttg cgagtctgga gacatcgggc cagaagtgtc 5700
   cgcgctaagc tactgtccca gggggggagg gccgccacgt gtggcaagta cctcttcaac 5760
   tgggccgtac ggaccaagct caaactcact ccaattccgg ctgcgtcccg gttggacttg 5820
   tccggctggt tcgtcgccgg ttacagcggg ggagacatat atcacagcct gtctcgcgcc 5880
   cgaccccgct ggttcatgtg gtgcctactc ctactttccg taggggtagg catctacctg 5940
   ctccccaacc ggtgaacggg gagctaaaca ctccaggcca ataggccatt cctttttttt 6000
   tttttttttt tttttttctt tttttttcct tttttttttt tttttttttt tttccttttc 6060
   tttcttcttt ggtggctcca tcttagccct agtcacggct agctgtgaaa ggtccgtgag 6120
   ccgcatgact gcagagagtg ctgatactgg cctctctgca gatcatgt               6168
<210> 4
   <211> 1984
   <212> PRT
   <213> Hepatitis C virus
<400> 4
   Ala Pro Ile Thr Ala Tyr Ser Gln Gln Thr Arg Gly Leu Leu Gly Cys
    1               5                  10                  15
   Ile Ile Thr Ser Leu Thr Gly Arg Asp Arg Asn Gln Val Glu Gly Glu
   20 25 30
   Val Gln Val Val Ser Thr Ala Thr Gln Ser Phe Leu Ala Thr Cys Ile
   35 40 45
   Asn Gly Val Cys Trp Thr Val Tyr His Gly Ala Gly Ser Lys Thr Leu
   50 55 60
   Ala Gly Pro Lys Gly Pro Ile Thr Gln Met Tyr Thr Asn Val Asp Gln
   65 70 75 80
   Asp Leu Val Gly Trp Arg Ala Pro Pro Gly Ala Arg Ser Leu Thr Pro
   85 90 95
   Cys Thr Cys Gly Ser Ser Asp Leu Tyr Leu Val Thr Arg His Ala Asp
   100 105 110
   Val Ile Pro Val Arg Arg Arg Gly Asp Asn Arg Gly Ser Leu Leu Ser
   115 120 125
   Pro Arg Pro Val Ser Tyr Leu Lys Gly Ser Ser Gly Gly Pro Leu Leu
   130 135 140
   Cys Pro Ser Gly His Ala Val Gly Ile Phe Arg Ala Ala Val Cys Thr
   145 150 155 160
   Arg Gly Val Ala Lys Ala Val Asp Phe Val Pro Val Glu Ser Met Glu
   165 170 175
   Thr Thr Met Arg Ser Pro Val Phe Thr Asp Asn Ser Ser Pro Pro Ala
   180 185 190
   Val Pro Gln Thr Phe Gln Val Ala His Leu His Ala Pro Thr Gly Ser
   195 200 205
   Gly Lys Ser Thr Lys Val Pro Ala Ala Tyr Ala Ala Gln Gly Tyr Lys
   210 215 220
   Val Leu Val Leu Asn Pro Ser Val Ala Ala Thr Leu Gly Phe Gly Ala
   225 230 235 240
   Tyr Met Ser Lys Ala His Gly Val Asp Pro Asn Ile Arg Thr Gly Val
   245 250 255
   Arg Thr Ile Thr Thr Gly Ala Pro Ile Thr Tyr Ser Thr Tyr Gly Lys
   260 265 270
   Phe Leu Ala Asp Gly Gly Cys Ser Gly Gly Ala Tyr Asp Ile Ile Ile
   275 280 285
   Cys Asp Glu Cys His Ser Thr Asp Ser Thr Ser Ile Leu Gly Ile Gly
   290 295 300
   Thr Val Leu Asp Gln Ala Glu Thr Ala Gly Ala Arg Leu Val Val Leu
   305 310 315 320
   Ala Thr Ala Thr Pro Pro Gly Ser Val Thr Val Pro His Pro Asn Ile
   325 330 335
   Glu Glu Val Ala Leu Ser Asn Thr Gly Glu Ile Pro Phe Tyr Gly Lys
   340 345 350
   Ala Phe Pro Ile Glu Ala Ile Lys Gly Gly Arg His Leu Ile Phe Cys
               355                 360                 365
   His Ser Lys Lys Lys Cys Asp Glu Leu Ala Thr Lys Leu Ser Ala Leu
       370                 375                 380
   Gly Ile Asn Ala Val Ala Tyr Tyr Arg Gly Leu Asp Val Ser Val Ile
   385                 390                 395                 400
   Pro Thr Ser Gly Asp Val Val Val Val Ala Thr Asp Ala Leu Met Thr
                   405                 410                 415
   Gly Tyr Thr Gly Asp Phe Asp Ser Val Ile Asp Cys Asn Thr Cys Val
               420                 425                 430
   Thr Gln Thr Val Asp Phe Ser Leu Asp Pro Thr Phe Thr Ile Glu Thr
           435                 440                 445
   Thr Thr Val Pro Gln Asp Ala Val Ser Arg Ser Gln Arg Arg Gly Arg
       450                 455                 460
   Thr Gly Arg Gly Arg Gly Gly Ile Tyr Arg Phe Val Thr Pro Gly Glu
   465                 470                 475                 480
   Arg Pro Ser Gly Met Phe Asp Ser Ser Val Leu Cys Glu Cys Tyr Asp
                   485                 490                 495
   Ala Gly Cys Ala Trp Tyr Glu Leu Thr Pro Ala Glu Thr Ser Val Arg
               500                 505                 510
   Leu Arg Ala Tyr Leu Asn Thr Pro Gly Leu Pro Val Cys Gln Asp His
           515                 520                 525
   Leu Glu Phe Trp Glu Ser Val Phe Thr Gly Leu Thr His Ile Asp Ala
       530                 535                 540
   His Phe Leu Ser Gln Thr Lys Gln Ala Gly Asp Asn Phe Pro Tyr Leu
   545                 550                 555                 560
   Val Ala Tyr Gln Ala Thr Val Cys Ala Arg Ala Gln Ala Pro Pro Pro
                   565                 570                 575
   Ser Trp Asp Gln Met Trp Glu Cys Leu Ile Arg Leu Lys Pro Thr Leu
               580                 585                 590
   His Gly Pro Thr Pro Leu Leu Tyr Arg Leu Gly Ala Val Gln Asn Glu
           595                 600                 605
   Val Thr Leu Thr His Pro Ile Thr Lys Phe Ile Met Ala Cys Met Ser
       610                 615                 620
   Ala Asp Leu Glu Val Val Thr Ser Thr Trp Val Leu Val Gly Gly Val
   625                 630                 635                 640
   Leu Ala Ala Leu Ala Ala Tyr Cys Leu Thr Thr Gly Ser Val Val Ile
                   645                 650                 655
   Val Gly Arg Ile Ile Leu Ser Gly Lys Pro Ala Val Ile Pro Asp Arg
               660                 665                 670
   Glu Val Leu Tyr Gln Glu Phe Asp Glu Met Glu Glu Cys Ala Ser His
           675                 680                 685
   Leu Pro Tyr Ile Glu Gln Gly Met Gln Leu Ala Glu Gln Phe Lys Gln
   690 695 700
   Lys Ala Leu Gly Leu Leu Gln Ala Ala Thr Lys Gln Ala Glu Ala Ala
   705 710 715 720
   Ala Pro Val Val Glu Ser Lys Trp Gln Ala Leu Glu Ala Phe Trp Ala
   725 730 735
   Lys His Met Trp Asn Phe Ile Ser Gly Ile Gln Tyr Leu Ala Gly Leu
   740 745 750
   Ser Thr Leu Pro Gly Asn Pro Pro Ile Ala Ser Leu Met Ala Phe Thr
   755 760 765
   Ala Ser Ile Thr Ser Pro Leu Thr Thr Gln His Thr Leu Leu Phe Asn
   770 775 780
   Ile Leu Gly Gly Trp Val Ala Ala Gln Leu Ala Pro Pro Ser Ala Ala
   785 790 795 800
   Ser Ala Phe Val Gly Ala Gly Ile Ala Gly Ala Ala Val Gly Ser Ile
   805 810 815
   Gly Leu Gly Lys Val Leu Val Asp Ile Leu Ala Gly Tyr Gly Ala Gly
   820 825 830
   Val Ala Gly Ala Leu Val Ala Phe Lys Val Met Ser Gly Glu Met Pro
   835 840 845
   Ser Thr Glu Asp Leu Val Asn Leu Leu Pro Ala Ile Leu Ser Pro Gly
   850 855 860
   Ala Leu Val Val Gly Val Val Cys Ala Ala Ile Leu Arg Arg His Val
   865 870 875 880
   Gly Pro Gly Glu Gly Ala Val Gln Trp Met Asn Arg Leu Ile Ala Phe
   885 890 895
   Ala Ser Arg Gly Asn His Val Ser Pro Thr His Tyr Val Pro Glu Ser
   900 905 910
   Asp Ala Ala Ala Arg Val Thr Gln Ile Leu Ser Ser Leu Thr Ile Thr
   915 920 925
   Gln Leu Leu Lys Arg Leu His Gln Trp Ile Asn Glu Asp Cys Ser Thr
   930 935 940
   Pro Cys Ser Gly Ser Trp Leu Arg Asp Val Trp Asp Trp Ile Cys Thr
   945 950 955 960
   Val Leu Thr Asp Phe Lys Thr Trp Leu Gln Ser Lys Leu Leu Pro Arg
   965 970 975
   Leu Pro Gly Val Pro Phe Leu Ser Cys Gln Arg Gly Tyr Lys Gly Val
   980 985 990
   Trp Arg Gly Asp Gly Val Met His Thr Thr Cys Pro Cys Gly Ala Gln
   995 1000 1005
   Ile Thr Gly His Val Lys Asn Gly Ser Met Arg Ile Val Gly Pro Lys
   1010 1015 1020
   Thr Cys Ser Asn Thr Trp His Gly Thr Phe Pro Ile Asn Ala Tyr Thr
   1025 1030 1035 1040
   Thr Gly Ser Cys Thr Pro Ser Pro Ala Pro Asn Tyr Ser Arg Ala Leu
   1045 1050 1055
   Trp Arg Val Ala Ala Glu Glu Tyr Val Glu Val Thr Arg Val Gly Asp
   1060 1065 1070
   Phe His Tyr Val Thr Gly Met Thr Thr Asp Asn Ile Lys Cys Pro Cys
   1075 1080 1085
   Gln Val Pro Ala Pro Glu Phe Phe Thr Glu Leu Asp Gly Val Arg Leu
   1090 1095 1100
   His Arg Tyr Ala Pro Ala Cys Lys Pro Leu Leu Arg Asp Glu Val Thr
   1105 1110 1115 1120
   Phe Gln Val Gly Leu Asn Gln Tyr Val Val Gly Ser Gln Leu Pro Cys
   1125 1130 1135
   Glu Pro Glu Pro Asp Val Thr Val Leu Thr Ser Met Leu Thr Asp Pro
   1140 1145 1150
   Ser His Ile Thr Ala Glu Thr Ala Lys Arg Arg Leu Ala Arg Gly Ser
   1155 1160 1165
   Pro Pro Ser Leu Ala Arg Ser Ser Ala Ser Gln Leu Ser Ala Pro Ser
   1170 1175 1180
   Leu Lys Ala Thr Cys Thr Thr His His Asp Ser Pro Asp Ala Asp Leu
   1185 1190 1195 1200
   Ile Glu Ala Asn Leu Leu Trp Arg Gln Glu Met Gly Gly Asn Ile Thr
   1205 1210 1215
   Arg Val Glu Ser Glu Asn Lys Val Val Ile Leu Asp Ser Phe Asp Pro
   1220 1225 1230
   Leu Arg Ala Glu Glu Asp Glu Arg Glu Val Ser Val Pro Ala Glu Ile
   1235 1240 1245
   Leu Arg Lys Thr Arg Lys Phe Pro Ser Ala Leu Pro Ile Trp Ala Arg
   1250 1255 1260
   Pro Asp Tyr Asn Pro Pro Leu Leu Glu Ser Trp Arg Asp Pro Asp Tyr
   1265 1270 1275 1280
   Val Pro Pro Val Val His Gly Cys Pro Leu Pro Pro Thr Lys Ala Pro
   1285 1290 1295
   Pro Ile Pro Pro Pro Arg Arg Lys Arg Thr Val Ile Leu Thr Glu Ser
   1300 1305 1310
   Thr Val Ser Ser Ala Leu Ala Glu Leu Ala Thr Lys Thr Phe Gly Ser
   1315 1320 1325
   Ser Gly Ser Ser Ala Val Asp Ser Gly Thr Ala Thr Ala Pro Pro Asp
   1330 1335 1340
   Gly Pro Ser Asp Asp Gly Asp Ala Gly Ser Asp Ala Glu Ser Tyr Ser
   1345 1350 1355 1360
   Ser Met Pro Pro Leu Glu Gly Glu Pro Gly Asp Pro Asp Leu Ser Asp
   1365 1370 1375
   Gly Ser Trp Ser Thr Val Ser Glu Glu Ala Ser Glu Asp Val Val Cys
   1380 1385 1390
   Cys Ser Met Ser Tyr Thr Trp Thr Gly Ala Leu Ile Thr Pro Cys Ala
   1395 1400 1405
   Ala Glu Glu Ser Lys Leu Pro Ile Asn Ala Leu Ser Asn Ser Leu Leu
   1410 1415 1420
   Arg His His Asn Met Val Tyr Ala Thr Thr Ser Arg Ser Ala Ser Gln
   1425 1430 1435 1440
   Arg Gln Lys Lys Val Thr Phe Asp Arg Leu Gln Val Leu Asp Asp His
   1445 1450 1455
   Tyr Arg Asp Val Leu Lys Glu Met Lys Ala Lys Ala Ser Thr Val Lys
   1460 1465 1470
   Ala Lys Leu Leu Ser Val Glu Glu Ala Cys Lys Leu Thr Pro Pro His
   1475 1480 1485
   Ser Ala Arg Ser Lys Phe Gly Tyr Gly Ala Lys Asp Val Arg Asn Leu
   1490 1495 1500
   Ser Ser Lys Ala Val Asn His Ile His Ser Val Trp Lys Asp Leu Leu
   1505 1510 1515 1520
   Glu Asp Thr Glu Thr Pro Ile Asp Thr Thr Val Met Ala Lys Asn Glu
   1525 1530 1535
   Val Phe Cys Val Gln Pro Glu Lys Gly Gly Arg Lys Pro Ala Arg Leu
   1540 1545 1550
   Ile Val Phe Pro Asp Leu Gly Val Arg Val Cys Glu Lys Met Ala Leu
   1555 1560 1565
   Tyr Asp Val Val Ser Thr Leu Pro Gln Ala Val Met Gly Ser Ser Tyr
   1570 1575 1580
   Gly Phe Gln Tyr Ser Pro Gly Gln Arg Val Glu Phe Leu Val Asn Ala
   1585 1590 1595 1600
   Trp Lys Ser Lys Lys Thr Pro Met Gly Phe Ala Tyr Asp Thr Arg Cys
   1605 1610 1615
   Phe Asp Ser Thr Val Thr Glu Asn Asp Ile Arg Val Glu Glu Ser Ile
   1620 1625 1630
   Tyr Gln Cys Cys Asp Leu Ala Pro Glu Ala Arg Gln Ala Ile Arg Ser
   1635 1640 1645
   Leu Thr Glu Arg Leu Tyr Ile Gly Gly Pro Leu Thr Asn Ser Lys Gly
   1650 1655 1660
   Gln Asn Cys Gly Tyr Arg Arg Cys Arg Ala Ser Gly Val Leu Thr Thr
   1665 1670 1675 1680
   Ser Cys Gly Asn Thr Leu Thr Cys Tyr Leu Lys Ala Ser Ala Ala Cys
   1685 1690 1695
   Arg Ala Ala Lys Leu Gln Asp Cys Thr Met Leu Val Cys Gly Asp Asp
               1700                1705                1710
   Leu Val Val Ile Cys Glu Ser Ala Gly Thr Gln Glu Asp Ala Ala Ser
           1715                1720                1725
   Leu Arg Val Phe Thr Glu Ala Met Thr Arg Tyr Ser Ala Pro Pro Gly
       1730                1735                1740
   Asp Pro Pro Gln Pro Glu Tyr Asp Leu Glu Leu Ile Thr Ser Cys Ser
   1745                1750                1755                1760
   Ser Asn Val Ser Val Ala His Asp Ala Ser Gly Lys Arg Val Tyr Tyr
                   1765                1770                1775
   Leu Thr Arg Asp Pro Thr Thr Pro Leu Ala Arg Ala Ala Trp Glu Thr
               1780                1785                1790
   Ala Arg His Thr Pro Val Asn Ser Trp Leu Gly Asn Ile Ile Met Tyr
           1795                1800                1805
   Ala Pro Thr Leu Trp Ala Arg Met Ile Leu Met Thr His Phe Phe Ser
       1810                1815                1820
   Ile Leu Leu Ala Gln Glu Gln Leu Glu Lys Ala Leu Asp Cys Gln Ile
   1825                1830                1835                1840
   Tyr Gly Ala Thr Tyr Ser Ile Glu Pro Leu Asp Leu Pro Gln Ile Ile
                   1845                1850                1855
   Gln Arg Leu His Gly Leu Ser Ala Phe Ser Leu His Ser Tyr Ser Pro
               1860                1865                1870
   Gly Glu Ile Asn Arg Val Ala Ser Cys Leu Arg Lys Leu Gly Val Pro
           1875                1880                1885
   Pro Leu Arg Val Trp Arg His Arg Ala Arg Ser Val Arg Ala Lys Leu
       1890                1895                1900
   Leu Ser Gln Gly Gly Arg Ala Ala Thr Cys Gly Lys Tyr Leu Phe Asn
   1905                1910                1915                1920
   Trp Ala Val Arg Thr Lys Leu Lys Leu Thr Pro Ile Pro Ala Ala Ser
                   1925                1930                1935
   Arg Leu Asp Leu Ser Gly Trp Phe Val Ala Gly Tyr Ser Gly Gly Asp
               1940                1945                1950
   Ile Tyr His Ser Leu Ser Arg Ala Arg Pro Arg Trp Phe Met Trp Cys
           1955                1960                1965
   Leu Leu Leu Leu Ser Val Gly Val Gly Ile Tyr Leu Leu Pro Asn Arg
       1970                1975                1980
<210> 5
   <211> 6168
   <212> DNA
   <213> Hepatitis C virus
<400> 5
   gcgcccatca cggcttactc ccaacagacg cggggcctac tcggttgcat catcactagt 60
   ctcacaggcc gggacaggaa ccaggtcgag ggggaggttc aggtggtttc caccgcaacg 120
   cagtccttct tggcgacctg catcaatggt gtgtgttgga ctgtctacca tggtgccggc 180
   tcaaagaccc tagccggccc aaagggtccg atcacccaaa tgtacaccaa tgtagaccag 240
   gacctcgtcg gctggcgggc gcccctcggg gcgcgttcct taacaccatg cacctgcggc 300
   agctcggacc tctacttggt cacgaggcat gctgatgtca ttccggtgcg ccggcggggc 360
   gataacaggg gcagcctgct ttcccccaga cctgtctctt acttaaaggg ctcctcgggt 420
   ggtccactgc tctgcccctc agggcacgct gtgggcatct tccgggccgc cgtgtgcacc 480
   aggggggttg cgaaggcggt ggactttgtg cccgttgagt ccatggaaac taccatgcgg 540
   tctccggtct tcacggacaa ttcatctccc ccggccgtac cgcagacatt ccaagtggcc 600
   catctacacg ctcccactgg cagcggtaag agcactaagg tgccggctgc ttatgcagcc 660
   caagggtaca aggtactcgt cctgaaccca tccgttgccg ccaccttagg ttttggggcg 720
   tatatgtcta aagcacatgg tgttgacccc aacatcagaa ctggggtaag gaccatcacc 780
   acaggcgctc ccattacgta ttccacttat ggcaagttcc ttgccgacgg tggttgctct 840
   gggggcgcct atgacatcat aatatgtgat gagtgccact caactgactc gacttccatc 900
   ttgggcatcg gcactgtctt ggaccaagcg gagacggctg gagcgcggct cgtcgtgctc 960
   gccaccgcta cacctccggg atcagtcacc gtgccacatc ccaatatcga ggaggtggct 1020
   ttgtccaaca ctggagagat ccccttttat ggtaaggcct ttcccatcga ggccatcaag 1080
   ggggggaggc atctcatttt ctgccactcc aagaagaaat gtgacgagct cgccacaaag 1140
   ctgtcggccc ttggaatcaa cgctgtagcg tactaccggg gtcttgatgt gtccgtcata 1200
   ccgacaagcg gagatgtcgt tgtcgtggca acagacgctc tgatgacggg ctacactggc 1260
   gactttgact cagtgatcga ctgtaacaca tgtgtcaccc agacagtcga cttcagcttg 1320
   gaccctacct tcaccattga gacgacgacc gtgccccaag acgcggtatc gcgctcgcaa 1380
   cggcgaggca ggactggtag gggcaggggg ggcatataca ggtttgtgac tccaggggaa 1440
   cggccctcgg gcatgttcga ttcttcggtc ctgtgcgagt gctatgacgc gggctgtgct 1500
   tggtacgagc tcacgcccgc cgagacctcg gttaggttgc gggcttacct aaatacacca 1560
   gggttgcccg tctgccagga ccatctggag ttctgggaga gcgtcttcac aggcctcacc 1620
   cacatagatg cccatttctt atcccagacc aagcaggcag gagacaactt cccctacctg 1680
   gtagcgtacc aggctacggt gtgcgccagg gcccaggccc caccaccatc gtgggaccaa 1740
   atgtggaagt gtctcatacg gctaaagcct acactgcacg ggccaacacc cctgctgtac 1800
   aggctaggag ccgtccaaaa cgaagtcact ctcacacacc ccataaccaa attcatcatg 1860
   gcatgcatgt cggctgacct agaggtcgtc acgagcacct gggtgctagt aggcggggtc 1920
   cttgcagctc tggccgcgta ctgcctgaca acgggcagcg tggtcattgt gggtaggatc 1980
   atcttgtctg ggaagccggc tgtcattccc gatagggaag tgctctacca ggagttcgat 2040
   gaaatggaag agtgcgcctc gcacctccct tacattgaac agggaatgca gctcgccgag 2100
   caattcaagc agaaggcgct cgggttgctg caagcagcca ccaagcaagc ggaggctgct 2160
   gctcccgtgg tggagtccaa gtggcaagcc cttgaggcct tttgggcgaa gcacatgtgg 2220
   aatttcatca gcgggataca gtacttagca ggcttgtcca ctctgcctgg gaaccccccg 2280
   atagcttcac tgatggcatt cacagcctct atcaccagcc cgctcaccac ccagcatacc 2340
   ctcttgttta acatcttggg gcggtgggtg gcagcccaac tcgccccccc cagcgctgct 2400
   tcagctttcg tgggcgccgg gatcgctggc gcggctgttg gcagcatagg ccttgggaag 2460
   gtgcttgtgg acatcctggc gggctatggg gcgggggtgg caggcgcgct cgtggccttt 2520
   aaggtcatga gcggcgaaat gccctccacc gaggacctag tcaacttgct ccctgccatc 2580
   ctctcccccg gtgccctggt tgtcggggtc gtgtgcgcgg caatactgcg ccggcacgtg 2640
   ggcccggggg agggggctgt gcagtggatg aaccggctga tagcgttcgc ttcgcggggt 2700
   aaccatgttt cccccacgca ctatgtgcct gagagcgacg cggccgcgcg tgtcactcag 2760
   atcctctcca gccttaccat cacccagctg ttgaagagac tccaccagtg gatcaatgag 2820
   gactgctcca cgccatgctc cggctcgtgg ctcagggatg tttgggactg gatatgcacg 2880
   gtgttgactg acttcaagac ctggctccag tccaagctcc tgccacggtt gccgggggtc 2940
   cctttccttt catgccaacg tggatataag ggagtctggc gaggagatgg cgtcatgcac 3000
   accacttgcc catgtggcgc acaaatcacc ggacatgtca aaaacggttc catgaggatc 3060
   gttgggccta aaacctgtag caacacgtgg cacggaacat tccccatcaa cgcgtacacc 3120
   acgggttcct gcacaccctc cccggcgcca aactattcca gggcgttgtg gcgggtagct 3180
   gctgaggagt acgtggaggt cacgcgggtg ggggatttcc actacgtgac gggcatgacc 3240
   actgacaaca taaaatgccc atgccaggtt ccggcccccg aatttttcac agagttggac 3300
   ggggtgcggc tgcacaggta cgctccggcg tgcaaacctc tcttacggga tgaggtcaca 3360
   ttccaggtcg ggctcaacca atacgtggtt gggtcacagc ttccatgcga gcccgaaccg 3420
   gatgtaacag tgctcacttc catgctcacc gacccctccc acattacagc agagacggct 3480
   aagcgtaggc tggccagggg gtctccccct tccttggccc gctcttcagc tagtcagttg 3540
   tctgcgcctt ctctgaaggc gacatgcacc acccatcatg actccccaga cgccgacctc 3600
   atcgaggcca acctcctgtg gcggcaggag atgggcggga acatcacccg cgtggagtca 3660
   gagaacaaag tagtgattct ggactctttc gacccgcttc gagcggagga ggatgagagg 3720
   gaagtatccg ttccggcgga gatcctgcgg aaaaccagga aattcccctc agcgttgccc 3780
   atatgggcac gcccggacta caacccacca ctgttagagt cttggagaga cccggactat 3840
   gtccctccgg tggtacacgg gtgcccgtta ccgcccacca aggcccctcc aataccacct 3900
   ccacggagaa agagaacagt tatcctgaca gaatccactg tgtcttctgc cttggcggag 3960
   cttgctacaa agaccttcgg cagctccgga tcgtcggccg tcgacagcgg cacagcgact 4020
   gcccctcctg atgggccctc cgacgacggt gatgcaggat cggacgctga gtcatactcc 4080
   tccatgcccc cccttgaggg ggagccgggg gatcccgatc tcagcgacgg gtcttggtcc 4140
   actgtgagcg aggaggccag tgaggacgtc gtctgctgct caatgtccta cacatggaca 4200
   ggtgccttaa tcacgccatg cgccgcggag gagagcaagc tgcccatcaa tgcgttgagc 4260
   aactctttgc tgcgccacca caacatggtc tatgctacaa catcccgcag cgcaagccag 4320
   cggcagaaaa aggtcacctt tgacagactg caagtcctgg acgaccacta ccgggacgtg 4380
   ctcaaggaga tgaaggcgaa ggcgtccaca gttaaggcca aacttctatc cgtagaagag 4440
   gcctgcaagc tgacgccccc acattcagcc agatctaagt ttggctatgg ggcaaaggac 4500
   gtccggaacc tatccagtaa ggccgttaac cacatccact ccgtgtggaa ggacttgctg 4560
   gaagacactg agacaccaat tgataccacc gtcatggcaa aaaatgaggt cttctgtgtc 4620
   caaccagaga aaggaggccg caagccagct cgccttatcg tgttcccaga cctgggggtg 4680
   cgtgtgtgcg agaaaatggc cctttatgat gtggtctcca ctcttcctca ggccgtgatg 4740
   ggctcctcat acggattcca atactctcct ggacagcggg tcgagttcct ggtgaatgcc 4800
   tggaaatcaa agaagacccc tatgggcttc gcatatgaca cccgctgttt tgactcaaca 4860
   gtcactgaga atgacatccg tgttgaggag tcaatttacc aatgttgtga cttggccccc 4920
   gaggccagac aggccataag gtcgctcaca gagcggcttt atatcggggg ccccctgact 4980
   aattcaaaag ggcagaactg cggttatcgc cggtgccgcg ccagcggcgt gctgacgact 5040
   agctgcggta acaccctcac atgttacttg aaggcctctg cagcctgtcg agctgcaaag 5100
   ctccaggact gcacgatgct cgtgtgcgga gacgacctcg tcgttatctg tgagagcgcg 5160
   gggacccagg aggacgcggc gagcctgcga gtcttcacgg aggctatgac caggtactct 5220
   gccccccccg gggacccgcc ccaaccggaa tacgacttgg agctgataac atcatgctcc 5280
   tccaacgtgt cggtcgcgca cgatgcatct ggcaagcggg tgtactatct cacccgcgac 5340
   cccaccaccc ctcttgctcg ggctgcgtgg gagacagcta gacacactcc agtcaactcc 5400
   tggctaggca acatcatcat gtatgcgccc accttatggg caaggatgat tctgatgacc 5460
   cacttcttct caatccttct agcccaggag caacttgaga aagccctaga ttgccagatc 5520
   tacggggcca cttactccat tgagccactt gacctacctc agatcattca acgactccat 5580
   ggtcttagcg cattttcact ccatagttac tctccaggtg agatcaatag ggtggcttca 5640
   tgcctcagga aacttggggt accgcccttg cgagtctgga gacatcgggc cagaagtgtc 5700
   cgcgctaagc tactgtccca gggggggagg gccgccacgt gtggcaagta cctcttcaac 5760
   tgggccgtac ggaccaagct caaactcact ccaattccgg ctgcgtcccg gttggacttg 5820
   tccggctggt tcgtcgccgg ttacagcggg ggagacatat atcacagcct gtctcgcgcc 5880
   cgaccccgct ggttcatgtg gtgcctactc ctactttccg taggggtagg catctacctg 5940
   ctccccaacc ggtgaacggg gagctaaaca ctccaggcca ataggccatt cctttttttt 6000
   tttttttttt tttttttctt tttttttcct tttttttttt tttttttttt tttccttttc 6060
   tttcttcttt ggtggctcca tcttagccct agtcacggct agctgtgaaa ggtccgtgag 6120
   ccgcatgact gcagagagtg ctgatactgg cctctctgca gatcatgt 6168
<210> 6
   <211> 1984
   <212> PRT
   <213> Hepatitis C virus
<400> 6
   Ala Pro Ile Thr Ala Tyr Ser Gln Gln Thr Arg Gly Leu Leu Gly Cys
    1               5                  10                  15
   Ile Ile Thr Ser Leu Thr Gly Arg Asp Arg Asn Gln Val Glu Gly Glu
               20                  25                  30
   Val Gln Val Val Ser Thr Ala Thr Gln Ser Phe Leu Ala Thr Cys Ile
           35                  40                  45
   Asn Gly Val Cys Trp Thr Val Tyr His Gly Ala Gly Ser Lys Thr Leu
       50                  55                  60
   Ala Gly Pro Lys Gly Pro Ile Thr Gln Met Tyr Thr Asn Val Asp Gln
   65                  70                  75                  80
   Asp Leu Val Gly Trp Arg Ala Pro Leu Gly Ala Arg Ser Leu Thr Pro
   85 90 95
   Cys Thr Cys Gly Ser Ser Asp Leu Tyr Leu Val Thr Arg His Ala Asp
   100 105 110
   Val Ile Pro Val Arg Arg Arg Gly Asp Asn Arg Gly Ser Leu Leu Ser
   115 120 125
   Pro Arg Pro Val Ser Tyr Leu Lys Gly Ser Ser Gly Gly Pro Leu Leu
   130 135 140
   Cys Pro Ser Gly His Ala Val Gly Ile Phe Arg Ala Ala Val Cys Thr
   145 150 155 160
   Arg Gly Val Ala Lys Ala Val Asp Phe Val Pro Val Glu Ser Met Glu
   165 170 175
   Thr Thr Met Arg Ser Pro Val Phe Thr Asp Asn Ser Ser Pro Pro Ala
   180 185 190
   Val Pro Gln Thr Phe Gln Val Ala His Leu His Ala Pro Thr Gly Ser
   195 200 205
   Gly Lys Ser Thr Lys Val Pro Ala Ala Tyr Ala Ala Gln Gly Tyr Lys
   210 215 220
   Val Leu Val Leu Asn Pro Ser Val Ala Ala Thr Leu Gly Phe Gly Ala
   225 230 235 240
   Tyr Met Ser Lys Ala His Gly Val Asp Pro Asn Ile Arg Thr Gly Val
   245 250 255
   Arg Thr Ile Thr Thr Gly Ala Pro Ile Thr Tyr Ser Thr Tyr Gly Lys
   260 265 270
   Phe Leu Ala Asp Gly Gly Cys Ser Gly Gly Ala Tyr Asp Ile Ile Ile
   275 280 285
   Cys Asp Glu Cys His Ser Thr Asp Ser Thr Ser Ile Leu Gly Ile Gly
   290 295 300
   Thr Val Leu Asp Gln Ala Glu Thr Ala Gly Ala Arg Leu Val Val Leu
   305 310 315 320
   Ala Thr Ala Thr Pro Pro Gly Ser Val Thr Val Pro His Pro Asn Ile
   325 330 335
   Glu Glu Val Ala Leu Ser Asn Thr Gly Glu Ile Pro Phe Tyr Gly Lys
   340 345 350
   Ala Phe Pro Ile Glu Ala Ile Lys Gly Gly Arg His Leu Ile Phe Cys
   355 360 365
   His Ser Lys Lys Lys Cys Asp Glu Leu Ala Thr Lys Leu Ser Ala Leu
   370 375 380
   Gly Ile Asn Ala Val Ala Tyr Tyr Arg Gly Leu Asp Val Ser Val Ile
   385 390 395 400
   Pro Thr Ser Gly Asp Val Val Val Val Ala Thr Asp Ala Leu Met Thr
   405 410 415
   Gly Tyr Thr Gly Asp Phe Asp Ser Val Ile Asp Cys Asn Thr Cys Val
               420                 425                 430
   Thr Gln Thr Val Asp Phe Ser Leu Asp Pro Thr Phe Thr Ile Glu Thr
           435                 440                 445
   Thr Thr Val Pro Gln Asp Ala Val Ser Arg Ser Gln Arg Arg Gly Arg
       450                 455                 460
   Thr Gly Arg Gly Arg Gly Gly Ile Tyr Arg Phe Val Thr Pro Gly Glu
   465                 470                 475                 480
   Arg Pro Ser Gly Met Phe Asp Ser Ser Val Leu Cys Glu Cys Tyr Asp
                   485                 490                 495
   Ala Gly Cys Ala Trp Tyr Glu Leu Thr Pro Ala Glu Thr Ser Val Arg
               500                 505                 510
   Leu Arg Ala Tyr Leu Asn Thr Pro Gly Leu Pro Val Cys Gln Asp His
           515                 520                 525
   Leu Glu Phe Trp Glu Ser Val Phe Thr Gly Leu Thr His Ile Asp Ala
       530                 535                 540
   His Phe Leu Ser Gln Thr Lys Gln Ala Gly Asp Asn Phe Pro Tyr Leu
   545                 550                 555                 560
   Val Ala Tyr Gln Ala Thr Val Cys Ala Arg Ala Gln Ala Pro Pro Pro
                   565                 570                 575
   Ser Trp Asp Gln Met Trp Lys Cys Leu Ile Arg Leu Lys Pro Thr Leu
               580                 585                 590
   His Gly Pro Thr Pro Leu Leu Tyr Arg Leu Gly Ala Val Gln Asn Glu
           595                 600                 605
   Val Thr Leu Thr His Pro Ile Thr Lys Phe Ile Met Ala Cys Met Ser
       610                 615                 620
   Ala Asp Leu Glu Val Val Thr Ser Thr Trp Val Leu Val Gly Gly Val
   625                 630                 635                 640
   Leu Ala Ala Leu Ala Ala Tyr Cys Leu Thr Thr Gly Ser Val Val Ile
                   645                 650                 655
   Val Gly Arg Ile Ile Leu Ser Gly Lys Pro Ala Val Ile Pro Asp Arg
               660                 665                 670
   Glu Val Leu Tyr Gln Glu Phe Asp Glu Met Glu Glu Cys Ala Ser His
           675                 680                 685
   Leu Pro Tyr Ile Glu Gln Gly Met Gln Leu Ala Glu Gln Phe Lys Gln
       690                 695                 700
   Lys Ala Leu Gly Leu Leu Gln Ala Ala Thr Lys Gln Ala Glu Ala Ala
   705                 710                 715                 720
   Ala Pro Val Val Glu Ser Lys Trp Gln Ala Leu Glu Ala Phe Trp Ala
                   725                 730                 735
   Lys His Met Trp Asn Phe Ile Ser Gly Ile Gln Tyr Leu Ala Gly Leu
               740                 745                 750
   Ser Thr Leu Pro Gly Asn Pro Pro Ile Ala Ser Leu Met Ala Phe Thr
   755 760 765
   Ala Ser Ile Thr Ser Pro Leu Thr Thr Gln His Thr Leu Leu Phe Asn
   770 775 780
   Ile Leu Gly Gly Trp Val Ala Ala Gln Leu Ala Pro Pro Ser Ala Ala
   785 790 795 800
   Ser Ala Phe Val Gly Ala Gly Ile Ala Gly Ala Ala Val Gly Ser Ile
   805 810 815
   Gly Leu Gly Lys Val Leu Val Asp Ile Leu Ala Gly Tyr Gly Ala Gly
   820 825 830
   Val Ala Gly Ala Leu Val Ala Phe Lys Val Met Ser Gly Glu Met Pro
   835 840 845
   Ser Thr Glu Asp Leu Val Asn Leu Leu Pro Ala Ile Leu Ser Pro Gly
   850 855 860
   Ala Leu Val Val Gly Val Val Cys Ala Ala Ile Leu Arg Arg His Val
   865 870 875 880
   Gly Pro Gly Glu Gly Ala Val Gln Trp Met Asn Arg Leu Ile Ala Phe
   885 890 895
   Ala Ser Arg Gly Asn His Val Ser Pro Thr His Tyr Val Pro Glu Ser
   900 905 910
   Asp Ala Ala Ala Arg Val Thr Gln Ile Leu Ser Ser Leu Thr Ile Thr
   915 920 925
   Gln Leu Leu Lys Arg Leu His Gln Trp Ile Asn Glu Asp Cys Ser Thr
   930 935 940
   Pro Cys Ser Gly Ser Trp Leu Arg Asp Val Trp Asp Trp Ile Cys Thr
   945 950 955 960
   Val Leu Thr Asp Phe Lys Thr Trp Leu Gln Ser Lys Leu Leu Pro Arg
   965 970 975
   Leu Pro Gly Val Pro Phe Leu Ser Cys Gln Arg Gly Tyr Lys Gly Val
   980 985 990
   Trp Arg Gly Asp Gly Val Met His Thr Thr Cys Pro Cys Gly Ala Gln
   995 1000 1005
   Ile Thr Gly His Val Lys Asn Gly Ser Met Arg Ile Val Gly Pro Lys
   1010 1015 1020
   Thr Cys Ser Asn Thr Trp His Gly Thr Phe Pro Ile Asn Ala Tyr Thr
   1025 1030 1035 1040
   Thr Gly Ser Cys Thr Pro Ser Pro Ala Pro Asn Tyr Ser Arg Ala Leu
   1045 1050 1055
   Trp Arg Val Ala Ala Glu Glu Tyr Val Glu Val Thr Arg Val Gly Asp
   1060 1065 1070
   Phe His Tyr Val Thr Gly Met Thr Thr Asp Asn Ile Lys Cys Pro Cys
   1075 1080 1085
   Gln Val Pro Ala Pro Glu Phe Phe Thr Glu Leu Asp Gly Val Arg Leu
   1090 1095 1100
   His Arg Tyr Ala Pro Ala Cys Lys Pro Leu Leu Arg Asp Glu Val Thr
   1105 1110 1115 1120
   Phe Gln Val Gly Leu Asn Gln Tyr Val Val Gly Ser Gln Leu Pro Cys
   1125 1130 1135
   Glu Pro Glu Pro Asp Val Thr Val Leu Thr Ser Met Leu Thr Asp Pro
   1140 1145 1150
   Ser His Ile Thr Ala Glu Thr Ala Lys Arg Arg Leu Ala Arg Gly Ser
   1155 1160 1165
   Pro Pro Ser Leu Ala Arg Ser Ser Ala Ser Gln Leu Ser Ala Pro Ser
   1170 1175 1180
   Leu Lys Ala Thr Cys Thr Thr His His Asp Ser Pro Asp Ala Asp Leu
   1185 1190 1195 1200
   Ile Glu Ala Asn Leu Leu Trp Arg Gln Glu Met Gly Gly Asn Ile Thr
   1205 1210 1215
   Arg Val Glu Ser Glu Asn Lys Val Val Ile Leu Asp Ser Phe Asp Pro
   1220 1225 1230
   Leu Arg Ala Glu Glu Asp Glu Arg Glu Val Ser Val Pro Ala Glu Ile
   1235 1240 1245
   Leu Arg Lys Thr Arg Lys Phe Pro Ser Ala Leu Pro Ile Trp Ala Arg
   1250 1255 1260
   Pro Asp Tyr Asn Pro Pro Leu Leu Glu Ser Trp Arg Asp Pro Asp Tyr
   1265 1270 1275 1280
   Val Pro Pro Val Val His Gly Cys Pro Leu Pro Pro Thr Lys Ala Pro
   1285 1290 1295
   Pro Ile Pro Pro Pro Arg Arg Lys Arg Thr Val Ile Leu Thr Glu Ser
   1300 1305 1310
   Thr Val Ser Ser Ala Leu Ala Glu Leu Ala Thr Lys Thr Phe Gly Ser
   1315 1320 1325
   Ser Gly Ser Ser Ala Val Asp Ser Gly Thr Ala Thr Ala Pro Pro Asp
   1330 1335 1340
   Gly Pro Ser Asp Asp Gly Asp Ala Gly Ser Asp Ala Glu Ser Tyr Ser
   1345 1350 1355 1360
   Ser Met Pro Pro Leu Glu Gly Glu Pro Gly Asp Pro Asp Leu Ser Asp
   1365 1370 1375
   Gly Ser Trp Ser Thr Val Ser Glu Glu Ala Ser Glu Asp Val Val Cys
   1380 1385 1390
   Cys Ser Met Ser Tyr Thr Trp Thr Gly Ala Leu Ile Thr Pro Cys Ala
   1395 1400 1405
   Ala Glu Glu Ser Lys Leu Pro Ile Asn Ala Leu Ser Asn Ser Leu Leu
   1410 1415 1420
   Arg His His Asn Met Val Tyr Ala Thr Thr Ser Arg Ser Ala Ser Gln
   1425 1430 1435 1440
   Arg Gln Lys Lys Val Thr Phe Asp Arg Leu Gln Val Leu Asp Asp His
   1445 1450 1455
   Tyr Arg Asp Val Leu Lys Glu Met Lys Ala Lys Ala Ser Thr Val Lys
   1460 1465 1470
   Ala Lys Leu Leu Ser Val Glu Glu Ala Cys Lys Leu Thr Pro Pro His
   1475 1480 1485
   Ser Ala Arg Ser Lys Phe Gly Tyr Gly Ala Lys Asp Val Arg Asn Leu
   1490 1495 1500
   Ser Ser Lys Ala Val Asn His Ile His Ser Val Trp Lys Asp Leu Leu
   1505 1510 1515 1520
   Glu Asp Thr Glu Thr Pro Ile Asp Thr Thr Val Met Ala Lys Asn Glu
   1525 1530 1535
   Val Phe Cys Val Gln Pro Glu Lys Gly Gly Arg Lys Pro Ala Arg Leu
   1540 1545 1550
   Ile Val Phe Pro Asp Leu Gly Val Arg Val Cys Glu Lys Met Ala Leu
   1555 1560 1565
   Tyr Asp Val Val Ser Thr Leu Pro Gln Ala Val Met Gly Ser Ser Tyr
   1570 1575 1580
   Gly Phe Gln Tyr Ser Pro Gly Gln Arg Val Glu Phe Leu Val Asn Ala
   1585 1590 1595 1600
   Trp Lys Ser Lys Lys Thr Pro Met Gly Phe Ala Tyr Asp Thr Arg Cys
   1605 1610 1615
   Phe Asp Ser Thr Val Thr Glu Asn Asp Ile Arg Val Glu Glu Ser Ile
   1620 1625 1630
   Tyr Gln Cys Cys Asp Leu Ala Pro Glu Ala Arg Gln Ala Ile Arg Ser
   1635 1640 1645
   Leu Thr Glu Arg Leu Tyr Ile Gly Gly Pro Leu Thr Asn Ser Lys Gly
   1650 1655 1660
   Gln Asn Cys Gly Tyr Arg Arg Cys Arg Ala Ser Gly Val Leu Thr Thr
   1665 1670 1675 1680
   Ser Cys Gly Asn Thr Leu Thr Cys Tyr Leu Lys Ala Ser Ala Ala Cys
   1685 1690 1695
   Arg Ala Ala Lys Leu Gln Asp Cys Thr Met Leu Val Cys Gly Asp Asp
   1700 1705 1710
   Leu Val Val Ile Cys Glu Ser Ala Gly Thr Gln Glu Asp Ala Ala Ser
   1715 1720 1725
   Leu Arg Val Phe Thr Glu Ala Met Thr Arg Tyr Ser Ala Pro Pro Gly
   1730 1735 1740
   Asp Pro Pro Gln Pro Glu Tyr Asp Leu Glu Leu Ile Thr Ser Cys Ser
   1745 1750 1755 1760
   Ser Asn Val Ser Val Ala His Asp Ala Ser Gly Lys Arg Val Tyr Tyr
                   1765                1770                1775
   Leu Thr Arg Asp Pro Thr Thr Pro Leu Ala Arg Ala Ala Trp Glu Thr
               1780                1785                1790
   Ala Arg His Thr Pro Val Asn Ser Trp Leu Gly Asn Ile Ile Met Tyr
           1795                1800                1805
   Ala Pro Thr Leu Trp Ala Arg Met Ile Leu Met Thr His Phe Phe Ser
       1810                1815                1820
   Ile Leu Leu Ala Gln Glu Gln Leu Glu Lys Ala Leu Asp Cys Gln Ile
   1825                1830                1835                1840
   Tyr Gly Ala Thr Tyr Ser Ile Glu Pro Leu Asp Leu Pro Gln Ile Ile
                   1845                1850                1855
   Gln Arg Leu His Gly Leu Ser Ala Phe Ser Leu His Ser Tyr Ser Pro
               1860                1865                1870
   Gly Glu Ile Asn Arg Val Ala Ser Cys Leu Arg Lys Leu Gly Val Pro
           1875                1880                1885
   Pro Leu Arg Val Trp Arg His Arg Ala Arg Ser Val Arg Ala Lys Leu
       1890                1895                1900
   Leu Ser Gln Gly Gly Arg Ala Ala Thr Cys Gly Lys Tyr Leu Phe Asn
   1905                1910                1915                1920
   Trp Ala Val Arg Thr Lys Leu Lys Leu Thr Pro Ile Pro Ala Ala Ser
                   1925                1930                1935
   Arg Leu Asp Leu Ser Gly Trp Phe Val Ala Gly Tyr Ser Gly Gly Asp
               1940                1945                1950
   Ile Tyr His Ser Leu Ser Arg Ala Arg Pro Arg Trp Phe Met Trp Cys
           1955                1960                1965
   Leu Leu Leu Leu Ser Val Gly Val Gly Ile Tyr Leu Leu Pro Asn Arg
       1970                1975                1980
<210> 7
   <211> 9587
   <212> DNA
   <213> Hepatitis C virus
<400> 7
   gccagccccc gattgggggc gacactccac catagatcac tcccctgtga ggaactactg 60
   tcttcacgca gaaagcgtct agccatggcg ttagtatgag tgtcgtgcag cctccaggac 120
   cccccctccc gggagagcca tagtggtctg cggaaccggt gagtacaccg gaattgccag 180
   gacgaccggg tcctttcttg gattaacccg ctcaatgcct ggagatttgg gcgtgccccc 240
   gcaagactgc tagccgagta gtgttgggtc gcgaaaggcc ttgtggtact gcctgatagg 300
   gtgcttgcga gtgccccggg aggtctcgta gaccgtgcac catgagcacg aatcctaaac 360
   ctcaaagaaa aaccaaacgt aacaccaacc gccgcccaca ggacgtcaag ttcccgggcg 420
   gtggtcagat cgttggtgga gtttacctgt tgccgcgcag gggccccagg ttgggtgtgc 480
   gcgcgactag gaagacttcc gagcggtcgc agcctcgtgg aaggcgacaa cctatcccca 540
   aggctcgcca gcccgagggc agggcctggg ctcagcccgg atacccttgg cccctctacg 600
   gcaatgaggg tctggggtgg gcaggatggc tcctgtcacc ccgtggctct cggcctagtt 660
   ggggccctac ggacccccgg cgtaggtcgc gtaatttggg taaggtcatc gataccctca 720
   catgcggctt cgccgacctc atggggtaca tcccgctcgt cggcgccccc ctaggaggcg 780
   ctgccagggc cctggcgcat ggcgtccggg ttctggagga cggcgtgaac tatgcaacag 840
   ggaatttgcc cggttgctct ttctctatct tcctcttggc tttgctgtcc tgtttgacca 900
   tcccagcttc cgcttacgaa gtgcgcaacg tatccggggt gtaccatgtc acgaacgact 960
   gctccaactc aagtattgtg tatgaggcag cggacatgat catgcacacc cccgggtgcg 1020
   tgccctgcgt tcgggaggac aattcctccc gttgctgggt agcgctcact cccacgcttg 1080
   cggccaggaa cagcagcgtc cccaccacga caatacgacg ccacgtcgac ttgctcgttg 1140
   gggcagctgc tttctgttcc gctatgtacg tgggagacct ctgcggatct gttttcctca 1200
   tttcccagct gttcaccttc tcacctcgcc ggtatgagac ggtgcaagac tgcaactgct 1260
   cgctctatcc cggccacgta tcaggtcacc gcatggcttg ggatatgatg atgaactggt 1320
   cacctacaac agccctggtg gtgtcgcagt tgctccggat cccacaagcc gtcgtggaca 1380
   tggtggcggg ggcccactgg ggagtcctgg cgggccttgc ctactattcc atggtgggga 1440
   actgggctaa ggtcttgatt gtgatgctac tttttgccgg cgtcgacggg aacactcgtg 1500
   tgtcaggggg ggaagcagcc aagaacacca tgggctttgc atccctcttt gtgtctggcc 1560
   cgtcgcagaa aatccaactt attaacacca atggcagctg gcacatcaac aggactgctc 1620
   tgaattgcga tgactccctc cacaccgggt tccttgctgc gctgttttac gcgcacaagt 1680
   tcaactcgtc cggatgctcg gggcgcatgg ccagctgccg ccccattgac gagtttgccc 1740
   aggggtgggg tcccatcact cacggtgtgc ctgacaacct ggaccagagg ccttactgct 1800
   ggcactacgc gcctcggccg tgcggtatcg tacccgcgtc gcaggtgtgt ggcccagtgt 1860
   attgcttcac cccgagccct gtcgtggtgg ggacgaccga tcgtttcggc gcccccacgt 1920
   acagctgggg ggagaatgag acggacgtgc tactccttaa caacacgcga ccgccacaag 1980
   gcaactggtt cggttgcaca tggatgaaca gcaccgggtt caccaagacg tgcgggggcc 2040
   ccccgtgcaa cattggaggg gtcggcaaca acaccttgac ctgtcctacg gactgcttcc 2100
   ggaagcaccc cgaggccact tacaccaaat gcggctcggg cccttggttg acacctaggt 2160
   gcatggttga ctacccatac agactctggc actacccttg cactgtcaat tttaccatct 2220
   tcaaggtcag gatgtatgta gggggtgtgg agcacaggct cgacgccgcg tgcaattgga 2280
   cccgaggaga gcgttgcaat gtggaggaca gggatagatc agagcttagc ccactgctac 2340
   tgtccacaac agagtggcag atactgccct gttccttcac caccctaccg gctctgtcca 2400
   ctggtttgat ccacctccac cagaacatcg tggacgtgca atacctgtac ggtgtggggt 2460
   cagtggttgt ctccgttgta atcagatggg agtatgtcgt gctgctcttc cttctcctgg 2520
   cggacgcacg cgtctgcgcc tgcttgtgga tgatgctgct gatagctcag gctgaggccg 2580
   ccttagagaa cttggtggtc ctcaatgcgg catctgtagc tggagcgcat ggcactctct 2640
   ccttccttgt gttcttctgt gccgcctggt acatcaaggg caagctggtc cctggagcgg 2700
   catatgcttt ctatggcgtg tggccgctgc tcctgctcct gctggcatta ccaccacgag 2760
   catacgccat ggaccgggag atggctgcat catgcggagg cgcggtcttc ataggtctgg 2820
   cactcctgac cttgtcacca cactataaag cgttcctcgc taggctcatt tggtggttgc 2880
   agtatcttat caccagggtc gaggcgctac tgcaagtgtg ggtcccccct cttaacgttc 2940
   gggggggccg cgatgccatc atcctcctca cgtgcgcggt ccatccagag ctaatctttg 3000
   aaatcaccaa aatcttgctc gccataattg gtccgctcat ggtgctccag gctggcttaa 3060
   ccagagtgcc gtacttcgtg cgcgctcagg ggctcatccg tgtgtgcatg ttggtgcgga 3120
   aagtcgctgg gggtcactac gtccagatgg ctctcatgag gctggccgcg ctgacgggca 3180
   cgtacgttta caaccatctt actccgctgc gggactgggc ccacgcgggc ttgcgggacc 3240
   ttgtggtggc agttgagccc gtcgtcttct ctgacatgga gaccaagatc atcacctggg 3300
   gggcagacac cgcggcgtgt ggggacatca tcttgggtct acccgtctcc gcccgaaggg 3360
   ggagggagat acttttggga ccggccgaca gttatgaagg gcagggatgg cggcttcttg 3420
   cgcctatcac ggcctattcc caacagacgc ggggcctact cggttgcatc atcactagtc 3480
   tcacaggccg ggacaggaac caggtcgagg gggaggttca ggtggtttcc accgcaacgc 3540
   agtccttctt ggcgacctgc atcaatggtg tgtgttggac tgtctaccat ggtgccggct 3600
   caaagaccct agccggccca aagggtccga tcacccaaat gtacaccaat gtagaccagg 3660
   acctcgtcgg ctggcgggcg ccccccgggg cgcgttcctt aacaccatgc acctgcggca 3720
   gctcggacct ctacttggtc acgaggcatg ctgatgtcat tccggtgcgc cggcggggcg 3780
   ataacagggg cagcctgctt tcccccagac ctgtctctta cttaaagggc tcctcgggtg 3840
   gtccactgct ctgcccctca gggcacgctg tgggcatctt ccgggccgcc gtgtgcacca 3900
   ggggggttgc gaaggcggtg gactttgtgc ccgttgagtc catggaaact accatgcggt 3960
   ctccggtctt cacggacaat tcatctcccc cggccgtacc gcagacattc caagtggccc 4020
   atctacacgc tcccactggc agcggtaaga gcactaaggt gccggctgct tatgcagccc 4080
   aagggtacaa ggtactcgtc ctgaacccat ccgttgccgc caccttaggt tttggggcgt 4140
   atatgtctaa agcacatggt gttgacccca acatcagaac tggggtaagg accatcacca 4200
   caggcgctcc cattacgtat tccacttatg gcaagttcct tgccgacggt ggttgctctg 4260
   ggggcgccta tgacatcata atatgtgatg agtgccactc aactgactcg acttccatct 4320
   tgggcatcgg cactgtcttg gaccaagcgg agacggctgg agcgcggctc gtcgtgctcg 4380
   ccaccgctac acctccggga tcagtcaccg tgccacatcc caatatcgag gaggtggctt 4440
   tgtccaacac tggagagatc cccttttatg gtaaggcctt tcccatcgag gccatcaagg 4500
   gggggaggca tctcattttc tgccactcca agaagaaatg tgacgagctc gccacaaagc 4560
   tgtcggccct tggaatcaac gctgtagcgt actaccgggg tcttgatgtg tccgtcatac 4620
   cgacaagcgg agatgtcgtt gtcgtggcaa cagacgctct gatgacgggc tacactggcg 4680
   actttgactc agtgatcgac tgtaacacat gtgtcaccca gacagtcgac ttcagcttgg 4740
   accctacctt caccattgag acgacgaccg tgccccaaga cgcggtatcg cgctcgcaac 4800
   ggcgaggcag gactggtagg ggcagggggg gcatatacag gtttgtgact ccaggggaac 4860
   ggccctcggg catgttcgat tcttcggtcc tgtgcgagtg ctatgacgcg ggctgtgctt 4920
   ggtacgagct cacgcccgcc gagacctcgg ttaggttgcg ggcttaccta aatacaccag 4980
   ggttgcccgt ctgccaggac catctggagt tctgggagag cgtcttcaca ggcctcaccc 5040
   acatagatgc ccatttctta tcccagacca agcaggcagg agacaacttc ccctacctgg 5100
   tagcgtacca ggctacggtg tgcgccaggg cccaggcccc accaccatcg tgggaccaaa 5160
   tgtgggagtg tctcatacgg ctaaagccta cactgcacgg gccaacaccc ctgctgtaca 5220
   ggctaggagc cgtccaaaac gaagtcactc tcacacaccc cataaccaaa ttcatcatgg 5280
   catgcatgtc ggctgaccta gaggtcgtca cgagcacctg ggtgctagta ggcggggtcc 5340
   ttgcagctct ggccgcgtac tgcctgacaa cgggcagcgt ggtcattgtg ggtaggatca 5400
   tcttgtctgg gaagccggct gtcattcccg atagggaagt gctctaccag gagttcgatg 5460
   aaatggaaga gtgcgcctcg cacctccctt acattgaaca gggaatgcag ctcgccgagc 5520
   aattcaagca gaaggcgctc gggttgctgc aagcagccac caagcaagcg gaggctgctg 5580
   ctcccgtggt ggagtccaag tggcaagccc ttgaggcctt ttgggcgaag cacatgtgga 5640
   atttcatcag cgggatacag tacttagcag gcttgtccac tctgcctggg aaccccccga 5700
   tagcttcact gatggcattc acagcctcta tcaccagccc gctcaccacc cagcataccc 5760
   tcttgtttaa catcttgggg gggtgggtgg cagcccaact cgcccccccc agcgctgctt 5820
   cagctttcgt gggcgccggg atcgctggcg cggctgttgg cagcataggc cttgggaagg 5880
   tgcttgtgga catcctggcg ggctatgggg cgggggtggc aggcgcgctc gtggccttta 5940
   aggtcatgag cggcgaaatg ccctccaccg aggacctagt caacttgctc cctgccatcc 6000
   tctcccccgg tgccctggtt gtcggggtcg tgtgcgcggc aatactgcgc cggcacgtgg 6060
   gcccggggga gggggctgtg cagtggatga accggctgat agcgttcgct tcgcggggta 6120
   accatgtttc ccccacgcac tatgtgcctg agagcgacgc ggccgcgcgt gtcactcaga 6180
   tcctctccag ccttaccatc acccagctgt tgaagagact ccaccagtgg atcaatgagg 6240
   actgctccac gccatgctcc ggctcgtggc tcagggatgt ttgggactgg atatgcacgg 6300
   tgttgactga cttcaagacc tggctccagt ccaagctcct gccacggttg ccgggggtcc 6360
   ctttcctttc atgccaacgt ggatataagg gagtctggcg aggagatggc gtcatgcaca 6420
   ccacttgccc atgtggcgca caaatcaccg gacatgtcaa aaacggttcc atgaggatcg 6480
   ttgggcctaa aacctgtagc aacacgtggc acggaacatt ccccatcaac gcgtacacca 6540
   cgggttcctg cacaccctcc ccggcgccaa actattccag ggcgttgtgg cgggtagctg 6600
   ctgaggagta cgtggaggtc acgcgggtgg gggatttcca ctacgtgacg ggcatgacca 6660
   ctgacaacat aaaatgccca tgccaggttc cggcccccga atttttcaca gagttggacg 6720
   gggtgcggct gcacaggtac gctccggcgt gcaaacctct cttacgggat gaggtcacat 6780
   tccaggtcgg gctcaaccaa tacgtggttg ggtcacagct tccatgcgag cccgaaccgg 6840
   atgtaacagt gctcacttcc atgctcaccg acccctccca cattacagca gagacggcta 6900
   agcgtaggct ggccaggggg tctccccctt ccttggcccg ctcttcagct agtcagttgt 6960
   ctgcgccttc tctgaaggcg acatgcacca cccatcatga ctccccagac gccgacctca 7020
   tcgaggccaa cctcctgtgg cggcaggaga tgggcgggaa catcacccgc gtggagtcag 7080
   agaacaaagt agtgattctg gactctttcg acccgcttcg agcggaggag gatgagaggg 7140
   aagtatccgt tccggcggag atcctgcgga aaaccaggaa attcccctca gcgttgccca 7200
   tatgggcacg cccggactac aacccaccac tgttagagtc ttggagagac ccggactatg 7260
   tccctccggt ggtacacggg tgcccgttac cgcccaccaa ggcccctcca ataccacctc 7320
   cacggagaaa gagaacagtt atcctgacag aatccactgt gtcttctgcc ttggcggagc 7380
   ttgctacaaa gaccttcggc agctccggat cgtcggccgt cgacagcggc acagcgactg 7440
   cccctcctga tgggccctcc gacgacggtg atgcaggatc ggacgctgag tcatactcct 7500
   ccatgccccc ccttgagggg gagccggggg atcccgatct cagcgacggg tcttggtcca 7560
   ctgtgagcga ggaggccagt gaggacgtcg tctgctgctc aatgtcctac acatggacag 7620
   gtgccttaat cacgccatgc gccgcggagg agagcaagct gcccatcaat gcgttgagca 7680
   actctttgct gcgccaccac aacatggtct atgctacaac atcccgcagc gcaagccagc 7740
   ggcagaaaaa ggtcaccttt gacagactgc aagtcctgga cgaccactac cgggacgtgc 7800
   tcaaggagat gaaggcgaag gcgtccacag ttaaggccaa acttctatcc gtagaagagg 7860
   cctgcaagct gacgccccca cattcagcca gatctaagtt tggctatggg gcaaaggacg 7920
   tccggaacct atccagtaag gccgttaacc acatccactc cgtgtggaag gacttgctgg 7980
   aagacactga gacaccaatt gataccaccg tcatggcaaa aaatgaggtc ttctgtgtcc 8040
   aaccagagaa aggaggccgc aagccagctc gccttatcgt gttcccagac ctgggggtgc 8100
   gtgtgtgcga gaaaatggcc ctttatgatg tggtctccac tcttcctcag gccgtgatgg 8160
   gctcctcata cggattccaa tactctcctg gacagcgggt cgagttcctg gtgaatgcct 8220
   ggaaatcaaa gaagacccct atgggcttcg catatgacac ccgctgtttt gactcaacag 8280
   tcactgagaa tgacatccgt gttgaggagt caatttacca atgttgtgac ttggcccccg 8340
   aggccagaca ggccataagg tcgctcacag agcggcttta tatcgggggc cccctgacta 8400
   attcaaaagg gcagaactgc ggttatcgcc ggtgccgcgc cagcggcgtg ctgacgacta 8460
   gctgcggtaa caccctcaca tgttacttga aggcctctgc agcctgtcga gctgcaaagc 8520
   tccaggactg cacgatgctc gtgtgcggag acgacctcgt cgttatctgt gagagcgcgg 8580
   ggacccagga ggacgcggcg agcctgcgag tcttcacgga ggctatgacc aggtactctg 8640
   ccccccccgg ggacccgccc caaccggaat acgacttgga gctgataaca tcatgctcct 8700
   ccaacgtgtc ggtcgcgcac gatgcatctg gcaagcgggt gtactatctc acccgcgacc 8760
   ccaccacccc tcttgctcgg gctgcgtggg agacagctag acacactcca gtcaactcct 8820
   ggctaggcaa catcatcatg tatgcgccca ccttatgggc aaggatgatt ctgatgaccc 8880
   acttcttctc aatccttcta gcccaggagc aacttgagaa agccctagat tgccagatct 8940
   acggggccac ttactccatt gagccacttg acctacctca gatcattcaa cgactccatg 9000
   gtcttagcgc attttcactc catagttact ctccaggtga gatcaatagg gtggcttcat 9060
   gcctcaggaa acttggggta ccgcccttgc gagtctggag acatcgggcc agaagtgtcc 9120
   gcgctaagct actgtcccag ggggggaggg ccgccacgtg tggcaagtac ctcttcaact 9180
   gggccgtacg gaccaagctc aaactcactc caattccggc tgcgtcccgg ttggacttgt 9240
   ccggctggtt cgtcgccggt tacagcgggg gagacatata tcacagcctg tctcgcgccc 9300
   gaccccgctg gttcatgtgg tgcctactcc tactttccgt aggggtaggc atctacctgc 9360
   tccccaaccg gtgaacgggg agctaaacac tccaggccaa taggccattc cttttttttt 9420
   tttttttttt tttttctttt tttttccttt tttttttttt tttttttttt ttccttttct 9480
   ttcttctttg gtggctccat cttagcccta gtcacggcta gctgtgaaag gtccgtgagc 9540
   cgcatgactg cagagagtgc tgatactggc ctctctgcag atcatgt               9587
<210> 8
   <211> 3010
   <212> PRT
   <213> Hepatitis C virus
<400> 8
   Met Ser Thr Asn Pro Lys Pro Gln Arg Lys Thr Lys Arg Asn Thr Asn
    1               5                  10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Gln Pro Glu Gly Arg Ala Trp Ala Gln Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Ile Pro Ala Ser Ala Tyr
           180                 185                 190
   Glu Val Arg Asn Val Ser Gly Val Tyr His Val Thr Asn Asp Cys Ser
       195                 200                 205
   Asn Ser Ser Ile Val Tyr Glu Ala Ala Asp Met Ile Met His Thr Pro
       210                 215                 220
   Gly Cys Val Pro Cys Val Arg Glu Asp Asn Ser Ser Arg Cys Trp Val
   225                 230                 235                 240
   Ala Leu Thr Pro Thr Leu Ala Ala Arg Asn Ser Ser Val Pro Thr Thr
                   245                 250                 255
   Thr Ile Arg Arg His Val Asp Leu Leu Val Gly Ala Ala Ala Phe Cys
               260                 265                 270
   Ser Ala Met Tyr Val Gly Asp Leu Cys Gly Ser Val Phe Leu Ile Ser
           275                 280                 285
   Gln Leu Phe Thr Phe Ser Pro Arg Arg Tyr Glu Thr Val Gln Asp Cys
       290                 295                 300
   Asn Cys Ser Leu Tyr Pro Gly His Val Ser Gly His Arg Met Ala Trp
   305                 310                 315                 320
   Asp Met Met Met Asn Trp Ser Pro Thr Thr Ala Leu Val Val Ser Gln
                   325                 330                 335
   Leu Leu Arg Ile Pro Gln Ala Val Val Asp Met Val Ala Gly Ala His
               340                 345                 350
   Trp Gly Val Leu Ala Gly Leu Ala Tyr Tyr Ser Met Val Gly Asn Trp
           355                 360                 365
   Ala Lys Val Leu Ile Val Met Leu Leu Phe Ala Gly Val Asp Gly Asn
       370                 375                 380
   Thr Arg Val Ser Gly Gly Glu Ala Ala Lys Asn Thr Met Gly Phe Ala
   385                 390                 395                 400
   Ser Leu Phe Val Ser Gly Pro Ser Gln Lys Ile Gln Leu Ile Asn Thr
                   405                 410                 415
   Asn Gly Ser Trp His Ile Asn Arg Thr Ala Leu Asn Cys Asp Asp Ser
               420                 425                 430
   Leu His Thr Gly Phe Leu Ala Ala Leu Phe Tyr Ala His Lys Phe Asn
           435                 440                 445
   Ser Ser Gly Cys Ser Gly Arg Met Ala Ser Cys Arg Pro Ile Asp Glu
       450                 455                 460
   Phe Ala Gln Gly Trp Gly Pro Ile Thr His Gly Val Pro Asp Asn Leu
   465                 470                 475                 480
   Asp Gln Arg Pro Tyr Cys Trp His Tyr Ala Pro Arg Pro Cys Gly Ile
                   485                 490                 495
   Val Pro Ala Ser Gln Val Cys Gly Pro Val Tyr Cys Phe Thr Pro Ser
               500                 505                 510
   Pro Val Val Val Gly Thr Thr Asp Arg Phe Gly Ala Pro Thr Tyr Ser
           515                 520                 525
   Trp Gly Glu Asn Glu Thr Asp Val Leu Leu Leu Asn Asn Thr Arg Pro
       530                 535                 540
   Pro Gln Gly Asn Trp Phe Gly Cys Thr Trp Met Asn Ser Thr Gly Phe
   545                 550                 555                 560
   Thr Lys Thr Cys Gly Gly Pro Pro Cys Asn Ile Gly Gly Val Gly Asn
                   565                 570                 575
   Asn Thr Leu Thr Cys Pro Thr Asp Cys Phe Arg Lys His Pro Glu Ala
               580                 585                 590
   Thr Tyr Thr Lys Cys Gly Ser Gly Pro Trp Leu Thr Pro Arg Cys Met
           595                 600                 605
   Val Asp Tyr Pro Tyr Arg Leu Trp His Tyr Pro Cys Thr Val Asn Phe
       610                 615                 620
   Thr Ile Phe Lys Val Arg Met Tyr Val Gly Gly Val Glu His Arg Leu
   625                 630                 635                 640
   Asp Ala Ala Cys Asn Trp Thr Arg Gly Glu Arg Cys Asn Val Glu Asp
                   645                 650                 655
   Arg Asp Arg Ser Glu Leu Ser Pro Leu Leu Leu Ser Thr Thr Glu Trp
               660                 665                 670
   Gln Ile Leu Pro Cys Ser Phe Thr Thr Leu Pro Ala Leu Ser Thr Gly
           675                 680                 685
   Leu Ile His Leu His Gln Asn Ile Val Asp Val Gln Tyr Leu Tyr Gly
       690                 695                 700
   Val Gly Ser Val Val Val Ser Val Val Ile Arg Trp Glu Tyr Val Val
   705                 710                 715                 720
   Leu Leu Phe Leu Leu Leu Ala Asp Ala Arg Val Cys Ala Cys Leu Trp
                   725                 730                 735
   Met Met Leu Leu Ile Ala Gln Ala Glu Ala Ala Leu Glu Asn Leu Val
               740                 745                 750
   Val Leu Asn Ala Ala Ser Val Ala Gly Ala His Gly Thr Leu Ser Phe
           755                 760                 765
   Leu Val Phe Phe Cys Ala Ala Trp Tyr Ile Lys Gly Lys Leu Val Pro
       770                 775                 780
   Gly Ala Ala Tyr Ala Phe Tyr Gly Val Trp Pro Leu Leu Leu Leu Leu
   785                 790                 795                 800
   Leu Ala Leu Pro Pro Arg Ala Tyr Ala Met Asp Arg Glu Met Ala Ala
                   805                 810                 815
   Ser Cys Gly Gly Ala Val Phe Ile Gly Leu Ala Leu Leu Thr Leu Ser
               820                 825                 830
   Pro His Tyr Lys Ala Phe Leu Ala Arg Leu Ile Trp Trp Leu Gln Tyr
           835                 840                 845
   Leu Ile Thr Arg Val Glu Ala Leu Leu Gln Val Trp Val Pro Pro Leu
       850                 855                 860
   Asn Val Arg Gly Gly Arg Asp Ala Ile Ile Leu Leu Thr Cys Ala Val
   865                 870                 875                 880
   His Pro Glu Leu Ile Phe Glu Ile Thr Lys Ile Leu Leu Ala Ile Ile
                   885                 890                 895
   Gly Pro Leu Met Val Leu Gln Ala Gly Leu Thr Arg Val Pro Tyr Phe
               900                 905                 910
   Val Arg Ala Gln Gly Leu Ile Arg Val Cys Met Leu Val Arg Lys Val
           915                 920                 925
   Ala Gly Gly His Tyr Val Gln Met Ala Leu Met Arg Leu Ala Ala Leu
       930                 935                 940
   Thr Gly Thr Tyr Val Tyr Asn His Leu Thr Pro Leu Arg Asp Trp Ala
   945                 950                 955                 960
   His Ala Gly Leu Arg Asp Leu Val Val Ala Val Glu Pro Val Val Phe
                   965                 970                 975
   Ser Asp Met Glu Thr Lys Ile Ile Thr Trp Gly Ala Asp Thr Ala Ala
               980                 985                 990
   Cys Gly Asp Ile Ile Leu Gly Leu Pro Val Ser Ala Arg Arg Gly Arg
           995                 1000                1005
   Glu Ile Leu Leu Gly Pro Ala Asp Ser Tyr Glu Gly Gln Gly Trp Arg
      1010                1015                1020
   Leu Leu Ala Pro Ile Thr Ala Tyr Ser Gln Gln Thr Arg Gly Leu Leu
   1025                1030                1035                1040
   Gly Cys Ile Ile Thr Ser Leu Thr Gly Arg Asp Arg Asn Gln Val Glu
                  1045                1050                1055
   Gly Glu Val Gln Val Val Ser Thr Ala Thr Gln Ser Phe Leu Ala Thr
              1060                1065                1070
   Cys Ile Asn Gly Val Cys Trp Thr Val Tyr His Gly Ala Gly Ser Lys
          1075                1080                1085
   Thr Leu Ala Gly Pro Lys Gly Pro Ile Thr Gln Met Tyr Thr Asn Val
      1090                1095                1100
   Asp Gln Asp Leu Val Gly Trp Arg Ala Pro Pro Gly Ala Arg Ser Leu
   1105                1110                1115                1120
   Thr Pro Cys Thr Cys Gly Ser Ser Asp Leu Tyr Leu Val Thr Arg His
                  1125                1130                1135
   Ala Asp Val Ile Pro Val Arg Arg Arg Gly Asp Asn Arg Gly Ser Leu
              1140                1145                1150
   Leu Ser Pro Arg Pro Val Ser Tyr Leu Lys Gly Ser Ser Gly Gly Pro
          1155                1160                1165
   Leu Leu Cys Pro Ser Gly His Ala Val Gly Ile Phe Arg Ala Ala Val
      1170                1175                1180
   Cys Thr Arg Gly Val Ala Lys Ala Val Asp Phe Val Pro Val Glu Ser
   1185                1190                1195                1200
   Met Glu Thr Thr Met Arg Ser Pro Val Phe Thr Asp Asn Ser Ser Pro
                   1205                1210                1215
   Pro Ala Val Pro Gln Thr Phe Gln Val Ala His Leu His Ala Pro Thr
               1220                1225                1230
   Gly Ser Gly Lys Ser Thr Lys Val Pro Ala Ala Tyr Ala Ala Gln Gly
           1235                1240                1245
   Tyr Lys Val Leu Val Leu Asn Pro Ser Val Ala Ala Thr Leu Gly Phe
       1250                1255                1260
   Gly Ala Tyr Met Ser Lys Ala His Gly Val Asp Pro Asn Ile Arg Thr
   1265                1270                1275                1280
   Gly Val Arg Thr Ile Thr Thr Gly Ala Pro Ile Thr Tyr Ser Thr Tyr
                   1285                1290                1295
   Gly Lys Phe Leu Ala Asp Gly Gly Cys Ser Gly Gly Ala Tyr Asp Ile
               1300                1305                1310
   Ile Ile Cys Asp Glu Cys His Ser Thr Asp Ser Thr Ser Ile Leu Gly
           1315                1320                1325
   Ile Gly Thr Val Leu Asp Gln Ala Glu Thr Ala Gly Ala Arg Leu Val
       1330                1335                1340
   Val Leu Ala Thr Ala Thr Pro Pro Gly Ser Val Thr Val Pro His Pro
   1345                1350                1355                1360
   Asn Ile Glu Glu Val Ala Leu Ser Asn Thr Gly Glu Ile Pro Phe Tyr
                   1365                1370                1375
   Gly Lys Ala Phe Pro Ile Glu Ala Ile Lys Gly Gly Arg His Leu Ile
               1380                1385                1390
   Phe Cys His Ser Lys Lys Lys Cys Asp Glu Leu Ala Thr Lys Leu Ser
           1395                1400                1405
   Ala Leu Gly Ile Asn Ala Val Ala Tyr Tyr Arg Gly Leu Asp Val Ser
       1410                1415                1420
   Val Ile Pro Thr Ser Gly Asp Val Val Val Val Ala Thr Asp Ala Leu
   1425                1430                1435                1440
   Met Thr Gly Tyr Thr Gly Asp Phe Asp Ser Val Ile Asp Cys Asn Thr
                   1445                1450                1455
   Cys Val Thr Gln Thr Val Asp Phe Ser Leu Asp Pro Thr Phe Thr Ile
               1460                1465                1470
   Glu Thr Thr Thr Val Pro Gln Asp Ala Val Ser Arg Ser Gln Arg Arg
           1475                1480                1485
   Gly Arg Thr Gly Arg Gly Arg Gly Gly Ile Tyr Arg Phe Val Thr Pro
       1490                1495                1500
   Gly Glu Arg Pro Ser Gly Met Phe Asp Ser Ser Val Leu Cys Glu Cys
   1505                1510                1515                1520
   Tyr Asp Ala Gly Cys Ala Trp Tyr Glu Leu Thr Pro Ala Glu Thr Ser
   1525 1530 1535
   Val Arg Leu Arg Ala Tyr Leu Asn Thr Pro Gly Leu Pro Val Cys Gln
   1540 1545 1550
   Asp His Leu Glu Phe Trp Glu Ser Val Phe Thr Gly Leu Thr His Ile
   1555 1560 1565
   Asp Ala His Phe Leu Ser Gln Thr Lys Gln Ala Gly Asp Asn Phe Pro
   1570 1575 1580
   Tyr Leu Val Ala Tyr Gln Ala Thr Val Cys Ala Arg Ala Gln Ala Pro
   1585 1590 1595 1600
   Pro Pro Ser Trp Asp Gln Met Trp Glu Cys Leu Ile Arg Leu Lys Pro
   1605 1610 1615
   Thr Leu His Gly Pro Thr Pro Leu Leu Tyr Arg Leu Gly Ala Val Gln
   1620 1625 1630
   Asn Glu Val Thr Leu Thr His Pro Ile Thr Lys Phe Ile Met Ala Cys
   1635 1640 1645
   Met Ser Ala Asp Leu Glu Val Val Thr Ser Thr Trp Val Leu Val Gly
   1650 1655 1660
   Gly Val Leu Ala Ala Leu Ala Ala Tyr Cys Leu Thr Thr Gly Ser Val
   1665 1670 1675 1680
   Val Ile Val Gly Arg Ile Ile Leu Ser Gly Lys Pro Ala Val Ile Pro
   1685 1690 1695
   Asp Arg Glu Val Leu Tyr Gln Glu Phe Asp Glu Met Glu Glu Cys Ala
   1700 1705 1710
   Ser His Leu Pro Tyr Ile Glu Gln Gly Met Gln Leu Ala Glu Gln Phe
   1715 1720 1725
   Lys Gln Lys Ala Leu Gly Leu Leu Gln Ala Ala Thr Lys Gln Ala Glu
   1730 1735 1740
   Ala Ala Ala Pro Val Val Glu Ser Lys Trp Gln Ala Leu Glu Ala Phe
   1745 1750 1755 1760
   Trp Ala Lys His Met Trp Asn Phe Ile Ser Gly Ile Gln Tyr Leu Ala
   1765 1770 1775
   Gly Leu Ser Thr Leu Pro Gly Asn Pro Pro Ile Ala Ser Leu Met Ala
   1780 1785 1790
   Phe Thr Ala Ser Ile Thr Ser Pro Leu Thr Thr Gln His Thr Leu Leu
   1795 1800 1805
   Phe Asn Ile Leu Gly Gly Trp Val Ala Ala Gln Leu Ala Pro Pro Ser
   1810 1815 1820
   Ala Ala Ser Ala Phe Val Gly Ala Gly Ile Ala Gly Ala Ala Val Gly
   1825 1830 1835 1840
   Ser Ile Gly Leu Gly Lys Val Leu Val Asp Ile Leu Ala Gly Tyr Gly
   1845 1850 1855
   Ala Gly Val Ala Gly Ala Leu Val Ala Phe Lys Val Met Ser Gly Glu
   1860 1865 1870
   Met Pro Ser Thr Glu Asp Leu Val Asn Leu Leu Pro Ala Ile Leu Ser
   1875 1880 1885
   Pro Gly Ala Leu Val Val Gly Val Val Cys Ala Ala Ile Leu Arg Arg
   1890 1895 1900
   His Val Gly Pro Gly Glu Gly Ala Val Gln Trp Met Asn Arg Leu Ile
   1905 1910 1915 1920
   Ala Phe Ala Ser Arg Gly Asn His Val Ser Pro Thr His Tyr Val Pro
   1925 1930 1935
   Glu Ser Asp Ala Ala Ala Arg Val Thr Gln Ile Leu Ser Ser Leu Thr
   1940 1945 1950
   Ile Thr Gln Leu Leu Lys Arg Leu His Gln Trp Ile Asn Glu Asp Cys
   1955 1960 1965
   Ser Thr Pro Cys Ser Gly Ser Trp Leu Arg Asp Val Trp Asp Trp Ile
   1970 1975 1980
   Cys Thr Val Leu Thr Asp Phe Lys Thr Trp Leu Gln Ser Lys Leu Leu
   1985 1990 1995 2000
   Pro Arg Leu Pro Gly Val Pro Phe Leu Ser Cys Gln Arg Gly Tyr Lys
   2005 2010 2015
   Gly Val Trp Arg Gly Asp Gly Val Met His Thr Thr Cys Pro Cys Gly
   2020 2025 2030
   Ala Gln Ile Thr Gly His Val Lys Asn Gly Ser Met Arg Ile Val Gly
   2035 2040 2045
   Pro Lys Thr Cys Ser Asn Thr Trp His Gly Thr Phe Pro Ile Asn Ala
   2050 2055 2060
   Tyr Thr Thr Gly Ser Cys Thr Pro Ser Pro Ala Pro Asn Tyr Ser Arg
   2065 2070 2075 2080
   Ala Leu Trp Arg Val Ala Ala Glu Glu Tyr Val Glu Val Thr Arg Val
   2085 2090 2095
   Gly Asp Phe His Tyr Val Thr Gly Met Thr Thr Asp Asn Ile Lys Cys
   2100 2105 2110
   Pro Cys Gln Val Pro Ala Pro Glu Phe Phe Thr Glu Leu Asp Gly Val
   2115 2120 2125
   Arg Leu His Arg Tyr Ala Pro Ala Cys Lys Pro Leu Leu Arg Asp Glu
   2130 2135 2140
   Val Thr Phe Gln Val Gly Leu Asn Gln Tyr Val Val Gly Ser Gln Leu
   2145 2150 2155 2160
   Pro Cys Glu Pro Glu Pro Asp Val Thr Val Leu Thr Ser Met Leu Thr
   2165 2170 2175
   Asp Pro Ser His Ile Thr Ala Glu Thr Ala Lys Arg Arg Leu Ala Arg
   2180 2185 2190
   Gly Ser Pro Pro Ser Leu Ala Arg Ser Ser Ala Ser Gln Leu Ser Ala
   2195 2200 2205
   Pro Ser Leu Lys Ala Thr Cys Thr Thr His His Asp Ser Pro Asp Ala
   2210 2215 2220
   Asp Leu Ile Glu Ala Asn Leu Leu Trp Arg Gln Glu Met Gly Gly Asn
   2225 2230 2235 2240
   Ile Thr Arg Val Glu Ser Glu Asn Lys Val Val Ile Leu Asp Ser Phe
   2245 2250 2255
   Asp Pro Leu Arg Ala Glu Glu Asp Glu Arg Glu Val Ser Val Pro Ala
   2260 2265 2270
   Glu Ile Leu Arg Lys Thr Arg Lys Phe Pro Ser Ala Leu Pro Ile Trp
   2275 2280 2285
   Ala Arg Pro Asp Tyr Asn Pro Pro Leu Leu Glu Ser Trp Arg Asp Pro
   2290 2295 2300
   Asp Tyr Val Pro Pro Val Val His Gly Cys Pro Leu Pro Pro Thr Lys
   2305 2310 2315 2320
   Ala Pro Pro Ile Pro Pro Pro Arg Arg Lys Arg Thr Val Ile Leu Thr
   2325 2330 2335
   Glu Ser Thr Val Ser Ser Ala Leu Ala Glu Leu Ala Thr Lys Thr Phe
   2340 2345 2350
   Gly Ser Ser Gly Ser Ser Ala Val Asp Ser Gly Thr Ala Thr Ala Pro
   2355 2360 2365
   Pro Asp Gly Pro Ser Asp Asp Gly Asp Ala Gly Ser Asp Ala Glu Ser
   2370 2375 2380
   Tyr Ser Ser Met Pro Pro Leu Glu Gly Glu Pro Gly Asp Pro Asp Leu
   2385 2390 2395 2400
   Ser Asp Gly Ser Trp Ser Thr Val Ser Glu Glu Ala Ser Glu Asp Val
   2405 2410 2415
   Val Cys Cys Ser Met Ser Tyr Thr Trp Thr Gly Ala Leu Ile Thr Pro
   2420 2425 2430
   Cys Ala Ala Glu Glu Ser Lys Leu Pro Ile Asn Ala Leu Ser Asn Ser
   2435 2440 2445
   Leu Leu Arg His His Asn Met Val Tyr Ala Thr Thr Ser Arg Ser Ala
   2450 2455 2460
   Ser Gln Arg Gln Lys Lys Val Thr Phe Asp Arg Leu Gln Val Leu Asp
   2465 2470 2475 2480
   Asp His Tyr Arg Asp Val Leu Lys Glu Met Lys Ala Lys Ala Ser Thr
   2485 2490 2495
   Val Lys Ala Lys Leu Leu Ser Val Glu Glu Ala Cys Lys Leu Thr Pro
   2500 2505 2510
   Pro His Ser Ala Arg Ser Lys Phe Gly Tyr Gly Ala Lys Asp Val Arg
   2515 2520 2525
   Asn Leu Ser Ser Lys Ala Val Asn His Ile His Ser Val Trp Lys Asp
   2530 2535 2540
   Leu Leu Glu Asp Thr Glu Thr Pro Ile Asp Thr Thr Val Met Ala Lys
   2545 2550 2555 2560
   Asn Glu Val Phe Cys Val Gln Pro Glu Lys Gly Gly Arg Lys Pro Ala
   2565 2570 2575
   Arg Leu Ile Val Phe Pro Asp Leu Gly Val Arg Val Cys Glu Lys Met
   2580 2585 2590
   Ala Leu Tyr Asp Val Val Ser Thr Leu Pro Gln Ala Val Met Gly Ser
   2595 2600 2605
   Ser Tyr Gly Phe Gln Tyr Ser Pro Gly Gln Arg Val Glu Phe Leu Val
   2610 2615 2620
   Asn Ala Trp Lys Ser Lys Lys Thr Pro Met Gly Phe Ala Tyr Asp Thr
   2625 2630 2635 2640
   Arg Cys Phe Asp Ser Thr Val Thr Glu Asn Asp Ile Arg Val Glu Glu
   2645 2650 2655
   Ser Ile Tyr Gln Cys Cys Asp Leu Ala Pro Glu Ala Arg Gln Ala Ile
   2660 2665 2670
   Arg Ser Leu Thr Glu Arg Leu Tyr Ile Gly Gly Pro Leu Thr Asn Ser
   2675 2680 2685
   Lys Gly Gln Asn Cys Gly Tyr Arg Arg Cys Arg Ala Ser Gly Val Leu
   2690 2695 2700
   Thr Thr Ser Cys Gly Asn Thr Leu Thr Cys Tyr Leu Lys Ala Ser Ala
   2705 2710 2715 2720
   Ala Cys Arg Ala Ala Lys Leu Gln Asp Cys Thr Met Leu Val Cys Gly
   2725 2730 2735
   Asp Asp Leu Val Val Ile Cys Glu Ser Ala Gly Thr Gln Glu Asp Ala
   2740 2745 2750
   Ala Ser Leu Arg Val Phe Thr Glu Ala Met Thr Arg Tyr Ser Ala Pro
   2755 2760 2765
   Pro Gly Asp Pro Pro Gln Pro Glu Tyr Asp Leu Glu Leu Ile Thr Ser
   2770 2775 2780
   Cys Ser Ser Asn Val Ser Val Ala His Asp Ala Ser Gly Lys Arg Val
   2785 2790 2795 2800
   Tyr Tyr Leu Thr Arg Asp Pro Thr Thr Pro Leu Ala Arg Ala Ala Trp
   2805 2810 2815
   Glu Thr Ala Arg His Thr Pro Val Asn Ser Trp Leu Gly Asn Ile Ile
   2820 2825 2830
   Met Tyr Ala Pro Thr Leu Trp Ala Arg Met Ile Leu Met Thr His Phe
   2835 2840 2845
   Phe Ser Ile Leu Leu Ala Gln Glu Gln Leu Glu Lys Ala Leu Asp Cys
   2850 2855 2860
   Gln Ile Tyr Gly Ala Thr Tyr Ser Ile Glu Pro Leu Asp Leu Pro Gln
   2865 2870 2875 2880
   Ile Ile Gln Arg Leu His Gly Leu Ser Ala Phe Ser Leu His Ser Tyr
   2885 2890 2895
   Ser Pro Gly Glu Ile Asn Arg Val Ala Ser Cys Leu Arg Lys Leu Gly
   2900 2905 2910
   Val Pro Pro Leu Arg Val Trp Arg His Arg Ala Arg Ser Val Arg Ala
   2915 2920 2925
   Lys Leu Leu Ser Gln Gly Gly Arg Ala Ala Thr Cys Gly Lys Tyr Leu
   2930 2935 2940
   Phe Asn Trp Ala Val Arg Thr Lys Leu Lys Leu Thr Pro Ile Pro Ala
   2945 2950 2955 2960
   Ala Ser Arg Leu Asp Leu Ser Gly Trp Phe Val Ala Gly Tyr Ser Gly
   2965 2970 2975
   Gly Asp Ile Tyr His Ser Leu Ser Arg Ala Arg Pro Arg Trp Phe Met
   2980 2985 2990
   Trp Cys Leu Leu Leu Leu Ser Val Gly Val Gly Ile Tyr Leu Leu Pro
   2995 3000 3005
   Asn Arg
   3010
<210> 9
   <211> 9587
   <212> DNA
   <213> Hepatitis C virus
<400> 9
   gccagccccc gattgggggc gacactccac catagatcac tcccctgtga ggaactactg 60
   tcttcacgca gaaagcgtct agccatggcg ttagtatgag tgtcgtgcag cctccaggac 120
   cccccctccc gggagagcca tagtggtctg cggaaccggt gagtacaccg gaattgccag 180
   gacgaccggg tcctttcttg gattaacccg ctcaatgcct ggagatttgg gcgtgccccc 240
   gcaagactgc tagccgagta gtgttgggtc gcgaaaggcc ttgtggtact gcctgatagg 300
   gtgcttgcga gtgccccggg aggtctcgta gaccgtgcac catgagcacg aatcctaaac 360
   ctcaaagaaa aaccaaacgt aacaccaacc gccgcccaca ggacgtcaag ttcccgggcg 420
   gtggtcagat cgttggtgga gtttacctgt tgccgcgcag gggccccagg ttgggtgtgc 480
   gcgcgactag gaagacttcc gagcggtcgc agcctcgtgg aaggcgacaa cctatcccca 540
   aggctcgcca gcccgagggc agggcctggg ctcagcccgg atacccttgg cccctctacg 600
   gcaatgaggg tctggggtgg gcaggatggc tcctgtcacc ccgtggctct cggcctagtt 660
   ggggccctac ggacccccgg cgtaggtcgc gtaatttggg taaggtcatc gataccctca 720
   catgcggctt cgccgacctc atggggtaca tcccgctcgt cggcgccccc ctaggaggcg 780
   ctgccagggc cctggcgcat ggcgtccggg ttctggagga cggcgtgaac tatgcaacag 840
   ggaatttgcc cggttgctct ttctctatct tcctcttggc tttgctgtcc tgtttgacca 900
   tcccagcttc cgcttacgaa gtgcgcaacg tatccggggt gtaccatgtc acgaacgact 960
   gctccaactc aagtattgtg tatgaggcag cggacatgat catgcacacc cccgggtgcg 1020
   tgccctgcgt tcgggaggac aattcctccc gttgctgggt agcgctcact cccacgcttg 1080
   cggccaggaa cagcagcgtc cccaccacga caatacgacg ccacgtcgac ttgctcgttg 1140
   gggcagctgc tttctgttcc gctatgtacg tgggagacct ctgcggatct gttttcctca 1200
   tttcccagct gttcaccttc tcacctcgcc ggtatgagac ggtgcaagac tgcaactgct 1260
   cgctctatcc cggccacgta tcaggtcacc gcatggcttg ggatatgatg atgaactggt 1320
   cacctacaac agccctggtg gtgtcgcagt tgctccggat cccacaagcc gtcgtggaca 1380
   tggtggcggg ggcccactgg ggagtcctgg cgggccttgc ctactattcc atggtgggga 1440
   actgggctaa ggtcttgatt gtgatgctac tttttgccgg cgtcgacggg aacactcgtg 1500
   tgtcaggggg ggaagcagcc aagaacacca tgggctttgc atccctcttt gtgtctggcc 1560
   cgtcgcagaa aatccaactt attaacacca atggcagctg gcacatcaac aggactgctc 1620
   tgaattgcga tgactccctc cacaccgggt tccttgctgc gctgttttac gcgcacaagt 1680
   tcaactcgtc cggatgctcg gggcgcatgg ccagctgccg ccccattgac gagtttgccc 1740
   aggggtgggg tcccatcact cacggtgtgc ctgacaacct ggaccagagg ccttactgct 1800
   ggcactacgc gcctcggccg tgcggtatcg tacccgcgtc gcaggtgtgt ggcccagtgt 1860
   attgcttcac cccgagccct gtcgtggtgg ggacgaccga tcgtttcggc gcccccacgt 1920
   acagctgggg ggagaatgag acggacgtgc tactccttaa caacacgcga ccgccacaag 1980
   gcaactggtt cggttgcaca tggatgaaca gcaccgggtt caccaagacg tgcgggggcc 2040
   ccccgtgcaa cattggaggg gtcggcaaca acaccttgac ctgtcctacg gactgcttcc 2100
   ggaagcaccc cgaggccact tacaccaaat gcggctcggg cccttggttg acacctaggt 2160
   gcatggttga ctacccatac agactctggc actacccttg cactgtcaat tttaccatct 2220
   tcaaggtcag gatgtatgta gggggtgtgg agcacaggct cgacgccgcg tgcaattgga 2280
   cccgaggaga gcgttgcaat gtggaggaca gggatagatc agagcttagc ccactgctac 2340
   tgtccacaac agagtggcag atactgccct gttccttcac caccctaccg gctctgtcca 2400
   ctggtttgat ccacctccac cagaacatcg tggacgtgca atacctgtac ggtgtggggt 2460
   cagtggttgt ctccgttgta atcagatggg agtatgtcgt gctgctcttc cttctcctgg 2520
   cggacgcacg cgtctgcgcc tgcttgtgga tgatgctgct gatagctcag gctgaggccg 2580
   ccttagagaa cttggtggtc ctcaatgcgg catctgtagc tggagcgcat ggcactctct 2640
   ccttccttgt gttcttctgt gccgcctggt acatcaaggg caagctggtc cctggagcgg 2700
   catatgcttt ctatggcgtg tggccgctgc tcctgctcct gctggcatta ccaccacgag 2760
   catacgccat ggaccgggag atggctgcat catgcggagg cgcggtcttc ataggtctgg 2820
   cactcctgac cttgtcacca cactataaag cgttcctcgc taggctcatt tggtggttgc 2880
   agtatcttat caccagggtc gaggcgctac tgcaagtgtg ggtcccccct cttaacgttc 2940
   gggggggccg cgatgccatc atcctcctca cgtgcgcggt ccatccagag ctaatctttg 3000
   aaatcaccaa aatcttgctc gccataattg gtccgctcat ggtgctccag gctggcttaa 3060
   ccagagtgcc gtacttcgtg cgcgctcagg ggctcatccg tgtgtgcatg ttggtgcgga 3120
   aagtcgctgg gggtcactac gtccagatgg ctctcatgag gctggccgcg ctgacgggca 3180
   cgtacgttta caaccatctt actccgctgc gggactgggc ccacgcgggc ttgcgggacc 3240
   ttgtggtggc agttgagccc gtcgtcttct ctgacatgga gaccaagatc atcacctggg 3300
   gggcagacac cgcggcgtgt ggggacatca tcttgggtct acccgtctcc gcccgaaggg 3360
   ggagggagat acttttggga ccggccgaca gttatgaagg gcagggatgg cggcttcttg 3420
   cgcctatcac ggcctattcc caacagacgc ggggcctact cggttgcatc atcactagtc 3480
   tcacaggccg ggacaggaac caggtcgagg gggaggttca ggtggtttcc accgcaacgc 3540
   agtccttctt ggcgacctgc atcaatggtg tgtgttggac tgtctaccat ggtgccggct 3600
   caaagaccct agccggccca aagggtccga tcacccaaat gtacaccaat gtagaccagg 3660
   acctcgtcgg ctggcgggcg cccctcgggg cgcgttcctt aacaccatgc acctgcggca 3720
   gctcggacct ctacttggtc acgaggcatg ctgatgtcat tccggtgcgc cggcggggcg 3780
   ataacagggg cagcctgctt tcccccagac ctgtctctta cttaaagggc tcctcgggtg 3840
   gtccactgct ctgcccctca gggcacgctg tgggcatctt ccgggccgcc gtgtgcacca 3900
   ggggggttgc gaaggcggtg gactttgtgc ccgttgagtc catggaaact accatgcggt 3960
   ctccggtctt cacggacaat tcatctcccc cggccgtacc gcagacattc caagtggccc 4020
   atctacacgc tcccactggc agcggtaaga gcactaaggt gccggctgct tatgcagccc 4080
   aagggtacaa ggtactcgtc ctgaacccat ccgttgccgc caccttaggt tttggggcgt 4140
   atatgtctaa agcacatggt gttgacccca acatcagaac tggggtaagg accatcacca 4200
   caggcgctcc cattacgtat tccacttatg gcaagttcct tgccgacggt ggttgctctg 4260
   ggggcgccta tgacatcata atatgtgatg agtgccactc aactgactcg acttccatct 4320
   tgggcatcgg cactgtcttg gaccaagcgg agacggctgg agcgcggctc gtcgtgctcg 4380
   ccaccgctac acctccggga tcagtcaccg tgccacatcc caatatcgag gaggtggctt 4440
   tgtccaacac tggagagatc cccttttatg gtaaggcctt tcccatcgag gccatcaagg 4500
   gggggaggca tctcattttc tgccactcca agaagaaatg tgacgagctc gccacaaagc 4560
   tgtcggccct tggaatcaac gctgtagcgt actaccgggg tcttgatgtg tccgtcatac 4620
   cgacaagcgg agatgtcgtt gtcgtggcaa cagacgctct gatgacgggc tacactggcg 4680
   actttgactc agtgatcgac tgtaacacat gtgtcaccca gacagtcgac ttcagcttgg 4740
   accctacctt caccattgag acgacgaccg tgccccaaga cgcggtatcg cgctcgcaac 4800
   ggcgaggcag gactggtagg ggcagggggg gcatatacag gtttgtgact ccaggggaac 4860
   ggccctcggg catgttcgat tcttcggtcc tgtgcgagtg ctatgacgcg ggctgtgctt 4920
   ggtacgagct cacgcccgcc gagacctcgg ttaggttgcg ggcttaccta aatacaccag 4980
   ggttgcccgt ctgccaggac catctggagt tctgggagag cgtcttcaca ggcctcaccc 5040
   acatagatgc ccatttctta tcccagacca agcaggcagg agacaacttc ccctacctgg 5100
   tagcgtacca ggctacggtg tgcgccaggg cccaggcccc accaccatcg tgggaccaaa 5160
   tgtggaagtg tctcatacgg ctaaagccta cactgcacgg gccaacaccc ctgctgtaca 5220
   ggctaggagc cgtccaaaac gaagtcactc tcacacaccc cataaccaaa ttcatcatgg 5280
   catgcatgtc ggctgaccta gaggtcgtca cgagcacctg ggtgctagta ggcggggtcc 5340
   ttgcagctct ggccgcgtac tgcctgacaa cgggcagcgt ggtcattgtg ggtaggatca 5400
   tcttgtctgg gaagccggct gtcattcccg atagggaagt gctctaccag gagttcgatg 5460
   aaatggaaga gtgcgcctcg cacctccctt acattgaaca gggaatgcag ctcgccgagc 5520
   aattcaagca gaaggcgctc gggttgctgc aagcagccac caagcaagcg gaggctgctg 5580
   ctcccgtggt ggagtccaag tggcaagccc ttgaggcctt ttgggcgaag cacatgtgga 5640
   atttcatcag cgggatacag tacttagcag gcttgtccac tctgcctggg aaccccccga 5700
   tagcttcact gatggcattc acagcctcta tcaccagccc gctcaccacc cagcataccc 5760
   tcttgtttaa catcttgggg gggtgggtgg cagcccaact cgcccccccc agcgctgctt 5820
   cagctttcgt gggcgccggg atcgctggcg cggctgttgg cagcataggc cttgggaagg 5880
   tgcttgtgga catcctggcg ggctatgggg cgggggtggc aggcgcgctc gtggccttta 5940
   aggtcatgag cggcgaaatg ccctccaccg aggacctagt caacttgctc cctgccatcc 6000
   tctcccccgg tgccctggtt gtcggggtcg tgtgcgcggc aatactgcgc cggcacgtgg 6060
   gcccggggga gggggctgtg cagtggatga accggctgat agcgttcgct tcgcggggta 6120
   accatgtttc ccccacgcac tatgtgcctg agagcgacgc ggccgcgcgt gtcactcaga 6180
   tcctctccag ccttaccatc acccagctgt tgaagagact ccaccagtgg atcaatgagg 6240
   actgctccac gccatgctcc ggctcgtggc tcagggatgt ttgggactgg atatgcacgg 6300
   tgttgactga cttcaagacc tggctccagt ccaagctcct gccacggttg ccgggggtcc 6360
   ctttcctttc atgccaacgt ggatataagg gagtctggcg aggagatggc gtcatgcaca 6420
   ccacttgccc atgtggcgca caaatcaccg gacatgtcaa aaacggttcc atgaggatcg 6480
   ttgggcctaa aacctgtagc aacacgtggc acggaacatt ccccatcaac gcgtacacca 6540
   cgggttcctg cacaccctcc ccggcgccaa actattccag ggcgttgtgg cgggtagctg 6600
   ctgaggagta cgtggaggtc acgcgggtgg gggatttcca ctacgtgacg ggcatgacca 6660
   ctgacaacat aaaatgccca tgccaggttc cggcccccga atttttcaca gagttggacg 6720
   gggtgcggct gcacaggtac gctccggcgt gcaaacctct cttacgggat gaggtcacat 6780
   tccaggtcgg gctcaaccaa tacgtggttg ggtcacagct tccatgcgag cccgaaccgg 6840
   atgtaacagt gctcacttcc atgctcaccg acccctccca cattacagca gagacggcta 6900
   agcgtaggct ggccaggggg tctccccctt ccttggcccg ctcttcagct agtcagttgt 6960
   ctgcgccttc tctgaaggcg acatgcacca cccatcatga ctccccagac gccgacctca 7020
   tcgaggccaa cctcctgtgg cggcaggaga tgggcgggaa catcacccgc gtggagtcag 7080
   agaacaaagt agtgattctg gactctttcg acccgcttcg agcggaggag gatgagaggg 7140
   aagtatccgt tccggcggag atcctgcgga aaaccaggaa attcccctca gcgttgccca 7200
   tatgggcacg cccggactac aacccaccac tgttagagtc ttggagagac ccggactatg 7260
   tccctccggt ggtacacggg tgcccgttac cgcccaccaa ggcccctcca ataccacctc 7320
   cacggagaaa gagaacagtt atcctgacag aatccactgt gtcttctgcc ttggcggagc 7380
   ttgctacaaa gaccttcggc agctccggat cgtcggccgt cgacagcggc acagcgactg 7440
   cccctcctga tgggccctcc gacgacggtg atgcaggatc ggacgctgag tcatactcct 7500
   ccatgccccc ccttgagggg gagccggggg atcccgatct cagcgacggg tcttggtcca 7560
   ctgtgagcga ggaggccagt gaggacgtcg tctgctgctc aatgtcctac acatggacag 7620
   gtgccttaat cacgccatgc gccgcggagg agagcaagct gcccatcaat gcgttgagca 7680
   actctttgct gcgccaccac aacatggtct atgctacaac atcccgcagc gcaagccagc 7740
   ggcagaaaaa ggtcaccttt gacagactgc aagtcctgga cgaccactac cgggacgtgc 7800
   tcaaggagat gaaggcgaag gcgtccacag ttaaggccaa acttctatcc gtagaagagg 7860
   cctgcaagct gacgccccca cattcagcca gatctaagtt tggctatggg gcaaaggacg 7920
   tccggaacct atccagtaag gccgttaacc acatccactc cgtgtggaag gacttgctgg 7980
   aagacactga gacaccaatt gataccaccg tcatggcaaa aaatgaggtc ttctgtgtcc 8040
   aaccagagaa aggaggccgc aagccagctc gccttatcgt gttcccagac ctgggggtgc 8100
   gtgtgtgcga gaaaatggcc ctttatgatg tggtctccac tcttcctcag gccgtgatgg 8160
   gctcctcata cggattccaa tactctcctg gacagcgggt cgagttcctg gtgaatgcct 8220
   ggaaatcaaa gaagacccct atgggcttcg catatgacac ccgctgtttt gactcaacag 8280
   tcactgagaa tgacatccgt gttgaggagt caatttacca atgttgtgac ttggcccccg 8340
   aggccagaca ggccataagg tcgctcacag agcggcttta tatcgggggc cccctgacta 8400
   attcaaaagg gcagaactgc ggttatcgcc ggtgccgcgc cagcggcgtg ctgacgacta 8460
   gctgcggtaa caccctcaca tgttacttga aggcctctgc agcctgtcga gctgcaaagc 8520
   tccaggactg cacgatgctc gtgtgcggag acgacctcgt cgttatctgt gagagcgcgg 8580
   ggacccagga ggacgcggcg agcctgcgag tcttcacgga ggctatgacc aggtactctg 8640
   ccccccccgg ggacccgccc caaccggaat acgacttgga gctgataaca tcatgctcct 8700
   ccaacgtgtc ggtcgcgcac gatgcatctg gcaagcgggt gtactatctc acccgcgacc 8760
   ccaccacccc tcttgctcgg gctgcgtggg agacagctag acacactcca gtcaactcct 8820
   ggctaggcaa catcatcatg tatgcgccca ccttatgggc aaggatgatt ctgatgaccc 8880
   acttcttctc aatccttcta gcccaggagc aacttgagaa agccctagat tgccagatct 8940
   acggggccac ttactccatt gagccacttg acctacctca gatcattcaa cgactccatg 9000
   gtcttagcgc attttcactc catagttact ctccaggtga gatcaatagg gtggcttcat 9060
   gcctcaggaa acttggggta ccgcccttgc gagtctggag acatcgggcc agaagtgtcc 9120
   gcgctaagct actgtcccag ggggggaggg ccgccacgtg tggcaagtac ctcttcaact 9180
   gggccgtacg gaccaagctc aaactcactc caattccggc tgcgtcccgg ttggacttgt 9240
   ccggctggtt cgtcgccggt tacagcgggg gagacatata tcacagcctg tctcgcgccc 9300
   gaccccgctg gttcatgtgg tgcctactcc tactttccgt aggggtaggc atctacctgc 9360
   tccccaaccg gtgaacgggg agctaaacac tccaggccaa taggccattc cttttttttt 9420
   tttttttttt ttttttcttt ttttttcctt tttttttttt tttttttttt ttccttttct 9480
   ttcttctttg gtggctccat cttagcccta gtcacggcta gctgtgaaag gtccgtgagc 9540
   cgcatgactg cagagagtgc tgatactggc ctctctgcag atcatgt 9587
<210> 10
   <211> 3010
   <212> PRT
   <213> Hepatitis C virus
<400> 10
   Met Ser Thr Asn Pro Lys Pro Gln Arg Lys Thr Lys Arg Asn Thr Asn
    1               5                  10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Gln Pro Glu Gly Arg Ala Trp Ala Gln Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Ile Pro Ala Ser Ala Tyr
               180                 185                 190
   Glu Val Arg Asn Val Ser Gly Val Tyr His Val Thr Asn Asp Cys Ser
           195                 200                 205
   Asn Ser Ser Ile Val Tyr Glu Ala Ala Asp Met Ile Met His Thr Pro
       210                 215                 220
   Gly Cys Val Pro Cys Val Arg Glu Asp Asn Ser Ser Arg Cys Trp Val
   225                 230                 235                 240
   Ala Leu Thr Pro Thr Leu Ala Ala Arg Asn Ser Ser Val Pro Thr Thr
                   245                 250                 255
   Thr Ile Arg Arg His Val Asp Leu Leu Val Gly Ala Ala Ala Phe Cys
               260                 265                 270
   Ser Ala Met Tyr Val Gly Asp Leu Cys Gly Ser Val Phe Leu Ile Ser
           275                 280                 285
   Gln Leu Phe Thr Phe Ser Pro Arg Arg Tyr Glu Thr Val Gln Asp Cys
       290                 295                 300
   Asn Cys Ser Leu Tyr Pro Gly His Val Ser Gly His Arg Met Ala Trp
   305                 310                 315                 320
   Asp Met Met Met Asn Trp Ser Pro Thr Thr Ala Leu Val Val Ser Gln
                   325                 330                 335
   Leu Leu Arg Ile Pro Gln Ala Val Val Asp Met Val Ala Gly Ala His
               340                 345                 350
   Trp Gly Val Leu Ala Gly Leu Ala Tyr Tyr Ser Met Val Gly Asn Trp
           355                 360                 365
   Ala Lys Val Leu Ile Val Met Leu Leu Phe Ala Gly Val Asp Gly Asn
       370                 375                 380
   Thr Arg Val Ser Gly Gly Glu Ala Ala Lys Asn Thr Met Gly Phe Ala
   385                 390                 395                 400
   Ser Leu Phe Val Ser Gly Pro Ser Gln Lys Ile Gln Leu Ile Asn Thr
                   405                 410                 415
   Asn Gly Ser Trp His Ile Asn Arg Thr Ala Leu Asn Cys Asp Asp Ser
               420                 425                 430
   Leu His Thr Gly Phe Leu Ala Ala Leu Phe Tyr Ala His Lys Phe Asn
           435                 440                 445
   Ser Ser Gly Cys Ser Gly Arg Met Ala Ser Cys Arg Pro Ile Asp Glu
       450                 455                 460
   Phe Ala Gln Gly Trp Gly Pro Ile Thr His Gly Val Pro Asp Asn Leu
   465                 470                 475                 480
   Asp Gln Arg Pro Tyr Cys Trp His Tyr Ala Pro Arg Pro Cys Gly Ile
                   485                 490                 495
   Val Pro Ala Ser Gln Val Cys Gly Pro Val Tyr Cys Phe Thr Pro Ser
               500                 505                 510
   Pro Val Val Val Gly Thr Thr Asp Arg Phe Gly Ala Pro Thr Tyr Ser
           515                 520                 525
   Trp Gly Glu Asn Glu Thr Asp Val Leu Leu Leu Asn Asn Thr Arg Pro
       530                 535                 540
   Pro Gln Gly Asn Trp Phe Gly Cys Thr Trp Met Asn Ser Thr Gly Phe
   545                 550                 555                 560
   Thr Lys Thr Cys Gly Gly Pro Pro Cys Asn Ile Gly Gly Val Gly Asn
                   565                 570                 575
   Asn Thr Leu Thr Cys Pro Thr Asp Cys Phe Arg Lys His Pro Glu Ala
   580 585 590
   Thr Tyr Thr Lys Cys Gly Ser Gly Pro Trp Leu Thr Pro Arg Cys Met
   595 600 605
   Val Asp Tyr Pro Tyr Arg Leu Trp His Tyr Pro Cys Thr Val Asn Phe
   610 615 620
   Thr Ile Phe Lys Val Arg Met Tyr Val Gly Gly Val Glu His Arg Leu
   625 630 635 640
   Asp Ala Ala Cys Asn Trp Thr Arg Gly Glu Arg Cys Asn Val Glu Asp
   645 650 655
   Arg Asp Arg Ser Glu Leu Ser Pro Leu Leu Leu Ser Thr Thr Glu Trp
   660 665 670
   Gln Ile Leu Pro Cys Ser Phe Thr Thr Leu Pro Ala Leu Ser Thr Gly
   675 680 685
   Leu Ile His Leu His Gln Asn Ile Val Asp Val Gln Tyr Leu Tyr Gly
   690 695 700
   Val Gly Ser Val Val Val Ser Val Val Ile Arg Trp Glu Tyr Val Val
   705 710 715 720
   Leu Leu Phe Leu Leu Leu Ala Asp Ala Arg Val Cys Ala Cys Leu Trp
   725 730 735
   Met Met Leu Leu Ile Ala Gln Ala Glu Ala Ala Leu Glu Asn Leu Val
   740 745 750
   Val Leu Asn Ala Ala Ser Val Ala Gly Ala His Gly Thr Leu Ser Phe
   755 760 765
   Leu Val Phe Phe Cys Ala Ala Trp Tyr Ile Lys Gly Lys Leu Val Pro
   770 775 780
   Gly Ala Ala Tyr Ala Phe Tyr Gly Val Trp Pro Leu Leu Leu Leu Leu
   785 790 795 800
   Leu Ala Leu Pro Pro Arg Ala Tyr Ala Met Asp Arg Glu Met Ala Ala
   805 810 815
   Ser Cys Gly Gly Ala Val Phe Ile Gly Leu Ala Leu Leu Thr Leu Ser
   820 825 830
   Pro His Tyr Lys Ala Phe Leu Ala Arg Leu Ile Trp Trp Leu Gln Tyr
   835 840 845
   Leu Ile Thr Arg Val Glu Ala Leu Leu Gln Val Trp Val Pro Pro Leu
   850 855 860
   Asn Val Arg Gly Gly Arg Asp Ala Ile Ile Leu Leu Thr Cys Ala Val
   865 870 875 880
   His Pro Glu Leu Ile Phe Glu Ile Thr Lys Ile Leu Leu Ala Ile Ile
   885 890 895
   Gly Pro Leu Met Val Leu Gln Ala Gly Leu Thr Arg Val Pro Tyr Phe
   900 905 910
   Val Arg Ala Gln Gly Leu Ile Arg Val Cys Met Leu Val Arg Lys Val
   915 920 925
   Ala Gly Gly His Tyr Val Gln Met Ala Leu Met Arg Leu Ala Ala Leu
   930 935 940
   Thr Gly Thr Tyr Val Tyr Asn His Leu Thr Pro Leu Arg Asp Trp Ala
   945 950 955 960
   His Ala Gly Leu Arg Asp Leu Val Val Ala Val Glu Pro Val Val Phe
   965 970 975
   Ser Asp Met Glu Thr Lys Ile Ile Thr Trp Gly Ala Asp Thr Ala Ala
   980 985 990
   Cys Gly Asp Ile Ile Leu Gly Leu Pro Val Ser Ala Arg Arg Gly Arg
   995 1000 1005
   Glu Ile Leu Leu Gly Pro Ala Asp Ser Tyr Glu Gly Gln Gly Trp Arg
   1010 1015 1020
   Leu Leu Ala Pro Ile Thr Ala Tyr Ser Gln Gln Thr Arg Gly Leu Leu
   1025 1030 1035 1040
   Gly Cys Ile Ile Thr Ser Leu Thr Gly Arg Asp Arg Asn Gln Val Glu
   1045 1050 1055
   Gly Glu Val Gln Val Val Ser Thr Ala Thr Gln Ser Phe Leu Ala Thr
   1060 1065 1070
   Cys Ile Asn Gly Val Cys Trp Thr Val Tyr His Gly Ala Gly Ser Lys
   1075 1080 1085
   Thr Leu Ala Gly Pro Lys Gly Pro Ile Thr Gln Met Tyr Thr Asn Val
   1090 1095 1100
   Asp Gln Asp Leu Val Gly Trp Arg Ala Pro Leu Gly Ala Arg Ser Leu
   1105 1110 1115 1120
   Thr Pro Cys Thr Cys Gly Ser Ser Asp Leu Tyr Leu Val Thr Arg His
   1125 1130 1135
   Ala Asp Val Ile Pro Val Arg Arg Arg Gly Asp Asn Arg Gly Ser Leu
   1140 1145 1150
   Leu Ser Pro Arg Pro Val Ser Tyr Leu Lys Gly Ser Ser Gly Gly Pro
   1155 1160 1165
   Leu Leu Cys Pro Ser Gly His Ala Val Gly Ile Phe Arg Ala Ala Val
   1170 1175 1180
   Cys Thr Arg Gly Val Ala Lys Ala Val Asp Phe Val Pro Val Glu Ser
   1185 1190 1195 1200
   Met Glu Thr Thr Met Arg Ser Pro Val Phe Thr Asp Asn Ser Ser Pro
   1205 1210 1215
   Pro Ala Val Pro Gln Thr Phe Gln Val Ala His Leu His Ala Pro Thr
   1220 1225 1230
   Gly Ser Gly Lys Ser Thr Lys Val Pro Ala Ala Tyr Ala Ala Gln Gly
   1235 1240 1245
   Tyr Lys Val Leu Val Leu Asn Pro Ser Val Ala Ala Thr Leu Gly Phe
   1250 1255 1260
   Gly Ala Tyr Met Ser Lys Ala His Gly Val Asp Pro Asn Ile Arg Thr
   1265 1270 1275 1280
   Gly Val Arg Thr Ile Thr Thr Gly Ala Pro Ile Thr Tyr Ser Thr Tyr
   1285 1290 1295
   Gly Lys Phe Leu Ala Asp Gly Gly Cys Ser Gly Gly Ala Tyr Asp Ile
   1300 1305 1310
   Ile Ile Cys Asp Glu Cys His Ser Thr Asp Ser Thr Ser Ile Leu Gly
   1315 1320 1325
   Ile Gly Thr Val Leu Asp Gln Ala Glu Thr Ala Gly Ala Arg Leu Val
   1330 1335 1340
   Val Leu Ala Thr Ala Thr Pro Pro Gly Ser Val Thr Val Pro His Pro
   1345 1350 1355 1360
   Asn Ile Glu Glu Val Ala Leu Ser Asn Thr Gly Glu Ile Pro Phe Tyr
   1365 1370 1375
   Gly Lys Ala Phe Pro Ile Glu Ala Ile Lys Gly Gly Arg His Leu Ile
   1380 1385 1390
   Phe Cys His Ser Lys Lys Lys Cys Asp Glu Leu Ala Thr Lys Leu Ser
   1395 1400 1405
   Ala Leu Gly Ile Asn Ala Val Ala Tyr Tyr Arg Gly Leu Asp Val Ser
   1410 1415 1420
   Val Ile Pro Thr Ser Gly Asp Val Val Val Val Ala Thr Asp Ala Leu
   1425 1430 1435 1440
   Met Thr Gly Tyr Thr Gly Asp Phe Asp Ser Val Ile Asp Cys Asn Thr
   1445 1450 1455
   Cys Val Thr Gln Thr Val Asp Phe Ser Leu Asp Pro Thr Phe Thr Ile
   1460 1465 1470
   Glu Thr Thr Thr Val Pro Gln Asp Ala Val Ser Arg Ser Gln Arg Arg
   1475 1480 1485
   Gly Arg Thr Gly Arg Gly Arg Gly Gly Ile Tyr Arg Phe Val Thr Pro
   1490 1495 1500
   Gly Glu Arg Pro Ser Gly Met Phe Asp Ser Ser Val Leu Cys Glu Cys
   1505 1510 1515 1520
   Tyr Asp Ala Gly Cys Ala Trp Tyr Glu Leu Thr Pro Ala Glu Thr Ser
   1525 1530 1535
   Val Arg Leu Arg Ala Tyr Leu Asn Thr Pro Gly Leu Pro Val Cys Gln
   1540 1545 1550
   Asp His Leu Glu Phe Trp Glu Ser Val Phe Thr Gly Leu Thr His Ile
   1555 1560 1565
   Asp Ala His Phe Leu Ser Gln Thr Lys Gln Ala Gly Asp Asn Phe Pro
   1570 1575 1580
   Tyr Leu Val Ala Tyr Gln Ala Thr Val Cys Ala Arg Ala Gln Ala Pro
   1585 1590 1595 1600
   Pro Pro Ser Trp Asp Gln Met Trp Lys Cys Leu Ile Arg Leu Lys Pro
   1605 1610 1615
   Thr Leu His Gly Pro Thr Pro Leu Leu Tyr Arg Leu Gly Ala Val Gln
   1620 1625 1630
   Asn Glu Val Thr Leu Thr His Pro Ile Thr Lys Phe Ile Met Ala Cys
   1635 1640 1645
   Met Ser Ala Asp Leu Glu Val Val Thr Ser Thr Trp Val Leu Val Gly
   1650 1655 1660
   Gly Val Leu Ala Ala Leu Ala Ala Tyr Cys Leu Thr Thr Gly Ser Val
   1665 1670 1675 1680
   Val Ile Val Gly Arg Ile Ile Leu Ser Gly Lys Pro Ala Val Ile Pro
   1685 1690 1695
   Asp Arg Glu Val Leu Tyr Gln Glu Phe Asp Glu Met Glu Glu Cys Ala
   1700 1705 1710
   Ser His Leu Pro Tyr Ile Glu Gln Gly Met Gln Leu Ala Glu Gln Phe
   1715 1720 1725
   Lys Gln Lys Ala Leu Gly Leu Leu Gln Ala Ala Thr Lys Gln Ala Glu
   1730 1735 1740
   Ala Ala Ala Pro Val Val Glu Ser Lys Trp Gln Ala Leu Glu Ala Phe
   1745 1750 1755 1760
   Trp Ala Lys His Met Trp Asn Phe Ile Ser Gly Ile Gln Tyr Leu Ala
   1765 1770 1775
   Gly Leu Ser Thr Leu Pro Gly Asn Pro Pro Ile Ala Ser Leu Met Ala
   1780 1785 1790
   Phe Thr Ala Ser Ile Thr Ser Pro Leu Thr Thr Gln His Thr Leu Leu
   1795 1800 1805
   Phe Asn Ile Leu Gly Gly Trp Val Ala Ala Gln Leu Ala Pro Pro Ser
   1810 1815 1820
   Ala Ala Ser Ala Phe Val Gly Ala Gly Ile Ala Gly Ala Ala Val Gly
   1825 1830 1835 1840
   Ser Ile Gly Leu Gly Lys Val Leu Val Asp Ile Leu Ala Gly Tyr Gly
   1845 1850 1855
   Ala Gly Val Ala Gly Ala Leu Val Ala Phe Lys Val Met Ser Gly Glu
   1860 1865 1870
   Met Pro Ser Thr Glu Asp Leu Val Asn Leu Leu Pro Ala Ile Leu Ser
   1875 1880 1885
   Pro Gly Ala Leu Val Val Gly Val Val Cys Ala Ala Ile Leu Arg Arg
   1890 1895 1900
   His Val Gly Pro Gly Glu Gly Ala Val Gln Trp Met Asn Arg Leu Ile
   1905 1910 1915 1920
   Ala Phe Ala Ser Arg Gly Asn His Val Ser Pro Thr His Tyr Val Pro
   1925 1930 1935
   Glu Ser Asp Ala Ala Ala Arg Val Thr Gln Ile Leu Ser Ser Leu Thr
   1940 1945 1950
   Ile Thr Gln Leu Leu Lys Arg Leu His Gln Trp Ile Asn Glu Asp Cys
   1955 1960 1965
   Ser Thr Pro Cys Ser Gly Ser Trp Leu Arg Asp Val Trp Asp Trp Ile
   1970 1975 1980
   Cys Thr Val Leu Thr Asp Phe Lys Thr Trp Leu Gln Ser Lys Leu Leu
   1985 1990 1995 2000
   Pro Arg Leu Pro Gly Val Pro Phe Leu Ser Cys Gln Arg Gly Tyr Lys
   2005 2010 2015
   Gly Val Trp Arg Gly Asp Gly Val Met His Thr Thr Cys Pro Cys Gly
   2020 2025 2030
   Ala Gln Ile Thr Gly His Val Lys Asn Gly Ser Met Arg Ile Val Gly
   2035 2040 2045
   Pro Lys Thr Cys Ser Asn Thr Trp His Gly Thr Phe Pro Ile Asn Ala
   2050 2055 2060
   Tyr Thr Thr Gly Ser Cys Thr Pro Ser Pro Ala Pro Asn Tyr Ser Arg
   2065 2070 2075 2080
   Ala Leu Trp Arg Val Ala Ala Glu Glu Tyr Val Glu Val Thr Arg Val
   2085 2090 2095
   Gly Asp Phe His Tyr Val Thr Gly Met Thr Thr Asp Asn Ile Lys Cys
   2100 2105 2110
   Pro Cys Gln Val Pro Ala Pro Glu Phe Phe Thr Glu Leu Asp Gly Val
   2115 2120 2125
   Arg Leu His Arg Tyr Ala Pro Ala Cys Lys Pro Leu Leu Arg Asp Glu
   2130 2135 2140
   Val Thr Phe Gln Val Gly Leu Asn Gln Tyr Val Val Gly Ser Gln Leu
   2145 2150 2155 2160
   Pro Cys Glu Pro Glu Pro Asp Val Thr Val Leu Thr Ser Met Leu Thr
   2165 2170 2175
   Asp Pro Ser His Ile Thr Ala Glu Thr Ala Lys Arg Arg Leu Ala Arg
   2180 2185 2190
   Gly Ser Pro Pro Ser Leu Ala Arg Ser Ser Ala Ser Gln Leu Ser Ala
   2195 2200 2205
   Pro Ser Leu Lys Ala Thr Cys Thr Thr His His Asp Ser Pro Asp Ala
   2210 2215 2220
   Asp Leu Ile Glu Ala Asn Leu Leu Trp Arg Gln Glu Met Gly Gly Asn
   2225 2230 2235 2240
   Ile Thr Arg Val Glu Ser Glu Asn Lys Val Val Ile Leu Asp Ser Phe
   2245 2250 2255
   Asp Pro Leu Arg Ala Glu Glu Asp Glu Arg Glu Val Ser Val Pro Ala
   2260 2265 2270
   Glu Ile Leu Arg Lys Thr Arg Lys Phe Pro Ser Ala Leu Pro Ile Trp
   2275 2280 2285
   Ala Arg Pro Asp Tyr Asn Pro Pro Leu Leu Glu Ser Trp Arg Asp Pro
   2290 2295 2300
   Asp Tyr Val Pro Pro Val Val His Gly Cys Pro Leu Pro Pro Thr Lys
   2305 2310 2315 2320
   Ala Pro Pro Ile Pro Pro Pro Arg Arg Lys Arg Thr Val Ile Leu Thr
   2325 2330 2335
   Glu Ser Thr Val Ser Ser Ala Leu Ala Glu Leu Ala Thr Lys Thr Phe
   2340 2345 2350
   Gly Ser Ser Gly Ser Ser Ala Val Asp Ser Gly Thr Ala Thr Ala Pro
   2355 2360 2365
   Pro Asp Gly Pro Ser Asp Asp Gly Asp Ala Gly Ser Asp Ala Glu Ser
   2370 2375 2380
   Tyr Ser Ser Met Pro Pro Leu Glu Gly Glu Pro Gly Asp Pro Asp Leu
   2385 2390 2395 2400
   Ser Asp Gly Ser Trp Ser Thr Val Ser Glu Glu Ala Ser Glu Asp Val
   2405 2410 2415
   Val Cys Cys Ser Met Ser Tyr Thr Trp Thr Gly Ala Leu Ile Thr Pro
   2420 2425 2430
   Cys Ala Ala Glu Glu Ser Lys Leu Pro Ile Asn Ala Leu Ser Asn Ser
   2435 2440 2445
   Leu Leu Arg His His Asn Met Val Tyr Ala Thr Thr Ser Arg Ser Ala
   2450 2455 2460
   Ser Gln Arg Gln Lys Lys Val Thr Phe Asp Arg Leu Gln Val Leu Asp
   2465 2470 2475 2480
   Asp His Tyr Arg Asp Val Leu Lys Glu Met Lys Ala Lys Ala Ser Thr
   2485 2490 2495
   Val Lys Ala Lys Leu Leu Ser Val Glu Glu Ala Cys Lys Leu Thr Pro
   2500 2505 2510
   Pro His Ser Ala Arg Ser Lys Phe Gly Tyr Gly Ala Lys Asp Val Arg
   2515 2520 2525
   Asn Leu Ser Ser Lys Ala Val Asn His Ile His Ser Val Trp Lys Asp
   2530 2535 2540
   Leu Leu Glu Asp Thr Glu Thr Pro Ile Asp Thr Thr Val Met Ala Lys
   2545 2550 2555 2560
   Asn Glu Val Phe Cys Val Gln Pro Glu Lys Gly Gly Arg Lys Pro Ala
   2565 2570 2575
   Arg Leu Ile Val Phe Pro Asp Leu Gly Val Arg Val Cys Glu Lys Met
   2580 2585 2590
   Ala Leu Tyr Asp Val Val Ser Thr Leu Pro Gln Ala Val Met Gly Ser
   2595 2600 2605
   Ser Tyr Gly Phe Gln Tyr Ser Pro Gly Gln Arg Val Glu Phe Leu Val
   2610 2615 2620
   Asn Ala Trp Lys Ser Lys Lys Thr Pro Met Gly Phe Ala Tyr Asp Thr
   2625 2630 2635 2640
   Arg Cys Phe Asp Ser Thr Val Thr Glu Asn Asp Ile Arg Val Glu Glu
   2645 2650 2655
   Ser Ile Tyr Gln Cys Cys Asp Leu Ala Pro Glu Ala Arg Gln Ala Ile
   2660 2665 2670
   Arg Ser Leu Thr Glu Arg Leu Tyr Ile Gly Gly Pro Leu Thr Asn Ser
   2675 2680 2685
   Lys Gly Gln Asn Cys Gly Tyr Arg Arg Cys Arg Ala Ser Gly Val Leu
   2690 2695 2700
   Thr Thr Ser Cys Gly Asn Thr Leu Thr Cys Tyr Leu Lys Ala Ser Ala
   2705 2710 2715 2720
   Ala Cys Arg Ala Ala Lys Leu Gln Asp Cys Thr Met Leu Val Cys Gly
   2725 2730 2735
   Asp Asp Leu Val Val Ile Cys Glu Ser Ala Gly Thr Gln Glu Asp Ala
   2740 2745 2750
   Ala Ser Leu Arg Val Phe Thr Glu Ala Met Thr Arg Tyr Ser Ala Pro
   2755 2760 2765
   Pro Gly Asp Pro Pro Gln Pro Glu Tyr Asp Leu Glu Leu Ile Thr Ser
   2770 2775 2780
   Cys Ser Ser Asn Val Ser Val Ala His Asp Ala Ser Gly Lys Arg Val
   2785 2790 2795 2800
   Tyr Tyr Leu Thr Arg Asp Pro Thr Thr Pro Leu Ala Arg Ala Ala Trp
   2805 2810 2815
   Glu Thr Ala Arg His Thr Pro Val Asn Ser Trp Leu Gly Asn Ile Ile
   2820 2825 2830
   Met Tyr Ala Pro Thr Leu Trp Ala Arg Met Ile Leu Met Thr His Phe
   2835 2840 2845
   Phe Ser Ile Leu Leu Ala Gln Glu Gln Leu Glu Lys Ala Leu Asp Cys
   2850 2855 2860
   Gln Ile Tyr Gly Ala Thr Tyr Ser Ile Glu Pro Leu Asp Leu Pro Gln
   2865 2870 2875 2880
   Ile Ile Gln Arg Leu His Gly Leu Ser Ala Phe Ser Leu His Ser Tyr
   2885 2890 2895
   Ser Pro Gly Glu Ile Asn Arg Val Ala Ser Cys Leu Arg Lys Leu Gly
   2900 2905 2910
   Val Pro Pro Leu Arg Val Trp Arg His Arg Ala Arg Ser Val Arg Ala
   2915 2920 2925
   Lys Leu Leu Ser Gln Gly Gly Arg Ala Ala Thr Cys Gly Lys Tyr Leu
   2930 2935 2940
   Phe Asn Trp Ala Val Arg Thr Lys Leu Lys Leu Thr Pro Ile Pro Ala
   2945 2950 2955 2960
   Ala Ser Arg Leu Asp Leu Ser Gly Trp Phe Val Ala Gly Tyr Ser Gly
   2965 2970 2975
   Gly Asp Ile Tyr His Ser Leu Ser Arg Ala Arg Pro Arg Trp Phe Met
   2980 2985 2990
   Trp Cys Leu Leu Leu Leu Ser Val Gly Val Gly Ile Tyr Leu Leu Pro
   2995 3000 3005
   Asn Arg
   3010
<210> 11
   <211> 9587
   <212> DNA
   <213> Hepatitis C virus
<400> 11
   gccagccccc gattgggggc gacactccac catagatcac tcccctgtga ggaactactg 60
   tcttcacgca gaaagcgtct agccatggcg ttagtatgag tgtcgtgcag cctccaggtc 120
   cccccctccc gggagagcca tagtggtctg cggaaccggt gagtacaccg gaattgccag 180
   gacgaccggg tcctttcttg gatcaacccg ctcaatgcct ggagatttgg gcgtgccccc 240
   gcgagactgc tagccgagta gtgttgggtc gcgaaaggcc ttgtggtact gcctgatagg 300
   gtgcttgcga gtgccccggg aggtctcgta gaccgtgcat catgagcaca aatcctaaac 360
   ctcaaagaaa aaccaaacgt aacaccaacc gccgcccaca ggacgtcaag ttcccgggcg 420
   gtggtcagat cgttggtgga gtttacctgt tgccgcgcag gggccccagg ttgggtgtgc 480
   gcgcgactag gaagacttcc gagcggtcgc aacctcgtgg aaggcgacaa cctatcccca 540
   aggctcgccg gcccgagggc agggcctggg ctcagcccgg gtacccctgg cccctctatg 600
   gcaatgaggg tctggggtgg gcaggatggc tcctgtctcc ccgtggctcc cggcctagtt 660
   ggggccccac ggacccccgg cgtaggtcgc gtaatttggg taaagtcatc gataccctca 720
   catgcggctt cgccgacctc atggggtaca ttccgctcgt cggcgctcct ctaggaggcg 780
   ctgccagggc cctggcgcat ggcgtccggg ttctggagga cggcgtgaac tacgcaacag 840
   ggaatctgcc cggttgctct ttctctatct tcctcttggc tttgctgtcc tgtttgacca 900
   ccccagcttc cgcttatgaa gtgcgcaacg tgtccggagt gtaccatgtc acgaacgact 960
   gctccaactc aagtattgtg tatgaggcag cggacatgat catgcacacc cctgggtgcg 1020
   taccctgcgt ccgggagaac aactcctccc gctgctgggt agcgctcact cccacgctcg 1080
   cggccaggaa cagcagcatc cccactacga caatacggcg ccatgtcgat ttgctcgtcg 1140
   gggcggctgc tctctgctcc gctatgtatg tgggggatct ctgcggatct gttttcctcg 1200
   tctcccagct gttcaccttt tcacctcgcc ggtacgagac ggtacaggac tgcaattgct 1260
   cactctatcc cggccacgta tcaggtcacc gcatggcctg ggatatgatg atgaactggt 1320
   cgcccacgac agccctagtg gtatcgcagt tactccggat tccacaatcc gtcgtggaca 1380
   tggtggcggg ggcccactgg ggagtcctgg cgggccttgc ctactattcc atggtgggga 1440
   actgggctaa ggtcttgatt gtgatgctac tctttgctgg cgttgacggg agcacccgcg 1500
   taacaggggg aacgacagcc tacaacgtca ggggccttac gtccctcttt acaccagggc 1560
   cgtctcagaa aatccagctc ataaattcta acggcagctg gcacatcaac agaactgcct 1620
   tgaattgcaa tgactccctt caaactgggt tccttgctgc gttgttctac acacatagtt 1680
   tcaacgcgtc cggatgccca gagcgcatgg ccagctgccg cccaattgac aagtttgctc 1740
   aggcgtgggg tcccatcacc catgttgtgc ctaacatctc agaccagagg ccctattgct 1800
   ggcactacgc acctcgaccg tgtggtatcg tacctgcgtc gcaggtgtgt ggtccggtgt 1860
   attgcttcac cccgagccct gttgtggtgg gaacgaccga tcgtttcggc gtccccacat 1920
   atacctgggg ggagaatgag acggacgtgc tgctcctcaa caacacgcgg ccgccgcaag 1980
   gcaactggtt cggctgtaca tggatgaata gcaccgggtt caccaagaca tgcgggggcc 2040
   ccccgtgtga catcgggggg gccggcaata gcaccttgac ctgccctacg gattgcttcc 2100
   ggaagcaccc cgaggctacc tacaccaagt gcggctcggg gccttggttg acacccaggt 2160
   gtatagttga ctacccatac aggctttggc actacccctg cactgtcaac tttaccatct 2220
   tcaaggtcag gatgtatgtg gggggcgtgg agcataggct caacgccgcg tgcaattgga 2280
   ctcgaggaga gcgttgcaac ctggaggaca gggacaggtc ggagctcagc ccgctgctgc 2340
   tgtctacaac agagtggcag atactgccct gttccttcac caccctaccg gctctgtcca 2400
   ctggtttgat tcacctccat cagaacatcg tggacgtgca atacctgtat ggtgtagggt 2460
   cagcagttgt ctccattgtg atcaaatggg agtatgtcct gctgctcttc cttctcctgg 2520
   cagatgcgcg cgtctgcgcc tgcttatgga tgatgctgct gatagcccag gccgaggctg 2580
   cattggagaa cctggtgatc ctcaatgcgg cgtccgtggc cggagcacac ggtatcctct 2640
   ccttccttgt gttcttctgt gctgcctggt acatcaaggg caagctggtc cctggggcgg 2700
   catatgccct ctatggcgtg tggccgctgc tcctgctcct gctggcgttg ccaccacggg 2760
   cgtacgccat ggaccgggag atggctgcat cgtgcggagg cgcggttttc ataggtctgg 2820
   tactcttgac cctgtcacca cactataaag cattcctcgc caggctcata tggtggttgc 2880
   aatattttac caccagggcc gaggcgcact tgcaagtgtg ggtttccccc ctcaacgttc 2940
   gggggggccg cgatgccatc atcctcctca cgtgcgtggt ccacccagag ctgatttttg 3000
   acatcaccaa aatcttgctc gccatatttg gtccgctcat ggtgctccag gctggcctaa 3060
   ctagagtgcc gtacttcgtg cgcgctcaag ggctcatccg tgcgtgcgtg ttggtgcgga 3120
   aggtcgctgg gggtcattat gtccaaatgg ctctcgtgaa gctggccgct ctgacgggca 3180
   cgtacgttta tgaccatctt actccgctgc gggactgggc ccacgcgagc ctgcaagacc 3240
   ttgtggtagc ggtcgagccc gtcgtcttct ctgacatgga gaccaagatc atcacctggg 3300
   gggcagacac cgcagcgtgt ggagatatca tttcgggcct acccgtctcc gctcgaaggg 3360
   ggagggagat acttctgggg ccggccgata gttttgaaga gcaagggtgg cggctcctcg 3420
   cgcccatcac ggcctactcc caacagacgc ggggcttgct tggctgcatc atcactagtc 3480
   tcacaggtcg ggacaagaac caggtcgagg gggaggtgca ggtggtttcc accgcgacgc 3540
   aatctttcct ggcgacctgt gtcaatggtg tgtgctggtc tgtttaccac ggcgccggct 3600
   caaagacctt agccggccca aaggggccag tcacccaaat gtacaccaat gtagatctgg 3660
   acctcgtcgg ctggcaggcg ccccccggga cgcgctccct gacaccgtgc acctgcggca 3720
   gctcggacct ttacttggtc acgagacatg ctgacgtcat tccggtgcgc cggcggggcg 3780
   acagcagggg gagcctactt tcccccaggc ccgtctccta cttgaagggc tcctcgggcg 3840
   gtccactgct ctgcccctcg ggcacgctgg tgggcatctt ccgggctgct gtgtgcaccc 3900
   ggggggttgc caaggcggtg gactttatac ccgttgagtc tatggaaacc accatgcggt 3960
   ctccggtcta cacggacaac tcatctcccc cggctgtacc gcagactttc caagtggccc 4020
   acctgcacgc tcccactggc agcggtaaga gcactaaggt gccggctgca tatgcagccc 4080
   aagggtacaa ggtgctcgtc ctgaacccgt ccgttgccgc taccctaggt tttggggcgt 4140
   acatgtccaa agcacatggt gttgacccta acatcaggac tggggtaagg accatcacca 4200
   cgggcgctcc catcacgtac tccacctacg gcaagttcct tgccgacggt ggttgctctg 4260
   ggggcgccta tgacatcata atatgtgatg agtgtcactc aactgactcg acttctatct 4320
   tgggcattgg tacggtcttg gaccaagcgg agacggctgg agcgcggctc gtcgtgctcg 4380
   ccaccgctac acctccggga tcggttactg tgccacatcc taacatcgag gaggtggcct 4440
   tgtccaacac tggagagatt cccttctatg gcaaagccat ccccatcgag accatcaagg 4500
   ggggaaggca tctcatcttc tgccattcta agaagaagtg tgatgaactc gctgcaaagc 4560
   tgtcggccct tggggtcaac gctgtagcgt actaccgggg tcttgatgtg tccgtcatac 4620
   cgacgagcgg agacgtcgtt gtcgtggcaa cagacgctct aatgacgggc tacaccggtg 4680
   actttgactc agtgatcgac tgtaacacat gtgtcaccca gacagtcgat ttcagcttgg 4740
   accccacctt caccattgac accacgactg tgccccagga cgccgtgtcg cgctcacagc 4800
   ggcgaggtag gactggtagg ggcagagggg gcatatatag gtttgtgact ccaggggaac 4860
   ggccctcggg catgttcgat tcctcggtcc tgtgtgaatg ctatgacgcg ggctgtgctt 4920
   ggtacgagct cacgcccgcc gagacctcag ttaggctgcg ggcttaccta aatacaccag 4980
   ggttgcccgt ttgccaggac catttggagt tctgggagag cgtcttcaca ggcctcaccc 5040
   acatagatgc tcacttcttg tcccagacta agcaggcagg agacaacttc ccctacctgg 5100
   tggcatacca ggctacagtg tgcgccaggg cccaggctcc acctccgtcg tgggatcaga 5160
   tgtggaagtg tctcatacgg ctaaagccta cgctgcacgg gccaacgccc ctgttgtata 5220
   ggctaggagc cgtccaaaat gaggtcaccc tcacacaccc cataaccaaa ttcatcatgg 5280
   catgcatgtc ggctgacctg gaggtcgtca cgagcacctg ggtgctagtg ggcggcgtcc 5340
   tcgcagcgct ggccgcgtac tgcctgacaa cgggcagcgt ggttatcgtg ggcaggatca 5400
   tcttgtccgg gaggccggct atcattcccg acagggaagt tctctaccag gagttcgatg 5460
   aaatggaaga gtgcgcctca caccttccct acatcgaaca aggaatgcag ctcgccgagc 5520
   aattcaaaca gaaggcgctc gggctgctgc agacagccac caagcaagca gaggctgctg 5580
   ctcccgtggt gcagtccaag tggcaagccc ttgaggcctt ctgggcgaag cacatgtgga 5640
   acttcatcag cgggatacag tatttggcag gcttatccac tctgcctggg aaccccgcga 5700
   tagcatcact gatggcattc acagcctcca tcaccagccc gctcaccacc caatataccc 5760
   tcctgtttaa catcttgggg ggatgggtgg ccgcccaact cgccccccct agcgccgctt 5820
   cagcttttgt gggcgccggt atcgctggcg cggctgttgg cagcataggc cttgggaagg 5880
   tgcttgtgga catcttggcg ggttatggag caggggtggc aggcgcactt gtggccttta 5940
   aggtcatgag cggcgaagtg ccttccactg aggacctggt caacttactc cctgccatcc 6000
   tctctcctgg tgccctggtc gtcggggtcg tgtgcgcagc aatactgcgt cggcatgtgg 6060
   gcccagggga gggggctgtg cagtggatga accggctgat agcgttcgct tcgcggggta 6120
   accacgtctc ccccacgcac tatgtgcctg agagcgacgc tgcggcgcgt gtcacccaga 6180
   tcctctccag ccttaccatc actcagctgt taaagaggct ccaccagtgg atcaatgagg 6240
   actgctccac gccatgctcc ggctcgtggc tcagggatgt ttgggactgg atatgcacgg 6300
   tgttgagtga cttcaagacc tggctccagt ccaagctcct gccgcggctg ccgggagtcc 6360
   cttttttctc atgtcaacgt ggatacaggg gagtctggcg aggagatggc gtcatgcaaa 6420
   ccatctgccc atgtggagca caaatcagcg gacatgtcaa gaacggttcc atgaggatcg 6480
   ttgggcctaa aacctgtagc aacacgtggc atggaacatt ccccatcaac gcgtacacca 6540
   cgggcccctg cacaccctcc ccagcgccca actattccag ggcgctgtgg cgggtgactg 6600
   ctgaggagta cgtggaggtc acgcgggtgg gggatttcca ctacgtgacg ggcatgacca 6660
   ctgacaacgt aaaatgccca tgccaggttc cggcccccga attcttcacg gaattggacg 6720
   gggtgcggct gcacaggtac gctccggcgt gcaaacctct cctacgggat gaggtcacat 6780
   tccaggtcgg gctcaaccac ttcccggttg ggtctcaact cccatgtgag cccgaaccgg 6840
   atgtaacagt ggtcacttcc atgctgaccg acccctccca cattacggca gaaacggcta 6900
   ggcgtaggct ggccagaggg tctccccctt ccttggccag ctcctcagct agtcagttgt 6960
   ctgcgccctc cttgaaggcg acatgcacta cccatcatga ctccccggac gccgacctca 7020
   tcgaggccaa cctcctgtgg cggcaggaga tgggcggaaa catcacccgt gtggagtcgg 7080
   agaataaggt agtaatccta gactcttttg atccgcttcg agcggaggag gatgagaggg 7140
   aagtatccgt agcggcggag atcctgcgga aaaccaggaa gttcccccca gcgatgccta 7200
   tatgggcacg cccggattac aacccaccac tcctagagtc ttggaagaac ccagattacg 7260
   tccctccggt ggtgcacggg tgtccattgc cacctaccaa ggcccctcca ataccacctc 7320
   cacggagaaa gaggacggtt gtcctgacag aatccaccgt gtcttctgcc ttggcggagc 7380
   ttgctaccaa gaccttcggc agctccggat catcagccgt cgacagcggc acagcgagcg 7440
   ccccccctga tcagccttcc gatgacggag atgcaggatc cgacgttgaa tcgtactcct 7500
   ccatgccccc ccttgaggga gagccggggg accccgatct cagcgacggg tcttggtcta 7560
   ctgtgagcga ggaggctagt gaggacgtcg tctgttgttc gatgtcctac acatggacag 7620
   gcgctttaat cacaccatgc gccgcggagg agagcaagct gcccatcaat gcgttgagca 7680
   actctttgct gcgtcaccat aacatggtct atgccacaac atcccgcagc gcaggccaac 7740
   ggcagaaaaa ggtcaccttt gacagactgc aggtcctgga cgaccactac cgggacgtgc 7800
   tcaaggagat gaaggcgaag gcgtccacag ttaaggctaa acttctatca gtagaagaag 7860
   cctgcaagct gacgccccca cattcggcca gatctaaatt tggctatggg gcaaaggacg 7920
   tccggaacct atccagcaag gccgttaacc acatccactc cgtgtggaag gacttgctgg 7980
   aagacactga gacaccaatt gacactacca tcatggcaaa aaatgaggtc ttctgtgttc 8040
   aaccagagaa aggaggccgc aagccagctc gcctcatcgt attcccagac ttgggggttc 8100
   gtgtatgcga gaaaatggcc ctttatgacg tggtctccac tcttcctcag gccgtgatgg 8160
   gctcctcata cggattccag tactctcctg ggcagcgggt cgagttcctg gtgaacgcct 8220
   ggaaatcaaa gaaaaaccct atgggctttg catatgacac ccgctgtttt gactcaacgg 8280
   tcactgagaa tgacatccgt gttgaggagt caatttacca atgttgtgac ttggcccccg 8340
   aggccaggca ggccataagg tcgctcacag agcggcttta tatcgggggc cccctgacta 8400
   attcaaaagg gcagaactgc ggttatcgcc ggtgccgcgc cagcggcgtg ctgacgacca 8460
   gctgcggtaa caccctcaca tgttacttga aggccaccgc agcctgtcga gctgcaaagc 8520
   tccaggactg cacgatgctc gtgtgcggag acgaccttgt cgttatctgt gaaagcgcgg 8580
   gaacccagga ggatgcggcg agcctacgag tcttcacgga ggctatgact aggtactctg 8640
   ccccccccgg ggacccgccc caaccggaat acgacttgga gttaataaca tcatgctcct 8700
   ccaacgtgtc ggtcgcgcac gatgcatctg gcaagcgggt gtactacctc acccgggacc 8760
   ccaccacccc ccttgcgcgg gctgcgtggg agacagcaag acacactcca gttaattcct 8820
   ggctaggcaa catcatcatg tatgcgccca cattgtgggc aaggatgatt ctgatgaccc 8880
   actttttctc cgtccttcta gctcaggaac aacttgaaaa agccctagat tgtcaaatct 8940
   acggggccac ttactccatt gagccacttg acctacctca gatcattcaa cgactccacg 9000
   gtcttagcgc attttcactc catagttact ctccaggtga gatcaatagg gtggcttcat 9060
   gcctcaggaa acttggggta ccacccttgc gagtctggag acatcgggcc agaagtgtcc 9120
   gcgctaagct actgtcccag aaggggaggg ctgccacttg tggcaagtac ctcttcaact 9180
   gggccgtacg gaccaagctc aaactcactc caattccggc tgcgtcccgg ttggacttgt 9240
   ccggctggtt cgtcgccggt tacagcgggg gagacatata tcacagcctg tctcgcgccc 9300
   gaccccgctg gttcatgtgg tgcctactcc tactttccgt aggggtaggc atctacctgc 9360
   tccccaaccg gtgaacgggg agctaaacac tccaggccaa taggccattc cttttttttt 9420
   tttttttttt ttttttcttt ttttttcctt tttttttttt tttttttttt ttccttttct 9480
   ttcttctttg gtggctccat cttagcccta gtcacggcta gctgtgaaag gtccgtgagc 9540
   cgcatgactg cagagagtgc tgatactggc ctctctgcag atcatgt 9587
<210> 12
   <211> 3010
   <212> PRT
   <213> Hepatitis C virus
<400> 12
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Ala Trp Ala Gln Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Thr Pro Ala Ser Ala Tyr
               180                 185                 190
   Glu Val Arg Asn Val Ser Gly Val Tyr His Val Thr Asn Asp Cys Ser
           195                 200                 205
   Asn Ser Ser Ile Val Tyr Glu Ala Ala Asp Met Ile Met His Thr Pro
       210                 215                 220
   Gly Cys Val Pro Cys Val Arg Glu Asn Asn Ser Ser Arg Cys Trp Val
   225                 230                 235                 240
   Ala Leu Thr Pro Thr Leu Ala Ala Arg Asn Ser Ser Ile Pro Thr Thr
                   245                 250                 255
   Thr Ile Arg Arg His Val Asp Leu Leu Val Gly Ala Ala Ala Leu Cys
               260                 265                 270
   Ser Ala Met Tyr Val Gly Asp Leu Cys Gly Ser Val Phe Leu Val Ser
           275                 280                 285
   Gln Leu Phe Thr Phe Ser Pro Arg Arg Tyr Glu Thr Val Gln Asp Cys
       290                 295                 300
   Asn Cys Ser Leu Tyr Pro Gly His Val Ser Gly His Arg Met Ala Trp
   305                 310                 315                 320
   Asp Met Met Met Asn Trp Ser Pro Thr Thr Ala Leu Val Val Ser Gln
   325 330 335
   Leu Leu Arg Ile Pro Gln Ser Val Val Asp Met Val Ala Gly Ala His
   340 345 350
   Trp Gly Val Leu Ala Gly Leu Ala Tyr Tyr Ser Met Val Gly Asn Trp
   355 360 365
   Ala Lys Val Leu Ile Val Met Leu Leu Phe Ala Gly Val Asp Gly Ser
   370 375 380
   Thr Arg Val Thr Gly Gly Thr Thr Ala Tyr Asn Val Arg Gly Leu Thr
   385 390 395 400
   Ser Leu Phe Thr Pro Gly Pro Ser Gln Lys Ile Gln Leu Ile Asn Ser
   405 410 415
   Asn Gly Ser Trp His Ile Asn Arg Thr Ala Leu Asn Cys Asn Asp Ser
   420 425 430
   Leu Gln Thr Gly Phe Leu Ala Ala Leu Phe Tyr Thr His Ser Phe Asn
   435 440 445
   Ala Ser Gly Cys Pro Glu Arg Met Ala Ser Cys Arg Pro Ile Asp Lys
   450 455 460
   Phe Ala Gln Gly Trp Gly Pro Ile Thr His Val Val Pro Asn Ile Ser
   465 470 475 480
   Asp Gln Arg Pro Tyr Cys Trp His Tyr Ala Pro Arg Pro Cys Gly Ile
   485 490 495
   Val Pro Ala Ser Gln Val Cys Gly Pro Val Tyr Cys Phe Thr Pro Ser
   500 505 510
   Pro Val Val Val Gly Thr Thr Asp Arg Phe Gly Val Pro Thr Tyr Thr
   515 520 525
   Trp Gly Glu Asn Glu Thr Asp Val Leu Leu Leu Asn Asn Thr Arg Pro
   530 535 540
   Pro Gln Gly Asn Trp Phe Gly Cys Thr Trp Met Asn Ser Thr Gly Phe
   545 550 555 560
   Thr Lys Thr Cys Gly Gly Pro Pro Cys Asp Ile Gly Gly Ala Gly Asn
   565 570 575
   Ser Thr Leu Thr Cys Pro Thr Asp Cys Phe Arg Lys His Pro Glu Ala
   580 585 590
   Thr Tyr Thr Lys Cys Gly Ser Gly Pro Trp Leu Thr Pro Arg Cys Ile
   595 600 605
   Val Asp Tyr Pro Tyr Arg Leu Trp His Tyr Pro Cys Thr Val Asn Phe
   610 615 620
   Thr Ile Phe Lys Val Arg Met Tyr Val Gly Gly Val Glu His Arg Leu
   625 630 635 640
   Asn Ala Ala Cys Asn Trp Thr Arg Gly Glu Arg Cys Asn Leu Glu Asp
   645 650 655
   Arg Asp Arg Ser Glu Leu Ser Pro Leu Leu Leu Ser Thr Thr Glu Trp
   660 665 670
   Gln Ile Leu Pro Cys Ser Phe Thr Thr Leu Pro Ala Leu Ser Thr Gly
   675 680 685
   Leu Ile His Leu His Gln Asn Ile Val Asp Val Gln Tyr Leu Tyr Gly
   690 695 700
   Val Gly Ser Ala Val Val Ser Ile Val Ile Lys Trp Glu Tyr Val Leu
   705 710 715 720
   Leu Leu Phe Leu Leu Leu Ala Asp Ala Arg Val Cys Ala Cys Leu Trp
   725 730 735
   Met Met Leu Leu Ile Ala Gln Ala Glu Ala Ala Leu Glu Asn Leu Val
   740 745 750
   Ile Leu Asn Ala Ala Ser Val Ala Gly Ala His Gly Ile Leu Ser Phe
   755 760 765
   Leu Val Phe Phe Cys Ala Ala Trp Tyr Ile Lys Gly Lys Leu Val Pro
   770 775 780
   Gly Ala Ala Tyr Ala Leu Tyr Gly Val Trp Pro Leu Leu Leu Leu Leu
   785 790 795 800
   Leu Ala Leu Pro Pro Arg Ala Tyr Ala Met Asp Arg Glu Met Ala Ala
   805 810 815
   Ser Cys Gly Gly Ala Val Phe Ile Gly Leu Val Leu Leu Thr Leu Ser
   820 825 830
   Pro His Tyr Lys Ala Phe Leu Ala Arg Leu Ile Trp Trp Leu Gln Tyr
   835 840 845
   Phe Thr Thr Arg Ala Glu Ala His Leu Gln Val Trp Val Ser Pro Leu
   850 855 860
   Asn Val Arg Gly Gly Arg Asp Ala Ile Ile Leu Leu Thr Cys Val Val
   865 870 875 880
   His Pro Glu Leu Ile Phe Asp Ile Thr Lys Ile Leu Leu Ala Ile Phe
   885 890 895
   Gly Pro Leu Met Val Leu Gln Ala Gly Leu Thr Arg Val Pro Tyr Phe
   900 905 910
   Val Arg Ala Gln Gly Leu Ile Arg Ala Cys Val Leu Val Arg Lys Val
   915 920 925
   Ala Gly Gly His Tyr Val Gln Met Ala Leu Val Lys Leu Ala Ala Leu
   930 935 940
   Thr Gly Thr Tyr Val Tyr Asp His Leu Thr Pro Leu Arg Asp Trp Ala
   945 950 955 960
   His Ala Ser Leu Gln Asp Leu Val Val Ala Val Glu Pro Val Val Phe
   965 970 975
   Ser Asp Met Glu Thr Lys Ile Ile Thr Trp Gly Ala Asp Thr Ala Ala
   980 985 990
   Cys Gly Asp Ile Ile Ser Gly Leu Pro Val Ser Ala Arg Arg Gly Arg
           995                 1000                1005
   Glu Ile Leu Leu Gly Pro Ala Asp Ser Phe Glu Glu Gln Gly Trp Arg
       1010                1015                1020
   Leu Leu Ala Pro Ile Thr Ala Tyr Ser Gln Gln Thr Arg Gly Leu Leu
   1025                1030                1035                1040
   Gly Cys Ile Ile Thr Ser Leu Thr Gly Arg Asp Lys Asn Gln Val Glu
                   1045                1050                1055
   Gly Glu Val Gln Val Val Ser Thr Ala Thr Gln Ser Phe Leu Ala Thr
               1060                1065                1070
   Cys Val Asn Gly Val Cys Trp Ser Val Tyr His Gly Ala Gly Ser Lys
           1075                1080                1085
   Thr Leu Ala Gly Pro Lys Gly Pro Val Thr Gln Met Tyr Thr Asn Val
       1090                1095                1100
   Asp Leu Asp Leu Val Gly Trp Gln Ala Pro Pro Gly Thr Arg Ser Leu
   1105                1110                1115                1120
   Thr Pro Cys Thr Cys Gly Ser Ser Asp Leu Tyr Leu Val Thr Arg His
                   1125                1130                1135
   Ala Asp Val Ile Pro Val Arg Arg Arg Gly Asp Ser Arg Gly Ser Leu
               1140                1145                1150
   Leu Ser Pro Arg Pro Val Ser Tyr Leu Lys Gly Ser Ser Gly Gly Pro
           1155                1160                1165
   Leu Leu Cys Pro Ser Gly His Ala Val Gly Ile Phe Arg Ala Ala Val
       1170                1175                1180
   Cys Thr Arg Gly Val Ala Lys Ala Val Asp Phe Ile Pro Val Glu Ser
   1185                1190                1195                1200
   Met Glu Thr Thr Met Arg Ser Pro Val Tyr Thr Asp Asn Ser Ser Pro
                   1205                1210                1215
   Pro Ala Val Pro Gln Thr Phe Gln Val Ala His Leu His Ala Pro Thr
               1220                1225                1230
   Gly Ser Gly Lys Ser Thr Lys Val Pro Ala Ala Tyr Ala Ala Gln Gly
           1235                1240                1245
   Tyr Lys Val Leu Val Leu Asn Pro Ser Val Ala Ala Thr Leu Gly Phe
       1250                1255                1260
   Gly Ala Tyr Met Ser Lys Ala His Gly Val Asp Pro Asn Ile Arg Thr
   1265                1270                1275                1280
   Gly Val Arg Thr Ile Thr Thr Gly Ala Pro Ile Thr Tyr Ser Thr Tyr
                   1285                1290                1295
   Gly Lys Phe Leu Ala Asp Gly Gly Cys Ser Gly Gly Ala Tyr Asp Ile
               1300                1305                1310
   Ile Ile Cys Asp Glu Cys His Ser Thr Asp Ser Thr Ser Ile Leu Gly
           1315                1320                1325
   Ile Gly Thr Val Leu Asp Gln Ala Glu Thr Ala Gly Ala Arg Leu Val
   1330 1335 1340
   Val Leu Ala Thr Ala Thr Pro Pro Gly Ser Val Thr Val Pro His Pro
   1345 1350 1355 1360
   Asn Ile Glu Glu Val Ala Leu Ser Asn Thr Gly Glu Ile Pro Phe Tyr
   1365 1370 1375
   Gly Lys Ala Ile Pro Ile Glu Thr Ile Lys Gly Gly Arg His Leu Ile
   1380 1385 1390
   Phe Cys His Ser Lys Lys Lys Cys Asp Glu Leu Ala Ala Lys Leu Ser
   1395 1400 1405
   Ala Leu Gly Val Asn Ala Val Ala Tyr Tyr Arg Gly Leu Asp Val Ser
   1410 1415 1420
   Val Ile Pro Thr Ser Gly Asp Val Val Val Val Ala Thr Asp Ala Leu
   1425 1430 1435 1440
   Met Thr Gly Tyr Thr Gly Asp Phe Asp Ser Val Ile Asp Cys Asn Thr
   1445 1450 1455
   Cys Val Thr Gln Thr Val Asp Phe Ser Leu Asp Pro Thr Phe Thr Ile
   1460 1465 1470
   Asp Thr Thr Thr Val Pro Gln Asp Ala Val Ser Arg Ser Gln Arg Arg
   1475 1480 1485
   Gly Arg Thr Gly Arg Gly Arg Gly Gly Ile Tyr Arg Phe Val Thr Pro
   1490 1495 1500
   Gly Glu Arg Pro Ser Gly Met Phe Asp Ser Ser Val Leu Cys Glu Cys
   1505 1510 1515 1520
   Tyr Asp Ala Gly Cys Ala Trp Tyr Glu Leu Thr Pro Ala Glu Thr Ser
   1525 1530 1535
   Val Arg Leu Arg Ala Tyr Leu Asn Thr Pro Gly Leu Pro Val Cys Gln
   1540 1545 1550
   Asp His Leu Glu Phe Trp Glu Ser Val Phe Thr Gly Leu Thr His Ile
   1555 1560 1565
   Asp Ala His Phe Leu Ser Gln Thr Lys Gln Ala Gly Asp Asn Phe Pro
   1570 1575 1580
   Tyr Leu Val Ala Tyr Gln Ala Thr Val Cys Ala Arg Ala Gln Ala Pro
   1585 1590 1595 1600
   Pro Pro Ser Trp Asp Gln Met Trp Lys Cys Leu Ile Arg Leu Lys Pro
   1605 1610 1615
   Thr Leu His Gly Pro Thr Pro Leu Leu Tyr Arg Leu Gly Ala Val Gln
   1620 1625 1630
   Asn Glu Val Thr Leu Thr His Pro Ile Thr Lys Phe Ile Met Ala Cys
   1635 1640 1645
   Met Ser Ala Asp Leu Glu Val Val Thr Ser Thr Trp Val Leu Val Gly
   1650 1655 1660
   Gly Val Leu Ala Ala Leu Ala Ala Tyr Cys Leu Thr Thr Gly Ser Val
   1665                1670                1675                1680
   Val Ile Val Gly Arg Ile Ile Leu Ser Gly Arg Pro Ala Ile Ile Pro
                   1685                1690                1695
   Asp Arg Glu Val Leu Tyr Gln Glu Phe Asp Glu Met Glu Glu Cys Ala
               1700                1705                1710
   Ser His Leu Pro Tyr Ile Glu Gln Gly Met Gln Leu Ala Glu Gln Phe
           1715                1720                1725
   Lys Gln Lys Ala Leu Gly Leu Leu Gln Thr Ala Thr Lys Gln Ala Glu
       1730                1735                1740
   Ala Ala Ala Pro Val Val Gln Ser Lys Trp Gln Ala Leu Glu Ala Phe
   1745                1750                1755                1760
   Trp Ala Lys His Met Trp Asn Phe Ile Ser Gly Ile Gln Tyr Leu Ala
                   1765                1770                1775
   Gly Leu Ser Thr Leu Pro Gly Asn Pro Ala Ile Ala Ser Leu Met Ala
               1780                1785                1790
   Phe Thr Ala Ser Ile Thr Ser Pro Leu Thr Thr Gln Tyr Thr Leu Leu
           1795                1800                1805
   Phe Asn Ile Leu Gly Gly Trp Val Ala Ala Gln Leu Ala Pro Pro Ser
       1810                1815                1820
   Ala Ala Ser Ala Phe Val Gly Ala Gly Ile Ala Gly Ala Ala Val Gly
   1825                1830                1835                1840
   Ser Ile Gly Leu Gly Lys Val Leu Val Asp Ile Leu Ala Gly Tyr Gly
                   1845                1850                1855
   Ala Gly Val Ala Gly Ala Leu Val Ala Phe Lys Val Met Ser Gly Glu
               1860                1865                1870
   Val Pro Ser Thr Glu Asp Leu Val Asn Leu Leu Pro Ala Ile Leu Ser
           1875                1880                1885
   Pro Gly Ala Leu Val Val Gly Val Val Cys Ala Ala Ile Leu Arg Arg
       1890                1895                1900
   His Val Gly Pro Gly Glu Gly Ala Val Gln Trp Met Asn Arg Leu Ile
   1905                1910                1915                1920
   Ala Phe Ala Ser Arg Gly Asn His Val Ser Pro Thr His Tyr Val Pro
                   1925                1930                1935
   Glu Ser Asp Ala Ala Ala Arg Val Thr Gln Ile Leu Ser Ser Leu Thr
               1940                1945                1950
   Ile Thr Gln Leu Leu Lys Arg Leu His Gln Trp Ile Asn Glu Asp Cys
           1955                1960                1965
   Ser Thr Pro Cys Ser Gly Ser Trp Leu Arg Asp Val Trp Asp Trp Ile
       1970                1975                1980
   Cys Thr Val Leu Ser Asp Phe Lys Thr Trp Leu Gln Ser Lys Leu Leu
   1985                1990                1995                2000
   Pro Arg Leu Pro Gly Val Pro Phe Phe Ser Cys Gln Arg Gly Tyr Arg
   2005 2010 2015
   Gly Val Trp Arg Gly Asp Gly Val Met Gln Thr Ile Cys Pro Cys Gly
   2020 2025 2030
   Ala Gln Ile Ser Gly His Val Lys Asn Gly Ser Met Arg Ile Val Gly
   2035 2040 2045
   Pro Lys Thr Cys Ser Asn Thr Trp His Gly Thr Phe Pro Ile Asn Ala
   2050 2055 2060
   Tyr Thr Thr Gly Pro Cys Thr Pro Ser Pro Ala Pro Asn Tyr Ser Arg
   2065 2070 2075 2080
   Ala Leu Trp Arg Val Thr Ala Glu Glu Tyr Val Glu Val Thr Arg Val
   2085 2090 2095
   Gly Asp Phe His Tyr Val Thr Gly Met Thr Thr Asp Asn Val Lys Cys
   2100 2105 2110
   Pro Cys Gln Val Pro Ala Pro Glu Phe Phe Thr Glu Leu Asp Gly Val
   2115 2120 2125
   Arg Leu His Arg Tyr Ala Pro Ala Cys Lys Pro Leu Leu Arg Asp Glu
   2130 2135 2140
   Val Thr Phe Gln Val Gly Leu Asn His Phe Pro Val Gly Ser Gln Leu
   2145 2150 2155 2160
   Pro Cys Glu Pro Glu Pro Asp Val Thr Val Val Thr Ser Met Leu Thr
   2165 2170 2175
   Asp Pro Ser His Ile Thr Ala Glu Thr Ala Arg Arg Arg Leu Ala Arg
   2180 2185 2190
   Gly Ser Pro Pro Ser Leu Ala Ser Ser Ser Ala Ser Gln Leu Ser Ala
   2195 2200 2205
   Pro Ser Leu Lys Ala Thr Cys Thr Thr His His Asp Ser Pro Asp Ala
   2210 2215 2220
   Asp Leu Ile Glu Ala Asn Leu Leu Trp Arg Gln Glu Met Gly Gly Asn
   2225 2230 2235 2240
   Ile Thr Arg Val Glu Ser Glu Asn Lys Val Val Ile Leu Asp Ser Phe
   2245 2250 2255
   Asp Pro Leu Arg Ala Glu Glu Asp Glu Arg Glu Val Ser Val Ala Ala
   2260 2265 2270
   Glu Ile Leu Arg Lys Thr Arg Lys Phe Pro Pro Ala Met Pro Ile Trp
   2275 2280 2285
   Ala Arg Pro Asp Tyr Asn Pro Pro Leu Leu Glu Ser Trp Lys Asn Pro
   2290 2295 2300
   Asp Tyr Val Pro Pro Val Val His Gly Cys Pro Leu Pro Pro Thr Lys
   2305 2310 2315 2320
   Ala Pro Pro Ile Pro Pro Pro Arg Arg Lys Arg Thr Val Val Leu Thr
   2325 2330 2335
   Glu Ser Thr Val Ser Ser Ala Leu Ala Glu Leu Ala Thr Lys Thr Phe
   2340 2345 2350
   Gly Ser Ser Gly Ser Ser Ala Val Asp Ser Gly Thr Ala Ser Ala Pro
   2355 2360 2365
   Pro Asp Gln Pro Ser Asp Asp Gly Asp Ala Gly Ser Asp Val Glu Ser
   2370 2375 2380
   Tyr Ser Ser Met Pro Pro Leu Glu Gly Glu Pro Gly Asp Pro Asp Leu
   2385 2390 2395 2400
   Ser Asp Gly Ser Trp Ser Thr Val Ser Glu Glu Ala Ser Glu Asp Val
   2405 2410 2415
   Val Cys Cys Ser Met Ser Tyr Thr Trp Thr Gly Ala Leu Ile Thr Pro
   2420 2425 2430
   Cys Ala Ala Glu Glu Ser Lys Leu Pro Ile Asn Ala Leu Ser Asn Ser
   2435 2440 2445
   Leu Leu Arg His His Asn Met Val Tyr Ala Thr Thr Ser Arg Ser Ala
   2450 2455 2460
   Gly Gln Arg Gln Lys Lys Val Thr Phe Asp Arg Leu Gln Val Leu Asp
   2465 2470 2475 2480
   Asp His Tyr Arg Asp Val Leu Lys Glu Met Lys Ala Lys Ala Ser Thr
   2485 2490 2495
   Val Lys Ala Lys Leu Leu Ser Val Glu Glu Ala Cys Lys Leu Thr Pro
   2500 2505 2510
   Pro His Ser Ala Arg Ser Lys Phe Gly Tyr Gly Ala Lys Asp Val Arg
   2515 2520 2525
   Asn Leu Ser Ser Lys Ala Val Asn His Ile His Ser Val Trp Lys Asp
   2530 2535 2540
   Leu Leu Glu Asp Thr Glu Thr Pro Ile Asp Thr Thr Ile Met Ala Lys
   2545 2550 2555 2560
   Asn Glu Val Phe Cys Val Gln Pro Glu Lys Gly Gly Arg Lys Pro Ala
   2565 2570 2575
   Arg Leu Ile Val Phe Pro Asp Leu Gly Val Arg Val Cys Glu Lys Met
   2580 2585 2590
   Ala Leu Tyr Asp Val Val Ser Thr Leu Pro Gln Ala Val Met Gly Ser
   2595 2600 2605
   Ser Tyr Gly Phe Gln Tyr Ser Pro Gly Gln Arg Val Glu Phe Leu Val
   2610 2615 2620
   Asn Ala Trp Lys Ser Lys Lys Asn Pro Met Gly Phe Ala Tyr Asp Thr
   2625 2630 2635 2640
   Arg Cys Phe Asp Ser Thr Val Thr Glu Asn Asp Ile Arg Val Glu Glu
   2645 2650 2655
   Ser Ile Tyr Gln Cys Cys Asp Leu Ala Pro Glu Ala Arg Gln Ala Ile
   2660 2665 2670
   Arg Ser Leu Thr Glu Arg Leu Tyr Ile Gly Gly Pro Leu Thr Asn Ser
   2675 2680 2685
   Lys Gly Gln Asn Cys Gly Tyr Arg Arg Cys Arg Ala Ser Gly Val Leu
   2690 2695 2700
   Thr Thr Ser Cys Gly Asn Thr Leu Thr Cys Tyr Leu Lys Ala Thr Ala
   2705 2710 2715 2720
   Ala Cys Arg Ala Ala Lys Leu Gln Asp Cys Thr Met Leu Val Cys Gly
   2725 2730 2735
   Asp Asp Leu Val Val Ile Cys Glu Ser Ala Gly Thr Gln Glu Asp Ala
   2740 2745 2750
   Ala Ser Leu Arg Val Phe Thr Glu Ala Met Thr Arg Tyr Ser Ala Pro
   2755 2760 2765
   Pro Gly Asp Pro Pro Gln Pro Glu Tyr Asp Leu Glu Leu Ile Thr Ser
   2770 2775 2780
   Cys Ser Ser Asn Val Ser Val Ala His Asp Ala Ser Gly Lys Arg Val
   2785 2790 2795 2800
   Tyr Tyr Leu Thr Arg Asp Pro Thr Thr Pro Leu Ala Arg Ala Ala Trp
   2805 2810 2815
   Glu Thr Ala Arg His Thr Pro Val Asn Ser Trp Leu Gly Asn Ile Ile
   2820 2825 2830
   Met Tyr Ala Pro Thr Leu Trp Ala Arg Met Ile Leu Met Thr His Phe
   2835 2840 2845
   Phe Ser Val Leu Leu Ala Gln Glu Gln Leu Glu Lys Ala Leu Asp Cys
   2850 2855 2860
   Gln Ile Tyr Gly Ala Thr Tyr Ser Ile Glu Pro Leu Asp Leu Pro Gln
   2865 2870 2875 2880
   Ile Ile Gln Arg Leu His Gly Leu Ser Ala Phe Ser Leu His Ser Tyr
   2885 2890 2895
   Ser Pro Gly Glu Ile Asn Arg Val Ala Ser Cys Leu Arg Lys Leu Gly
   2900 2905 2910
   Val Pro Pro Leu Arg Val Trp Arg His Arg Ala Arg Ser Val Arg Ala
   2915 2920 2925
   Lys Leu Leu Ser Gln Lys Gly Arg Ala Ala Thr Cys Gly Lys Tyr Leu
   2930 2935 2940
   Phe Asn Trp Ala Val Arg Thr Lys Leu Lys Leu Thr Pro Ile Pro Ala
   2945 2950 2955 2960
   Ala Ser Arg Leu Asp Leu Ser Gly Trp Phe Val Ala Gly Tyr Ser Gly
   2965 2970 2975
   Gly Asp Ile Tyr His Ser Leu Ser Arg Ala Arg Pro Arg Trp Phe Met
   2980 2985 2990
   Trp Cys Leu Leu Leu Leu Ser Val Gly Val Gly Ile Tyr Leu Leu Pro
   2995 3000 3005
   Asn Arg
   3010
<210> 13
   <211> 9587
   <212> DNA
   <213> Hepatitis C virus
<400> 13
   gccagccccc gattgggggc gacactccac catagatcac tcccctgtga ggaactactg 60
   tcttcacgca gaaagcgtct agccatggcg ttagtatgag tgtcgtgcag cctccaggac 120
   cccccctccc gggagagcca tagtggtctg cggaaccggt gagtacaccg gaattgccag 180
   gacgaccggg tcctttcttg gatcaacccg ctcaatgcct ggagatttgg gcgtgccccc 240
   gcgagactgc tagccgagta gtgttgggtc gcgaaaggcc ttgtggtact gcctgatagg 300
   gtgcttgcga gtgccccggg aggtctcgta gaccgtgcat catgagcaca aatcctaaac 360
   ctcaaagaaa aaccaaacgt aacaccaacc gccgcccaca ggacgtcaag ttcccgggcg 420
   gtggtcagat cgttggtgga gtttacctgt tgccgcgcag gggccccagg ttgggtgtgc 480
   gcgcgactag gaagacttcc gagcggtcgc aacctcgtgg aaggcgacaa cctatcccca 540
   aggctcgcca gcccgagggc agggcctggg ctcagcccgg gtacccttgg cccctctatg 600
   gcaatgaggg tctggggtgg gcaggatggc tcctgtcacc ccgaggctct cggcctagtt 660
   ggggccccac ggacccccgg cgtaggtcgc gtaatctggg taaggtcatc gataccctta 720
   catgcggctt cgccgacctc atggggtaca ttccgctcgt cggcgccccc ctagggggcg 780
   ttgccagggc cctggcgcaa ggcgtccggg ttctggagga cggcgtgaac tacgcaacag 840
   ggaatttgcc cggttgctcc ttttctatct tcctcttggc tttgctgtct tgcttgacca 900
   ttccaacttc cgcttacgaa gtgcgcaacg tgtccggggt gtaccatgtc acgaacgact 960
   gctccaactc aagtatagtg tatgaggcag cggacatgat catgcacacc cccgggtgcg 1020
   tgccctgcgt ccgggagagt aatttctccc gctgctgggt ggcgctcact cccacgctcg 1080
   cggccaggaa cagcagcatc cccaccacga caatacgacg ccacgtcgat ttgctcgttg 1140
   gggcagccgc tttctgctct gctatgtacg tgggagatct ctgcggatcc gtcttcctcg 1200
   tctctcagct gttcaccttc tcacctcgcc ggtacgagac ggtacaagac tgcaactgct 1260
   cactctatcc cggccacgta tcaggtcacc gcatggcttg ggatatgatg atgaattggt 1320
   cacctacagc agccctagtg gtatcgcagt tactccggat cccacaagct atcgtggata 1380
   tggtggcggg ggcccactgg ggagtcctag cgggccttgc ctactattcc atggtgggga 1440
   actgggctaa ggttttgatt gtgatgctac tttttgccgg cgttgacggg agcacccgcg 1500
   tgtcgggggg gcaggtagcc tacaacacca ggggctttgc atccctcttt acaccagggg 1560
   cgagtcagaa aatccaactc ataaacacca acggcagctg gcacatcaac aggactgctc 1620
   tgaactgcaa tgactccctc cacactgggt tccttgccgc gctgttctac acacacaagt 1680
   tcaacgcgtc cggatgccca gagcgcatgg ccagctgccg ccccattgac aagttcgctc 1740
   aggggtgggg tcccatcact catgtcgagc ctgagatctc ggaccagagg ccatattgct 1800
   ggcactacgc accccgaccg tgcggtatag tacccgcgtc gcaggtgtgt ggtccagtgt 1860
   attgcttcac cccaagccct gttgtagtgg ggacgactga tcgttccggc gtccccacgt 1920
   acacctgggg ggagaatgag acggacgtgc tgctccttaa caacacgcgg ccaccccacg 1980
   gcaactggtt cggctgcaca tggatgaata gcaccgggtt caccaagacg tgcgggggcc 2040
   ccccgtgcaa catcgggggg gtcggcaaca acaccttgac ctgccccacg gattgcttcc 2100
   ggaaacaccc cgaagccact tacaccaaat gcggctcggg cccttggttg acacctaggt 2160
   gtatagttga ctacccatac agactttggc actacccctg cactgtcaac tttaccatct 2220
   tcaaggtcag gatgtatgtg gggggcgtgg agcacaggct caacgccgcg tgcaactgga 2280
   ctcggggaga gcgctgtgac ctggaggaca gggataggtc agaactcagt ccgctgctac 2340
   tgtccacaac agagtggcag atactgccct gttccttcac caccctaccg gctttgtcca 2400
   ctggcttgat ccacctccat cagaacatcg tggatgtgca atacttgtat ggcatagggt 2460
   cagccgttgt ctccgttgta atcaaatggg agtacgtcgt gctgcttttc cttctcctgg 2520
   cagacgcgcg cgtttgcgcc tgcttatgga tgatgctgct ggtagcccag gctgaggccg 2580
   ccttagagaa cctggtggtc ctcaatgcag cgtccgtggc tggagcgcat ggcattctct 2640
   ccttccttgt gttcttctgt gctgcctggt acatcaaagg caagctggtc cctggagcgg 2700
   catatgcttt ctatggcgta tggccgctgc tcctgctcct gctggcgtta ccaccacgag 2760
   catacgccat ggaccgggag atggccgcat cgtgcggagg cgcggttttt gtaggtctgg 2820
   cactcttgac cttgtcacca cactataaag tgtttctcgc taggctcata tggtggttac 2880
   aatatttaat caccagggcc gaagcgctct tgcaagtgtg gatcccccct ctcaacgttc 2940
   gcgggggccg cgatgccatc atcctcctca cgtgcgcggt ccatccagag ctaatttttg 3000
   aaatcaccaa aatcttgctc gccatactcg gaccgctcac agtgctccag gcaggcctaa 3060
   ctagagtgcc gtactttgta cgcgctcaag ggctcattcg tgcatgcatg ttggtgcgga 3120
   aagtcgctgg gggtcattat gtccaaatgg ctctcatgaa gctaggcgca ctgacgggca 3180
   cgtatcttta tgaccatctt actccactgc gggactgggc ccacgcgggc ctacgagacc 3240
   ttgcgatggc agttgagccc gttatctttt ctgacatgga gaccaagatc atcacctggg 3300
   gggcagacac cgcggcgtgc ggggacatca tctcaggtct acctgtctcc gcccgaaggg 3360
   ggagggagat actcctagga ccggccgata gttttgaagg gcaggggtgg cggctccttg 3420
   cgcctatcac ggcctattcc caacaaacta ggggcctact tggctgtatc atcactagtc 3480
   tcacaggtcg ggacaagaac cgggtcgagg gggaggttca ggtggtctcc accgcaacac 3540
   aatcttttct ggcgacctgc gtcaatggcg tgtgttggac cgtctaccac ggcgctggca 3600
   cgaagacttt ggccggcccg aaaggtccaa tcacccaaat gtataccaat gtagaccaag 3660
   acctcgtcgg ctggcaggcg ccccccgggg cgcgttcctt gacaccatgc acctgcggca 3720
   gctcggacct ctacttggtc acgaggcatg ccgatgtcat tccggtgcgc cggcggggcg 3780
   acagcagggg gagcctgctc tcccccaggc ctgtctccta cttgaagggc tcctcaggtg 3840
   gtccattgct ttgcccctcg ggacacgccg tgggcatctt ccgggctgct gtgtgcaccc 3900
   ggggggtcgc aaaggcggtg gactttgtac ccgttgagtc tatggaaact actatgcggt 3960
   ctccggtctt tacggataat tcatctcccc cggccgtacc gcagacattc caagtggccc 4020
   atctacacgc tcccactggc agcggcaaga gcactaaggt gccggctgca tacgcagccc 4080
   aagggtacaa ggtactcgtc ctgaacccgt ccgttgccgc caccttgggt tttggggcgt 4140
   acatgtctaa ggcacatggt gtcgacccta acatcagaac tggggtaagg accatcacta 4200
   cgggcgctcc cattacgtac tccacctacg gcaagttcct tgccgacggt ggttgctctg 4260
   ggggtgccta cgacatcata atatgtgatg agtgccactc aactgactcg acttctattt 4320
   tgggcattgg cacggtcctg gaccaagcgg agacggctgg agcgcgactc gtcgtgctcg 4380
   ccaccgctac acctccggga tcggtcactg tgccacatcc caacatcgag gaggtggccc 4440
   tgtccaacac tggagagatt cccttctatg gcaaagccat ccccatcgag accatcaagg 4500
   gggggaggca tctcatcttc tgccattcca agaagaaatg tgacgagctc gccgcaaagt 4560
   tgtcgggcct cggaatcaac gccgtagcgt actaccgggg tcttgatgtg tccgttatac 4620
   caacaagcgg agacgtcgtt gtcgtggcaa cagacgctct aatgacgggc tataccggcg 4680
   actttgactc ggtgatcgac tgtaatacat gcgtcaccca gacagtcgat tttagcttgg 4740
   accctacctt caccattgag acgacgaccg tgcctcaaga cgcggtgtcg cgctcgcagc 4800
   ggcgaggcag gactggtagg ggcagagggg gcatatacag atttgtgact ccaggggaac 4860
   ggccctcggg catgttcgat tcttcggtcc tgtgtgagtg ctatgacgcg ggctgtgctt 4920
   ggtatgagct cacgcccgcc gagacctcag ttaggttgcg ggcttacctg aatacaccag 4980
   ggttacccgt ctgccaggac catctggagt tctgggagag cgtcttcaca ggcctcaccc 5040
   acatcgatgc ccatttcttg tcccagacta aacaggcagg agacaacttc ccctacctgg 5100
   tggcatacca ggctacggtg tgcgccaggg cccaggctcc acctccatcg tgggatcaaa 5160
   tgtggaagtg tctcatacgg cttaagccta cactacacgg gccaacaccc ctgttgtaca 5220
   ggctaggggc cgtacaaaat gaggtcaccc tcacacaccc cataaccaaa tacatcatgg 5280
   catgcatgtc ggctgaccta gaggtcgtca ccagcacctg ggtgctggta ggcggggtcc 5340
   ttgcggctct ggccgcatac tgcctgacaa cgggcagtgt ggtcattgtg ggcaggatca 5400
   tcttgtcagg gaagccggct atcattcccg acagggaagt tctctaccga gagttcgacg 5460
   aaatggagga gtgcgcctca cacctccctt acatcgaaca gggaatgcag ctcgccgagc 5520
   aattcaagca gaaggcgctc gggttgctgc agacagccac caagcaagcg gaggctgctg 5580
   ctcccgtggt ggagtccaag tggcggaccc ttgaggcttt ctgggcgaag cacatgtgga 5640
   acttcatcag cgggatacag tacttagcag gcttgtccac tctgcctggg aaccccgcga 5700
   tagcatccct gatggcattc acagcctcta tcaccagccc gctcaccacc caacataccc 5760
   tcttgtttaa catcttgggg ggatgggtgg ccgcccaact cgcccccccc agcgctgcct 5820
   cagctttcgt gggcgccggt atcgctggcg cggctgttgg cagcataggt cttgggaagg 5880
   tgcttgtgga catcctggcg ggttatggag caggggtggc aggcgcactc gtggccttta 5940
   aggtcatgag tggcgacatg ccctccaccg aggatctggt caacttgctc cctgccatcc 6000
   tctctcccgg tgccctggtc gtcggagtag tgtgcgcagc aatactgcgt cggcacgtgg 6060
   gcccagggga gggggctgtg cagtggatga accggctgat agcgttcgct tcgcggggta 6120
   accacgtctc ccccacgcac tatgtgcctg agagcgacgc tgcagcacgt gtcacccaga 6180
   tcctctccag ccttaccatc actcagctgt tgaagaggct ccaccagtgg atcaatgagg 6240
   actgctccac gccatgctcc ggctcgtggc tcagggatgt atgggactgg atatgcacgg 6300
   tgttgagtga cttcaagacc tggctccagt ccaaactcct gccgcggtta ccaggagtcc 6360
   ctttcctttc atgtcaacgt ggatacaagg gagtctggcg gggagatggc gtcatgcaaa 6420
   ccacctgccc atgtggagca caaatcgctg gacatgtcaa aaacggttcc atgaggatcg 6480
   ttgggcctaa aacctgcagc aacacgtggc acggaacatt ccccatcaac gcgtacacca 6540
   caggcccctg cacaccctcc ccggcgccca actattctag ggcgctgtgg cgggtggctg 6600
   ctgaggagta cgtggagatc acgcgggtgg gggatttcca ctacgtgacg ggcatgaccg 6660
   ctgacaacgt gaaatgccca tgccaggttc cggcccccga attcttcaca gagttggacg 6720
   gggtacggct gcacaggtac gctccggcgt gcaaacctct cctacgggat gaggtcacat 6780
   tccaggtcgg gctcaaccaa tacccggttg ggtcacagct cccatgtgag cccgaaccgg 6840
   atgtaacagt ggtcacctcc atgctcaccg acccctccca cattacagca gaaacggcca 6900
   agcgtaggct ggccaggggg tcgccccctt ctttggccag ctcttcagcc agccagttgt 6960
   ctgcgccttc tttgaaggcg acttgcacta cccatcatga ctccccagat gccgaactca 7020
   tcgaggccaa cctcctgtgg cggcaggaga tgggcgggaa catcacccgc gtggagtcag 7080
   agaataaggt agtaatccta gactcttttg acccgctccg agcggaggag gatgagaggg 7140
   aagtatccgt tgcggcggag atcctgcgaa agaccaggaa attcccccct gcgatgccca 7200
   tatgggcacg accggattac aacccaccac tgatagagtc ttggagggac ccggactacg 7260
   tccctccggt ggtacacggg tgcccattgc cacctgccaa gacccctcca ataccacctc 7320
   cacggagaaa gaggacggtt gtcctgacag aatccaccgt gtcttctgcc ttggcggagc 7380
   ttgctacaaa gtccttcggc agttccggat cgtcggccgt cgacagcggc acggcaactg 7440
   cccctcctga tcagcccctc gacagcggag acacaggatc cgacgttgag tcgtactcct 7500
   ccatgccccc ccttgagggg gaaccggggg accccgatct cagcgacggg tcttggtcta 7560
   ctgtgagcga ggaggctggc gaggacgtcg tctgctgctc gatgtcctac acatggacag 7620
   gcgccttgat cacgccatgc gccgcggagg agagcaagct gcccatcaat gcgttgagca 7680
   actctttgct gcgacaccac aacatggtct atgccacaac atcccgcagc gcaagccaac 7740
   ggcagaaaaa ggtcaccttt gacagactgc aagtcctgga cgaccactac cgggacgtgc 7800
   tcaaggagat gaaggcgaag gcgtccacag ttaaggctaa gcttctatcc gtagaggaag 7860
   cctgcaagct gacgccccca cattcggcca aatcaaaatt tggctatggg gcgaaggacg 7920
   tccggaacct gtccagcaag gccgtcaacc acatccactc cgtgtggaag gacttgctgg 7980
   aagacgctga aacaccaatt gacaccacca tcatggcgaa gaatgaggtc ttctgtgttc 8040
   aaccagagaa aggaggccgc aagccagctc gtcttatcgt attcccagac ttgggagttc 8100
   gtgtgtgcga gaaaatggcc ctctacgacg tagtctccac tcttcctcag gccgtgatgg 8160
   gctcctcata cggattccag tactctcctg ggcagcgggt cgagttcctg gtgaatgcct 8220
   ggaaatcaaa gaagacccct atgggcttcg catatgacac ccgctgcttt gactcaacgg 8280
   tcactgagaa cgacatccgc gttgaggagt caatttacca atgttgtgac ttggcccccg 8340
   aggccagaca ggccataagg tcgctcacag agcggcttta tattgggggc cccctgacta 8400
   attcaaaagg gcagaactgc ggttatcgcc ggtgccgcgc cagcggcgtg ctgacgacca 8460
   gctgcggtaa tacccttaca tgttacttga aggcctctgc agcctgtcga gctgcaaagc 8520
   tccaggactg cacgatgctc gtgtgcggag acgaccttgt cgttatctgt gaaagcgcgg 8580
   gaactcagga ggacgcggcg agcctacgag tcttcacgga ggctatgact aggtactccg 8640
   ccccccccgg ggacccgccc cgaccggaat acgacctgga gttgataaca tcatgctcct 8700
   ccaacgtgtc ggtcgcgcac gatgcaactg gcaaacgggt gtactacctc acccgggacc 8760
   ccaccacccc ccttgcgcgg gctgcgtggg agacagctag acacactcca gtcaactcct 8820
   ggctaggcaa catcatcatg tatgcgccca ctttgtgggc aaggatgatt ctgatgactc 8880
   acttcttctc catccttcta gcccaggagc agcttgaaaa agccctagat tgtcagatct 8940
   acggggccac ttactccatt gaaccacttg acctacctca aatcattcaa cgactccacg 9000
   gtcttagcgc attttcactc catagttact ctccaggtga aatcaatagg gtggcttcat 9060
   gcctcaggaa acttggggta ccgcccttgc gagtctggag acatcgggcc agaagtgtcc 9120
   gcgctaagct actgtcccag ggggggaggg ctgcaatatg tggcaagtac ctcttcaact 9180
   gggccgtacg gaccaagctc aaactcactc caattccggc tgcgtcccgg ttggacttgt 9240
   ccggctggtt cgtcgccggt tacagcgggg gagacatata tcacagcctg tctcgcgccc 9300
   gaccccgctg gttcatgtgg tgcctactcc tactttccgt aggggtaggc atctacctgc 9360
   tccccaaccg gtgaacgggg agctaaacac tccaggccaa taggccattc cttttttttt 9420
   tttttttttt ttttttcttt ttttttcctt tttttttttt tttttttttt ttccttttct 9480
   ttcttctttg gtggctccat cttagcccta gtcacggcta gctgtgaaag gtccgtgagc 9540
   cgcatgactg cagagagtgc tgatactggc ctctctgcag atcatgt              9587
<210> 14
   <211> 3010
   <212> PRT
   <213> Hepatitis C virus
<400> 14
   Met Ser Thr Asn Pro Lys Pro Gln Arg Lys Thr Lys Arg Asn Thr Asn
    1               5                  10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Gln Pro Glu Gly Arg Ala Trp Ala Gln Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Val Ala Arg Ala Leu Ala Gln Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Ile Pro Thr Ser Ala Tyr
               180                 185                 190
   Glu Val Arg Asn Val Ser Gly Val Tyr His Val Thr Asn Asp Cys Ser
           195                 200                 205
   Asn Ser Ser Ile Val Tyr Glu Ala Ala Asp Met Ile Met His Thr Pro
       210                 215                 220
   Gly Cys Val Pro Cys Val Arg Glu Ser Asn Phe Ser Arg Cys Trp Val
   225                 230                 235                 240
   Ala Leu Thr Pro Thr Leu Ala Ala Arg Asn Ser Ser Ile Pro Thr Thr
                   245                 250                 255
   Thr Ile Arg Arg His Val Asp Leu Leu Val Gly Ala Ala Ala Phe Cys
               260                 265                 270
   Ser Ala Met Tyr Val Gly Asp Leu Cys Gly Ser Val Phe Leu Val Ser
           275                 280                 285
   Gln Leu Phe Thr Phe Ser Pro Arg Arg Tyr Glu Thr Val Gln Asp Cys
       290                 295                 300
   Asn Cys Ser Leu Tyr Pro Gly His Val Ser Gly His Arg Met Ala Trp
   305                 310                 315                 320
   Asp Met Met Met Asn Trp Ser Pro Thr Ala Ala Leu Val Val Ser Gln
                   325                 330                 335
   Leu Leu Arg Ile Pro Gln Ala Ile Val Asp Met Val Ala Gly Ala His
               340                 345                 350
   Trp Gly Val Leu Ala Gly Leu Ala Tyr Tyr Ser Met Val Gly Asn Trp
           355                 360                 365
   Ala Lys Val Leu Ile Val Met Leu Leu Phe Ala Gly Val Asp Gly Ser
       370                 375                 380
   Thr Arg Val Ser Gly Gly Gln Val Ala Tyr Asn Thr Arg Gly Phe Ala
   385                 390                 395                 400
   Ser Leu Phe Thr Pro Gly Ala Ser Gln Lys Ile Gln Leu Ile Asn Thr
   405 410 415
   Asn Gly Ser Trp His Ile Asn Arg Thr Ala Leu Asn Cys Asn Asp Ser
   420 425 430
   Leu His Thr Gly Phe Leu Ala Ala Leu Phe Tyr Thr His Lys Phe Asn
   435 440 445
   Ala Ser Gly Cys Pro Glu Arg Met Ala Ser Cys Arg Pro Ile Asp Lys
   450 455 460
   Phe Ala Gln Gly Trp Gly Pro Ile Thr His Val Glu Pro Glu Ile Ser
   465 470 475 480
   Asp Gln Arg Pro Tyr Cys Trp His Tyr Ala Pro Arg Pro Cys Gly Ile
   485 490 495
   Val Pro Ala Ser Gln Val Cys Gly Pro Val Tyr Cys Phe Thr Pro Ser
   500 505 510
   Pro Val Val Val Gly Thr Thr Asp Arg Ser Gly Val Pro Thr Tyr Thr
   515 520 525
   Trp Gly Glu Asn Glu Thr Asp Val Leu Leu Leu Asn Asn Thr Arg Pro
   530 535 540
   Pro His Gly Asn Trp Phe Gly Cys Thr Trp Met Asn Ser Thr Gly Phe
   545 550 555 560
   Thr Lys Thr Cys Gly Gly Pro Pro Cys Asn Ile Gly Gly Val Gly Asn
   565 570 575
   Asn Thr Leu Thr Cys Pro Thr Asp Cys Phe Arg Lys His Pro Glu Ala
   580 585 590
   Thr Tyr Thr Lys Cys Gly Ser Gly Pro Trp Leu Thr Pro Arg Cys Ile
   595 600 605
   Val Asp Tyr Pro Tyr Arg Leu Trp His Tyr Pro Cys Thr Val Asn Phe
   610 615 620
   Thr Ile Phe Lys Val Arg Met Tyr Val Gly Gly Val Glu His Arg Leu
   625 630 635 640
   Asn Ala Ala Cys Asn Trp Thr Arg Gly Glu Arg Cys Asp Leu Glu Asp
   645 650 655
   Arg Asp Arg Ser Glu Leu Ser Pro Leu Leu Leu Ser Thr Thr Glu Trp
   660 665 670
   Gln Ile Leu Pro Cys Ser Phe Thr Thr Leu Pro Ala Leu Ser Thr Gly
   675 680 685
   Leu Ile His Leu His Gln Asn Ile Val Asp Val Gln Tyr Leu Tyr Gly
   690 695 700
   Ile Gly Ser Ala Val Val Ser Val Val Ile Lys Trp Glu Tyr Val Val
   705 710 715 720
   Leu Leu Phe Leu Leu Leu Ala Asp Ala Arg Val Cys Ala Cys Leu Trp
   725 730 735
   Met Met Leu Leu Val Ala Gln Ala Glu Ala Ala Leu Glu Asn Leu Val
   740 745 750
   Val Leu Asn Ala Ala Ser Val Ala Gly Ala His Gly Ile Leu Ser Phe
   755 760 765
   Leu Val Phe Phe Cys Ala Ala Trp Tyr Ile Lys Gly Lys Leu Val Pro
   770 775 780
   Gly Ala Ala Tyr Ala Phe Tyr Gly Val Trp Pro Leu Leu Leu Leu Leu
   785 790 795 800
   Leu Ala Leu Pro Pro Arg Ala Tyr Ala Met Asp Arg Glu Met Ala Ala
   805 810 815
   Ser Cys Gly Gly Ala Val Phe Val Gly Leu Ala Leu Leu Thr Leu Ser
   820 825 830
   Pro His Tyr Lys Val Phe Leu Ala Arg Leu Ile Trp Trp Leu Gln Tyr
   835 840 845
   Leu Ile Thr Arg Ala Glu Ala Leu Leu Gln Val Trp Ile Pro Pro Leu
   850 855 860
   Asn Val Arg Gly Gly Arg Asp Ala Ile Ile Leu Leu Thr Cys Ala Val
   865 870 875 880
   His Pro Glu Leu Ile Phe Glu Ile Thr Lys Ile Leu Leu Ala Ile Leu
   885 890 895
   Gly Pro Leu Thr Val Leu Gln Ala Gly Leu Thr Arg Val Pro Tyr Phe
   900 905 910
   Val Arg Ala Gln Gly Leu Ile Arg Ala Cys Met Leu Val Arg Lys Val
   915 920 925
   Ala Gly Gly His Tyr Val Gln Met Ala Leu Met Lys Leu Gly Ala Leu
   930 935 940
   Thr Gly Thr Tyr Leu Tyr Asp His Leu Thr Pro Leu Arg Asp Trp Ala
   945 950 955 960
   His Ala Gly Leu Arg Asp Leu Ala Met Ala Val Glu Pro Val Ile Phe
   965 970 975
   Ser Asp Met Glu Thr Lys Ile Ile Thr Trp Gly Ala Asp Thr Ala Ala
   980 985 990
   Cys Gly Asp Ile Ile Ser Gly Leu Pro Val Ser Ala Arg Arg Gly Arg
   995 1000 1005
   Glu Ile Leu Leu Gly Pro Ala Asp Ser Phe Glu Gly Gln Gly Trp Arg
   1010 1015 1020
   Leu Leu Ala Pro Ile Thr Ala Tyr Ser Gln Gln Thr Arg Gly Leu Leu
   1025 1030 1035 1040
   Gly Cys Ile Ile Thr Ser Leu Thr Gly Arg Asp Lys Asn Arg Val Glu
   1045 1050 1055
   Gly Glu Val Gln Val Val Ser Thr Ala Thr Gln Ser Phe Leu Ala Thr
   1060 1065 1070
   Cys Val Asn Gly Val Cys Trp Thr Val Tyr His Gly Ala Gly Thr Lys
           1075                1080                1085
   Thr Leu Ala Gly Pro Lys Gly Pro Ile Thr Gln Met Tyr Thr Asn Val
       1090                1095                1100
   Asp Gln Asp Leu Val Gly Trp Gln Ala Pro Pro Gly Ala Arg Ser Leu
   1105                1110                1115                1120
   Thr Pro Cys Thr Cys Gly Ser Ser Asp Leu Tyr Leu Val Thr Arg His
                   1125                1130                1135
   Ala Asp Val Ile Pro Val Arg Arg Arg Gly Asp Ser Arg Gly Ser Leu
               1140                1145                1150
   Leu Ser Pro Arg Pro Val Ser Tyr Leu Lys Gly Ser Ser Gly Gly Pro
           1155                1160                1165
   Leu Leu Cys Pro Ser Gly His Ala Val Gly Ile Phe Arg Ala Ala Val
       1170                1175                1180
   Cys Thr Arg Gly Val Ala Lys Ala Val Asp Phe Val Pro Val Glu Ser
   1185                1190                1195                1200
   Met Glu Thr Thr Met Arg Ser Pro Val Phe Thr Asp Asn Ser Ser Pro
                   1205                1210                1215
   Pro Ala Val Pro Gln Thr Phe Gln Val Ala His Leu His Ala Pro Thr
               1220                1225                1230
   Gly Ser Gly Lys Ser Thr Lys Val Pro Ala Ala Tyr Ala Ala Gln Gly
           1235                1240                1245
   Tyr Lys Val Leu Val Leu Asn Pro Ser Val Ala Ala Thr Leu Gly Phe
       1250                1255                1260
   Gly Ala Tyr Met Ser Lys Ala His Gly Val Asp Pro Asn Ile Arg Thr
   1265                1270                1275                1280
   Gly Val Arg Thr Ile Thr Thr Gly Ala Pro Ile Thr Tyr Ser Thr Tyr
                   1285                1290                1295
   Gly Lys Phe Leu Ala Asp Gly Gly Cys Ser Gly Gly Ala Tyr Asp Ile
               1300                1305                1310
   Ile Ile Cys Asp Glu Cys His Ser Thr Asp Ser Thr Ser Ile Leu Gly
           1315                1320                1325
   Ile Gly Thr Val Leu Asp Gln Ala Glu Thr Ala Gly Ala Arg Leu Val
       1330                1335                1340
   Val Leu Ala Thr Ala Thr Pro Pro Gly Ser Val Thr Val Pro His Pro
   1345                1350                1355                1360
   Asn Ile Glu Glu Val Ala Leu Ser Asn Thr Gly Glu Ile Pro Phe Tyr
                   1365                1370                1375
   Gly Lys Ala Ile Pro Ile Glu Thr Ile Lys Gly Gly Arg His Leu Ile
               1380                1385                1390
   Phe Cys His Ser Lys Lys Lys Cys Asp Glu Leu Ala Ala Lys Leu Ser
           1395                1400                1405
   Gly Leu Gly Ile Asn Ala Val Ala Tyr Tyr Arg Gly Leu Asp Val Ser
      1410                1415                1420
   Val Ile Pro Thr Ser Gly Asp Val Val Val Val Ala Thr Asp Ala Leu
   1425                1430                1435                1440
   Met Thr Gly Tyr Thr Gly Asp Phe Asp Ser Val Ile Asp Cys Asn Thr
                   1445                1450                1455
   Cys Val Thr Gln Thr Val Asp Phe Ser Leu Asp Pro Thr Phe Thr Ile
               1460                1465                1470
   Glu Thr Thr Thr Val Pro Gln Asp Ala Val Ser Arg Ser Gln Arg Arg
           1475                1480                1485
   Gly Arg Thr Gly Arg Gly Arg Gly Gly Ile Tyr Arg Phe Val Thr Pro
      1490                1495                1500
   Gly Glu Arg Pro Ser Gly Met Phe Asp Ser Ser Val Leu Cys Glu Cys
   1505                1510                1515                1520
   Tyr Asp Ala Gly Cys Ala Trp Tyr Glu Leu Thr Pro Ala Glu Thr Ser
                   1525                1530                1535
   Val Arg Leu Arg Ala Tyr Leu Asn Thr Pro Gly Leu Pro Val Cys Gln
               1540                1545                1550
   Asp His Leu Glu Phe Trp Glu Ser Val Phe Thr Gly Leu Thr His Ile
           1555                1560                1565
   Asp Ala His Phe Leu Ser Gln Thr Lys Gln Ala Gly Asp Asn Phe Pro
      1570                1575                1580
   Tyr Leu Val Ala Tyr Gln Ala Thr Val Cys Ala Arg Ala Gln Ala Pro
   1585                1590                1595                1600
   Pro Pro Ser Trp Asp Gln Met Trp Lys Cys Leu Ile Arg Leu Lys Pro
                   1605                1610                1615
   Thr Leu His Gly Pro Thr Pro Leu Leu Tyr Arg Leu Gly Ala Val Gln
               1620                1625                1630
   Asn Glu Val Thr Leu Thr His Pro Ile Thr Lys Tyr Ile Met Ala Cys
           1635                1640                1645
   Met Ser Ala Asp Leu Glu Val Val Thr Ser Thr Trp Val Leu Val Gly
      1650                1655                1660
   Gly Val Leu Ala Ala Leu Ala Ala Tyr Cys Leu Thr Thr Gly Ser Val
   1665                1670                1675                1680
   Val Ile Val Gly Arg Ile Ile Leu Ser Gly Lys Pro Ala Ile Ile Pro
                   1685                1690                1695
   Asp Arg Glu Val Leu Tyr Arg Glu Phe Asp Glu Met Glu Glu Cys Ala
               1700                1705                1710
   Ser His Leu Pro Tyr Ile Glu Gln Gly Met Gln Leu Ala Glu Gln Phe
           1715                1720                1725
   Lys Gln Lys Ala Leu Gly Leu Leu Gln Thr Ala Thr Lys Gln Ala Glu
      1730                1735                1740
   Ala Ala Ala Pro Val Val Glu Ser Lys Trp Arg Thr Leu Glu Ala Phe
   1745 1750 1755 1760
   Trp Ala Lys His Met Trp Asn Phe Ile Ser Gly Ile Gln Tyr Leu Ala
   1765 1770 1775
   Gly Leu Ser Thr Leu Pro Gly Asn Pro Ala Ile Ala Ser Leu Met Ala
   1780 1785 1790
   Phe Thr Ala Ser Ile Thr Ser Pro Leu Thr Thr Gln His Thr Leu Leu
   1795 1800 1805
   Phe Asn Ile Leu Gly Gly Trp Val Ala Ala Gln Leu Ala Pro Pro Ser
   1810 1815 1820
   Ala Ala Ser Ala Phe Val Gly Ala Gly Ile Ala Gly Ala Ala Val Gly
   1825 1830 1835 1840
   Ser Ile Gly Leu Gly Lys Val Leu Val Asp Ile Leu Ala Gly Tyr Gly
   1845 1850 1855
   Ala Gly Val Ala Gly Ala Leu Val Ala Phe Lys Val Met Ser Gly Asp
   1860 1865 1870
   Met Pro Ser Thr Glu Asp Leu Val Asn Leu Leu Pro Ala Ile Leu Ser
   1875 1880 1885
   Pro Gly Ala Leu Val Val Gly Val Val Cys Ala Ala Ile Leu Arg Arg
   1890 1895 1900
   His Val Gly Pro Gly Glu Gly Ala Val Gln Trp Met Asn Arg Leu Ile
   1905 1910 1915 1920
   Ala Phe Ala Ser Arg Gly Asn His Val Ser Pro Thr His Tyr Val Pro
   1925 1930 1935
   Glu Ser Asp Ala Ala Ala Arg Val Thr Gln Ile Leu Ser Ser Leu Thr
   1940 1945 1950
   Ile Thr Gln Leu Leu Lys Arg Leu His Gln Trp Ile Asn Glu Asp Cys
   1955 1960 1965
   Ser Thr Pro Cys Ser Gly Ser Trp Leu Arg Asp Val Trp Asp Trp Ile
   1970 1975 1980
   Cys Thr Val Leu Ser Asp Phe Lys Thr Trp Leu Gln Ser Lys Leu Leu
   1985 1990 1995 2000
   Pro Arg Leu Pro Gly Val Pro Phe Leu Ser Cys Gln Arg Gly Tyr Lys
   2005 2010 2015
   Gly Val Trp Arg Gly Asp Gly Val Met Gln Thr Thr Cys Pro Cys Gly
   2020 2025 2030
   Ala Gln Ile Ala Gly His Val Lys Asn Gly Ser Met Arg Ile Val Gly
   2035 2040 2045
   Pro Lys Thr Cys Ser Asn Thr Trp His Gly Thr Phe Pro Ile Asn Ala
   2050 2055 2060
   Tyr Thr Thr Gly Pro Cys Thr Pro Ser Pro Ala Pro Asn Tyr Ser Arg
   2065 2070 2075 2080
   Ala Leu Trp Arg Val Ala Ala Glu Glu Tyr Val Glu Ile Thr Arg Val
   2085 2090 2095
   Gly Asp Phe His Tyr Val Thr Gly Met Thr Ala Asp Asn Val Lys Cys
   2100 2105 2110
   Pro Cys Gln Val Pro Ala Pro Glu Phe Phe Thr Glu Leu Asp Gly Val
   2115 2120 2125
   Arg Leu His Arg Tyr Ala Pro Ala Cys Lys Pro Leu Leu Arg Asp Glu
   2130 2135 2140
   Val Thr Phe Gln Val Gly Leu Asn Gln Tyr Pro Val Gly Ser Gln Leu
   2145 2150 2155 2160
   Pro Cys Glu Pro Glu Pro Asp Val Thr Val Val Thr Ser Met Leu Thr
   2165 2170 2175
   Asp Pro Ser His Ile Thr Ala Glu Thr Ala Lys Arg Arg Leu Ala Arg
   2180 2185 2190
   Gly Ser Pro Pro Ser Leu Ala Ser Ser Ser Ala Ser Gln Leu Ser Ala
   2195 2200 2205
   Pro Ser Leu Lys Ala Thr Cys Thr Thr His His Asp Ser Pro Asp Ala
   2210 2215 2220
   Glu Leu Ile Glu Ala Asn Leu Leu Trp Arg Gln Glu Met Gly Gly Asn
   2225 2230 2235 2240
   Ile Thr Arg Val Glu Ser Glu Asn Lys Val Val Ile Leu Asp Ser Phe
   2245 2250 2255
   Asp Pro Leu Arg Ala Glu Glu Asp Glu Arg Glu Val Ser Val Ala Ala
   2260 2265 2270
   Glu Ile Leu Arg Lys Thr Arg Lys Phe Pro Pro Ala Met Pro Ile Trp
   2275 2280 2285
   Ala Arg Pro Asp Tyr Asn Pro Pro Leu Ile Glu Ser Trp Arg Asp Pro
   2290 2295 2300
   Asp Tyr Val Pro Pro Val Val His Gly Cys Pro Leu Pro Pro Ala Lys
   2305 2310 2315 2320
   Thr Pro Pro Ile Pro Pro Pro Arg Arg Lys Arg Thr Val Val Leu Thr
   2325 2330 2335
   Glu Ser Thr Val Ser Ser Ala Leu Ala Glu Leu Ala Thr Lys Ser Phe
   2340 2345 2350
   Gly Ser Ser Gly Ser Ser Ala Val Asp Ser Gly Thr Ala Thr Ala Pro
   2355 2360 2365
   Pro Asp Gln Pro Leu Asp Ser Gly Asp Thr Gly Ser Asp Val Glu Ser
   2370 2375 2380
   Tyr Ser Ser Met Pro Pro Leu Glu Gly Glu Pro Gly Asp Pro Asp Leu
   2385 2390 2395 2400
   Ser Asp Gly Ser Trp Ser Thr Val Ser Glu Glu Ala Gly Glu Asp Val
   2405 2410 2415
   Val Cys Cys Ser Met Ser Tyr Thr Trp Thr Gly Ala Leu Ile Thr Pro
   2420 2425 2430
   Cys Ala Ala Glu Glu Ser Lys Leu Pro Ile Asn Ala Leu Ser Asn Ser
   2435 2440 2445
   Leu Leu Arg His His Asn Met Val Tyr Ala Thr Thr Ser Arg Ser Ala
   2450 2455 2460
   Ser Gln Arg Gln Lys Lys Val Thr Phe Asp Arg Leu Gln Val Leu Asp
   2465 2470 2475 2480
   Asp His Tyr Arg Asp Val Leu Lys Glu Met Lys Ala Lys Ala Ser Thr
   2485 2490 2495
   Val Lys Ala Lys Leu Leu Ser Val Glu Glu Ala Cys Lys Leu Thr Pro
   2500 2505 2510
   Pro His Ser Ala Lys Ser Lys Phe Gly Tyr Gly Ala Lys Asp Val Arg
   2515 2520 2525
   Asn Leu Ser Ser Lys Ala Val Asn His Ile His Ser Val Trp Lys Asp
   2530 2535 2540
   Leu Leu Glu Asp Ala Glu Thr Pro Ile Asp Thr Thr Ile Met Ala Lys
   2545 2550 2555 2560
   Asn Glu Val Phe Cys Val Gln Pro Glu Lys Gly Gly Arg Lys Pro Ala
   2565 2570 2575
   Arg Leu Ile Val Phe Pro Asp Leu Gly Val Arg Val Cys Glu Lys Met
   2580 2585 2590
   Ala Leu Tyr Asp Val Val Ser Thr Leu Pro Gln Ala Val Met Gly Ser
   2595 2600 2605
   Ser Tyr Gly Phe Gln Tyr Ser Pro Gly Gln Arg Val Glu Phe Leu Val
   2610 2615 2620
   Asn Ala Trp Lys Ser Lys Lys Thr Pro Met Gly Phe Ala Tyr Asp Thr
   2625 2630 2635 2640
   Arg Cys Phe Asp Ser Thr Val Thr Glu Asn Asp Ile Arg Val Glu Glu
   2645 2650 2655
   Ser Ile Tyr Gln Cys Cys Asp Leu Ala Pro Glu Ala Arg Gln Ala Ile
   2660 2665 2670
   Arg Ser Leu Thr Glu Arg Leu Tyr Ile Gly Gly Pro Leu Thr Asn Ser
   2675 2680 2685
   Lys Gly Gln Asn Cys Gly Tyr Arg Arg Cys Arg Ala Ser Gly Val Leu
   2690 2695 2700
   Thr Thr Ser Cys Gly Asn Thr Leu Thr Cys Tyr Leu Lys Ala Ser Ala
   2705 2710 2715 2720
   Ala Cys Arg Ala Ala Lys Leu Gln Asp Cys Thr Met Leu Val Cys Gly
   2725 2730 2735
   Asp Asp Leu Val Val Ile Cys Glu Ser Ala Gly Thr Gln Glu Asp Ala
   2740 2745 2750
   Ala Ser Leu Arg Val Phe Thr Glu Ala Met Thr Arg Tyr Ser Ala Pro
   2755 2760 2765
   Pro Gly Asp Pro Pro Arg Pro Glu Tyr Asp Leu Glu Leu Ile Thr Ser
   2770 2775 2780
   Cys Ser Ser Asn Val Ser Val Ala His Asp Ala Thr Gly Lys Arg Val
   2785 2790 2795 2800
   Tyr Tyr Leu Thr Arg Asp Pro Thr Thr Pro Leu Ala Arg Ala Ala Trp
   2805 2810 2815
   Glu Thr Ala Arg His Thr Pro Val Asn Ser Trp Leu Gly Asn Ile Ile
   2820 2825 2830
   Met Tyr Ala Pro Thr Leu Trp Ala Arg Met Ile Leu Met Thr His Phe
   2835 2840 2845
   Phe Ser Ile Leu Leu Ala Gln Glu Gln Leu Glu Lys Ala Leu Asp Cys
   2850 2855 2860
   Gln Ile Tyr Gly Ala Thr Tyr Ser Ile Glu Pro Leu Asp Leu Pro Gln
   2865 2870 2875 2880
   Ile Ile Gln Arg Leu His Gly Leu Ser Ala Phe Ser Leu His Ser Tyr
   2885 2890 2895
   Ser Pro Gly Glu Ile Asn Arg Val Ala Ser Cys Leu Arg Lys Leu Gly
   2900 2905 2910
   Val Pro Pro Leu Arg Val Trp Arg His Arg Ala Arg Ser Val Arg Ala
   2915 2920 2925
   Lys Leu Leu Ser Gln Gly Gly Arg Ala Ala Ile Cys Gly Lys Tyr Leu
   2930 2935 2940
   Phe Asn Trp Ala Val Arg Thr Lys Leu Lys Leu Thr Pro Ile Pro Ala
   2945 2950 2955 2960
   Ala Ser Arg Leu Asp Leu Ser Gly Trp Phe Val Ala Gly Tyr Ser Gly
   2965 2970 2975
   Gly Asp Ile Tyr His Ser Leu Ser Arg Ala Arg Pro Arg Trp Phe Met
   2980 2985 2990
   Trp Cys Leu Leu Leu Leu Ser Val Gly Val Gly Ile Tyr Leu Leu Pro
   2995 3000 3005
   Asn Arg
   3010
<210> 15
   <211> 20
   <212> DNA
   <213> Artificial Sequence
<220>
   <223> Description of Artificial Sequence: primer
<400> 15
   tgctcatggt gcacggtcta 20
<210> 16
   <211> 20
   <212> DNA
   <213> Artificial Sequence
<220>
   <223> Description of Artificial Sequence: primer
<400> 16
   agagccatag tggtctgcgg 20
<210> 17
   <211> 20
   <212> DNA
   <213> Artificial Sequence
<220>
   <223> Description of Artificial Sequence: primer
<400> 17
   ctttcgcgac ccaacactac 20
<210> 18
   <211> 18
   <212> DNA
   <213> Artificial Sequence
<220>
   <223> Description of Artificial Sequence: primer
<400> 18
   accttccacg agacgctt 18
<210> 19
   <211> 22
   <212> DNA
   <213> Artificial Sequence
<220>
   <223> Description of Artificial Sequence: primer
<400> 19
   caggatcatc tcgaagatca tg 22
<210> 20
   <211> 18
   <212> DNA
   <213> Artificial Sequence
<220>
   <223> Description of Artificial Sequence: primer
<400> 20
   ggtgcggcgg tgatgatg 18
<210> 21
   <211> 21
   <212> DNA
   <213> Artificial Sequence
<220>
   <223> Description of Artificial Sequence: primer
<400> 21
   cccagagtcg gagttgttga g 21
<210> 22
   <211> 21
   <212> DNA
   <213> Artificial Sequence
<220>
   <223> Description of Artificial Sequence: primer
<400> 22
   ggggtgcgat atttcttctt g 21
<210> 23
   <211> 21
   <212> DNA
   <213> Artificial Sequence
<220>
   <223> Description of Artificial Sequence: primer
<400> 23
   gagcctgacc aaattcgtac c 21
<210> 24
   <211> 20
   <212> DNA
   <213> Artificial Sequence
<220>
   <223> Description of Artificial Sequence: primer
<400> 24
   ttcacttcta caaatggacc 20
<210> 25
   <211> 20
   <212> DNA
   <213> Artificial Sequence
<220>
   <223> Description of Artificial Sequence: primer
<400> 25
   tagcctccgt agccatagcc 20
<210> 26
   <211> 22
   <212> DNA
   <213> Artificial Sequence
<220>
   <223> Description of Artificial Sequence: primer
<400> 26
   gactcatgac cacagtccat gc 22
<21C> 27
   <211> 22
   <212> DNA
   <213> Artificial Sequence
<220>
   <223> Description of Artificial Sequence: primer
<400> 27
   gaggagacca cctggtgctc ag 22

## Description

### Technical Field

The present invention relates to a novel HCV replicon-replicating cell, a novel full-length HCV RNA-replicating cell, and use of these cells. More specifically, the invention relates to a HCV RNA replication system and a HCV particle production system using a novel HCV replicon-replicating cell and a novel full-length HCV RNA-replicating cell.

### BACKGROUND ART

Hepatitis C virus (hereinafter, "HCV") is an RNA virus of the family *Flaviviridae,* discovered and identified as a causative virus of non-A non-B hepatitis in 1989. Because HCV is a virus that establishes persistent infection, the hepatitis (hepatitis C) caused by HCV infection develops into chronic hepatitis with high probability. It has been elucidated that the hepatitis leads to cirrhosis over the time span of some 20 years, before finally developing into hepatocellular carcinoma.

The estimated number of HCV-infected patients is about two million in Japan alone, and about two hundred million worldwide. The figure becomes even greater with large numbers of so-called asymptomatic carriers unaware of being infected with HCV. This has become a matter of social concern, as seen in the incidence of HCV infection caused by fibrinogen preparations. Currently, the victims of hepatocellular carcinoma in Japan totals about 35,000 per year, about 80% of which is due to HCV infection. The preceding cirrhosis victimizes about 20,000 people annually. Indeed, HCV is a virus that causes serious infections.

A system that can reproduce the repeated cycle of infection, replication, particle production, and reinfection following HCV propagation (HCV lifecycle) would be highly useful for the development of anti-HCV techniques. After the discovery of HCV, many attempts have been made to develop an artificial propagation system using cultured cells and animals; however, no practical system is available. For example, the susceptibility of various cultured cell lines to HCV infection has been described by Kato et al., Biochem. Biophys. Res. Commun. 206:863-869 (1995). Further, the only model animal of HCV infection is the chimpanzee, and no alternative animal has been found. Use of chimpanzees for drug screening is not practical in terms of scarcity and economy.

In 1999, an HCV replicon system was introduced as a new experiment system that clears the foregoing problems to some extent (see Non-Patent Document 1). An HCV replicon includes HCV genes (non-structural proteins NS3 to NS5B), excluding the genes coding HCV structural proteins. In this system, the HCV subgenome including the NS3 to NS5B regions and the both ends of the genome, essential for HCV genome replication replicates in the cells. The copy number of the HCV subgenome per cell reaches several thousands. Several other HCV strain-derived subgenomic HCV replicon cells have been established afterwards (see, for example, Non-Patent Document 2). For example, Abe et al. established three kinds of genome-length HCV RNA-replicating cell lines (Abe et al., Virus Research 125:88-97 (2007)). In the development of hepatitis C therapeutic drugs, efficacy assessment using such subgenomic HCV replicon systems is necessary, because it is practically impossible to conduct pharmacological tests using large numbers of model animals (chimpanzees).

The influence of HCV structural proteins cannot be assessed in the foregoing subgenomic HCV replicon systems. To overcome this problem, replication systems of full-length HCV genome have been developed, and, thus far, establishment of cells that can replicate the full-length genomes of three HCV strains (N strain, Con-1 strain, and H77 strain) has been reported (full-length HCV RNA replication system; see Non-Patent Documents 3 to 5). Assay systems that can monitor the replication level of HCV genome with a reporter gene are also developed (Non-Patent Document 6, and Patent Document 1). Further, infectious HCV particle-producing cells using JFH1 strain HCV of genotype 2a (HuH-7 cell-derived cloned cells) are established (Non-Patent Document 7).

There is an ongoing global effort directed to developing a specific antiviral agent for HCV using the foregoing techniques (HCV replicon replicating cells, full-length HCV RNA replicating cells, and HCV particle-producing cells that use JFH1 strain HCV).

### Citation List

### Patent Documents

Patent Document 1: Japanese Unexamined Patent Publication No. 2006-325582 (published on December 7, 2006)

### Non-Patent Documents

Non-Patent Document 1: Lohmann et al., Science 285: 110-113 (1999)
Non-Patent Document 2: Kato et al., Biochem. Biophys. Res. Commun. 306: 756-766 (2003)
Non-Patent Document 3: Blight et al., J. Virol. 77: 3181-3190 (2003)
Non-Patent Document 4: Ikeda et al., J. Virol. 76: 2997-3006 (2002)
Non-Patent Document 5: Pietschmann et al., J. Virol. 76: 4008-4021 (2002)
Non-Patent Document 6: Ikeda et al., Biochem. Biophys. Res. Commun. 329: 1350-1359 (2005)
Non-Patent Document 7: Wakita et al., Nat. Med. 11: 791-796 (2005)

### Summary of Invention

### Technical Problem

A specific human hepatoma cell-derived cloned cell line, called HuH-7, is the only cell line that has been used to reproduce the HCV life cycle. It has been elucidated that only a few cell clones among the HuH-7 cells can permit replication of the HCV genome. To verify the results that have been obtained by using HuH-7-derived cells, it is necessary to develop systems that enable reproduction of the HCV life cycle in various cell lines. However, the levels of replication of the HCV replicon and full-length HCV RNA in non-HuH-7 cells are much lower than those in HuH-7 cells, and are not practical for actual use.

The present invention was made in view of the foregoing problems. An object of the present invention is to construct a non-HuH-7 cell-derived HCV life cycle reproduction system that has capabilities equivalent to those of a HuH-7 cell-derived HCV life cycle reproduction system.

### Solution to Problem

The present inventors found that when using specific HCV replicon RNA that is different from the one used to construct the HuH-7 cell-based technique disclosed in Patent Literature 1, the RNA successfully replicates in a specific type of cell that is different from HuH-7 cells. The present invention has been accomplished based on this finding.

A feature of the method of producing an HCV replicon-replicating cell according to the present invention is that the method comprises introducing RNA containing an HCV replicon sequence and a selectable marker gene sequence into a Li23 cell or a cured cell derived from a Li23 cell, and the HCV replicon sequence contains a base sequence encoding an amino acid sequence as set forth in SEQ ID NO: 2 containing amino acid substitutions at Q1112R, K1609E and S2200R or at Q1112R, P1115L and S2200R.

A feature of the method of preparing a full-length HCV RNA-replicating cell is that the method comprises a step of introducing RNA containing an HCV replicon sequence and a selectable marker gene sequence into a cured cell derived from a Li23 cell. The full-length HCV genome sequence may be a base sequence encoding an amino acid sequence as set forth in SEQ ID NO: 2 containing amino acid substitutions at Q1112R, K1609E and S2200R, or at Q1112R, P1115L and S2200R. The base sequence is preferably a sequence as set forth in SEQ ID NO: 7 or 9. The full-length HCV genome sequence may be a base sequence as set forth in SEQ ID NO: 11 or 13.

In the method of producing full-length HCV RNA according to the present invention, the above RNA preferably further contains a reporter gene sequence, and/or preferably further contains an exogenous internal ribosomal entry site (IRES) sequence.

A feature of the screening method according to the present invention is that the method comprises a step of incubating the cell prepared by any one of the above methods with a candidate agent and a step of measuring the level of an HCV gene product or the level of a reporter gene product.

A feature of the method of producing a cured cell according to the present invention is that the method comprises a step of culturing a cell prepared by the above method in a medium containing a pharmaceutical agent having an antiviral action.

A feature of the method of producing an infectious HCV particle according to the present invention is that the method comprises a step of incubating the cured cell with infectious HCV

RNA.

### Advantageous Effects of Invention

According to the present invention, a cell capable of replicating an HCV replicon or a cell capable of replicating full-length HCV genome can be produced, and a cell that permits infection with HCV can also be obtained.

### Brief Description of Drawings

FIG. 1(a) represents the structure of HCV-O strain (genotype 1b) HCV genome.
FIG. 1(b) represents the structure of HCV-O strain-derived HCV replicon RNA (ON/3-5B/QR,KE,SR) that includes two adaptive mutations (Q1112R and K1609E) introduced into the NS3 region, and the adaptive mutation S2200R introduced into the NS5A region.
FIG. 1(c) represents the structure of full-length HCV RNA (ON/C-5B/QR,KE,SR) that includes HCV-O strain-derived C (core) to NS2 inserted between the IRES of EMCV and NS3 in the replicon of FIG. 1(b).
FIG. 1(d) represents the structure of RNA (ORN/C-5B/QR,KE,SR) modified to include RL gene inserted between the HCV IRES and NeoR gene of FIG. 1(c) so as to be produced as a fusion protein.
FIG. 1(e) represents the structure of JFH1 strain (genotype 2a)-derived infectious HCV RNA (JFH1) that originates in a fulminant hepatitis patient, and has the structure of the original HCV genome.
FIG. 2 is a diagram representing the procedure of preparing HCV replicon replicating cells and full-length HCV RNA replicating cells.
FIG. 3(a) is a diagram representing the expression of HCV proteins in HCV replicon replicating cells.
FIG. 3(b) is a diagram representing the effects of anti-HCV agents on the replication of an HCV-O strain replicon in HCV replicon replicating cells.
FIG. 4 is a diagram representing the result of full-length HCV RNA quantification in Li23 cell-derived full-length HCV RNA replicating cells.
FIG. 5 is a diagram representing the effect of HCV protein expression in Li23 cell-derived full-length HCV RNA replicating cells.
FIG. 6 is a diagram representing the results of the detection of double-stranded RNA (dsRNA), a replication intermediate of HCV RNA, in OL8 cells, the positive control O cells, and the negative control Li23 cells, using an immunofluorescent technique with anti-dsRNA antibodies.
FIG. 7 is a diagram representing the effect of IFN-α on the replication of full-length HCV RNA in Li23 cell-derived full-length HCV RNA replicating cells.
FIG. 8 is a diagram representing the result of gene analysis for HCV replicated in Li23 cell-derived full-length HCV RNA replicating cells.
FIG. 9 is a diagram representing the procedure of preparing reporter gene-carrying HCV replicon-replicating cells derived from Li23 cells, and reporter gene-carrying full-length HCV RNA-replicating cells, shown with the results obtained from these cells.
FIG. 10(a) is a diagram representing the result of full-length HCV RNA quantification in cloned ORL8 cells.
FIG. 10(b) is a diagram representing the result of full-length HCV RNA quantification in cloned ORL11 cells.
FIG. 11 is a diagram representing the result of gene analysis for HCV replicated in ORL8 cells.
FIG. 12 is a diagram representing the result of gene analysis for HCV replicated in ORL11 cells.
FIG. 13 is a diagram representing HCV protein expression in ORL8 cells and ORL11 cells.
FIG. 14 is a diagram representing the correlation between luciferase activity and HCV RNA level in ORL8 cells and ORL11 cells.
FIG. 15 is a diagram representing the time-dependent anti-HCV activity of IFN-α, using ORL8 cells and ORL11 cells.
FIG. 16 is a diagram comparing the anti-HCV activities of IFN-α using ORL8, ORL11, and OR6 cells.
FIG. 17 is a diagram comparing the anti-HCV activities of IFN-α using sORL8 (pool) cells and sORL11 (pool) cells.
FIG. 18 is a diagram comparing the anti-HCV activities of IFN-β using ORL8, ORL11, and OR6 cells.
FIG. 19 is a diagram comparing the anti-HCV activities of IFN-γ using ORL8, ORL11, and OR6 cells.
FIG. 20 is a diagram comparing the anti-HCV activities of Cyclosporin A (CsA) using ORL8, ORL11, and OR6 cells.
FIG. 21 is a diagram comparing the anti-HCV activities of fluvastatin (FLV) using ORL8, ORL11, and OR6 cells.
FIG. 22 is a diagram comparing the anti-HCV activities of fluvastatin (FLV) using ORL8, ORL11, and OR6 cells.
FIG. 23 is a diagram comparing the anti-HCV activities of simvastatin (SMV) using ORL8, ORL11, and OR6 cells.
FIG. 24 is a diagram comparing the anti-HCV activities of lovastatin (LOV) using ORL8, ORL11, and OR6 cells.
FIG. 25 is a diagram comparing the anti-HCV activities of pitavastatin (PTV) using ORL8, ORL11, and OR6 cells.
FIG. 26 is a diagram comparing the anti-HCV activities of ribavirin (RBV) using ORL8, ORL11, and OR6 cells.
FIG. 27 is a diagram comparing the anti-HCV activities of mizoribine using ORL8, ORL11, and OR6 cells.
FIG. 28 is a diagram comparing the anti-HCV activities of geldanamycin using ORL8, ORL11, and OR6 cells.
FIG. 29 is a diagram comparing the anti-HCV activities of myriocin using ORL8, ORL11, and OR6 cells.
FIG. 30 is a diagram comparing the anti-HCV activities of acetylsalicylic acid (ASA) using ORL8, ORL11, and OR6 cells.
FIG. 31 is a diagram comparing anti-HCV activities by the combined use of IFN-α and CsA using ORL8, ORL11, and OR6 cells.
FIG. 32 is a diagram comparing anti-HCV activities by the combined use of IFN-α and FLV using ORL8, ORL11, and OR6 cells.
FIG. 33 is a diagram comparing anti-HCV activities by the combined use of IFN-α and FLV using ORL8 cells and OR6 cells.
FIG. 34 is a diagram comparing anti-HCV activities by the combined use of IFN-α and FLV using ORL11 cells and OR6 cells.
FIG. 35 is a diagram comparing anti-HCV activities by the combined use of IFN-α and FLV using ORL8 cells and ORL11 cells.
FIG. 36 is a diagram comparing anti-HCV activities by the combined use of IFN-α and FLV using ORL8, ORL11, and OR6 cells.
FIG. 37 is a diagram comparing anti-HCV activities by the combined use of IFN-α and ribavirin (RBV) using ORL8, ORL11, and OR6 cells.
FIG. 38 is a diagram representing the results of the examination of HCV core protein expression in JFH1 strain HCV RNA-introduced Li23 cells, OL8c cells, and OL11c cells.
FIG. 39 is a diagram representing the results of JFH1 strain HCV infection experiment for Li23 cells and OL8c cells.
FIG. 40 is a diagram representing the results of JFH1 strain HCV infection experiment for the clones of various OL cured cells.
FIG. 41 is a diagram representing the results of the examination of infectious HCV particle production from JFH1 strain HCV-infected OL8c and OL11c cells.
FIG. 42 is a diagram representing the results of the examination of infectious HCV particle production from JFH11 strain HCV-infected OL8c cells.
FIG. 43 is a diagram representing the procedure of preparing cured cells by the IFN-γ treatment of OL8c cells and OL11c cells, and the results obtained from these cured cells.
FIG. 44 is a diagram representing the results of the examination of infectious HCV particle production from JFH1 strain HCV-infected ORL8c cells and ORL11c cells.
FIG. 45 is a diagram representing the results of the detection of double-stranded RNA (dsRNA), a replication intermediate of HCV RNA, for ORL8c cells, the positive control JFH1 strain HCV-infected RSc cells, and the negative control mock-infected ORL8c cells, using an immunofluorescent technique with anti-dsRNA antibodies.
FIG. 46(a) is a diagram representing the results of the ELISA measurement of the secretion level of HCV core protein released into the culture supernatants of JFH1 strain HCV-infected ORL8c cells and RSc cells.
FIG. 46(b) is a diagram representing the results of the quantification of HCV RNA level in JFH1 strain HCV-infected ORL8c cells and RSc cells using real-time LightCycler PCR.
FIG. 47(a) is a diagram representing the results of the qualitative comparison of HCV receptor mRNA expression levels in HuH-7 cells, RSc cells, Li23 cells, ORL8c cells, and ORL11c cells.
FIG. 47(b) is a diagram representing the results of the quantitative comparison of HCV receptor mRNA expression levels in HuH-7 cells, RSc cells, Li23 cells, ORL8c cells, and ORL11c cells.
FIG. 48(a) represents the results of the analysis of the expression levels of 1B-4 strain core protein and NS5A protein by Western blotting, confirming the establishment of a cell line capable of replicating full-length HCV RNA derived from r.on-HCV-O HCV strains.
FIG. 48(b) represents the results of the analysis of the expression levels of KAH5 strain core protein and NS5A protein by Western blotting, confirming the establishment of a cell line capable of replicating full-length HCV RNA derived from non-HCV-O HCV strains.
FIG. 49 is a diagram representing the correlation between luciferase activity and HCV RNA level in cell lines capable of replicating full-length HCV RNA derived from non-HCV-O HCV strains.

### Description of Embodiments

### [1] HCV replicon-replicating cell

The present disclosure provides an HCV replicon-replicating cell. A feature of the HCV replicon-replicating cell according to the present disclosure is that the cell is produced by introducing HCV replicon RNA having specific adaptive mutations into a specific cell. As used herein, the term "HCV replicon" is interchangeable with the term "subgenomic HCV replicon". These terms refer to a structural gene comprising the NS3-to-NS5B region of the HCV genome sequence.

A mutation present in HCV ORF may enhance the intracellular replication efficiency of the HCV genome. A mutation having this effect is known as an "adaptive mutation". A large number of HCV adaptive mutations are known. However, what adaptive mutations are suitable for what conditions is unknown. The present inventors have already established an HCV life cycle reproduction system derived from the HuH-7 cell line (see Patent Document 1). To construct an HCV life cycle reproduction system derived from a non-HuH-7 cell line, the present inventors tried to introduce HCV replicon RNA into various non-HuH-7 cell lines (for example, human hepatoma cell lines, human immortalized liver cell lines, human cholangiocarcinoma cell lines), but were not able to produce a desired transformant. However, as a result of trial-and-error experiments conducted from a unique viewpoint, the present inventors found that when using an HCV replicon sequence that comprises the NS3-to-NS5 region of the HCV genome and contains specific adaptive mutations (Q1112R and K1609E or Q1112R and P1115L) in the NS3 region and a specific adaptive mutation (S2200R) in the NS5A region, the target RNA can be introduced into a human hepatoma cell line, Li23. The combination of adaptive mutations that can be used in the present invention are a combination of "Q1112R, K1609E and S2200R", or a combination of "Q1112R, P1115L and S2200R". When using two of the three mutations in each of the above combinations or using a combination different from the above combinations (for example, "P1115L, K1609E and S2200R", "Q1112R, E1202G and S2200R", or "E1202G, K1609E and S2200R"), the present invention could not be accomplished. Furthermore, even when using an HCV replicon sequence having such a specific combination of adaptive mutations, the target RNA could not be introduced into cells other than Li23 cells.

A feature of the HCV replicon-replicating cell according to one embodiment of the present disclosure is that a human hepatoma cell line, Li23, is used as the parent cell and the HCV replicon-replicating cell is prepared by introducing RNA containing an HCV replicon sequence (containing adaptive mutations at Q1112R, K1609E and S2200R or at Q1112R, P1115L and S2200R) and a selectable marker gene sequence into the parent cell.

The RNA to be introduced into the cell according to the present invention is not particularly limited insofar as the RNA contains a selectable marker gene sequence and an HCV replicon sequence. There is no limitation on the selectable marker gene, but drug resistance genes are preferable because of convenience. The drug resistance gene is not particularly limited, and may be suitably selected from known drug resistance genes that can be used for the selection of transformed cells. Specific examples thereof include neomycin resistance genes (neomycin phosphotransferase genes), puromycin resistance genes, blasticidin resistance genes, hygromycin resistance genes, and the like. The HCV replicon sequence is preferably a base sequence as set forth in SEQ ID NO: 3 or 5, and may further contain mutations. In this case, the mutations are not limited to adaptive mutations.

The order of the sequences in RNA introduced into the cell of the present disclosure is not particularly limited, insofar as a selectable marker gene product and a protein encoded by the HCV replicon sequence can be expressed. The RNA preferably contains two IRESs, which are an IRES for translation of the selectable marker gene and an IRES for translation of the ORF of HCV, thereby maintaining a high level of the translated protein. Although both of the IRESs may be derived from HCV, at least one of the IRESs is preferably a foreign IRES. The mode of HCV genome replication can be maintained by using a foreign IRES. There is no particular limitation on the foreign IRES, and examples thereof include an IRES derived from encephalomyocarditis virus (EMCV), bovine viral diarrhea virus (BVDV) IRES, poliovirus IRES, and the like. EMCV IRES is preferable because of its high activity and wide use.

One example of the order of the sequences in RNA introduced into the cell of the present disclosure is, from the 5' end, the HCV IRES sequence, the selectable marker gene sequence, the foreign IRES sequence, the HCV ORF sequence, and the HCV3' untranslated sequence. However, this example is not limitative. The HCV IRES is an RNA comprising a 5'-untranslated region and a part of the core on the 5' side. For example, the region from positions 1 to 377 (wherein the 5'-untranslated region is at positions 1 to 341) of the base sequence of the HCV-O strain as set forth in SEQ ID NO: 1 is used in the Examples below. However, this example is not limitative.

The HCV genome sequence contained in RNA introduced into the cell of the present disclosure may be any sequence derived from HCV. HCV includes attenuated strains and mutant strains as well as pathogenic strains that cause hepatitis C. Although HCV has many genotypes, a sequence derived from any genotype of HCV may be used. Since about 70% of hepatitis C patients in Japan are infected with HCV genotype 1b, genotype 1b is preferable.

Examples of known HCV genotype 1b strains include the HCV-O strain, N strain, Con-1 strain, JT strain, and the like. The present inventors produced the cell of the present disclosure by using genomic RNA of the HCV-O strain. However, the production method is not limited thereto. The base sequence and the amino acid sequence of the HCV-O strain are as set forth in SEQ ID NO: 1 and SEQ ID NO: 2.

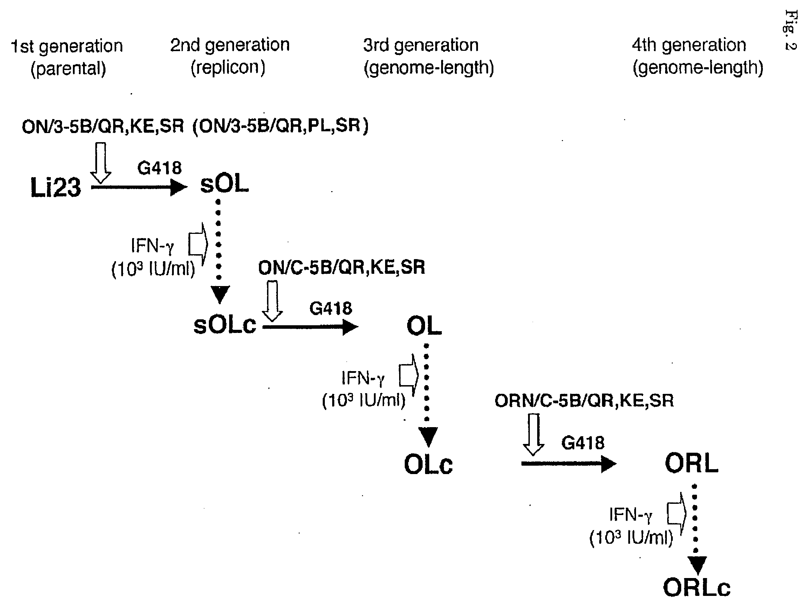

A feature of the HCV replicon-replicating cell according to another embodiment of the present disclosure is that a cured cell of a Li23-derived full-length HCV RNA-replicating cell (described later) is used as a parent cell, and the HCV replicon-replicating cell is prepared by introducing RNA containing an HCV replicon sequence (containing adaptive mutations at Q1112R, K1609E and S2200R or at Q1112R, P1115L and S2200R) and a selectable marker gene sequence into the parent cell. As used herein, the cell line "derived from a Li23 cell" is interchangeable with the "Li23-derived" cell line. These terms refer to a Li23-derived HCV replicon-replicating cell, a Li23-derived full-length HCV RNA-replicating cell, a cured cell of a Li23-derived HCV replicon-replicating cell, or a cured cell of a Li23-derived full-length HCV RNA-replicating cell. The parent cell used according to this embodiment is preferably an OLc cell described later (see Fig. 2), and more preferably an OL8c, OL11c, or OL14c cell.

The "cured cell" as used herein refers to a cell obtained by culturing a subgenomic HCV replicon-replicating cell or a full-length HCV genome-replicating cell in the presence of a pharmaceutical agent having an antiviral action. The "cured cell" indicates a cell from which the subgenomic HCV replicon has been completely removed, or a cell from which the full-length HCV genome has been completely removed. The term "completely removed" means that no HCV RNA and/or HCV protein is expressed in the cell. Persons skilled in the art can easily confirm whether the cell contains HCV-derived RNA by using a method such as RT-PCR or Northern blotting, and can easily confirm whether HCV protein is expressed by using a method such as Western blotting. Such a cured cell is indicated as "sOLc", "OLc", and "ORLc" in Fig. 2, and referred to as a "cured cell derived from a Li23 cell" in the specification. Particularly, "OLc" and "ORLc" are also referred to as "cured cells of Li23-derived full-length HCV RNA-replicating cells".

The pharmaceutical agent having an antiviral action is not particularly limited, insofar as a cured cell can be obtained by adding the agent to a medium. However, the pharmaceutical agent is preferably an agent having an anti-HCV action, more preferably IFN, Cyclosporin A (CsA), or the like, and particularly preferably IFN. Examples of IFN include IFN-α, IFN-β, IFN-γ, and the like. One example of the method of treating the cell using IFN comprises culturing the cell in a medium containing IFN-α in a concentration of 500 IU/ml for 2 weeks. However, the concentration and duration of the treatment may be suitably changed, while confirming whether the desired cured cell has been obtained.

The present invention further provides a method of producing an HCV replicon-replicating cell. A feature of the production method according to one embodiment of the present invention is that the method comprises a step of introducing RNA containing an HCV replicon sequence (containing adaptive mutations at Q1112R, K1609E and S2200R or at Q1112R, P1115L and S2200R) and a selectable marker gene sequence into a Li23 cell. A feature of the production method according to another embodiment of the present invention is that the method comprises a step of introducing RNA containing an HCV replicon sequence (containing adaptive mutations at Q1112R, K1609E and S2200R or at Q1112R, P1115L and S2200R) and a selectable marker gene sequence into a cured cell derived from a Li23 cell.

The cells prepared by the present inventors have been deposited in a depository for Okayama University, the National University Corporation Okayama University Intellectual Property Headquarters (1-1, Tsushima-naka 1-chome, Okayama-shi). The deposit numbers are as shown below.

**[Table 1]**

| Cell Name | Deposit Number |
|---|---|
| Li23 | OP-KITAKU-0001 |
| sOL | OP-KITAKU-0002 |
| OL8 | OP-KITAKU-0003 |
| OL11 | OP-KITAKU-0004 |
| OL14 | OP-KITAKU-0005 |
| sORL8(pool) | OP-KITAKU-0006 |
| sORL11 (pool) | OP-KITAKU-0007 |
| ORL8 | OP-KITAKU-0008 |
| ORL11 | OP-KITAKU-0009 |
| 1B-4RL8 | OP-KITAKU-0010 |
| KAH5RL8 | OP-KITAKU-0011 |
| 1B-4RN/C-5B | OP-KITAKU-0012 |
| OR6 | OP-KITAKU-0013 |

These cells were also deposited at the National Institute of Advanced Industrial Science and Technology, International Patent Organism Depository (Tsukuba Central 6, 1-1-1 Higashi, Tsukuba, Ibaraki, 305-8566, Japan) on July 31, 2008. A request for a transfer to the international deposit was received on July 30, 2009. The accession numbers are as shown below.

**[Table 2]**

| Cell Name | Deposit Number |
|---|---|
| Li23 | FERM ABP-11150 |
| sOL | FERM ABP-11151 |
| OL8 | FERM ABP-11152 |
| OL11 | FERM ABP-11153 |
| OL14 | FERM ABP-11154 |
| sORL8(pool) | FERM ABP-11155 |
| sORL11(pool) | FERM ABP-11156 |
| ORL8 | FERM ABP-11157 |
| ORL11 | FERM ABP-11158 |
| 1B-4RL8 | FERM ABP-11159 |
| KAH5RL8 | FERM ABP-11160 |
| OR6 | FERM ABP-11161 |
| 1B-4RN/C-5B | FERM ABP-11162 |

### [2] Full-length HCV RNA-Replicating Cell

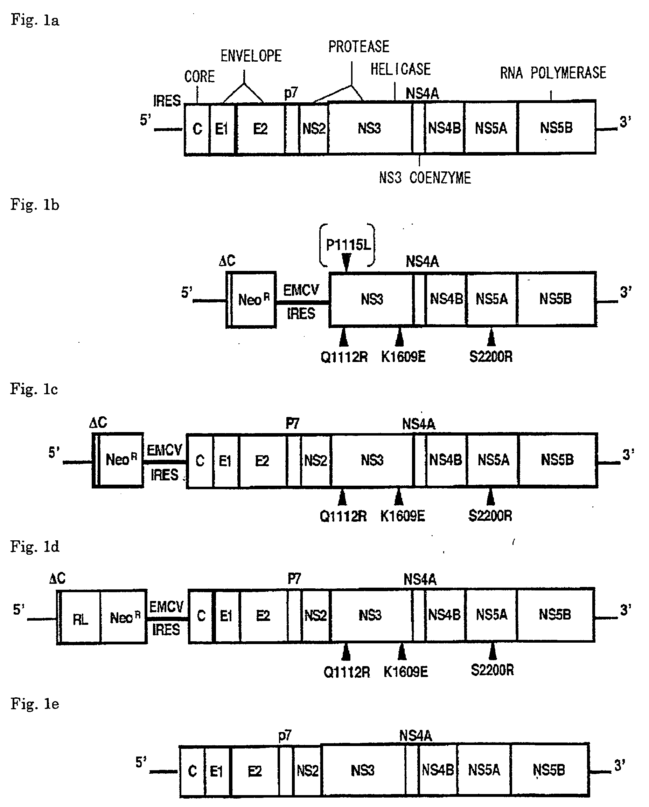

The present disclosure provides a full-length HCV RNA-replicating cell. A feature of the full-length HCV RNA-replicating cell according to the present disclosure is that a full-length HCV genome is introduced into a specific cell. The term "full-length HCV genome" as used herein refers to RNA containing all regions (C region to NS5B region) of the HCV genome shown in Fig. 1 (a), and is interchangeable with "full-length HCV RNA".

The RNA introduced into the full-length HCV RNA-replicating cell according to the present disclosure is not particularly limited insofar as the RNA contains a selectable marker gene sequence and a full-length RNA sequence. Preferably, the RNA further contains a reporter gene because a reporter assay can thereby easily be performed with high sensitivity. There is no particular limitation on the selectable marker gene. However, drug resistance genes are preferable because of convenience. The drug resistance gene is not particularly limited, and can be suitably selected from known drug resistance genes that are usable for the selection of transformed cells. Specific examples thereof include neomycin resistance genes (neomycin phosphotransferase genes), puromycin resistance genes, blasticidin resistance genes, hygromycin resistance genes, and the like. The full-length HCV RNA sequence is preferably a base sequence as set forth in SEQ ID NO: 1, 7, 9, 11, or 13, and may further contain mutations. In this case, the mutations are not limited to adaptive mutations.

The reporter gene that is optionally contained in RNA introduced into the full-length HCV RNA-replicating cell according to the present disclosure is not particularly limited. Examples thereof include luciferase genes, alkaline phosphatase genes, β-lactamase genes, chloramphenicol acetyltransferase genes, and the like. Luciferase genes are preferable. In general, firefly luciferase genes or Renilla luciferase genes are used as luciferase genes. Any of them may be used in the present invention. Renilla luciferase genes are preferable in view of the short length of the gene.

The RNA introduced into the full-length HCV RNA-replicating cell of the present disclosure preferably comprises two IRESs, which are an IRES for translation of a selectable marker gene and an IRES for translation of the ORF of HCV, thereby maintaining a high level of the translated protein. Although both of the IRESs may be derived from HCV, at least one of them is preferably a foreign IRES. The mode of replication of the HCV genome can be maintained by using a foreign IRES. The foreign IRES is not particularly limited, and examples thereof include an IRES from encephalomyocarditis virus (EMCV), bovine viral diarrhea virus (BVDV) IRES, poliovirus IRES, and the like. EMCV IRES is preferable because of its high activity and wide use.

One example of the order of the sequences in RNA introduced into the full-length HCV RNA-replicating cell is, from the 5' end, the HCV IRES sequence, (optionally) the reporter gene sequence, the selectable marker gene sequence, the foreign IRES sequence, the HCV ORF sequence, and the HCV3' untranslated sequence. However, this example is not limitative.

The full-length HCV RNA-replicating cell according to one embodiment of the present disclosure comprises an sOLc cell into which a full-length HCV genome has been introduced. The sOLc cell is obtained by introducing RNA containing an HCV replicon sequence (containing adaptive mutations at Q1112R, K1609E and S2200R) and a selectable marker gene sequence into a Li23 cell to produce an HCV replicon-replicating cell (sOL cell) and culturing the HCV replicon-replicating cell in the presence of a pharmaceutical agent having an antiviral action (see Fig. 2). The full-length HCV RNA-replicating cell according to this embodiment is an OL cell as set forth in Fig. 2. OL1 to OL14 cells are preferable, and OL8, OL11, and OL14 cells are more preferable.

The full-length HCV RNA-replicating cell according to another embodiment of the present disclosure comprises an OLc cell into which a full-length HCV genome has been introduced. The OLc cell is obtained by culturing an OL cell in the presence of a pharmaceutical agent having an antiviral action (see Fig. 2). The full-length HCV RNA-replicating cell according to this embodiment comprises RNA containing a full-length HCV genomic sequence and a selectable marker gene sequence, and is preferably an ORL cell as set forth in Fig. 2 (containing adaptive mutations at Q1112R, K1609E and S2200R). ORL8-1 to ORL8-9 cells and ORL11-1 to ORL11-16 cells are more preferable, and an ORL8-9 cell (hereinafter referred to as an "ORL8 cell") or an ORL11-5 cell (hereinafter referred to as an "ORL11 cell") are particularly preferable.

The full-length HCV RNA-replicating cell according to another embodiment of the present disclosure comprises an ORLc cell into which a full-length HCV genome has been introduced. The ORLc cell is obtained by culturing an ORL cell in the presence of a pharmaceutical agent having an antiviral action (see Fig. 2). The full-length HCV RNA-replicating cell according to this embodiment is produced by introducing RNA containing a full-length HCV genomic sequence and a selectable marker gene sequence. The full-length HCV RNA-replicating cell according to this embodiment permits replication of RNA of the infectious HCV JFH1 strain as well as a novel HCV strain (particularly replication of a long RNA having, for example, a luciferase gene (12 kb)). More specifically, the full-length HCV genomic sequence used in this embodiment may be derived from any HCV strain. The HCV strain to be used is preferably an HCV-O, 1B-4, or KAH-5 strain. The full-length HCV RNA-replicating cell according to this embodiment is preferably a 1B-4RL8 or KAH5RL8 cell. The base sequence (SEQ ID NO: 11) and the amino acid sequence (SEQ ID NO: 12) of the HCV 1B-4 strain, and the base sequence (SEQ ID NO: 13) and the amino acid sequence (SEQ ID NO: 14) of the KAH5 strain have been registered in GenBank under the accession numbers AB442219 and AB442220, respectively.

The full-length HCV RNA-replicating cell according to the present disclosure can replicate a full-length HCV genome, and express a reporter gene product when necessary. The full-length HCV RNA-replicating cell according to the present invention is a cell into which RNA containing a selectable marker gene sequence and a full-length HCV genomic sequence (and optionally a reporter gene sequence) has been introduced, and may be any cell that can replicate a full-length HCV genome, preferably a cell that can express a reporter gene product. When the RNA contains a reporter gene sequence, the expression level of the reporter gene product and the amount of HCV RNA replication in the full-length HCV RNA-replicating cell according to the present invention are very closely correlated with each other. Accordingly, quantification of the reporter gene product enables easy monitoring of the replication level of the full-length HCV genome. Furthermore, the full-length HCV RNA-replicating cell according to the present invention is very useful for functional analysis of HCV, including the effects of structural proteins, which is not possible with a subgenomic HCV replicon. More specifically, the full-length HCV RNA-replicating cell according to the present invention enables easy and quick functional analysis of HCV, screening of substances having anti-HCV action, etc.

The present invention provides a method of producing a full-length HCV RNA-replicating cell. A feature of the production method according to one embodiment of the present invention is that the method comprises a step of introducing into an sOLc cell RNA containing a full-length HCV genomic sequence and a selectable marker gene sequence. The RNA may further contain a reporter gene sequence. A feature of the production method according to another embodiment of the present invention is that the method comprises introducing into an OLc cell RNA containing a full-length HCV genomic sequence and a selectable marker gene sequence. The RNA may further contain a reporter gene sequence. A feature of the production method according to another embodiment of the present invention is that the method comprises introducing into an ORLc cell RNA containing a full-length HCV genomic sequence and a selectable marker gene sequence. The RNA may further contain a reporter gene sequence.

### [3] Screening method

The present invention provides a method of screening a substance having an anti-HCV action. The screening method according to the present invention is not particularly limited, insofar as the method includes a step of incubating the cell of the present invention with a candidate agent. When the RNA introduced into the above cell contains a reporter gene sequence, the method may further include a step of measuring the level of a reporter gene product. When RNA introduced into the cell does not contain a reporter gene sequence, the method may further include a step of measuring the level of an HCV gene product. The measured levels may be compared with preset reference values. More preferably, the reporter gene product level or the HCV gene product level achieved by not incubating the cell with a candidate agent is measured, and is compared with the corresponding level achieved by incubating the cell with a candidate agent. This screening method enables easy and quick screening of a large number of test substances.

In the screening method according to the present invention, the anti-HCV action is an inhibitory effect on the replication of a full-length HCV genome. Thus, the screening method according to the present invention is a method of screening a substance having an inhibitory action on the replication of a full-length HCV genome.

The screening method according to the present invention can screen a substance that suppresses the replication of viruses closely related to HCV, and viruses whose mode of replication is similar to that of HCV. Examples of viruses closely related to HCV include viruses belonging to the *Pestivirus* genus and the *Flavivirus* genus of the *Flaviviridae* family. Examples of viruses belonging to the *Flavivirus* genus include Japanese encephalitis virus, yellow fever virus, West Nile virus, and the like. Examples of viruses belonging to the *Pestivirus* genus include hog colera virus, bovine viral diarrhea virus, and the like.

Further, the screening method according to the present invention enables the screening of a substance that suppresses the replication of a virus whose mode of replication is similar to that of HCV. A feature of HCV replication is that the replication occurs entirely within the cytoplasm, and no viral genome is present in the nucleus. Accordingly, the screening method according to the present invention can screen a substance that suppresses the replication of a virus whose mode of replication is as described above.

The test substance and the cell can be brought into contact with each other by dissolving or suspending the test substance in a medium. Accordingly, the test substance may be any material that can be dissolved or suspended in a medium.

The method of measuring the level of a reporter gene product can be selected from known methods according to the reporter gene used. For example, when a luciferase gene is used as a reporter gene, a cell lysate prepared by dissolving the cell in a buffer containing a surfactant or the like is used as a test sample, and the amount of emission may be measured by an apparatus, such as a luminometer. In this measurement, commercially available luciferase assay reagents and luciferase assay kits can be suitably used.

Whether the test substance has an anti-HCV action or not can be determined by comparing the thus obtained level of the reporter gene product with the level of a reporter gene product in a cell not brought into contact with the test substance. Further, if the level of the reporter gene product in the cell brought into contact with a test substance in a concentration that does not reduce the cell growth potential is lower than that in the cell not brought into contact with the test substance, the test substance is evaluated as having an anti-HCV action. Preferably, when the level is not more than 50%, and more preferably 10% or less, the test substance is evaluated as having an anti-HCV action.

A more preferable criterion is the level obtained by applying IFN-α, to the screening method according to the present invention, or the level obtained by applying IFN-α and ribavirin to the screening method according to the present invention, both being standard methods for chronic hepatitis C treatment. A highly useful therapeutic agent for hepatitis C can be found if the HCV replication level achieved by the therapeutic agent is lower than the levels achieved by pharmaceuticals used in the current standard therapeutic method for hepatitis C.

The screening method according to the present invention enables at least selection of candidates for the active ingredient of a hepatitis C therapeutic agent. Since the only animal model for HCV infection is the chimpanzee, it is currently impossible to perform a pharmacological test using a large number of animals. It is thus expected that the screening method according to the present invention will be a vital method for the evaluation of drug efficacy in the development of a therapeutic agents for hepatitis C.

### [4] Screening Kit

The present invention provides a screening kit for screening a substance having an anti-HCV action. The screening kit according to the present invention is not particularly limited, insofar as the kit contains the cell of the present invention. When RNA introduced into the cell contains a reporter gene sequence, the kit may further contain a reagent for measuring the level of a reporter gene product. When RNA introduced into the cell does not contain a reporter gene sequence, the kit may further contain a reagent for measuring the level of an HCV gene product. This screening kit enables simple and effective implementation of the screening method according to the present invention.

The term "kit" as used herein refers to a package (e.g., a bottle, a plate, a tube, a dish, or the like) containing a specified material, and includes instructions for use of the specified material. The instructions may be written or printed on paper or other media, or committed to electronic media such as magnetic tape, computer-readable disks or tape, CD-ROM, and the like.

The screening kit according to the present invention may further contain items other than the cell of the present invention. Such components of the kit other than the cell are not particularly limited; necessary reagents, apparatuses, etc., may be selectively incorporated as components of the kit.

A person skilled in the art who reads this specification will easily understand that the screening kit according to the present invention can be used in the same manner as the screening method of the present invention described above.

### [5] Method of producing an infectious HCV particle

The present invention provides a method of producing an infectious HCV particle. The method of producing an infectious HCV particle according to the present invention may be any method comprising a step of introducing infectious HCV RNA into a cell (a "cured cell derived from a Li23 cell") obtained by culturing the cell of the present invention in a medium containing a pharmaceutical agent having an antiviral action, or a step of incubating the cell with infectious HCV. Examples of cells preferably used in the method of the present invention include, but are not limited to, ORL8c and ORL11c cells.

### Examples

### 1: Reagents and Procedures

### Reagents Used

Fluvastatin was purchased from Calbiochem and LKT Laboratories. IFN-α, IFN-β, and IFN-γ were purchased from Sigma. Cyclosporin A (CsA), myriocin, and acetylsalicylic acid were purchased from Sigma. Pravastatin, simvastatin, lovastatin, and geldanamycin were purchased from Wako chemical. Pitavastatin was purchased from Tronto Research. Ribavirin was purchased from Yamasa. Mizoribine was provided by Asahi Kasei.

### Li23 cells

The human hepatoma cell line Li23 was cultured using 500 ml of F-12 medium (Invitrogen 11765-054) and 500 ml of D-MEM medium (Sigma D5796) (a total volume of 1 L), with addition of the following materials.

| Materials Added (Group A) | Final Concentration |
|---|---|
| EGF (Toyobo EGF-201) | 50 ng/ml |
| Insulin (Sigma I-6634) | 10 µg/ml |
| Hydrocortison (Sigma H-0888) | 0.36 µg/ml |
| Transferrin (Sigma T-2252) | 5 µg/ml |
| Linoleic acid (Sigma L-1012) | 5 µg/ml |
| Selenium (Sigma S-9133) | 20 ng/ml |
| Prolactin (Sigma L6520) | 10 ng/ml |
| FBS (Biological Industries 04-001-1A) | 1% (v/v) |

| Material Added (Group B) | Final Concentration |
|---|---|
| Gentamycin (Invitrogen 15750-060) | 10 µg/ml |
| Kanamycun-monosulfate (Sigma K-4000) | 0.2 mg/ml |
| Fungizone (IBL 33605) | 0.5 µg/ml |

FBS was used after 30-min incubation at 56°C. The cell line Li23 shows EGF-dependent proliferation, and does not easily proliferate under the HuH-7 cell medium conditions (10% Fetal Bovine Serum (FBS)) commonly used for the HCV replication model. The Li23 cell line containing the HCV subgenomic replicon or full-length HCV genome was maintained in a medium that contained G418 at a concentration of 0.3 mg/ml (Invitrogen). The same culture was used without G418 for the cured cells (described later).

It was confirmed that the Li23 cells had the characteristics of the liver cells as do the HuH-7 cells, as follows. Li23 cells were examined with regard to expression of genes specific to the liver cells, or expression of 11 genes reported to have high expression levels in the liver cells (Aly HH et al., J. Hepatology, 46: 26-36,2007), using a standard RT-PCR method. The results were compared with those from the human hepatoma cell line HuH-7, human cervical cancer cells (HeLa cells), or human embryonic kidneys (HEK293 cells). It was found that the expression level of each gene in the Li23 cells was about the same as that in HuH-7 cells, whereas the genes were not detected in HeLa and HEK293 cells (the results are not presented).

### Northern Blot Analysis

Total RNA was extracted from the subject cells using an RNeasy Mini Kit (Qiagen), according to the manufacturer's experiment protocol. The extracted RNA was quantified by absorbance measurement at the wavelength of 260 nm. HCV RNA and β-actin RNA were detected with 4 µg of the RNA. Specifically, specific RNA detection was made using a Northern Max Kit (Ambion), according to the manufacturer's experiment protocol. The RNA sample was subjected to electrophorisis, and the gel was blotted on a Hybond-N+nylon membrane (Amersham-Pharmacia Biotech). The RNA was fixed to the membrane using a UV Crosslinker (Stratagene), and the 28S rRNA portion on the membrane was stained with ethidium bromide. The membrane was cut about 1 cm below the 28S rRNA band. HCV RNA was contained in the upper part of the removed membrane. β-actin mRNA was contained in the lower part. For the specific detection of HCV RNA, a minus-strand riboprobe complementary to the digoxigenine-labeled HCV NS5B region was synthesized and used according to the manufacturer's experiment protocol attached to a digoxigenine labeling kit (Roche). Alkali phosphatase-labeled anti-digoxigenin antibodies were used for the detection of the riboprobe that had specifically bound to the HCV RNA. After reaction using a CSPD (Roche), the patterns were exposed on an X-ray film for specific detection of HCV RNA. β-actin RNA was detected in the same manner.

### Western Blot Analysis

An SDS-containing sample buffer (100 µl) was added to the cells cultured in a 6-well culture plate, and the cell lysate was collected. After 10-min sonication using an ultrasonic homogenizer, each sample was supplemented with 10 µl of 2-mercaptoethanol, and treated at 100°C for 3 min. 10 to 20 µl of the sample was subjected to 10% SDS-PAGE, and the proteins were transferred to a membrane (PVDF membrane). The protein-transferred membrane was blocked for 60 min with 0.1% Tris buffer that contained 5% skim milk (10 mM Tris (pH 7.5), 150 mM NaCl, 0.1% Tween20). Then, the membrane was contacted with a solution of antibodies against the HCV proteins and the β-actin protein diluted 1,000 times with 0.1% Tris buffer, and a reaction was allowed for 60 min. After washing the membrane three times with 0.1% Tris buffer 5 min each time, the membrane was contacted with a 0.1% Tris buffer supplemented with HRP-labeled mouse secondary antibodies diluted 1,000 times, and a reaction was allowed for 60 min. The membrane was washed three times with 0.1% Tris buffer, 20 min each time. The proteins were allowed to chemiluminesce with a Renaissance^{™} Luminol Western Blot Chemiluminescence Reagent Plus (NEN Life Science), and exposed on an X-ray film (KODAK BioMax).

The antibodies used in the experiments were anti-core antibodies (Institute of Immunology), anti-E1 antibodies (a gift from Dr. Kohara, Tokyo Metropolitan Institute of Medical Science), anti-E2 antibodies (see the reference: Microbiol. Immunolo. 42, 875-877, 1998), anti-NS3 antibodies (Novocastera Laboratories), anti-NS4A antibodies (a gift from Dr. Takamizawa, Osaka University), anti-NS5A antibodies (a gift from Dr. Takamizawa, Osaka University), anti-NS5B antibodies (a gift from Dr. Kohara, Tokyo Metropolitan Institute of Medical Science), and anti-β-actin antibodies (Sigma).

### Plasmid Construction

Plasmid pON/C-5B includes a neomycin phosphotransferase (Neo)-encoding sequence downstream of the HCV IRES (internal ribosomal entry site), and a full-length HCV-O protein-encoding sequence downstream of the Encephalomyocarditis virus (EMCV) IRES.

First, a plasmid pHCV-O that contained a genotype 1b HCV-O full-length cDNA was constructed from HCV positive serum, using two fragments. The two fragments are EcoRI-MluI fragment (corresponding to position 45-2528 of the HCV genome) derived from the pBR322/16-6 described in the reference (Kato et al. J.Gen.Virol.79: 1859-1869 (1998)), and MluI-SpeI fragment (corresponding to position 2528-3420 of the HCV genome) derived from the PCR product of serum 1B-2. These fragments were ligated to the EcoRI-SpeI site of pNSS1RZ2RU having a 1B-2R1 sequence (see Non-Patent Document 2) to construct pHCV-O.

To obtain a fragment for constructing pON/C-5B, the EMCV IRES was fused with the coding sequence of core protein using overlapping PCR. The resulting DNA was digested with RsrII and ClaI, and ligated to the ClaI-XbaI site of pHCV-O with a XbaI-RsrII fragment of pNSSIRZ2RU.

Plasmid pON/3-5B was constructed in a manner described in Non-Patent Document 2 in detail. Specifically, as described in Non-Patent Document 2, the region of the RNA extracted from sO cells corresponding to position 3474-9185 of HCV-O gene was amplified by RT-PCR method, and the amplified RNA was ligated to the SpeI-BsiwI site of pNSSIRZ2RU to construct pON/3-5B.

Further, according to the method of Ikeda et al. (see

Non-Patent Document 4), Q1112R, K1609E, and S2200R mutations, or Q1112R, P1115L, and S2200R mutations were introduced into the pON/3-5B using QuickChange mutagenesis (Stratagene) to construct pON/3-5B/QR,KE,SR, or pON/3-5B/QR,PL,SR. Further, Q1112R, K1609E, and S2200R mutations, or Q1112R, P1115L, and S2200R mutations were introduced into the pON/C-5B to construct pON/C-5B/QR,KE,SR, or pON/C-SB/QR,PL,SR. The plasmid pORN/C-5B/QR,KE,SR was constructed by introducing the PCR product of Renilla luciferase gene (Promega) to the AscI site upstream of the Neo gene in pON/C-5B/QR,KE,SR. Each plasmid contains a T7 promoter sequence on the 5' side of the inserted gene.

### RNA synthesis

The plasmid DNA was linearized by cutting with XbaI, and RNA synthesis was performed using a T7 MEGAscript Kit (Ambion) according to the manufacturer's experiment protocol. After being precipitated with lithium chloride, the RNA was washed with 75% ethanol, and dissolved in RNase-free water.

### Quantification of HCV RNA

Total RNA was extracted from the HCV RNA replicating cells using an RNeasy Mini Kit (Qiagen) according to the manufacturer's experiment protocol. First, using 2 µg of RNA as a template, reverse transcription (RT) reaction was performed with superscript™ II reverse transcriptase (Invitrogen) and the primer 319R below according to the manufacturer's experiment protocol. HCV RNA was quantified by real-time LightCycler PCR, using the resulting cDNA as a template. Real-Time LightCycler PCR was performed based on the method previously reported by the present inventors (reference: Acta Med. Okayama 56, 107-110, 2002), using the primers 104 and 197R below.
319R: 5'-TGCTCATGGTGCACGGTCTA-3'(SEQ ID NO: 15)
104: 5'-AGAGCCATAGTGGTCTGCGG-3'(SEQ ID NO: 16)
197R: 5'-CTTTCGCGACCCAACACTAC-3'(SEQ ID NO: 17).

### Luciferase Reporter Assay

Renilla luciferase was quantified by collecting cells using a Renilla Luciferase Assay System (Promega) according to the manufacturer's experiment protocol.

### 2: HCV genome

FIG. 1 is a schematic illustration showing the structure of HCV genome, the structure of RNA introduced to subgenomic HCV replicon cells, the structure of RNA introduced to full-length HCV genome replicating cells, and the structure of RNA introduced to full-length HCV genome replicating cells expressing a luciferase gene product.

FIG. 1(a) shows an HCV-O strain (genotype 1lb). The HCV genome is a positive-chain, single-stranded RNA of about 9.6 kb (SEQ ID NO: 1), 90% of which is a single large ORF producing a polyprotein of about 3,000 amino acids (SEQ ID NO: 2). The polyprotein is processed by host protease in the first half that ends with p7, whereas the remaining portion is processed by two proteases encoded by NS2 and NS3. In the end, at least 10 virus proteins are produced. The region ending with E2 is called a structural region that forms viral particles, and NS2 to NS5B are called a non-structural region. The regions necessary for HCV RNA replication are known to include the 5' untranslated region (HCV IRES) including a region that encodes the first 12 amino acids of the core, a region from NS3 to NS5B, and a 3' end region. The HCV-O strain is an HCV strain that belongs to genotype 1b, which accounts for about 70% of patients in Japan, and was isolated from healthy carriers (HCV-infected individuals with normal liver function).

The RNAs used in the present invention are shown in FIG. 1(b) to FIG. 1(e). FIG. 1(b) shows HCV-O strain-derived HCV replicon RNA (ON/3-5B/QR,KE,SR) that includes two adaptive mutations (Q1112R and K1609E) introduced into the NS3 region, and adaptive mutation S2200R introduced into the NS5A region. The HCV-O strain-derived HCV replicon RNA (ON/3-5B/QR,PL,SR) to which P1115L adaptive mutation is introduced in place of K1609E also has this structure. The full-length HCV RNA (ON/C-5B/QR,KE,SR) shown in FIG. 1(c) includes HCV-O strain-derived C (core) to NS2 inserted between the internal ribosomal entry site (IRES) of Encephalomyocarditis virus (EMCV) and NS3 in the replicon (ON/3-5B/QR,KE,SR (FIG. 1(b)), and thus has a structure 1.4 kb longer than the original HCV genome (a full-length of 11 kb). The RNA (ORN/C-5B/QR,KE,SR) shown in FIG. 1(d) is modified to include Renilla luciferase gene (RL gene) between the HCV IRES and NeoR gene of the full-length HCV RNA (ON/C-5B/QR,KE,SR (FIG. 1(c)) so as to be produced as a fusion protein, and has a structure 2.4 kb longer than the original HCV genome (a full-length of 12 kb). The RL gene allows for the quantification of HCV RNA replication levels through RL activity measurement (referred to as "reporter assay"). The HCV RNA (JFH1 strain) shown in FIG. 1(e) has the structure of the original HCV genome, specifically, infectious HCV RNA derived from the JFH1 strain (genotype 2a) that originates in a fulminant hepatitis patient.

These five kinds of RNA were obtained by in vitro synthesis using a T7 MEGAscript (Ambion) after the plasmids (pON/3-5B/QR,KE,SR; pON/3-5B/QR,PL,SR; pON/C-5B/QR,KE,SR; pORN/C-5B/QR,KE,SR; pJFH1) containing these RNA sequences were linearized by cutting with restriction enzyme XbaI. The plasmid containing the full-length JFH1 cDNA was provided by Tokyo Metropolitan Organization for Medical Research based on research material transfer agreement.

### 3: HCV Replicon Replicating Cell Line

### 3-1 Preparation of Cell Line

The RNA (10 µg) synthesized in vitro from pON/3-5B/QR,KE,SR or pON/3-5B/QR,PL,SR was introduced into Li23 cells (8 x 10⁶) according to the method described in Non-Patent Document 2. After 2 days, the medium was replaced with medium containing G418 (0.3 mg/ml) and NaHCO₃ (0.15%). The cells were cultured for 3 weeks with medium replacement every 4 days, and cells that showed sustained high levels of replicon RNA replication were obtained as G418-resistant colonies. Some of the colonies (one plate) were stained with Coomassie Brilliant Blue (CBB). Similar replicon RNA introduction experiments were conducted using other cell lines, including other human hepatoma cell lines (HuH-6, Li21, Li24), human immortalized liver cell lines (PH5CH, OUMS29, IHH10.3, IHH12), and human bile duct cancer cell line (HuH28). However, no G418-resistant cell colonies were obtained (the results are not presented). The Li23 cells to which the RNA was not introduced were completely killed by the G418 contained in the medium (the results are not presented).

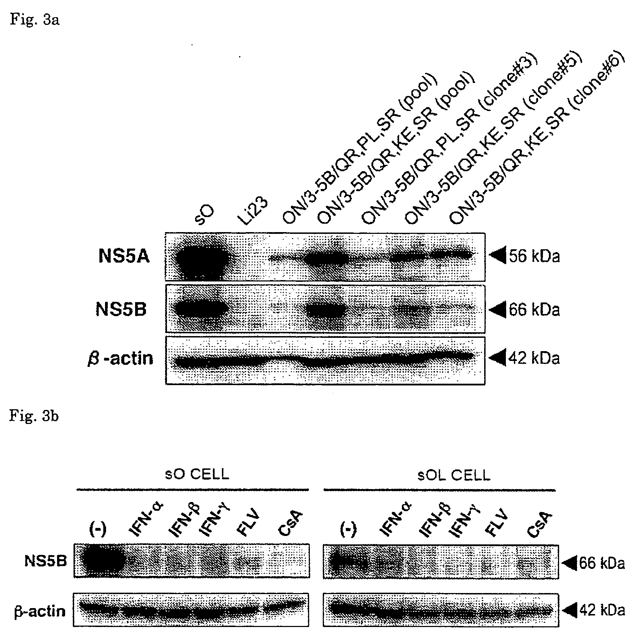

The expression levels of the HCV proteins in the G418-resistant cells were analyzed by Western blotting. The NS5A and NS5B proteins were detected by an ordinary method using NS5A and NS5B antibodies, respectively. The sO cells (HuH-7 cell-derived cells that show efficient replication of HCV-O strain-derived replicon RNA) reported in Non-Patent Document 2 by the present inventors were used for comparison. β-actin detection using β-actin antibody was performed in parallel to find the amounts of proteins used in the analysis. FIG. 3(a) shows that the expression levels of NS5A and NS5B proteins are far greater in cells obtained by introducing ON/3-5B/QR,KE,SR RNA than in cells obtained by introducing ON/3-5B/QR,PL,SR RNA. In the case of ON/3-5B/QR,KE,SR, it was also found that colony-pooled cells had higher expression levels than cloned cells. The pooled cells (ON/3-5B/QR,KE,SR (pool)) were used as sOL cells in the next step without selecting cell clones at this stage, because the purpose of the experiment was to establish a full-length HCV RNA replicating cell line.

### 3-2 Drug Sensitivity of Cell Line

Sensitivity of an HCV replicon in sOL cells was examined with respect to drugs reported to have anti-HCV activity in HuH-7 cell-derived cells. It was also investigated whether treatment with such drugs would enable production of sOL cured cells (cells expected to have an intracelluler environment suited for HCV RNA replication) considered to be necessary for the production of full-length HCV RNA replicating cells. Note that HuH-7 cell-derived sO cells were compared with sOL cells in an experiment conducted to compare effects.

A 6-well plate was inoculated with 1 x 10⁵ cells, and various drugs were added 24 hours later. The drugs were added so that the final concentration was 20 IU/ml for IFN-α, IFN-β, and IFN-γ, 5 µM for fluvastatin (FLV), and 0.5 µg/ml for Cyclosporin A (CsA). After 5 days, the expression levels of NS5B protein in the cells were analyzed by Western blotting. As shown in FIG. 3(b), the sOL cells had about the same level of sensitivity as the sO cells to each anti-HCV agent. It was therefore considered possible to obtain cured sOL cells by treatment with these drugs. 4: Full-Length HCV RNA Replicating Cell Line

### 4-1 Preparation of Cell Line

IFN-γ (10³ IU/ml) was added to sOL cells five times at 4-day intervals in the absence of G418 to obtain cured cells OLc from which HCV-O strain replicon RNA was excluded. Note that the resulting cells were identified as cured cells by the absence of HCV RNA, and by the lack of expression of the proteins encoded by the HCV genome.

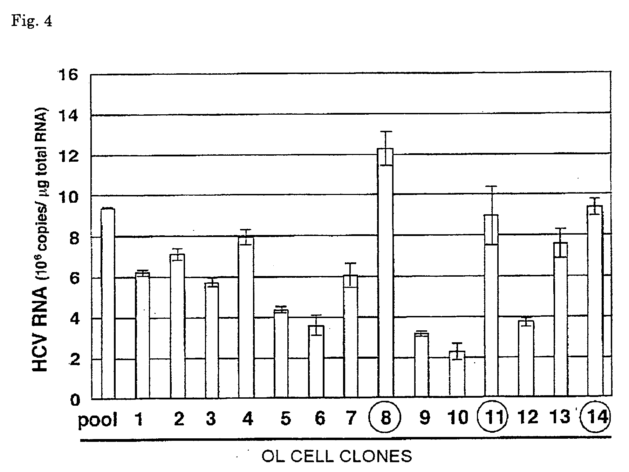

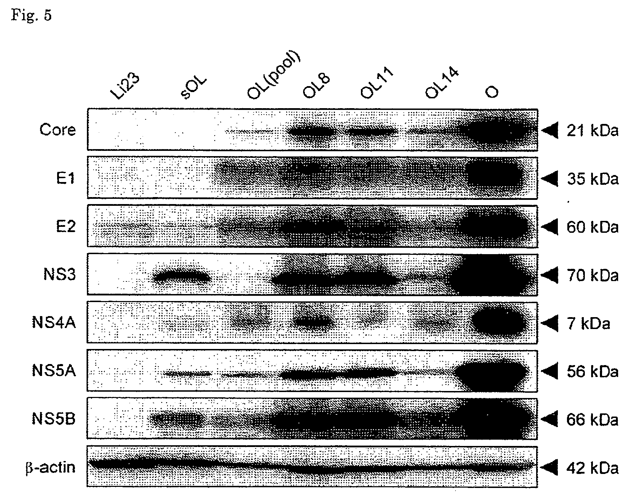

The RNA (2 µg or 4 µg) synthesized in vitro from pON/C-5B/QR,KE,SR was introduced to sOLc cells (8 x 10⁶) according to the method described in Non-Patent Document 2. After 2 days, the medium was replaced with a medium that contained G418 (0.3 mg/µl) and NaHCO₃ (0.15%). The cells were cultured for 3 weeks with medium replacement every 4 days. As a result, G418-resistant colonies were obtained at each RNA level (the results are not presented). Some of the colonies (1 plate) were stained with CBB. The number of stained colonies was counted, and the rate of colony formation per 1 µg RNA was calculated to be about 100 colonies/µg RNA. Considering the possibility of incomplete drug selection by G418 and the possibility of minute amounts of remaining HCV-O strain replicon, large colonies were selected for cloning, and cells that showed sustained high levels of full-length HCV RNA replication were obtained as G418-resistant colonies (OL1 to OL14). By the quantitative comparison of HCV RNA in the cells using LightCycler PCR, the top three clones (OL8 cells, OL11 cells, and OL14 cells) were selected, and used for HCV protein detection and HCV gene analysis (FIG. 4). About 200 remaining colonies were mixed, and used as OL (pool) cells for further analysis. Note that replication of 11-kb full-length HCV RNA and the absence of 8-kb replicon RNA in the OL cells were confirmed in all OL cell clones and in OL (pool) cells (the results are not presented). It was also confirmed from the result of Western blotting for various HCV proteins that OL8, OL11, and OL14 cells expressed proteins in amounts considered to be sufficient for various experiments, though the expression levels were lower than that in O cells (FIG. 5). Note that OL14 cells had lower expression levels of HCV proteins than OL8 cells or OL11 cells. Further, PCR conducted for the HCV 5' UTR confirmed the lack of HCV genome incorporation in the host genome in OL8, OL11, and OL14 cells (the results are not presented).

Subsequently, OL8 cells were observed using an immunofluorescent technique with anti-dsRNA antibodies, in order to detect double-stranded RNA (dsRNA), a replication intermediate of HCV RNA, in the full-length HCV RNA-replicating OL8 cells. The O cells and Li23 cells were used as positive control and negative control, respectively.

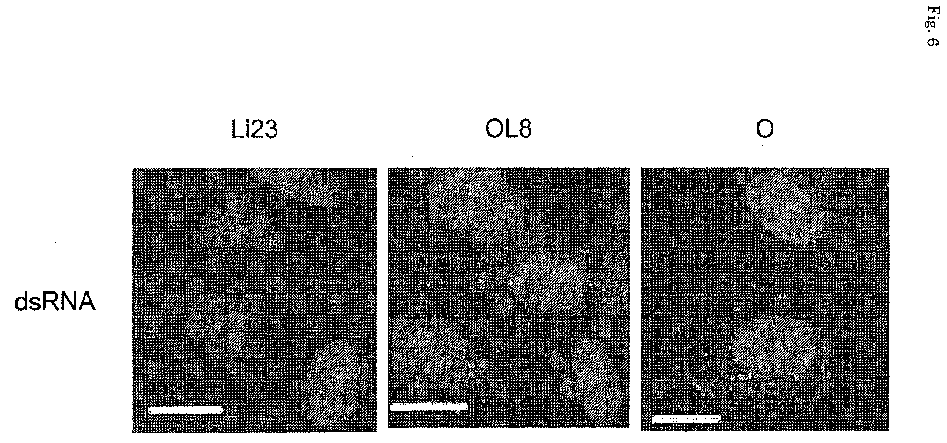

The cells cultured for 4 days after being inoculated on a collagen-coated cover slip were fixed at room temperature with a PBS solution containing 3% paraformaldehyde, and treated with 0.1% Triton X-100 to prepare permeable cells. After treatment with 1% (v/v) bovine serum albumin (BSA), the cells were treated with anti-dsRNA antibodies (K1: English and Scientific Consulting; primary antibodies), and then with Cy2-conjugated anti-mouse antibodies (Jackson Immuno Research, West Grove; secondary antibodies). The cell nuclei were stained with 4',6-diamidino-2-phenylindole (Sigma). The cover slip was placed on a glass slide using a PermaFluor Aqueous Mountant (ThermoFisher), and observed with a confocal laser scanning microscope (LSM510; Carl Zeiss). Photographed images are shown in FIG. 6 (bar length, 20 µm).

As shown in the figure, small dot-like fluorescence scattered over the cytoplasm was observed in the OL8 and O cells. L23 cells did not show any such fluorescence. The fact that the replication intermediate double-stranded RNA was observed in OL8 cells as in O cells can be taken as evidence of efficient HCV RNA replication in the OL8 cells.

### 4-2 Drug Sensitivity of Cell Lines

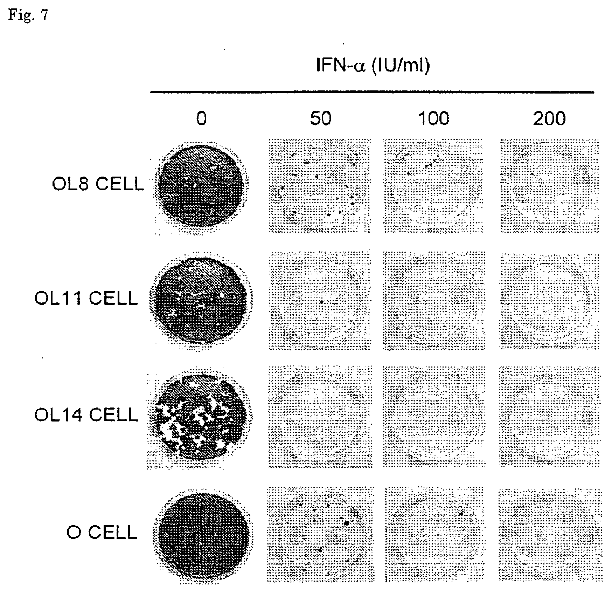

Whether IFN-α suppresses the full-length HCV RNA replication in OL8, OL11, and OL14 cells was determined by colony assay. Cells (1 x 10⁴) were inoculated on dishes having an outer diameter of 10 cm, and cultured for 25 days in the presence of G418 (0.3 mg/ml) while adding IFN-α (0, 50, 100, or 200 IU/ml) every 4 days. The colonies that appeared as G418 resistant cells were stained with CBB solution (FIG. 7). Some G418-resistant cell colonies were obtained in the OL8 and OL11 cells; however, the number did not differ much from that obtained in the O cells used as control. No G418-resistant cell colonies were obtained in OL14 cells. The results demonstrated that the full-length HCV RNA replication in the OL8, OL11, and OL14 cells were highly sensitive to IFN-α as in O cells.

### 4-3 HCV Gene Analysis in Cell Lines

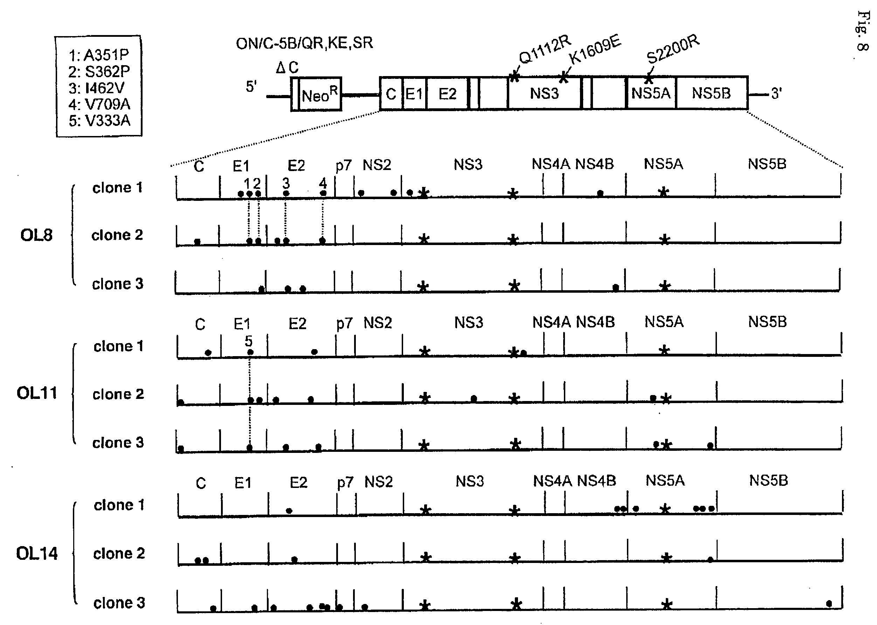

The presence or absence of new adaptive mutations other than the three adaptive mutations (Q1112R, K1609E, and S2200R) introduced in the full-length HCV RNA replicated in the OL8, OL11, and OL14 cells was examined. Total RNA was prepared from each cell line, and the full-length HCV RNA was amplified according to the RT-PCR method described in Non-Patent Document 7 (from the 5' UTR-NS2 5.1-kb first half to the NS3-NS5B 6-kb second half). The RT primer 290ROK was used for the amplification of the first half, and a primer set (21X and NS3RXOK) was used for PCR. The RT primer 386R was used for the amplification of the second half, and a primer set (NS2XOK and 9388RX) was used for PCR. A Primscript (Takara) was used for RT, and a KOD-plus DNA polymerase (Toyobo) for PCR. The amplification product was inserted into a plasmid vector (pBR322MC), and the base sequence of the inserted portion was determined, and compared with the base sequence of ON/C-5B/QR,KE,SR originally introduced into the cells (FIG. 8).

As a result, the following became clear. Note that no mutation was recognized in the 5' UTR (341 bases) base sequences of the analyzed 9 clones, though not shown in the figure.
(1) The mutations Q1112R, K1609E, and S2200R originally introduced were conserved in the total of 9 clones analyzed (3 clones for each cell line).
(2) The NS3 to NS5B region essential for the HCV RNA replication did not contain any new mutation that accompanied amino acid substitutions conserved in the three clones derived from eeach cell line. The mutations were all clone specific. The number of mutations in the NS3 to NS5B region per clone was low: 1 in OL8 cells, 1.3 in OL11 cells, and 2.6 in OL14 cells. The clone specific mutations (Q1067R, K1397R, I1612M, V1864A, V1929L, E1937D, C1968W, T1989S, T2169A, L2171P, P2322L, T2332A, S2380P, and W2404R) detected in the NS3 to NS5B region did not classify as any of the adaptive mutations reported thus far. However, a previous report (Lohmann et al., JVI, 77:3007-3019, 2003) indicates that the Q2933R detected in clone 3 of OL14 cells is a weak adaptive mutation (1.6-fold increase in replication level). It was therefore considered that the Q1112R, K1609E, and S2200R mutations were essential for the full-length HCV RNA replication in OL cell lines, and that no additional adaptive mutation was necessary.
(3) On the other hand, relatively larger numbers of mutations were observed in the core to NS2 region. In OL11 cells, a mutation (V333A) accompanied by a common amino acid substitution across the three clones was detected in the E1 region. In OL8 cells, no common mutation was detected in the three clones; however, four mutations (A351P and S362P in the E1 region, and 462V and V709A in the E2 region) were detected and recognized in two of the three clones. The number of mutations in the core to NS2 region per clone was higher than in the NS3 to NS5B region: 5.3 in the OL8 cells, 4 in the OL11 cells, and 4 in the OL14 cells. The results therefore suggested that this region was not necessary for HCV RNA replication.

Neighbour-joining analysis was performed with GENETYX-MAC (Software Development) for the three clones derived from OL8, OL11, and OL14 cells, using the base sequence and amino acid sequence in the HCV polyprotein, and a genetic phylogenetic tree was created based on the parental clone ON/C-5B/QR,KE,SR (the results are not presented). The phylogenetic tree appeared the same at the base sequence level or the amino acid sequence level. However, the OL14 cells did not form genetically-independent clusters. As described above, OL14 cells had considerably lower HCV protein expression levels than the other two cells, and as such the OL8 cells and OL11 cells were used for the subsequent analyses.

### 5: Cell Line Capable of Replicating Reporter Gene-Containing HCV Replicon RNA or Full-Length HCV RNA

### 5-1 Preparation of Cell Lines

Cured cells OLc required for JFH1 strain HCV infection experiment were prepared by adding IFN-γ (10³ IU/ml) five times at 4-day intervals to the three clones of the OL cells (OL8, OL11, and OL14 cells), and to other clones (OL cells) in the absence of G418. About 4 to 5 x 10⁵ OL8 cells and OL11 cells were inoculated on dishes having an outer diameter of 10 cm, and IFN-γ (10³ IU/ml) was added four times at 4-day intervals. The cells were appropriately subcultured when the dish became full. The cells subcultured after the 4th addition of IFN-γ were divided into two groups of dishes, one containing medium supplemented with G418 (0.3 mg/ml) and NaHCO₃ (0.15%), and one continuously used to culture the cells with the medium alone. IFN-γ was then added once to each group, and the cells were cultured for at least 4 days before stained with CBB. While the cells cultured in the G418-free medium grew and filled the dish, the cells were completely killed when cultured in the medium supplemented with G418 (the results are not presented).

Cell lines capable of replicating reporter gene-carrying HCV RNA (replicon or full-length) were prepared using OL8c cells and OL11c cells. The RNAs (10 µg; ORN/3-5B/QR,KE,SR, or 20 µg; ORN/C-5B/QR,KE,SR) synthesized in vitro from pORN/3-5B/QR,KE,SR and pORN/C-5B/QR,KE,SR were introduced into OL8c cells or OL11c cells (8 x 10⁶) according to the method described in Non-Patent Document 2. After 2 days, the medium was replaced with a medium containing G418 (0.3 mg/ml) and NaHCO₃ (0.15%). The medium was replaced every 4 days, and the cells were cultured for 2 to 3 weeks.

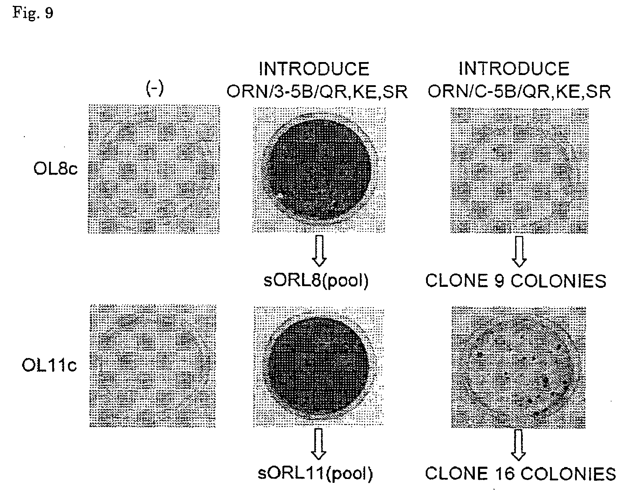

In the ORN/3-5B/QR,KE,SR (replicon RNA)-introduced OL8c and OL11c cells, G418-resistant cells filled the dish in 2 weeks (FIG. 9). Because the whole cells were estimated to be several tens of thousands of colonies, the G418-resistant cells were mixed without cloning, and used as reporter gene-carrying replicon-replicating cells (sORL8 (pool) cells and sORL11 (pool) cells). These cells had luciferase activities measuring 8 x 10⁵ and 15 x 10⁵, respectively, in terms of actual measurement values per 2 x 10⁵ cells (Promega assay kit). It was therefore found that the both of these cells were sufficient for the activity evaluation of anti-HCV agents. Note that the doubling time of the cells using a common subculture medium (in the presence of G418) was calculated as 53 hours and 40 hours. It was also confirmed that the replicon RNA in these cells were not incorporated into the host DNA (the results are not presented).

In the ORN/C-5B/QR,KE,SR (full-length HCV RNA)-introduced cells, only small numbers of G418-resistant colonies appeared even after about 3 weeks from the introduction, about 30 from OL8c cells, and about 400 from OL11c cells. Nine cell colonies were cloned from OL8c cells (ORL8-1 to ORL8-9), and 16 cell colonies were cloned from OL11c cells (ORL11-1 to ORL11-16) (FIG. 9). The remaining uncloned cell colonies from OL11c cells were mixed, and used as ORL11 (pool) cells. The measured luciferase activity of the ORL11 (pool) cells was 7 x 10⁵ (Promega assay kit). The cells were therefore found to be sufficient for the activity evaluation of anti-HCV agents. The doubling time of the cells using a common subculture medium (in the presence of G418) was calculated as 44 hours. It was also confirmed that the HCV genome in the cells was not incorporated into the host DNA (the results are not presented).

The expression level of HCV core protein in the ORL11 (pool) cells was determined by Western blotting. The result suggested that the expression level of core protein in the ORL11 (pool) cells was considerably lower than that in OR6 cells. It was therefore considered that selection of cloned cells having higher expression levels was necessary.

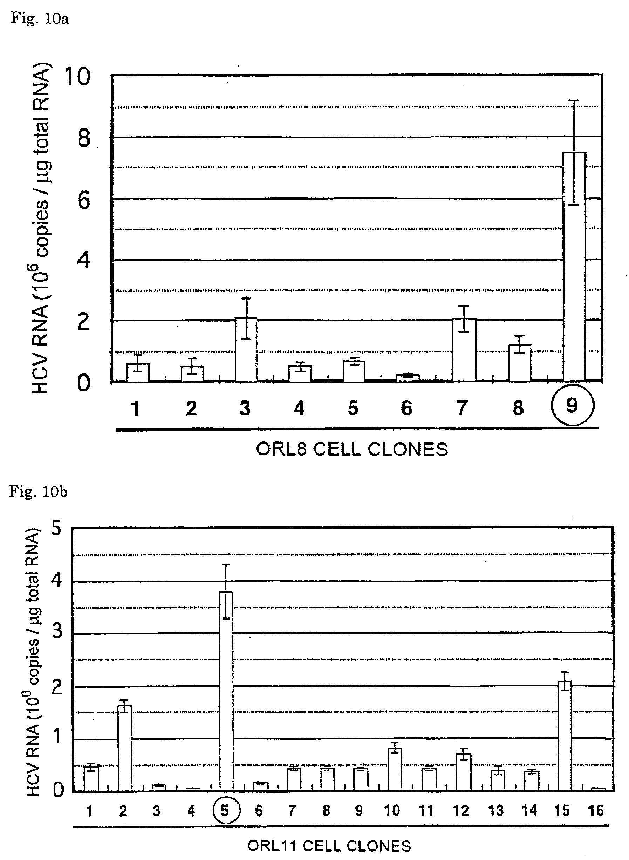

Cells that showed sustained high levels of RL gene-carrying full-length HCV RNA replication were obtained as G418-resistant colonies (FIG. 10(a) and FIG. 10(b)). Specifically, the top two clones (ORL8-9 cells and ORL11-5 cells; referred to as ORL8 cells and ORL11 cells, respectively) were selected by the quantitative comparison of the HCV RNAs in the cells using LightCycler PCR. The luciferase reporter assays of these cells are considered useful for the screening and evaluation of various anti-HCV agents.

### 5-2 HCV Gene Analysis in Cell Lines

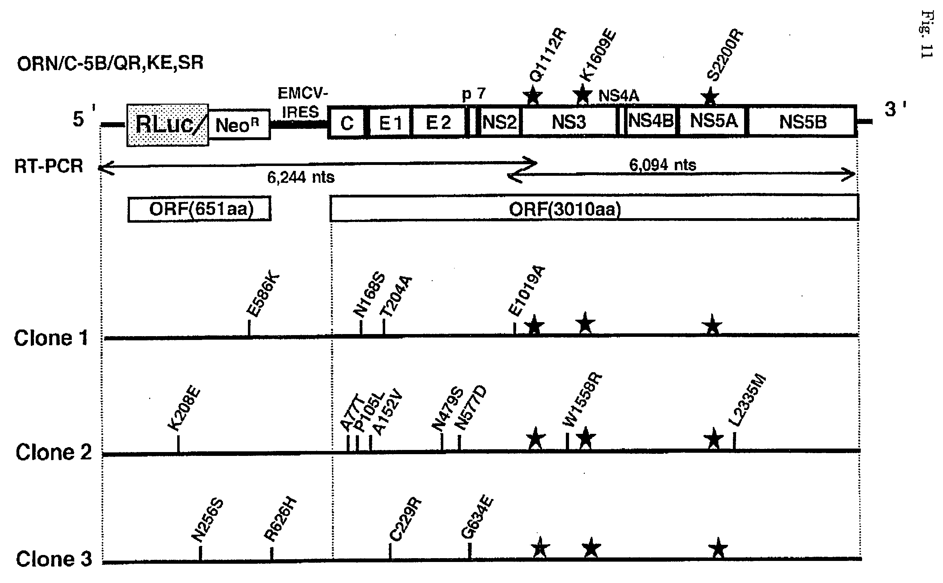

The presence or absence of new adaptive mutations other than the three adaptive mutations (Q1112R, K1609E, and S2200R) originally introduced to the reporter gene-carrying full-length HCV RNA replicated in the ORL8 cells was examined. Total RNA was prepared from ORL8 cells, and the full-length HCV RNA was amplified according to the RT-PCR method described in Non-Patent Document 7 (from the 5' UTR-NS2 6.2-kb first half to the NS3-NS5B 6.1-kb second half). The RT primer 290ROK was used for the amplification of the first half, and a primer set (21X and NS3RXOK) was used for PCR. The RT primer 386R was used for the amplification of the second half, and a primer set (NS2XOK and 9388RX) was used for PCR. A Primscript (Takara) was used for RT, and a KOD-plus DNA polymerase (Toyobo) for PCR. The amplification product was inserted into a plasmid vector (pBR322MC), and the base sequence of the inserted portion was determined, and compared with the base sequence of ORN/C-5B/QR,KE,SR originally introduced into the cells (FIG. 11). As a result, the following became clear.
(1) The mutations Q1112R, K1609E, and S2200R originally introduced were conserved in the three clones analyzed.
(2) New mutations that accompanied amino acid substitutions conserved in the three clones were not detected in the 5' UTR to NS5B region for which the base sequence was determined. All new mutations accompanied by amino acid substitutions (4 in clone 1; 8 in clone 2; and 4 in clone 3) were specific to the clones analyzed. It was therefore considered that the Q1112R, K1609E, and S2200R mutations were essential for the reporter gene-carrying full-length HCV RNA replication in the ORL8 cells, and that no additional adaptive mutation was necessary.
(3) Mutations that accompanied amino acid substitutions were found in larger numbers in the core to NS2 region than in the NS3 to NS5B region. The same phenomenon was also seen in the results from the full-length HCV RNA replicating OL8 cells, OL11 cells, and OL14 cells.
(4) The clone specific mutations W1558R and L2335M detected in the NS3 to NS5B region did not classify as any of the adaptive mutations reported thus far.

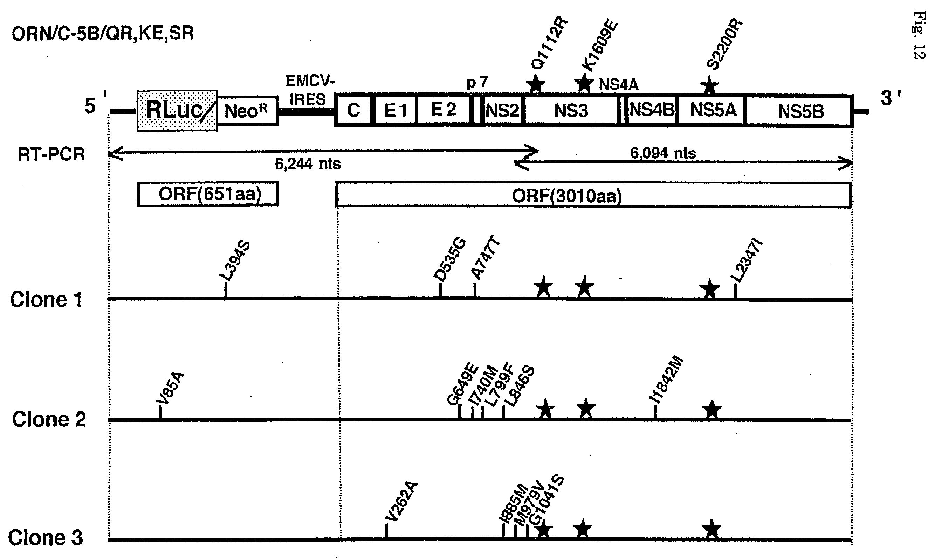

The presence or absence of new adaptive mutations other than the three adaptive mutations (Q1112R, K1609E, and S2200R) originally introduced to the reporter gene-carrying full-length HCV RNA replicated in the ORL11 cells was determined in the same manner as in ORL8 cells (FIG. 12). As a result, the following became clear.
(1) As for the ORL8 cells, the mutations Q1112R, K1609E, and S2200R originally introduced were conserved in the three clones analyzed.
(2) New mutations that accompanied amino acid substitutions conserved in the three clones were not detected in the 5' UTR to NS5B region for which the base sequence was determined. All new mutations accompanied by amino acid substitutions (4 in clone 1; 6 in clone 2; and 4 in clone 3) were specific to the clones analyzed. It was therefore considered that the Q1112R, K1609E, and S2200R mutations were essential for the reporter gene-carrying full-length HCV RNA replication in the ORL11 cells, and that no additional adaptive mutation was necessary.
(3) Mutations that accompanied amino acid substitutions were found in larger numbers in the core to NS2 region than in the NS3 to NS5B region. The same phenomenon was also seen in the results from the full-length HCV RNA replicating OL8 cells, OL11 cells, and OL14 cells.
(4) The clone specific mutations G1041S, I1842M, and L2347I detected in the NS3 to NS5B region did not classify as any of the adaptive mutations reported thus far.

### 5-3 Cell Line Behavior Analysis

ORL8, ORL11, and OR6 cells were each inoculated in three wells of a 24-well plate, each well containing 2 x 10⁴ cells (1-ml medium: assay medium containing no G418, fungizone, or NaHC0₃). The cells were cultured for 4 days, and luciferase activity was measured. The measured values were, on average, 1 x 10⁶ in ORL8 cells, 2 x 10⁶ cells in ORL11 cells, and 2 x 10⁶ cells in OR6 cells (the results are not presented). This level of luciferase activity was considered sufficient for experiments that study effectiveness after addition of anti-HCV agents. The value was about 100 in OL8c cells and OL11c cells used as controls.

To examine the doubling time of the cells, the cells were inoculated under the conditions of the assay system used to measure luciferase activity, and the cells after 24, 48, 72, and 96 hours were counted using a trypan blue staining technique. Measurements were made in 3 wells at each point, and a mean value was determined for the measurement of doubling time in a logarithmic growth phase. For comparison and contrast, the OR6 cells were also counted in the same manner. The doubling time of cells were 23 hours in ORL8 cells, 26 hours in ORL11 cells, and 34 hours in OR6 cells (the results are not presented).

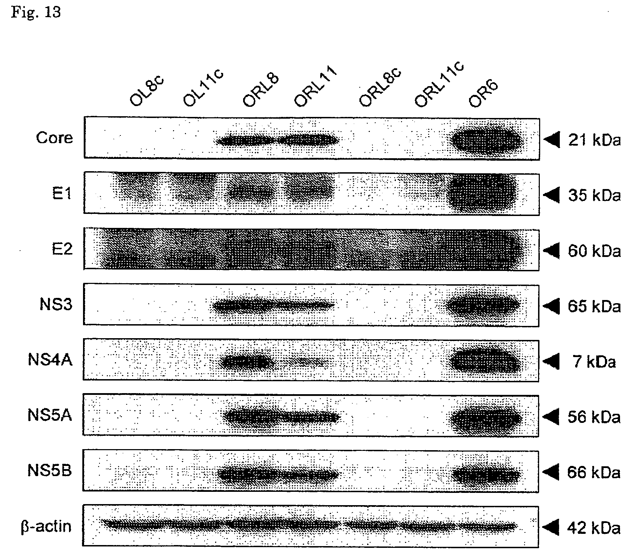

The expression level of each HCV protein (core , E1, E2, NS3, NS4A, NSSA, and NS5B) in the ORL8 and ORL11 cells was compared with that in the OR6 cells using Western blot analysis (FIG. 13). As controls, the same analysis was also made for the cured OL8c, OL11c, ORL8c, and ORL11c cells. β-actin detection using β-actin antibody was also performed to find the amounts of proteins used in the analysis. The expression level of each HCV protein in ORL8 and ORL11 cells was considerably lower than that in OR6, but the expression level was considered sufficient for various analyses. The lower expression levels relative to OR6 cells was in accord with the lower HCV RNA levels of ORL8 and ORL11 cells in comparison with OR6 cells (> 1 x 10⁷ copies/mg total RNA; FIG. 10(a) and 10(b)).

### 5-4 Drug Sensitivity of Cell Lines

Analysis was made as to the usefulness of the cell lines ORL8 and ORL11 for the evaluation of drug effects, as in OR6 cells (see Patent Document 1). As described in Patent Document 1, there is a correlation between cell-derived luciferase activity and HCV RNA level in OR6 cells, making the OR6 cells a convenient cell line for the evaluation of drug effects.

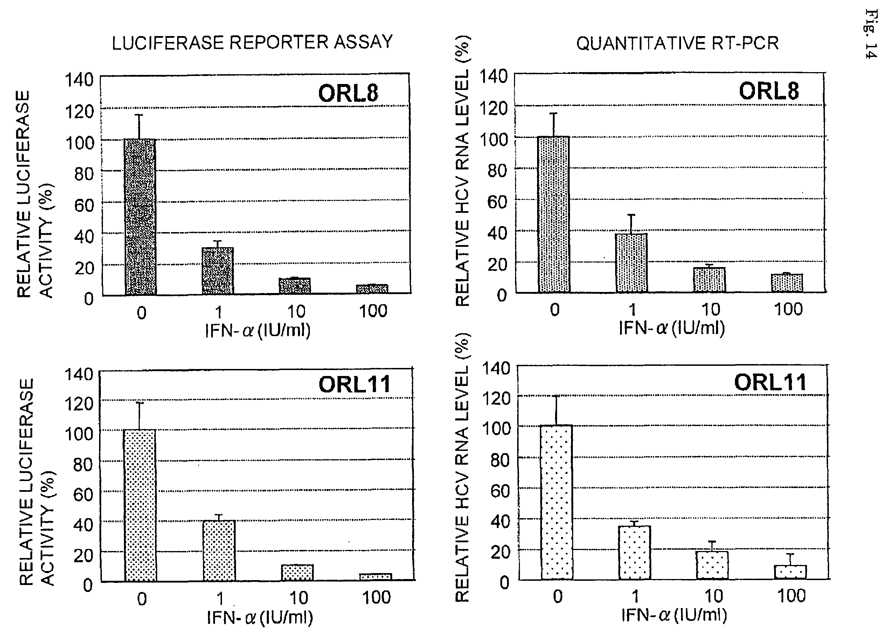

The results are shown in FIG. 14. The graphs on the left represent the measured luciferase activities 24 hours after the addition of IFN-α (0, 1, 10, 100 IU/ml) to the ORL8 and ORL11 cells (each cultured for 1 day after inoculating 2 x 10⁴ cells in a 24-well plate). Measurements were made in at least 3 wells at each point. SD values are also shown in the figure. One hundred percent measurement values of 400,000 and 500,000 were obtained for the ORL8 cells and ORL11 cells, respectively. The graphs on the right represent the results of quantitative HCV RNA measurements by LightCycler PCR 24 hours after the addition of IFN-α (0, 1, 10, 100 IU/ml) to the ORL8 and ORL11 cells (each cultured for 2 days after inoculating 2 x 10⁵ cells in a 6-well plate). Measurements were made in at least 3 wells at each point. SD values are also shown in the figure. As represented in the figure, the luciferase activity and the HCV RNA level decreased in a manner that depended on IFN-α concentration, and the results of these measurements had a good correlation as did the result from OR6 cells. The results thus demonstrated that the ORL8 and ORL11 cells were useful for the quantification of HCV RNA replication level with a simple luciferase assay.

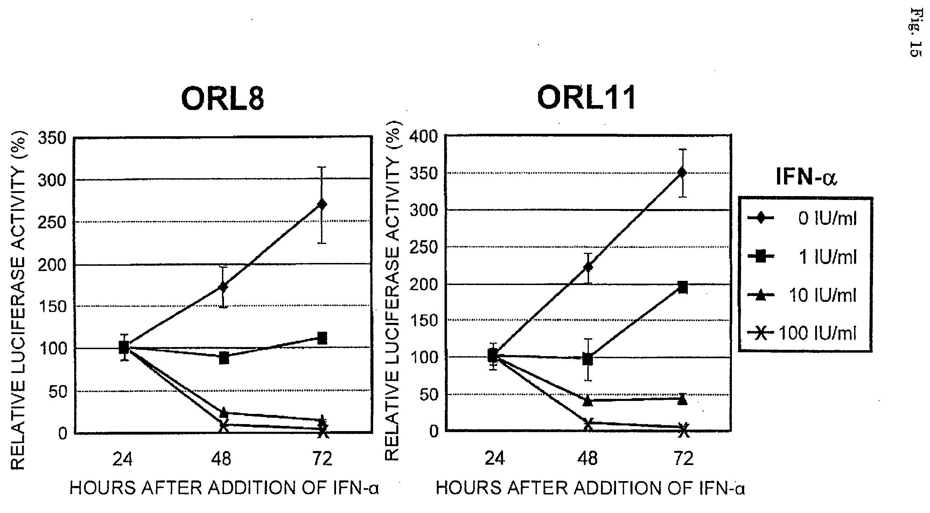

Time-dependent viral effect is confirmed in OR6 cells (Naka et al., BBRC, 330: 871-879, 2005). It was investigated whether similar viral effects also can be seen in ORL8 and ORL11 cells (FIG. 15). Cells (2 x 10⁴) were inoculated on a 24-well plate, and the predetermined quantities of IFN-α (0, 1, 10, 100 IU/ml) were added 24 hours later. Luciferase activity of each cell line was then measured at hour 24, 48, and 72. A separately prepared Li23 cell medium containing 10% FBS was used for the dilution of IFN-α. SD values were calculated based on the results from three samples at each point.

From the reference luciferase value of 100 at twenty-four hours after the addition of IFN-α, the activity showed no change up until hour 48 in response to the addition of 1 IU/ml IFN-α. At hour 72, a slight increase was observed in ORL8 cells, and a clear increase again in ORL11 cells. These results indicate the high HCV RNA replication levels of these cells. However, the reincrease after 72 hours was not so evident in cells treated with 10 IU/ml or 100 IU/ml IFN-α. Further, a decrease in luciferase activity, and the strong anti-HCV effect of IFN-α were observed as early as 24 hours after the addition of IFN-α, though not presented in the figure. The measured luciferase activity values were 380,000 (no addition of IFN-α), 113,000 (addition of 1 IU/ml IFN-α), 36,000 (addition of 10 IU/ml IFN-α), and 19,000 (addition of 100 IU/ml IFN-α) for the ORL8 cells. Because the luciferase activity 72 hours after the addition of 1 IU/ml IFN-α is 50% or less of the luciferase activity obtained without IFN-α, EC₅₀ (50% effective drug concentration) is estimated to be 1 IU/ml or less. The measured values for the ORL11 cells were 750,000 without addition of IFN-α, 300,000 with 1 IU/ml IFN-α, 76,000 with 10 IU/ml IFN-α, and 27,000 with 100 IU/ml IFN-α. As in the ORL8 cells, the luciferase activity 72 hours after the addition of 1 IU/ml IFN-α is 50% or less of the luciferase activity obtained without IFN-α, and thus EC₅₀ (50% effective drug concentration) is estimated to be 1 IU/ml or less.

Thus, at least for IFN-α, the results suggested the potential of ORL8 or ORL11 cells as a convenient assay system capable of monitoring the HCV RNA replication level solely by the measurement of luciferase activity 72 hours after the addition of the drug, as also suggested for OR6 cells.

### 5-5 Anti-HCV Effect of Various Drugs

Drugs reported to have anti-HCV activities were evaluated using an ORL8- or ORL11-cell assay system. An OR6-cell assay system was used as a control. Note that, depending on compounds, use of DMSO or ethanol as a solvent is needed; however, it has been confirmed that the assay system is not affected as long as the DMSO concentration is 0.5% or less, and that the ethanol concentration is 0.2 to 0.25% (data not presented).

### (A) IFN-α

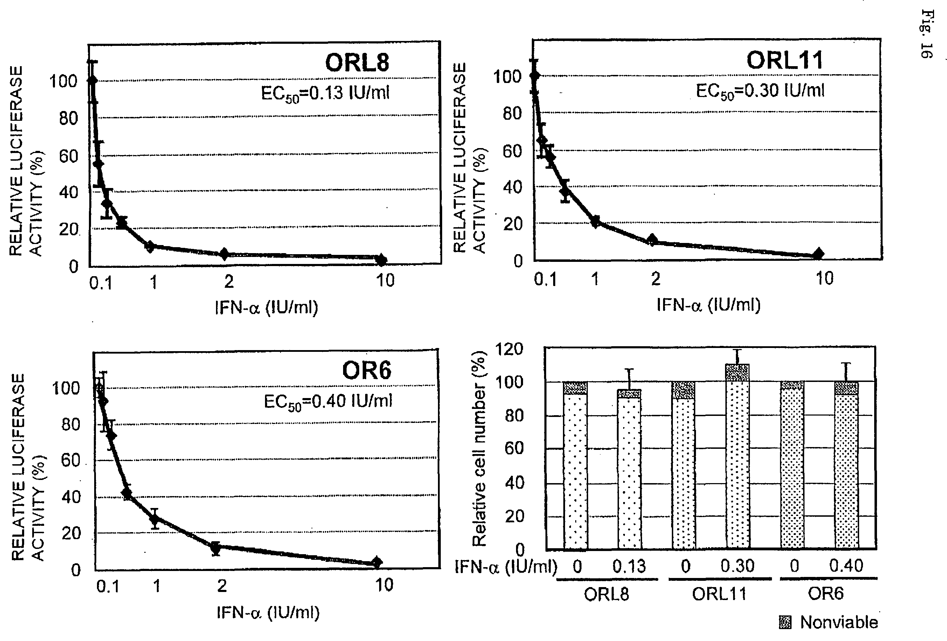

IFN-α (0, 0.1, 0.2, 0.5, 1, 2, 10 IU/ml: Sigma, 12396) was added to each cell line, and luciferase activity after 72 hours was measured. By analysis, the EC₅₀ values of the ORL8, ORL11, and OR6 cells were calculated as 0.13 IU/ml, 0.30 IU/ml, and 0.40 IU/ml, respectively. Values close to these were obtained in the same experiment repeated three times, yielding good reproducibility. The ORL8 cells had the highest sensitivity for IFN-α, followed by ORL11 cells and OR6 cells. Representative results are presented in FIG. 16.

In order to ascertain that the decrease in luciferase activity in response to the addition of IFN-α was not due to the cell growth inhibition or cytotoxicity by IFN-α, the cells were cultured under the same conditions used for the luciferase assay, and counted using a trypan blue staining technique. The effect of adding IFN-α in the concentration corresponding to the EC₅₀ value of each cell line was examined. The results for all cells were 95% or higher over the control cells, and cytotoxicity by IFN-α was hardly recognized.

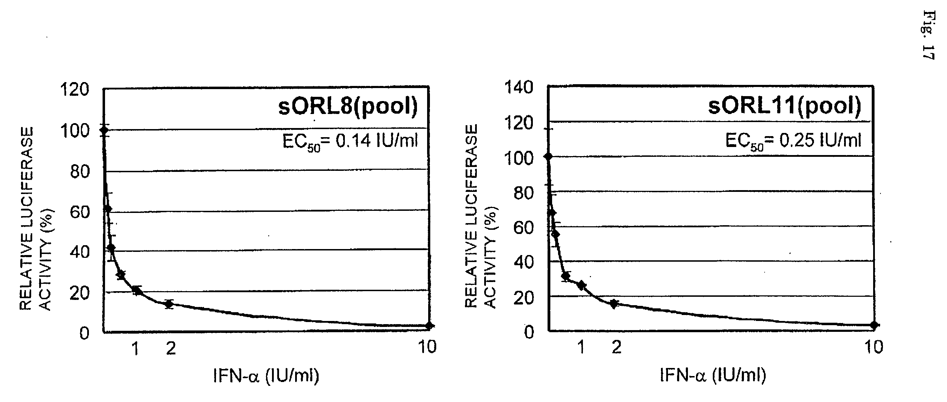

The same experiments were conducted using sORL8 (pool) cells and sORL11 (pool) cells. By analysis, the EC₅₀ values of sORL8 (pool) cells and sORL11 (pool) cells were calculated as 0.14 IU/ml and 0.25 IU/ml, respectively, about the same values obtained from ORL8 and ORL11 cells (0.13 IU/ml and 0.30 IU/ml, respectively). The results suggest that the influence of IFN-α does not differ greatly for the replication of HCV replicon RNA and full-length HCV RNA. Representative results are presented in FIG. 17.

### (B) IFN-β

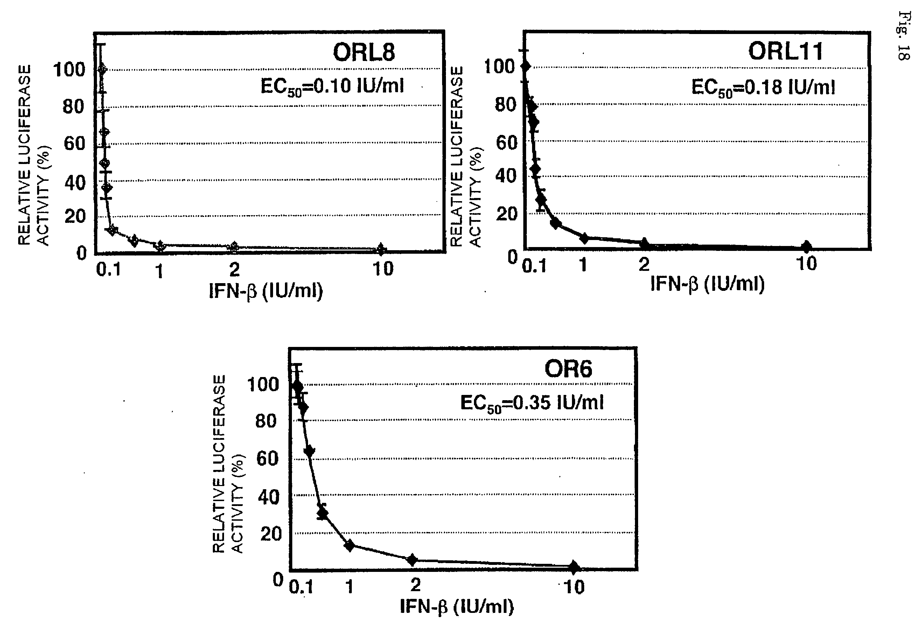

IFN-β (0, 0.05, 0.1, 0.2, 0.5, 1, 2, 10 IU/ml: provided by Toray) was added to each cell line, and luciferase activity after 72 hours was measured. By analysis, the EC₅₀ values of ORL8, ORL11, and OR6 cells were calculated as 0.10 IU/ml, 0.18 IU/ml, and 0.35 IU/ml, respectively. Representative results are presented in FIG. 18. The trend seen in IFN-α, was also observed in IFN-β, with the ORL8 cells showing the highest sensitivity.

### (C) IFN-γ

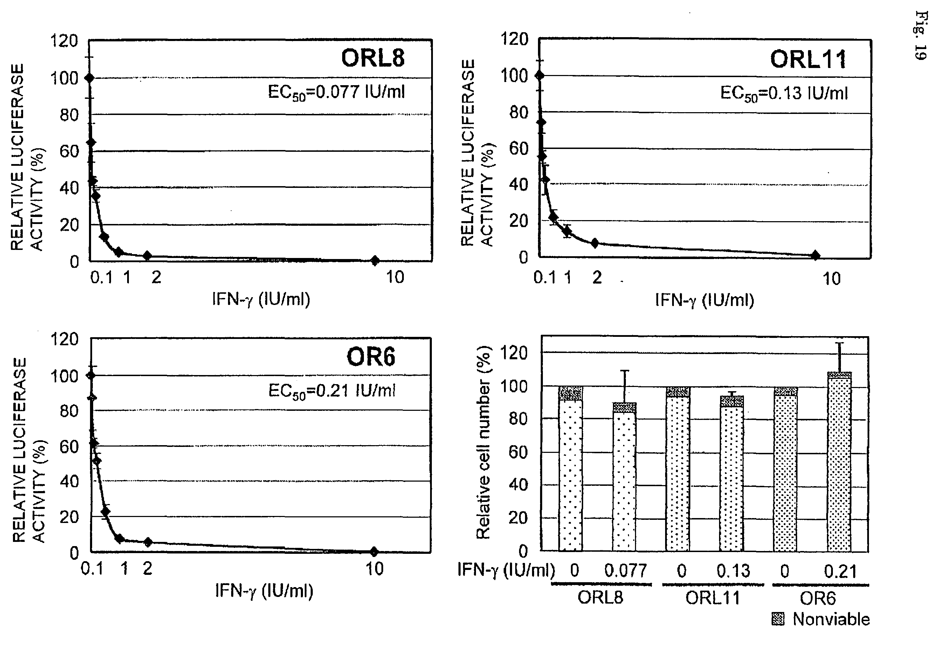

IFN-γ (0, 0.05, 0.1, 0.2, 0.5, 1, 2, 10 IU/ml: Sigma, I1520) was added to each cell line, and luciferase activity after 72 hours was measured. By analysis, the EC₅₀ values of ORL8, ORL11, and OR6 cells were calculated as 0.077 IU/ml, 0.13 IU/ml, and 0.21 IU/ml, respectively. Representative results are presented in FIG. 19. The same trend seen in IFN-α or IFN-β was also observed in IFN-γ, with the ORL8 cells showing the highest sensitivity.

In order to ascertain that the decrease in luciferase activity in response to the addition of IFN-γ was not due to the cell growth inhibition or cytotoxicity by IFN-γ, the cells were cultured under the same conditions used for the luciferase assay, and counted using a trypan blue staining technique. The effect of adding IFN-γ in the concentration corresponding to the EC₅₀ value of each cell line was examined. The results for all cells were 90% or higher over the control cells, and cytotoxicity by IFN-γ was hardly recognized.

### (D) Cyclosporin A (CsA)

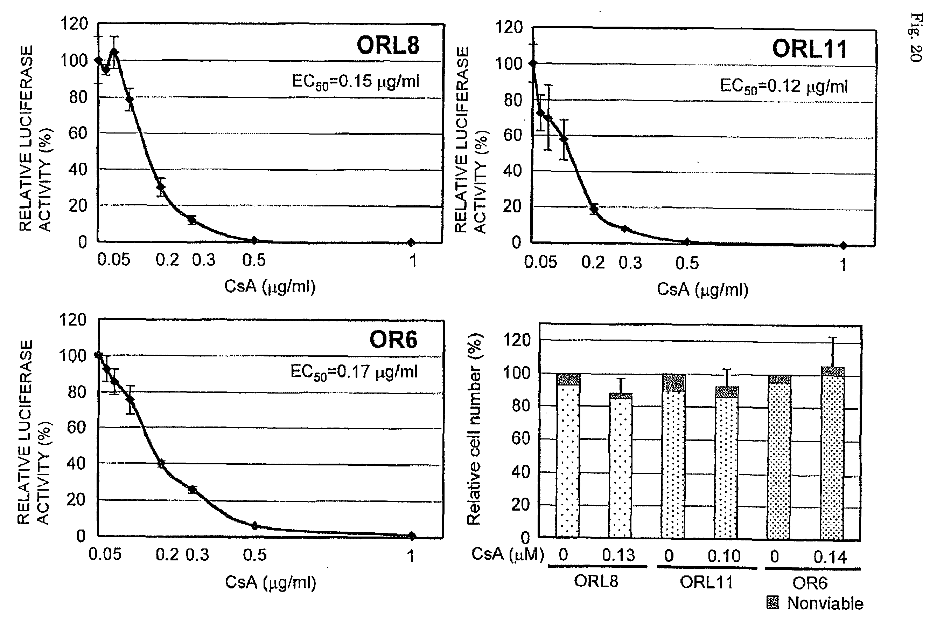

CsA (0, 0.025, 0.05, 0.1, 0.2, 0.3, 0.5, 1 µg/ml: Sigma, C3662) was added to each cell line, and luciferase activity after 72 hours was measured. By analysis, the EC₅₀ values of the ORL8, ORL11, and OR6 cells were calculated as 0.15 µg/ml, 0.12 µg/ml, and 0.17 µg/ml, respectively. Representative results are presented in FIG. 20. Unlike IFN-α, IFN-β, or IFN-γ, the sensitivity of the ORL11 cells to CsA was only slightly higher than those of the other cells, and the differences between the three were smaller than those observed in IFNs.

In order to ascertain that the decrease in luciferase activity in response to the addition of CsA was not due to the cell growth inhibition or cytotoxicity by CsA, the cells were cultured under the same conditions used for the luciferase assay, and counted using a trypan blue staining technique. The effect of adding CsA in the concentration corresponding to the E₅₀ value of each cell line was examined. The results for all cells were 87% or higher over the control cells, and cytotoxicity by CsA was hardly recognized.

### (E) Fluvastatin (FLV)

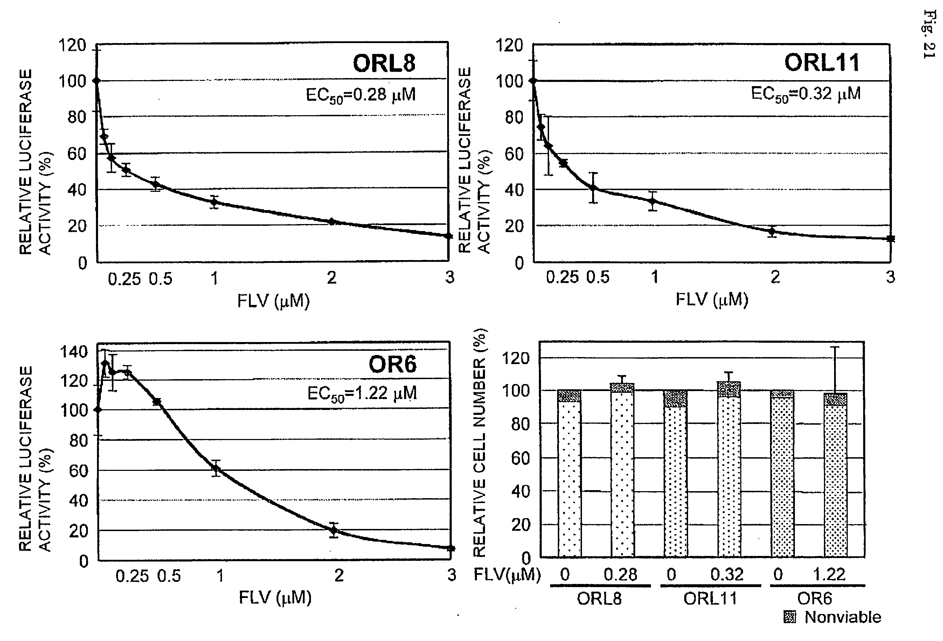

FLV (0, 0.063, 0.125, 0.25, 0.5, 1, 2, 3 µM: Calbiochem, 344095) was added to each cell line, and luciferase activity after 72 hours was measured. By analysis, the EC₅₀ values of the ORL8, ORL11, and OR6 cells were calculated as 0.28 µM, 0.32 µM, and 1.22 µM, respectively. Representative results are presented in FIG. 21.

The anti-HCV effect of FLV is found with an assay using OR6 cells (see Patent Document 1). The ORL8 and ORL11 cells had EC₅₀ values (0.28 µM and 0.32 µM, respectively) considerably smaller than the EC₅₀ value (1.22 µM) of OR6 cells. There is an increasing trend for clinical trials that additionally use FLV in the PEG-IFN+ribavirin combination therapy, and these tests are producing good results (Sezaki et al. Kanzo, 49:22-24, 2008). The effectiveness of FLV in clinical trials provide supportive evidence for the credibility of the EC₅₀ values of FLV obtained in the cell assay systems using ORL8 and ORL11 cells, and support the usefulness of the cell assay systems that use ORL8 and ORL11 cells.

In order to ascertain that the decrease in luciferase activity in response to the addition of FLV was not due to the cell growth inhibition or cytotoxicity by FLV, the cells were cultured under the same conditions used for the luciferase assay, and counted using a trypan blue staining technique. The effect of adding FLV in the concentration corresponding to the EC₅₀ value of each cell line was examined. The results for all cells were 97% or higher over the control cells, and cytotoxicity by FLV was hardly recognized.

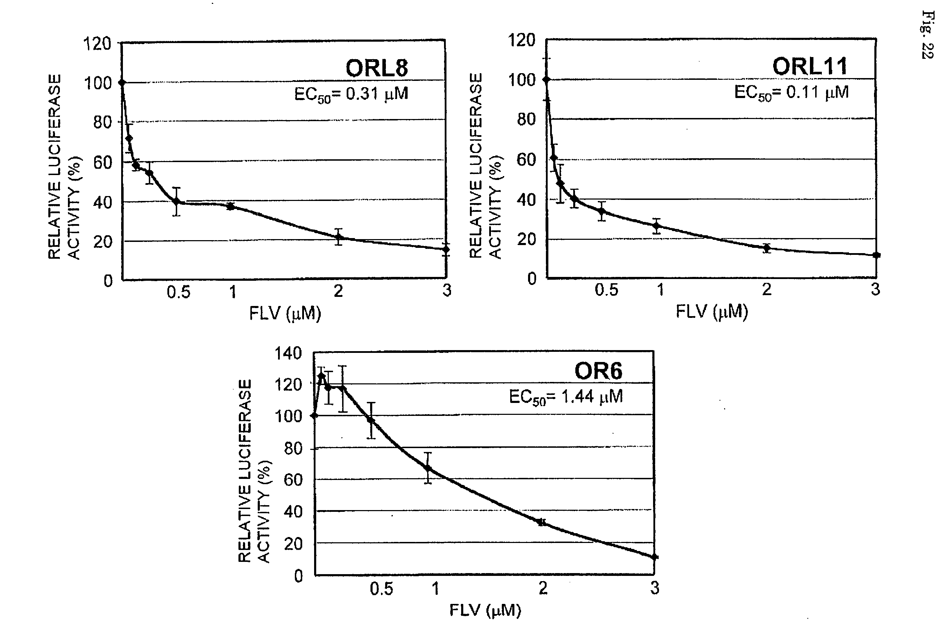

The same experiment was conducted using FLV obtained from a different manufacturer (LKT laboratories Inc., F4482, purity 99.5%), and EC₅₀ was calculated. The EC₅₀ values of ORL8, ORL11, and OR6 cells were 0.31 µM, 0.11 µM, and 1.44 µM, respectively (FIG. 22). By comparing these with the foregoing results, the values of the two results were about the same for ORL8 cells, whereas the current result had a higher effect for ORL11 cells, and the previous result had a slightly higher effect for OR6 cells. What is notable is the EC₅₀ value 0.11 µM in ORL11 cells, because it shows that the effect of FLV is maximized in this assay system. Reproducibility was confirmed by the EC₅₀ value of 0.14 µM obtained in a separate experiment (the results are not presented).

### (F) Pravastatin

Pravastatin (0, 0.25, 0.5, 1, 2, 3, 5, 10 µM) was added to each cell line, and luciferase activity after 72 hours was measured. There are previous reports that the anti-HCV effect by pravastatin was unconfirmable in a cell assay system using OR6 cells. As in OR6 cells, the anti-HCV effect by pravastatin was not confirmable in ORL8 and ORL11 cells in this experiment. In this respect, it can be said that there is no large difference between the HuH-7 cell line and the Li23 cell line (the results are not presented).

### (G) Simvastatin (SMV)

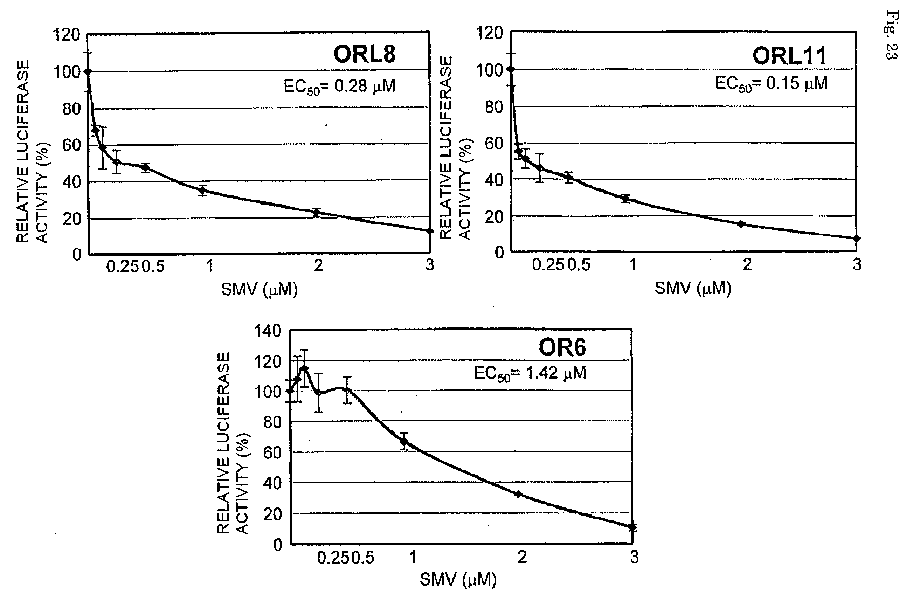

SMV (0, 0.063, 0.125, 0.25, 0.5, 1, 2, 3 µM: Wako chemical, 193-12051) was added to each cell line, and Luciferase activity after 72 hours was measured. By analysis, the EC₅₀ values of ORL8, ORL11, and OR6 cells were 0.28 µM, 0.15 µM, and 1.42 µM, respectively. Representative results are presented in FIG. 23.

The anti-HCV effect of SMV is found with an assay using OR6 cells (see Ikeda et al., Hepatology 44:117-125 (2006)). The ORL8 and ORL11 cells had EC₅₀ values (0.28 µM and 0.15 µM, respectively) considerably smaller than the EC₅₀ value (1.42 µM) of OR6 cells. This result suggests that SMV also has potential use in the treatment of patients with hepatitis C. What is notable is that SMV had the same level of activity as FLV in ORL8 cells, and that the anti-HCV activity of SMV was stronger than that of FLV in ORL11 cells. FLV topped SMV in anti-HCV activity in OR6 cells, but the order of anti-HCV activity was reversed in ORL11 cells. This result suggests the need for a comprehensive approach using ORL8 or ORL11 cells in addition to OR6 cells, and supports the usefulness of the cell assay systems that use ORL8 and ORL11 cells.

### (H) Lovastatin (LOV)

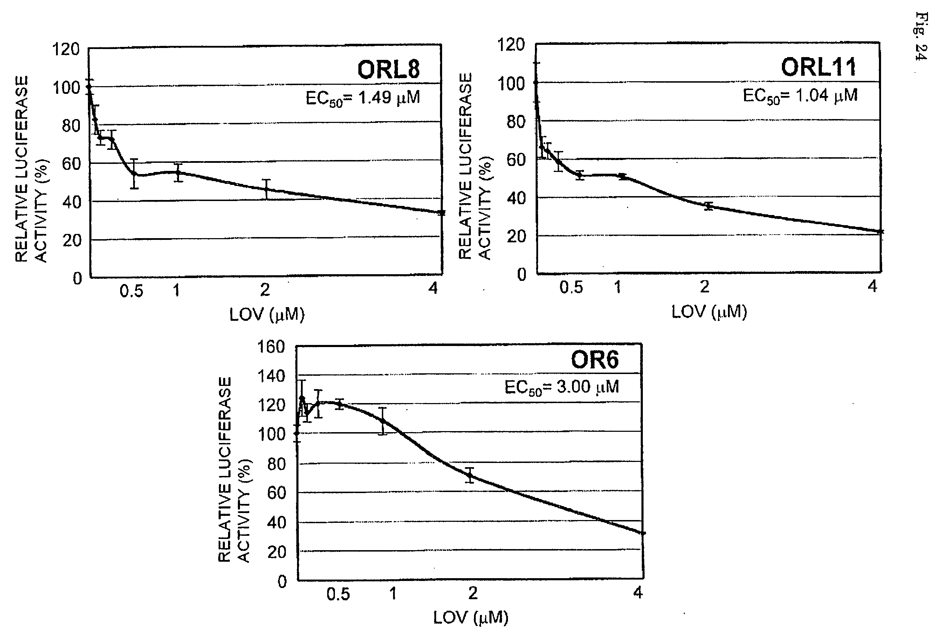

LOV (0, 0.063, 0.125, 0.25, 0.5, 1, 2, 4 µM: Wako chemical, 125-04581) was added to each cell line, and luciferase activity after 72 hours was measured. By analysis, the EC₅₀ values of ORL8, ORL11, and OR6 cells were calculated as 1.49 µM, 1.04 µM, and 3.0 µM, respectively. Representative results are presented in FIG. 24.

As also pointed out in other reports, LOV has high EC₅₀ values in replicon assays (meaning weak anti-HCV activities), and is not suited for clinical treatment. However, all of these reports use cell assay systems derived from HuH-7 cells. The results obtained in this experiment using the Li23 cell line-derived cell assay system suggest that it might be possible to find anti-HCV activities in drugs that are considered to show only weak anti-HCV activities, if any, in conventional cell assay systems.

### (I) Pitavastatin (PTV)

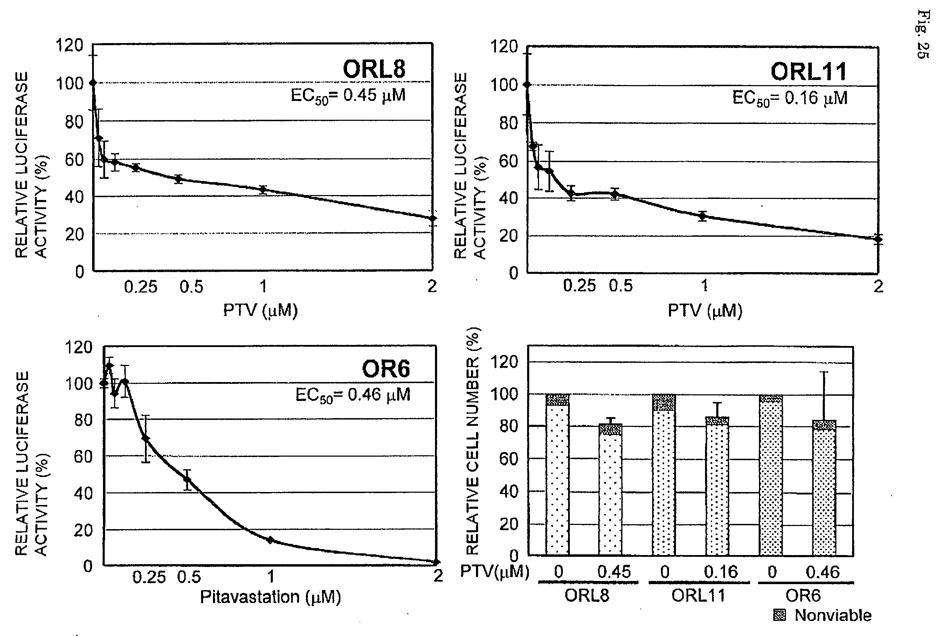

PTV (0, 0.032, 0.063, 0.125, 0.25, 0.5, 1, 2 µM: Tronto Research Inc., P531005 PTV lactone (prodrug)) was added to each cell line, and luciferase activity after 72 hours was measured. By analysis, the EC₅₀ values of ORL8, ORL11, and OR6 cells were calculated as 0.45 µM, 0.16 µM, and 0.46 µM, respectively. Representative results are presented in FIG. 25.

In order to ascertain that the decrease in luciferase activity in response to the addition of PTV was not due to the cell growth inhibition or cytotoxicity by PTV, the cells were cultured under the same conditions used for the luciferase assay, and counted using a trypan blue staining technique. The effect of adding PTV in the concentration corresponding to the EC₅₀ value of each cell line was examined. The results for all cells were 80% or higher over the control cells, and cytotoxicity by PTV was hardly recognized.

### (J) Ribavirin

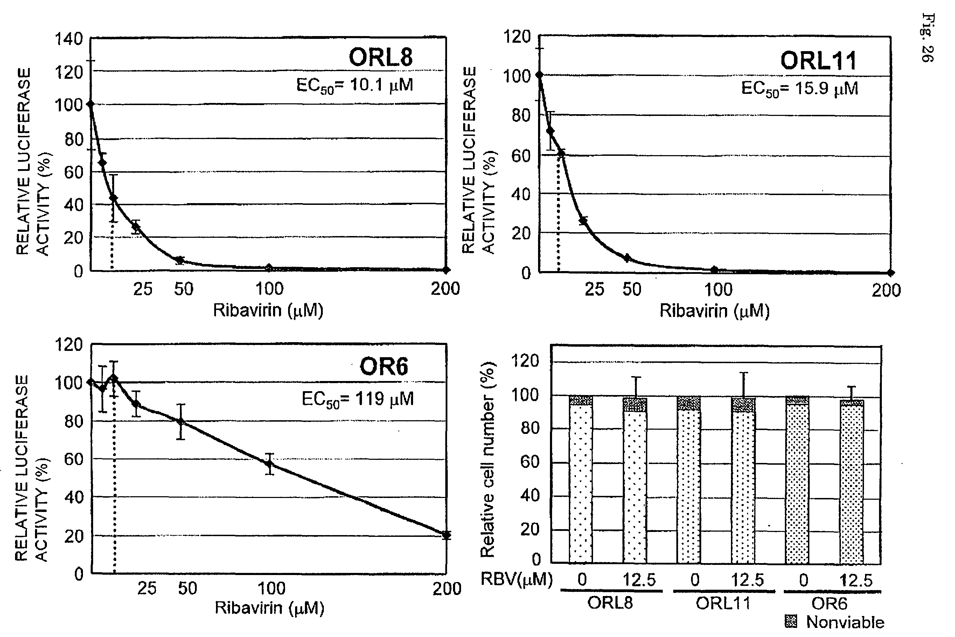

Ribavirin (0, 3.13, 6.25, 12.5, 25, 50, 100, 200 µM: provided by Yamasa; purity > 99.0%) was added to each cell line, and luciferase activity after 72 hours was measured. By analysis, the EC₅₀ values of ORL8, ORL11, and OR6 cells were calculated as 10.1 µM, 15.9 µM, and 119 µM, respectively. Representative results are presented in FIG. 26.

Ribavirin is currently used with PEG-IFN, and, despite side effects such as anemia, the drug has been used as a standard therapy because it provides better therapeutic effect than using PEG-IFN alone. However, what provides the anti-HCV effect of ribavirin remains elusive. In the measurement using an OR6 cell assay system prepared by the present inventors, ribavirin was shown to have anti-HCV effect with a high EC₅₀ value (76 µM) (Naka et al., BBRC, 330; 871-879, 2005). Broadly, four possibilities have been proposed concerning the anti-HCV effect of ribavirin:
(1) Ribavirin has RNA mutation inducing activity, and induces mutation in the HCV genome (causes error catastrophe at 100 µM or more);
(2) Inhibitory effect for the RNA-dependent RNA polymerase (NS5B) of HCV;
(3) Cell immunity enhancing effect, and effect by promotion of IFN-γ production; and
(4) Inhibitory effect for inosine-5'-monophosphate dehydrogenase (IMPDH).

An assay using ORL8 or ORL11 cells produced an unexpected result. Compared with the EC₅₀ (119 µM) obtained in the assay using OR6 cells, the assays using ORL8 and ORL11 cells yielded considerably lower EC₅₀ values of 10.1 µM and 15.9 µM, respectively.

In order to ascertain that the decrease in luciferase activity in response to the addition of ribavirin was not due to the cell growth inhibition or cytotoxicity by ribavirin, the cells were cultured under the same conditions used for the luciferase assay, and counted using a trypan blue staining technique. The effect of adding ribavirin in the concentration corresponding to the EC₅₀ value of each cell line was examined. The results for all cells were 98% or higher over the control cells, and cytotoxicity by ribavirin was hardly recognized. Cytotoxicity by ribavirin was not recognized at a concentration of 12.5 µM (the results are not presented).

The EC₅₀ value of 10 µM cannot be said as having a strong anti-HCV effect. However, the blood concentrations of patients undergoing ribavirin therapy are about 10 to 14 µM, and this range of concentration was found to be sufficient for enhancing the anti-HCV effect when PEG-IFN is used in combination. From the experiment results using the conventional HuH-7 cell line, it had been believed that HCV RNA replication could not be inhibited at the ribavirin blood concentration of a drug-administered patient. However, it was found from the foregoing results that ribavirin has activity as an HCV RNA replication inhibitor. It has been confirmed by Western blotting that, in ORL8 and ORL11 cells, the amounts of HCV proteins decrease correlatively with decrease in luciferase activity by ribavirin (data not presented). The results suggest that it might be possible to actually find anti-HCV activity in drugs that are considered to show only weak anti-HCV activities, if any, in conventional cell assay systems. It can therefore be said from the perspective of finding such anti-HCV agents that the present assay system can be a useful assay system.

### (K) Mizoribine

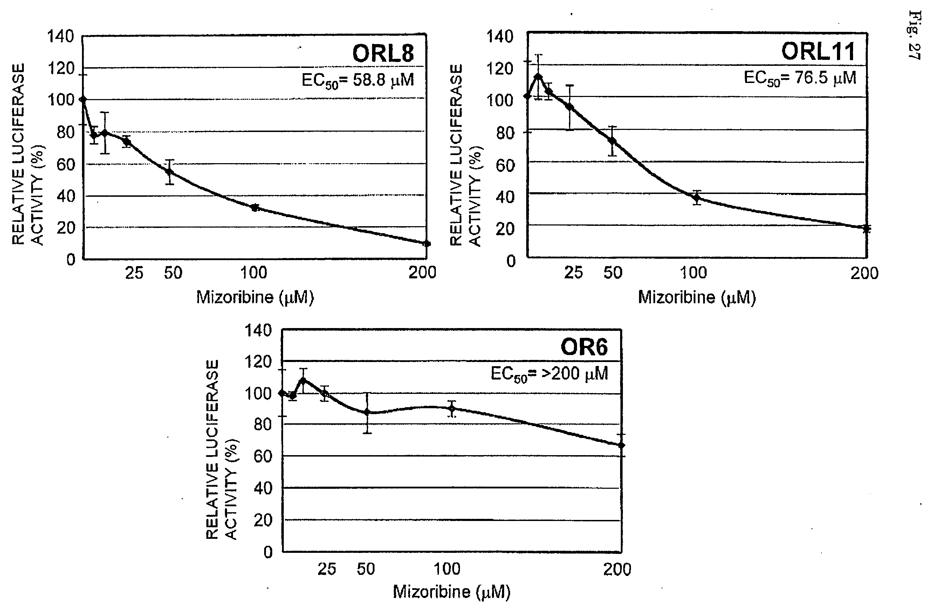

Mizoribine (0, 3.13, 6.25, 12.5, 25, 50, 100, 200 µM) was added to each cell line, and luciferase activity after 72 hours was measured. By analysis, the EC₅₀ values of ORL8 and ORL11 cells were calculated as 58.8 µM and 76.5 µM, respectively. In an assay system using OR6 cells, the value remained above 50% even at 200 µM. Representative results are presented in FIG. 27.

### (L) Geldanamycin

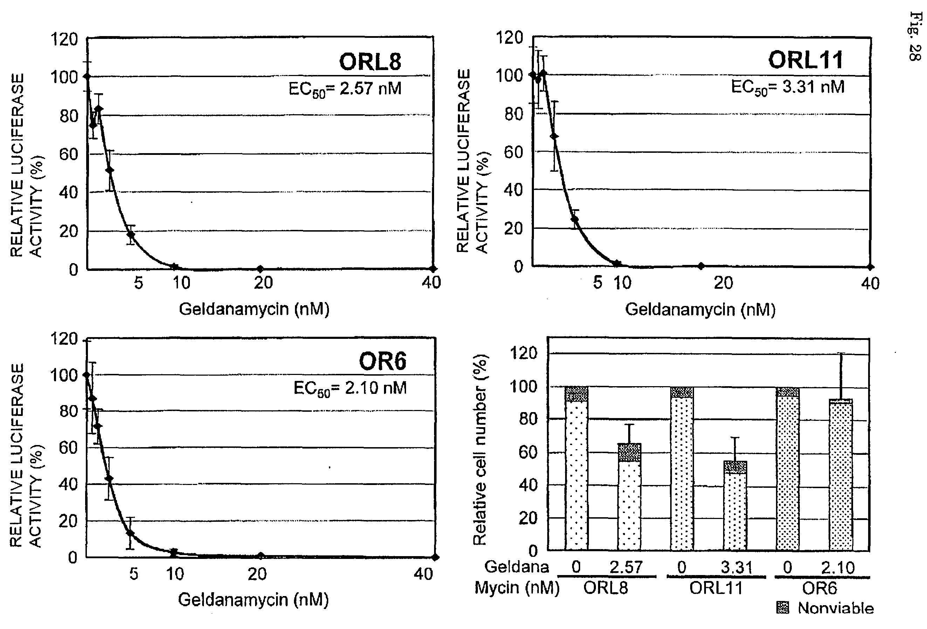

Geldanamycin (0, 0.63, 1.25, 2.5, 5, 10, 20, 40 nM: Wako chemical, 077-04571) was added to each cell line, and luciferase activity after 72 hours was measured. Geldanamycin (heat shock protein (Hsp90) inhibitor) is reported in a paper from other laboratory in which HCV replicon assay (using HuH-7 cell-derived cells) is used (Nakagawa et al., BBRC 353; 882-888, 2007), and the EC₅₀ value (analyzed 72 hours after the addition of the drug) has been calculated as 5.5 nM in Con1 strain, and 7.8 nM in N strain. Thus, in this experiment, assay was performed in such a manner that these values fell at the center of the drug concentration range. By analysis, the EC₅₀ values of ORL8, ORL11, and OR6 cells were calculated as 2.57 nM, 3.31 nM, and 2.10 nM, respectively. Representative results are presented in FIG. 28.

In this manner, the anti-HCV activity of Geldanamycin was also confirmed in this assay system. The values obtained for the replication of full-length HCV RNA in HCV-O strain were lower than the reported values, but did not differ greatly among the different cells.

### (M) Myriocin

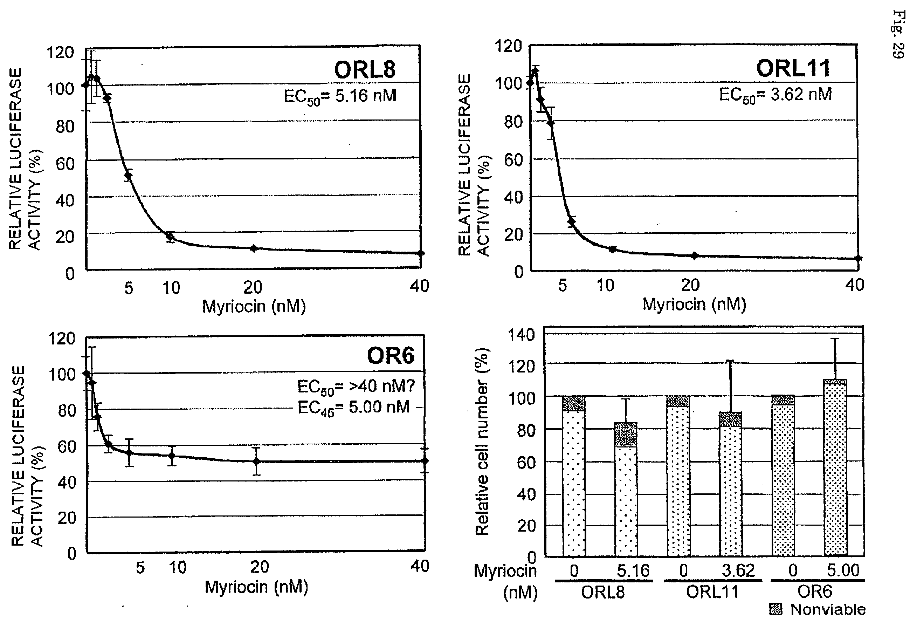

Myriocin (0, 0.63, 1.25, 2.5, 5, 10, 20, 40 nM: Sigma, M1177) was added to each cell line, and luciferase activity after 72 hours was measured. Myriocin (serine palmitoyl transferase inhibitor) is reported in a paper from other laboratory in which HCV replicon assay (using HuH-7 cell-derived cells) is used (Umehara et al., BBRC 346; 67-73, 2006), and the EC₅₀ value (analyzed 72 hours after the addition of the drug) has been calculated as 5.8 nM in Conl strain. Thus, in this experiment, assay was performed in such a manner that these values fell at the center of the drug concentration range. By analysis, the EC₅₀ values of ORL8 and ORL11 cells were calculated as 5.16 nM and 3.62 nM, respectively. The results for these cells were almost the same as the values obtained in the Con-1 strain HCV replicon assay. However, in the assay using the HuH-7 cell-derived OR6 cells, activities slightly below 60% were maintained even at 5 nM to 40 nM, and, because the value did not fall below 50%, it was not possible to calculate EC₅₀ value. Although the reasons for these results remain unclear, it can be said that the ORL8 and ORL11 in the Li23 cell line that does not show the phenomenon observed in the cells of HuH-7 cell line provide cell assay systems suited for the evaluation of myriocin-related drugs. Representative results are presented in FIG. 29.

In order to ascertain that the decrease in luciferase activity in response to the addition of myriocin was not due to the cell growth inhibition or cytotoxicity by myriocin, the cells were cultured under the same conditions used for the luciferase assay, and counted using a trypan blue staining technique. The effect of adding myriocin in the concentration corresponding to the EC₅₀ value of each cell line was examined. The number of ORL8 cells was 83.5%, slightly below the result from the control cells. However, the results were 91% or higher in the other cells. It was considered from these results that the decrease in luciferase activity was due to the anti-HCV activity of myriocin.

### (N) Acetylsalicylic Acid (ASA)

There is a recent report describing the anti-HCV activity of ASA as revealed by experiments using the HCV replicons of Con1 strain (Trujillo-Murillo K, et al., Hepatology 47:1462-1472 (2008)). Thus, the anti-HCV activity of ASA was investigated in the present assay system, and EC₅₀ was calculated.

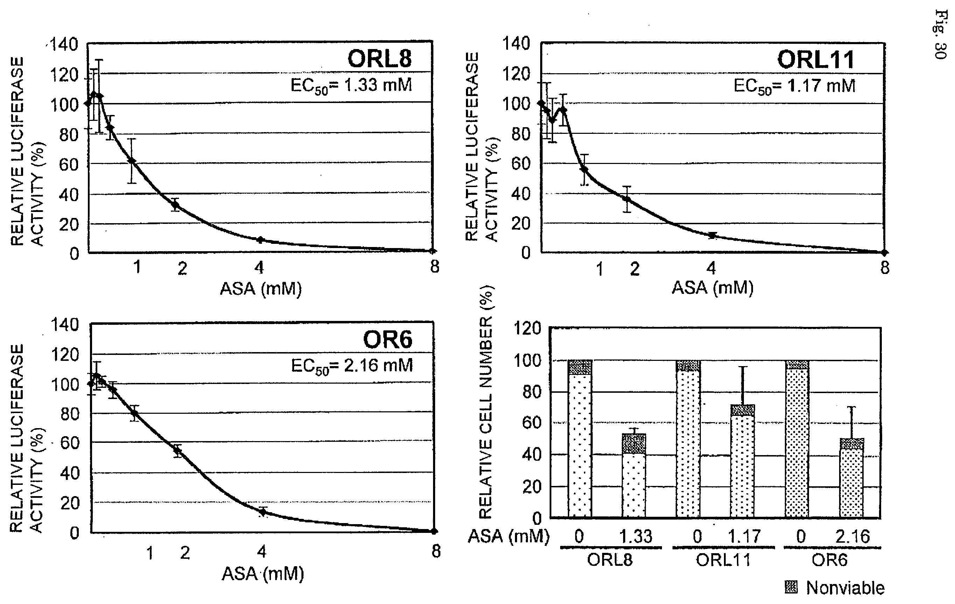

Myriocin (0, 0.125, 0.25, 0.5, 1, 2, 4, 8 mM) was added to each cell line, and luciferase activity after 72 hours was measured (the EC₅₀ value of ASA is reported to be 4 mM in the foregoing paper). By analysis, the EC₅₀ values of ORL8, ORL11, and OR6 cells were calculated as 1.33 mM, 1.17 mM, and 2.16 mM, respectively. These concentration values are slightly below the values obtained in the Corn-1 strain HCV replicon assay, and apparently confirm the anti-HCV activity of ASA. However, because there was a clear decrease in cell count in cells treated at a concentration of 2 mM, the effect of the drug on cell growth was examined by treating the ORL8, ORL11, and OR6 cells with the foregoing concentrations of ASA. As a result, the cell count decreased to 53%, 72%, and 51% of the control cells. Representative results are presented in FIG. 30. The results suggest that the decrease in luciferase activity in at least ORL8 and OR6 cells are almost completely due to cell growth inhibition. The foregoing report does not give any consideration to the cell growth inhibitory effect of ASA, and it cannot be concluded that the ASA itself has anti-HCV activity. Thus, when presented with data showing a clear decrease in cell count in the drug concentration range used in the assay, it is necessary to count and compare the cells, and test whether the decrease in luciferase activity is due to a decrease in cell count.

### 5-6 Anti-HCV Effect by Combined Use of Drugs

The assay system using OR6 cells (see Patent Document 1) also can be used to measure the combined effect of drugs with IFN-α. Thus, assessment was made whether the present assay systems using the ORL8 cells and ORL11 cells were usable for the measurement of combined effect. Note that the concentrations of the drugs used are based on the EC₅₀ values above.

### (i) Combined Use of IFN-α and CsA

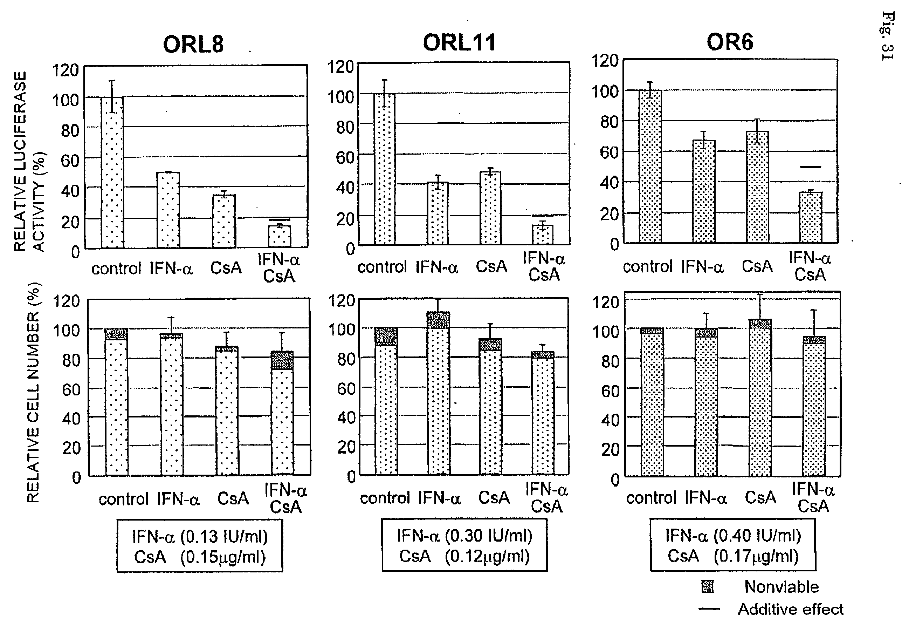

The results are presented in FIG. 31. The luciferase activities after the IFN-α treatment alone were 50% (ORL8), 41% (ORL11), and 67% (OR6). The values were 35% (ORL8), 48% (ORL11), and 73% (OR6) for CsA. The result for OR6 cells is slightly below the expected value (50%); however, this does not present itself as a problem for the measurement of combined effect. The luciferase activities after the combined use of the drugs decreased to 14% (ORL8), 13% (ORL11), and 33% (ORL6), respectively. The values of the additive effect by the combined use expected from the results of single-agent treatment were 18% (ORL8), 20% (ORL11), and 49% (OR6). The actual values of the cell assay systems were lower than the expected values by 22% (ORL8), 35% (ORL11), and 33% (OR6), so the combined use of IFN-α and CsA was found to show some synergistic effect. The synergistic effect by the combined use of IFN-α and CsA (OR6 cell assay system) is described in Patent Document 1. Thus, the ORL8-cell and ORL11-cell assay systems also can be used to assay the combined effect of IFN-α and CsA as with the OR6-cell assay system.

### (ii) Combined Use of IFN-α and FLV

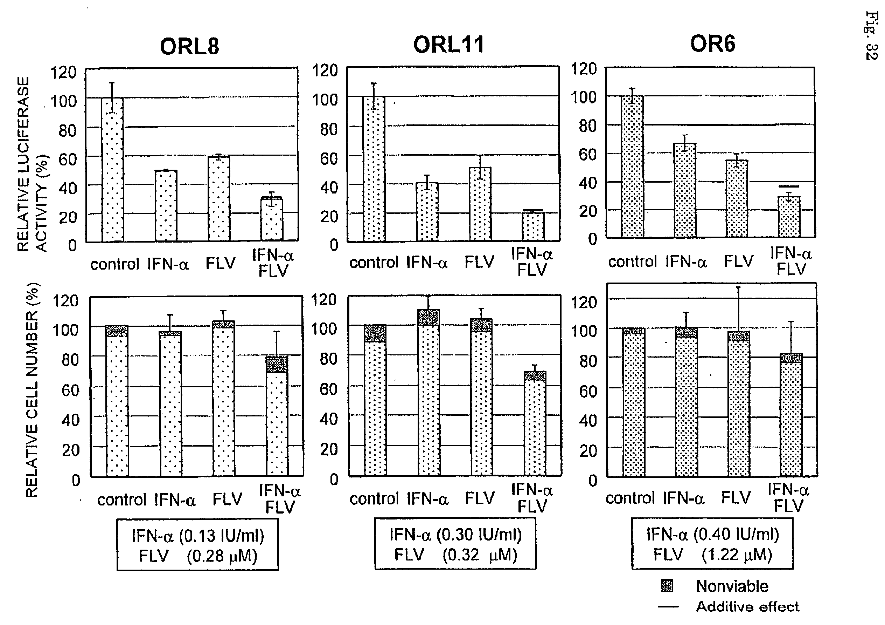

FLV (Calbiochem, 344095) was used. The results are presented in FIG. 32. The luciferase activities after the IFN-α treatment alone were 50% (ORL8), 41% (ORL11), and 67% (OR6). The values were 59% (ORL8), 51% (ORL11), and 55% (OR6) for FLV. The luciferase activities after the combined use of the drugs decreased to 29% (ORL8), 20% (ORL11), and 37% (OR6). The values of the additive effect by the combined use expected from the results of single-agent treatment were 30% (ORL8), 21% (ORL11), and 37% (OR6). These effects are considered additive because the results have good match with the actual values. However, upon checking the cell count, it was found that the drugs alone had the tendency not to inhibit almost any cell growth, but inhibit cell growth when used in combination.

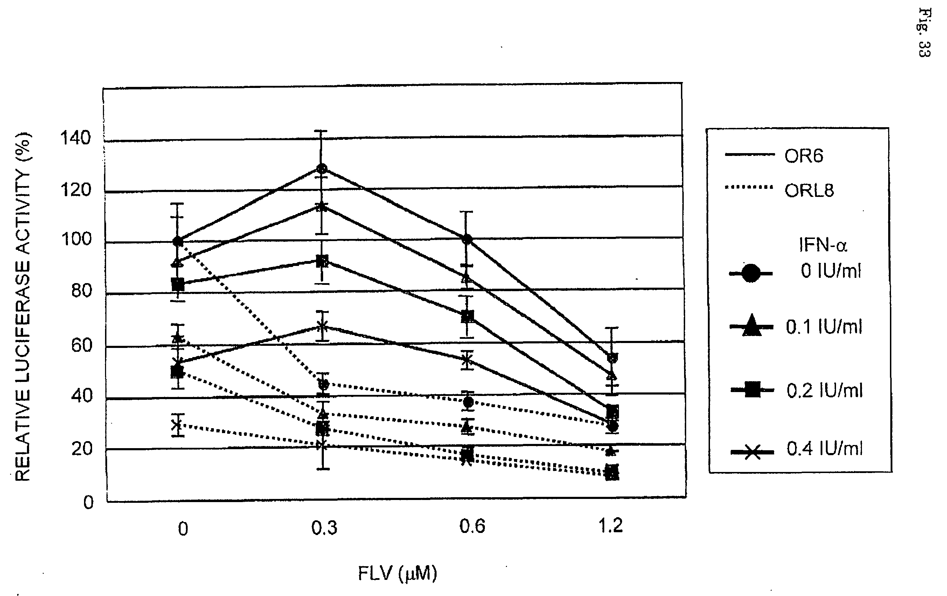

For the combined effect of IFN-α and FLV, assay was performed at different drug concentrations. FIG. 33 shows the results of assays using ORL8 cells and OR6 cells. IFN-α was added to make the final concentrations 0, 0.1, 0.2, and 0.4 IU/ml. FLV was added to make the final concentrations 0, 0.3, 0.6, and 1.2 µM. Luciferase activity was measured after 72 hours from the addition. The results were close to those expected from the results obtained from adding these agents alone. The combined effect of IFN-α and FLV represented by these results is considered additive, and the ORL8-cell assay system is considered to have better sensitivity than the OR6-cell assay system.

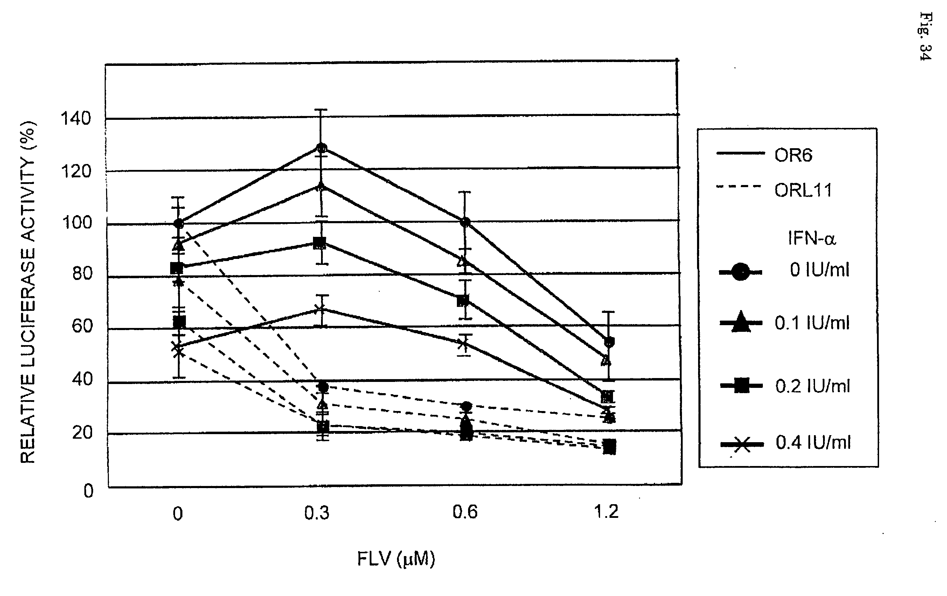

FIG. 34 shows the results of ORL11- and OR6-cell assays. The results were close to those expected from the results of single-agent treatment, and the luciferase activity had concentration-dependent attenuation patterns as did the ORL11 cells. It can also be considered from these results that the ORL11-cell assay system has better sensitivity than the OR6-cell assay system.

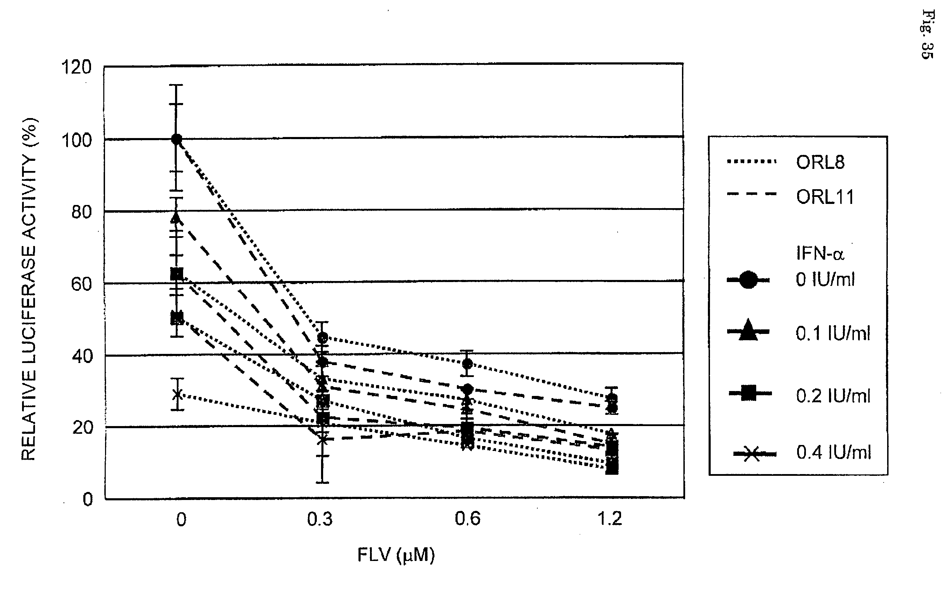

FIG. 35 shows the assay results from ORL8 and ORL11 cells. There is an overlap between the attenuation curves of luciferase activity between the two assay systems. The ORL8 cell line appeared to have slightly higher sensitivity to IFN-α; however, these assay systems were substantially the same in terms of sensitivity to the combined effect.

### (iii) Combined Use of IFN-α and PTV

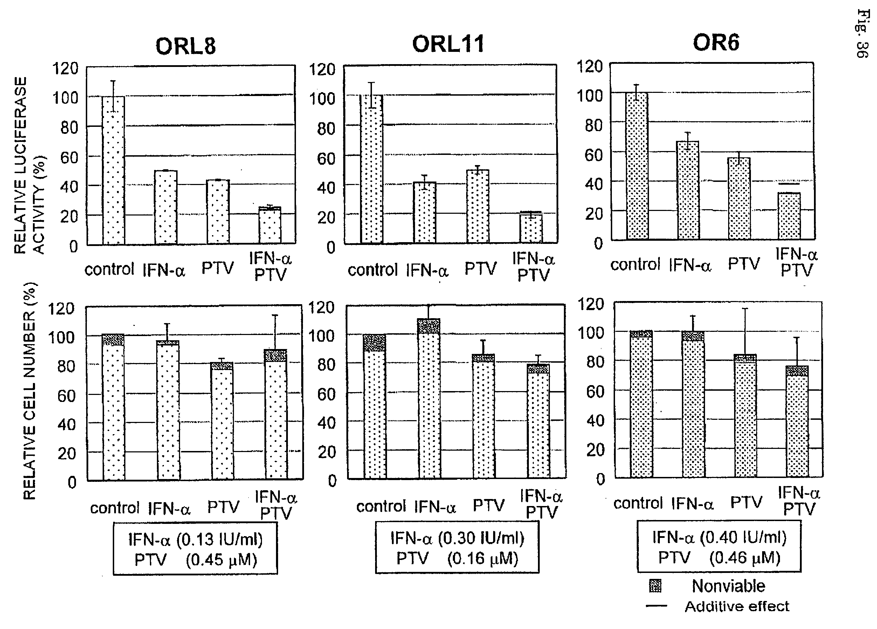

PTV (PTV lactone (prodrug)) was used. The results are presented in FIG. 36. The luciferase activities after the IFN-α treatment alone were 50% (ORL8), 41% (ORL11), and 67% (OR6). The values were 43% (ORL8), 49% (ORL11), and 56% (OR6) for PTV. The luciferase activities after the combined use of the drugs decreased to 24% (ORL8), 19% (ORL11), and 31% (OR6). The values of the additive effect by the combined use expected from the results of single-agent treatment were 22% (ORL8), 20% (ORL11), and 38% (ORL6). The value of the OR6-cell assay system was lower than the expected value by little less than 20%. However, the effect was considered additive considering the results of the other cell assay systems together.

### (iv) Combined Use of IFN-α and Ribavirin

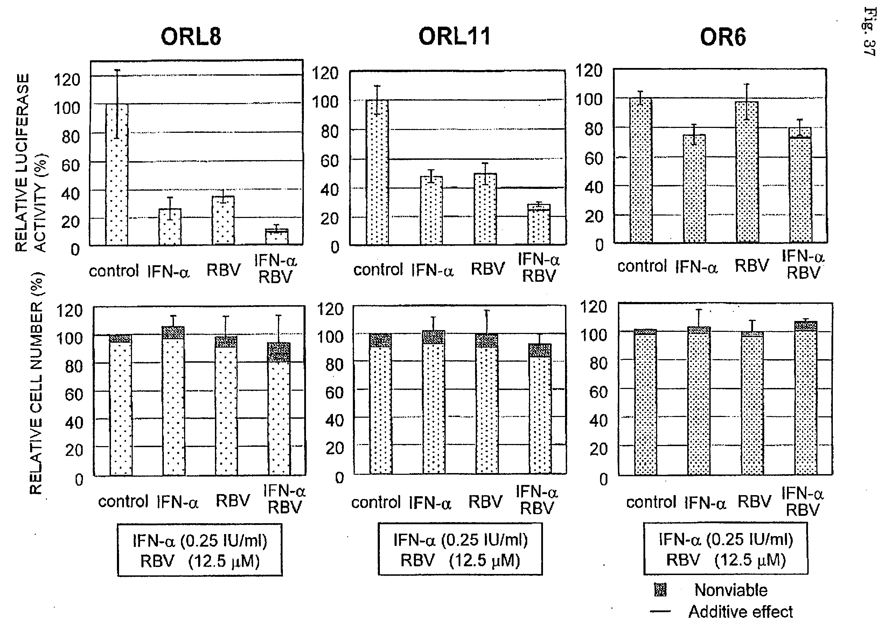

The results are presented in FIG. 37. It should be noted that the combined effect was examined under the fixed conditions of IFN-α at 0.25 IU/ml (the concentration expected to cause 35%, 55%, and 70% decreases in ORL8, ORL11, and OR6 cells, respectively), and ribavirin at 12.5 µM (the concentration expected to cause 40% and 60% decreases in ORL8 and ORL11 cells, respectively, and no decrease in OR6 cells). The luciferase activities after the IFN-α treatment alone were 26% (ORL8), 48% (ORL11), and 75% (OR6). The values were 35% (ORL8), 49% (ORL11), and 97% (OR6) for ribavirin. The luciferase activity after the combined use of the drugs decreased to 11% (ORL8), 28% (ORL11), and 80% (OR6). The values of the additive effect by the combined use expected from the results of single-agent treatment were 9% (ORL8), 24% (ORL11), and 73% (OR6). The effect represented by these results was considered additive, even though the actual values in these cell assay systems were slightly higher (9 to 18%) than the expected values.

### 6: Infectious HCV Particle-Producing Cell Line

### 6-1 HCV Infection of OL Cured Cells

When HuH-7 cell-derived cells are used, only the HCV type 2a-derived JFH11 strain HCV can reproduce the HCV lifecycle. The ability of Li23 cell-derived cells to reproduce the HCV lifecycle was investigated. First, it was investigated whether the cured cells prepared from OL8 cells and OL11 cells obtained as the full-length HCV RNA replicating cells were capable of JFH1 strain HCV RNA replication.

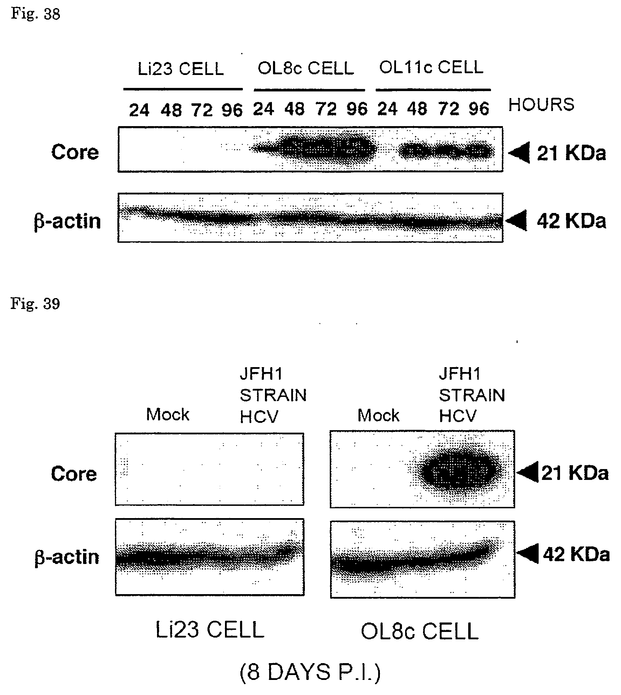

JFH1 strain HCV RNA (20 µg) was introduced into Li23 cells (parental strain), OL8c cells, and OL11c cells (2 x 10⁶ each), using the electroporation technique. Following the introduction, the cells were transferred to a 6-well plate (about 4 x 10⁵ each), and the expression levels of the core protein in each cell line (1/20 of the cells in each well was used for the analysis) were analyzed by Western blotting after 24, 48, 72, and 96 hours (FIG. 38). The core protein was not recognized at all in Li23 cells. In OL8c cells, the core protein was recognized 24 hours after the introduction, and enhancement of expression level was observed after 48 hours. The core protein was not detected in OL11c cells after 24 hours from the introduction; however, expression of the core protein was recognized after 48 hours. These results appear to be solely due to the replication and propagation of the JFH1 strain HCV RNA in the OL8c cells and OL11c cells, suggesting that the OL8c and OL11c cells are permissive for the replication of the HCV RNAs of not only the HCV-O strain but also the JFH1 strain.

RSc cells were infected with JFH1 strain HCV, and the supernatant after 145 days was used as virus fluid. The virus fluid was expected to contain 10^{5.3} TCID₅₀ of infectious HCV particles per ml. The RSc cells are HuH-7 cell-derived cloned cells, and efficiently and persistently produce infectious HCV particles with the JFH1 strain HCV RNA introduced into the cells (Ariumi et al., JVI 81:13922-13926, 2007).

The virus fluid was added to Li23 cells (2 x 10⁴ cells/24 wells) and to OL8c cells (2 x 10⁴ cells/24 wells), and the medium was replaced after 2 hours. After 8 days from the infection, the expression level of core protein in each cell line was determined by Western blotting. Mock experiment was conducted in parallel using culture. An equivalent of 2 x 10⁴ cells was used for the assay. While the core protein was not detected at all in Li23 cells, strong core protein expression was recognized in OL8c cells (FIG. 39). The results suggest that the OL8c cells are permissive not only for the replication of JFH1 strain HCV RNA but also for the infection of JFH1 strain HCV.

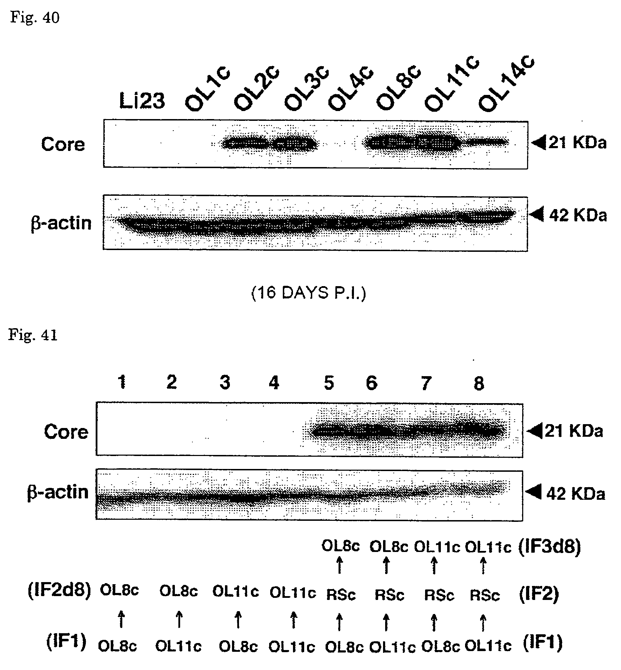

Non-OL8c cured cells were also examined with respect to JFH1 strain HCV infection and propagation. JFH1 strain HCV infection experiment was conducted using Li23, and cured cells (OL1c cells, OL2c cells, OL3c cells, OL4c cells, OL8c cells, OL11c cells, and OL14c cells) prepared from various cloned OL cells. The expression level of core protein in each cell line after 16 days from the infection was determined by Western blotting. While the core protein was not detected at all in Li23 cells, strong core protein expression was recognized in OL8c cells even after 16 days from infection (FIG. 40). This suggests sustained HCV RNA replication in the cells after the infection. The OL2c cells, OL3c cells, and OL11c cells had the same level of core protein expression as OL8c cells, whereas expression was weak in OL14c cells. The core protein was not detected at all in OL1c cells and OL4c cells. Given the lack of correlation with the amount of full-length HCV RNA (the replication level of HCV-O strain HCV RNA) in the cells, the differences among the cell clones may be due to differences in permissiveness in the HCV infection step, or differences in replication efficiency based on different HCV strains.

### 6-2 Production of Infectious HCV Particles from OL Cured Cells

Production of infectious HCV particles from JFH1 strain HCV-infected OL8c and OL11c cells was examined. Note that the cells were infected by replacing medium after 2 hours from the addition of virus fluid to the cells (FIG. 41).

The culture supernatants of OL8c and OL11c cells (2 x 10⁴ cells/24 wells each) 7 days post infection (100 µl each, IF1 in the figure) were used to infect OL8c and OL11c cells (2 x 10⁴ cells/24 wells each). The core protein expression level of each cell line 8 days post infection (IF2d8 in the figure) was analyzed by Western blotting. The results are shown in lanes 1 to 4. The core protein was not detected in OL8c and OL11c cells, and it was not possible to confirm production of infectious HCV particles from OL8c and OL11c cells.

The culture supernatants of OL8c and OL11c cells (2 x 10⁴ cells/24 wells each) 7 days post infection (100 µl each, IF1 in the figure) were used to infect RSc cells (2 x 10⁴ cells/24 wells). The culture supernatant 7 days post infection (100 µl, IF2 in the figure) was used to infect separately prepared OL8c and OL11c cells (2 x 10⁴ cells/24 wells each). The core protein expression level in each cell line 8 days post infection (IF3d8 in the figure) was analyzed by Western blotting. The results are shown in lanes 5 to 8. The core protein was detected in OL8c and OL11c cells. The result suggests small production of infectious HCV particles from OL8c and OL11c cells, and production and amplification of infectious HCV particles via RSc cells.

Because small production of infectious HCV particles from OL8c and OL11c cells was indicated, the HCV infected cells were continuously cultured for 27 days, and analyzed by Western blotting.

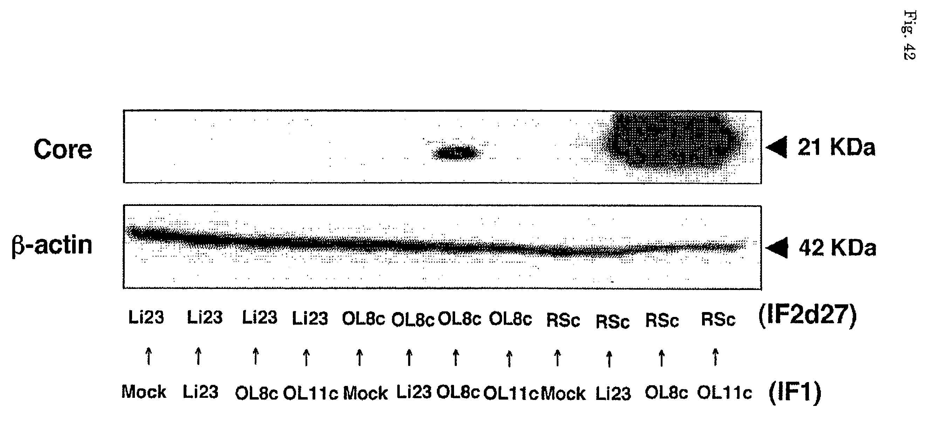

The culture supernatants of OL8c and OL11c cells (2 x 10⁴ cells/24 wells each) 7 days post infection (100 µl each, IF1 in the figure) were used to infect OL8c and OL11c cells (2 x 10⁴ cells/24 wells each). As a control of HCV-producing cells, Mock experiment using a Li23 cell supernatant or culture was also conducted (FIG. 42). The core protein expression level in each cell line 27 days post infection (IF2d27 in the figure) was analyzed by Western blotting. It was found that the expression level of core protein in the cells was very high in the infection of RSc cells with the supernatants of the JFH1 strain HCV-infected OL8c and OL11c cells. Conceivably, this is due to the propagation of infectious HCV particles via RSc cells.

The core protein 27 days post infection reached the detectable level by Western blot analysis only when the culture supernatant derived from the JFH1 strain HCV-infected ORL8c cells were used to reinfect OL8c cells. This fact suggests the completion of the HCV lifecycle in OL8c cells, specifically, reproduction of JFH1 strain HCV as infectious particles after replication and propagation in the infected OL8c cells, and replication and propagation of the infectious particles in the reinfected OL8c cells.

### 6-3 Preparation of ORL Cured Cell Lines

Though OL8c cells were shown to be capable of reproducing the HCV lifecycle, the capability is far below those of other cells such as RSc cells. Further, from the practical standpoint, experiment takes time. Thus, ORL8c and ORL11c cells considered to be superior to OL8c and OL11c cells in terms of HCV RNA replication environment were prepared by adding IFN-γ (10³ IU/µl) to ORL8 and ORL11 cells in the absence of G418.

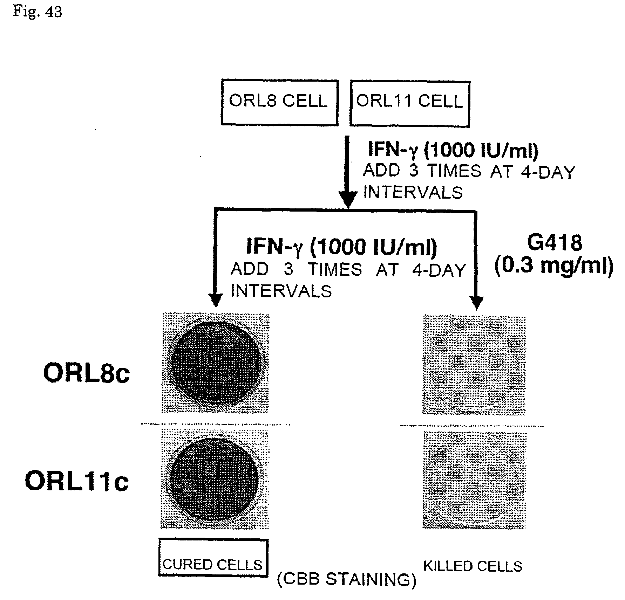

About 5 x 10⁵ ORL8 cells and ORL11 cells were inoculated on dishes having an outer diameter of 10 cm, and IFN-γ (1,000 IU/ml) was added three times at 4-day intervals. The cells were appropriately subcultured when the dish became full. The cells subcultured after the 3rd addition of IFN-γ were divided into two groups of dishes. One group contained medium supplemented with G418 (0.3 mg/ml) and NaHCO₃ (0.15%). The petri dish in the other group was continuously used to culture the cells with the medium alone. IFN-γ was then added three times to each group while the cells were subcultured as required, followed by CBB staining. While the cells cultured in the G418-free medium grew and filled the dish (cured cells), the cells were completely killed when continuously cultured in the medium supplemented with G418 (FIG. 43).

### 6-4 Production of Infectious HCV Particles from ORL Cured Cells

The prepared cured cells (ORL8c or ORL11c cells) were used for JFH1 strain HCV infection experiment, and HCV particle production capability was examined. As noted above, the Rsc cells used as control are HuH-7 cell-derived cloned cells, and efficiently and persistently produce infectious HCV particles with the JFH1 strain HCV RNA introduced into the cells (Ariumi et al., JVI 81:13922-13926, 2007).

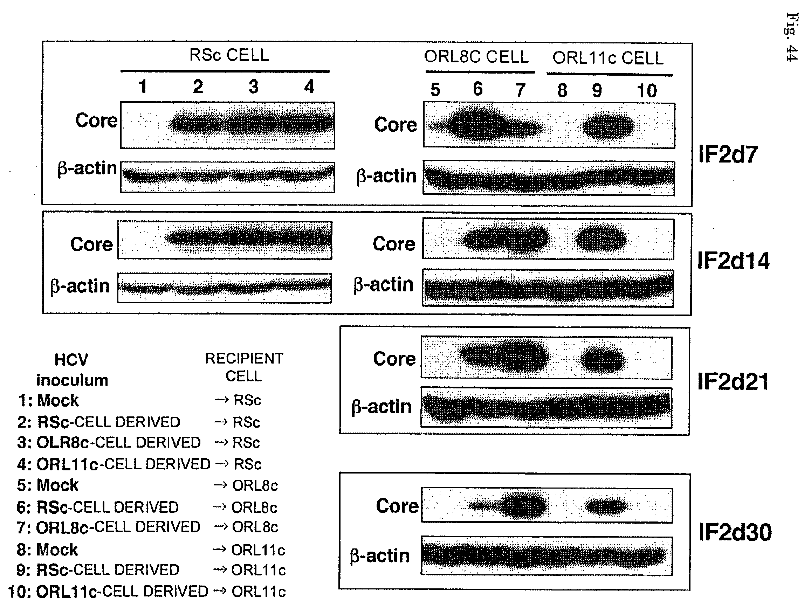

The culture supernatants of RSc cells, ORL8c cells, and ORL11c cells (2 x 10⁴ cells each (3 x 10⁴ cells for ORL11c cells)/24 wells; 100 µl each) 7 days post infection were used to infect separately prepared RSc cells (2 x 10⁴ cells/24 wells). Mock infection experiment was also conducted using culture. The core protein expression levels in each cell line 7 days and 14 days post infection (IF2d7 and IF2d14, respectively, in the figure) were analyzed by Western blotting (lanes 1 to 4 in FIG. 44). The core protein expression levels were substantially the same in lanes 2, 3, and 4. That is, the JFH1 strain HCV-infected RSc, ORL8c, and ORL11c cells produced substantially the same level of infectious HCV, and the ORL8c and ORL11c cells were shown to have higher infectious HCV production capability than OL8c and OL11c cells, comparable to that of HuH-7 cell-derived RSc cells.

The culture supernatants of RSc and ORL8c cells (2 x 10⁴ cells/24 wells each) 7 days post infection (100 µl each) were used to infect separately prepared ORL8c cells (2 x 10⁴ cells/24 wells). Mock infection experiment was also conducted using culture. The core protein expression levels in each of the cells 7 days, 14 days, 21 days, and 30 days post infection (IF2d7, IF2d14, IF2d21, and IF2d30, respectively, in the figure) were analyzed by Western blotting (lanes 5 to 7 in FIG. 44). The level of the core protein produced in the ORL8c cells infected with HCV produced from RSc cells became maximum after 7 days from infection, remained at almost the same level until day 21, and decreased by day 30 (lane 6). On the other hand, the core protein produced in the ORL8c cells infected with HCV produced from ORL8c cells had a substantial expression level by 7 days post infection, and the expression level increased time-dependently, and was maintained at higher levels even after 30 days relative to day 7 (lane 7). These results suggest that the HCV produced in ORL8c cells reinfects ORL8c cells, and that HCV production is maintained for at least 1 month. ORL8c cells were found to have infectious HCV particle production capability far superior to that of OL8c cells, which is the parental cell line of ORL8c cells, and such HCV production capability was comparable to that of HuH-7 cell-derived RSc cells.

The culture supernatants of RSc and ORL11c cells (2 x 10⁴ cells or 3 x 10⁴ cells/24 wells each) 7 days post infection (100 µl each) were used to infect separately prepared ORL11c cells (3 x 10⁴ cells/24 wells). Mock experiment was also conducted using culture. The core protein expression levels in each cell line 7 days, 14 days, 21 days, and 30 days post infection (IF2d7, IF2d14, IF2d21, and IF2d30, respectively, in the figure) were analyzed by Western blotting (lanes 8 to 10 in FIG. 44). As in the case of ORL8c cells, the level of the core protein produced in the ORL11c cells infected with HCV produced from RSc cells became maximum after 7 days from infection, remained at about the same level until day 21, and decreased by day 30 (lane 9). On the other hand, the core protein was not produced in the ORL11c cells infected with HCV produced from ORL11c cells (lane 10). It was found from these results that, unlike ORL8c cells, the ORL11c cells were permissive for infection and propagation of HCV produced from RSc cells, but not permissive for reinfection of ORL11c cells by the ORL11c cell-produced HCV and for propagation of the ORL11c cell-produced HCV.

### 6-5 HCV RNA Replication Level in ORL Cured Cells

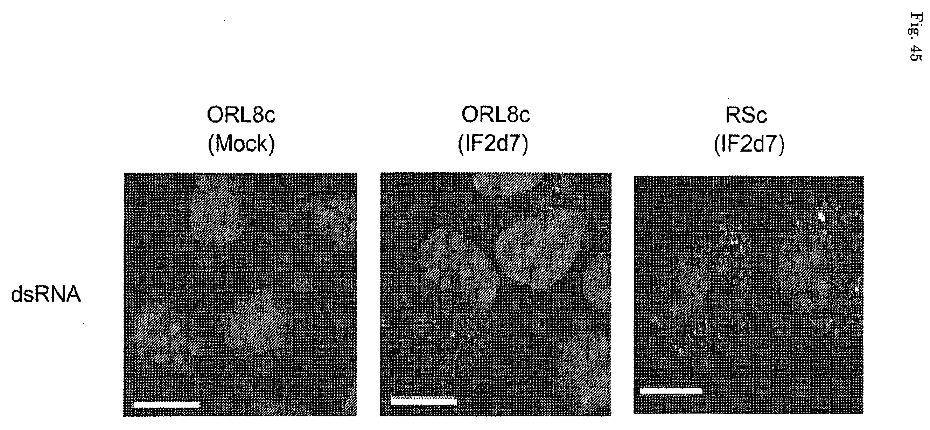

For detection of double-stranded RNA (dsRNA), a replication intermediate of HCV RNA, in JFH1 strain HCV-infected ORL8c cells (IF2d7), ORL8c cells were observed using the immunofluorescent technique with anti-dsRNA antibodies, according to the foregoing procedure. JFH1 strain HCV-infected RSc cells were used as positive control, and mock-infected ORL8c cells as negative control. The results of observation using a confocal laser scanning microscope are shown in FIG. 45 (bar length, 20 µm).

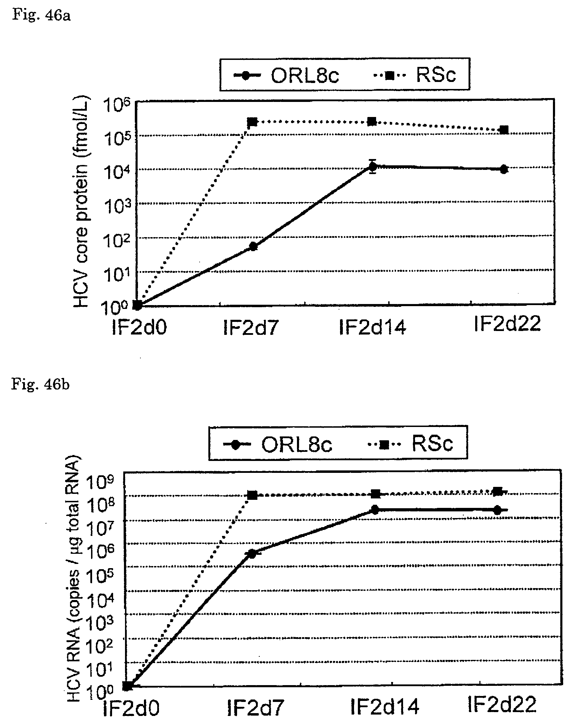

As shown in the figure, dot-like fluorescence scattered over the cytoplasm was observed in HCV-infected ORL8c and RSc cells. Such fluorescence was not observed at all in the mock-infected ORL8c cells. These results can be taken as the basis for the specific detection of dsRNA (replication intermediate of HCV RNA) in ORL8c cells. Note that the fact that the fluorescence intensity observed in ORL8c cells was comparable to that of RSc cells suggests that the HCV RNA replication level in ORL8c cells does not differ greatly from that in RSc cells, as shown in FIG. 46(b).

The HCV replication levels in ORL8c cells and RSc cells were compared. In order to detect and quantify the HCV particles released into the culture supernatants of ORL8c cells and RSc cells at the same time points used in FIG. 44 (7 days, 14 days, 21 days, and 30 days post infection; IF2d7, IF2d14, IF2d21, and IF2d30, respectively, in the figure), the secretion levels of HCV core protein in the culture supernatants of JFH1 strain HCV-infected ORL8c and RSc cells (IF2d7, IF2dl4, IF2d22) were measured by ELISA (measurement was made by Mitsubishi BCL). Experiment was conducted three times, and 1 ml of supernatant was used for the measurement (FIG. 46(a)).

As shown in the figure, in RSc cells, the level of HCV particles released into the culture supernatant reached 10⁵ fmol/L or more 7 days post injection, and leveled off or decreased thereafter. On the other hand, in ORL8c cells, the HCV particle level remained low at 10² fmol/L 7 days post infection, increased to 10⁴ fmol/L by 14 days post infection, and remained at the same level until day 22 post infection. Taken together, it was found that the HCV particle production capability of ORL8c cells was one order of magnitude smaller than that of RSc cells. However, this level was found to be sufficient for the behavior analysis of various viruses.

The HCV RNAs in these cells were quantified according to the procedure of real-time LightCycler PCR described above. Experiment was conducted three times (FIG. 46(b)). In RSc cells, the RNA level reached 10⁸ copies/µg total RNA at day 7 post infection, and remained at almost the same level until day 22 post infection, as with the released level of core protein into the culture supernatant. On the other hand, in ORL8c cells, the RNA level was only about 5 x 10⁵ copies/µg total RNA at day 7 post infection. However, the RNA level reached about 5 x 10⁷ copies/µg total RNA on day 14 post injection, and remained at the same level until day 22. It was found from these results that high HCV RNA replication levels were maintained in the both cells.

### 6-6 The Correlation between HCV Receptor Expression Level and HCV Particle Production Capability

For comparison of HCV receptor expression levels in various cells, the expression level of mRNA was measured using a RT-PCR method and a quantitative RT-PCR method.

HuH-7, RSc, Li23, ORL8c, and ORL11c cells were cultured in 10-cm plates (medium, 10 ml). Total RNA was extracted from these cells using an RNeasy Mini Kit (Qiagen) according to the manufacturer's experiment protocol attached to the kit. Using 2 µg of RNA as a template, reverse transcription (RT) reaction was performed with superscript^{™} II reverse transcriptase (Invitrogen) and oligo dT (Invitrogen) according to the manufacturer's experiment protocols. The resulting cDNA was used as template, and PCR was performed with the primer sets shown in Table 3. PCR amplification products were detected by ethidium bromide staining after 3% agarose gel electrophoresis.

**[table 3]**

| Primers used for RT-PCR analysis | | | | | |
|---|---|---|---|---|---|
| Gene (Accession No.) | Direction | Base sequence | | Amplification product (bp) | Number of cycles |
| CD81 | Forward | ACCTTCCACGAGACGCTT | SEQ ID NO: 18 | 222 | 25 |
| (NM_004356) | Reverse | CAGGATCATCTCGAAGATCATG | SEQ ID NO: 19 | | |
| | | | | | |
| SR-B1 | Forward | GGTGCGGCGGTGATGATG | SEQ ID NO: 20 | 225 | 25 |
| (NM_005505) | Reverse | CCCAGAGTCGGAGTTGTTGAG | SEQ ID NO: 21 | | |
| | | | | | |
| CLDN1 | Forward | GGGGTGCGATATTTCTTCTTG | SEQ ID NO: 22 | 129 | 25 |
| (NM_021101) | Reverse | GAGCCTGACCAAATTCGTACC | SEQ ID NO: 23 | | |
| | | | | | |
| OCLN | Forward | TTCACTTCTACAAATGGACC | SEQ ID NO: 24 | 235 | 25 |
| (NM_002538) | Reverse | TAGCCTCCGTAGCCATAGCC | SEQ ID NO: 25 | | |
| | | | | | |
| GAPDH | Forward | GACTCATGACCACAGTCCATGC | SEQ ID NO: 26 | 334 | 22 |
| (NM_002046) | Reverse | GAGGAGACCACCTGGTGCTCAG | SEQ ID NO: 27 | | |

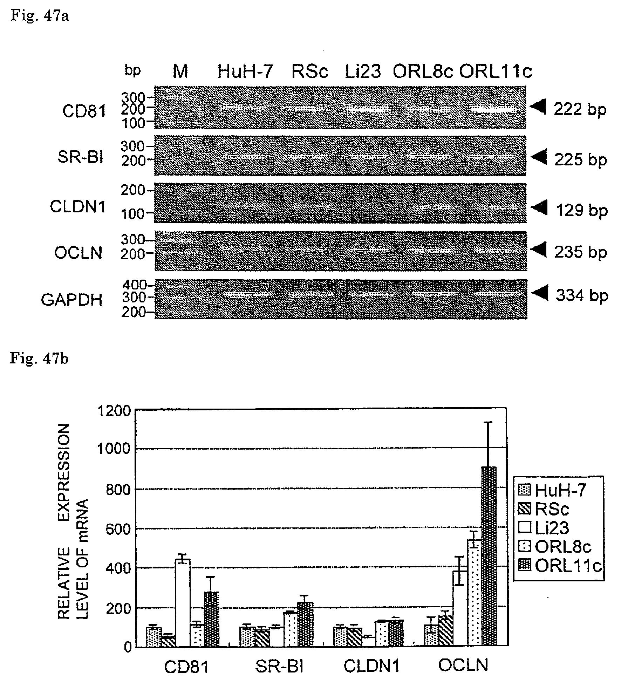

Expression of CD81, SR-B1, CLDN1 (Claudin-1), and OCLN (Occludin), reported to be HCV receptors, was confirmed in all cells along with GAPDH as internal control, though the detected bands had slightly different shades (FIG. 47(a)). For details of these HCV receptors, see Burlone M.E. and Budkowska A. Hepatitis C virus cell entry: role of lipoproteins and cellular receptor, Journal of General Virology 90, 1055-1070 (2009).

The cDNA was also used for quantification of HCV RNA according to the procedure of real-time LightCycler PCR. As a result, HCV receptor expression was confirmed in all cells as in the result of qualitative experiment presented in FIG. 47(a), and there was no HCV receptor that had a lower expression level only in ORL8c cells relative to RSc cells. Further, CLDN1 and OCLN had higher expression levels in ORL8c cells than in RSc cells (FIG. 47(b)).

### 6-7 Establishment of Full-Length HCV RNA Replicating Cell Line Derived from Non-HCV-O HCV Strains

ORL8c cells are cured cells produced by IFN-γ treatment that excludes HCV RNA from cells capable of replicating luciferase gene-carrying full-length HCV RNA derived from HCV-O strain. Thus, with ORL8c cells, it might be possible to establish cell lines capable of replicating full-length HCV RNA that derives from non-HCV-O HCV strains.

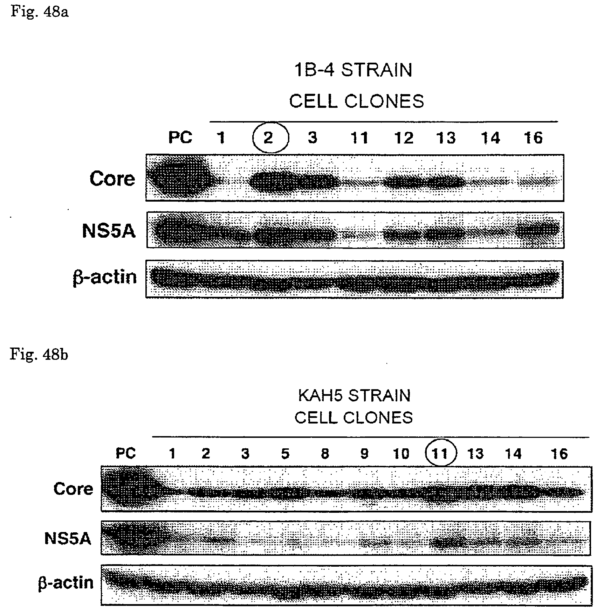

Luciferase gene-carrying full-length HCV RNA (10 µg) synthesized in vitro using the template plasmids (p1B-4RN/C-5B and pKAH5RN/C-5B) prepared from the HCV carrier strain 1B-4 (genotype 1b) and the acute hepatitis patient-derived HCV strain KAH5 (genotype 1b), respectively, was introduced into 2 x 10⁶ ORL8c cells using the electroporation technique. The plasmid p1B-4RN/C-5B had mutations Q1067R and S2200R introduced at two locations. The plasmid pKAH5RN/C-5B had mutations Ins2040K (insertion of lysine at position 2040 of the HCV polyprotein), R2328Q, E2401G, and V2416A introduced at four locations. S2200R and Ins2040K are known adaptive mutations. Starting from day 2 after the introduction of RNA, the medium was replaced with G418 (0.3 mg/ml)-containing medium every 4 days, and the cells where cultured for about 3 weeks. Cells believed to have high levels of sustained replication of luciferase gene-carrying full-length HCV RNA were obtained as G418-resistant colonies (clones). For the selection of cell clones having high HCV RNA replication levels from these clones, the expression levels of core protein and NS5A protein were analyzed by Western blotting (FIG. 48). OR6c cell-derived cells that showed efficient replication of HCV-O strain-derived full-length HCV RNA (with four adaptive mutations) were used as positive control (PC in the figure).

As shown in FIG. 48(a), the 1B-4 strain had the highest level of HCV protein expression in clone #2. Cell clone #2 was grown into a new cell line (will be referred to as "1B-4RL8 cells"). As shown in FIG. 48(b), the KAH5 strain had the highest level of HCV protein expression in clone #11. Cell clone #11 was grown into a new cell line (will be referred to as "KAH5RL8 cells").

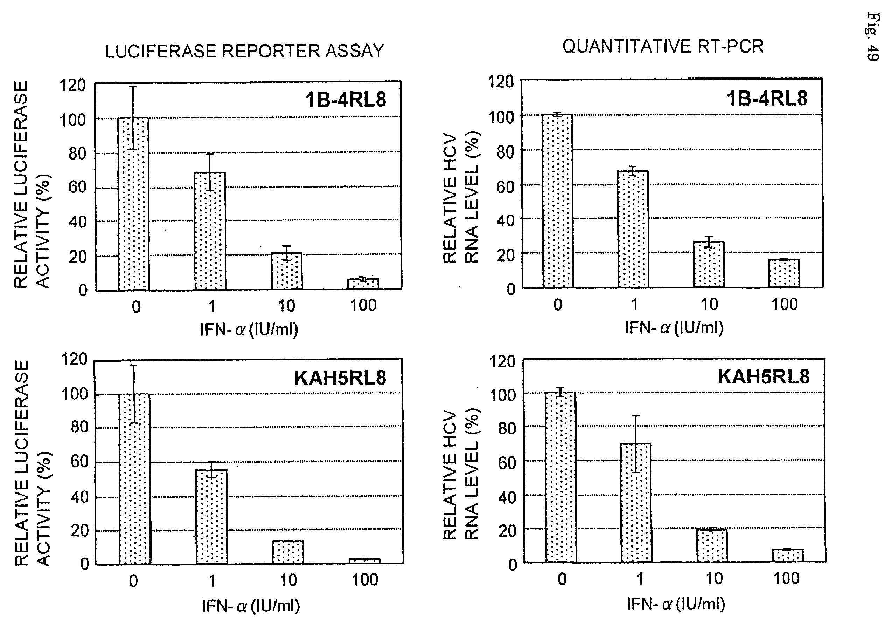

The 1B-4RL8 and KAH5RL8 cells were examined with regard to a possible correlation between luciferase activity and HCV RNA level (FIG. 49). The graphs on the left represent the measured luciferase activities 24 hours after the addition of IFN-α (0, 1, 10, 100 IU/ml) to the 1B-4RL8 and KAH5RL8 cells (each cultured for 2 days after inoculating 2 x 10⁴ cells in a 24-well plate). Measurements were made in at least 3 wells at each point. SD values are also shown in the figure. A one hundred percent measurement value of 300,000 was obtained for the 1B-4RL8 and KAH5RL8 cells. Though the result is slightly below the results obtained for ORL8 and ORL11 cells (FIG. 14), the value is sufficient for the analysis of drug anti-HCV effects. The graphs on the right represent the results of quantitative HCV RNA measurements by LightCycler PCR 24 hours after the addition of IFN-α (0, 1, 10, 100 IU/ml) to the 1B-4RL8 and KAH5RL8 cells (each cultured for 2 days after inoculating 2 x 10⁵ cells in a 6-well plate). Measurements were made in at least 3 wells at each point. SD values are also shown in the figure. As represented in the figure, the luciferase activity and the HCV RNA level decreased in a manner that depended on IFN-α concentration.

The results of these measurements had a good correlation as did the results from OR6 cells. It was therefore demonstrated that the 1B-4RL8 and KAH5RL8 cells were useful for the quantification of HCV RNA replication level with a simple luciferase assay, and could provide desirable assay systems for the activity evaluation of anti-HCV agents against 1B-4 strain HCV and KAH5 strain HCV. Specifically, it can be said that ORL8c cells have not only infectious JFH1 strain HCV production capability, but the ability to permit replication of HCV RNAs of new HCV strains (particularly, replication of RNAs longer than the original HCV RNA (9.6 kb), as in the 12-kb RNA having a luciferase gene).

The present invention is not limited to the description of the embodiments above, but may be altered within the scope of the claims. An embodiment based on a proper combination of technical means disclosed in different embodiments is encompassed in the technical scope of the present invention.

### Industrial Applicability

According to the present invention, an HCV life cycle reproduction system derived from a non-HuH-7 cell line can be constructed. Further, combined use of this system with an HCV life cycle reproduction system derived from a known HuH-7 cell line enables the detection of a substance that is known to have an anti-HCV action, and also enables the detection of the anti-HCV action of a substance that is considered not to have an anti-HCV action. Accordingly, the present invention can provide a method of screening a substance having an anti-HCV action, and a therapeutic agent for hepatitis C, and thus contributes to the development of the reagent industry, the pharmaceutical industry, etc.

## Claims

1. A method of producing an HCV replicon-replicating cell, comprising a step of introducing RNA containing an HCV replicon sequence and a selectable marker gene sequence into a Li23 cell (FERM-BP-11150) or a cured cell of a Li23-derived HCV replicon-replicating cell or a cured cell of a Li23-derived full-length HCV RNA-replicating cell wherein the HCV replicon sequence comprises a base sequence encoding an amino acid sequence as set forth in SEQ ID NO: 2 containing amino acid substitutions at Q1112R, K1609E and S2200R or at Q1112R, P1115L and S2200R.

2. The method according to claim 1 wherein the HCV replicon sequence is as set forth in SEQ ID NO: 3 or 5.

3. A method of producing a full-length HCV RNA-replicating cell, comprising a step of introducing RNA containing a full-length HCV genome sequence and a selective marker into a cured cell of a Li23 (FERM-BP-11150)-derived HCV replicon-replicating cell or a cured cell of a Li23 (FERM-BP-11150)-derived full-length HCV RNA-replicating cell;
wherein the Li23-derived HCV replicon-replicating cell is produced by a method comprising
a step of introducing RNA containing an HCV replicon sequence and a selectable marker gene sequence into a Li23 cell (FERM-BP-11150), a cured cell of a Li23-derived HCV replicon-replicating cell, or a cured cell of a Li23-derived full-length HCV RNA-replicating cell,
wherein the HCV replicon sequence comprises a base sequence encoding an amino acid sequence as set forth in SEQ ID NO: 2 containing animo acid substitutions at Q1112R, K1609E and S2200R or at Q1112R, P1115L and S2200R.

4. The method according to claim 3 wherein the full-length HCV genome sequence comprises a base sequence encoding an amino acid sequence as set forth in SEQ ID NO: 2 containing amino acid substitutions at Q1112R, K1609E and S2200R or at Q1112R, P1115L and S2200R.

5. The method according to claim 4 wherein the base sequence is as set forth in SEQ ID NO: 7 or 9.

6. The method according to claim 4 wherein the full-length HCV genome sequence contains a base sequence as set forth in SEQ ID NO: 11 or 13.

7. The method according to any one of claims 3 to 6 wherein the RNA further contains a reporter gene sequence.

8. The method according to any one of claims 3 to 6 wherein the RNA further contains an exogenous internal ribosomal entry site (IRES) sequence.

9. A method of screening a substance having an anti-HCV action, comprising
a step of incubating a cell prepared by the method of any one of claims 1 to 6 with a candidate agent; and
a step of measuring the level of an HCV gene product.

10. A kit for screening a substance having an anti-HCV action, comprising
a cell prepared by the method of any one of claims 1 to 6; and
a reagent for measuring the level of an HCV gene product.

11. A method of screening a substance having an anti-HCV action, comprising
a step of incubating a cell prepared by the method of claim 7 with a candidate agent; and
a step of measuring the level of a reporter gene product.

12. A kit for screening a substance having an anti-HCV action, comprising
a cell prepared by the method of claim 7; and
a reagent for measuring the level of a reporter gene product.

13. A method of producing a cured cell derived from a Li23 cell, comprising a step of culturing the cell prepared by the method of any one of claims 1 to 8 in a medium containing a pharmaceutical agent having an anti-viral action.

14. A method of producing an infectious HCV particle, comprising a step of incubating the cured cell prepared by the method of claim 13 with infectious HCV RNA.

## Patentansprüche

1. Verfahren zur Herstellung einer HCV-Replikon-replizierenden Zelle, umfassend einen Schritt des Einführens von RNA, die eine HCV-Replikonsequenz und eine selektierbare Marker-Gensequenz enthält, in eine Li23-Zelle (FERM-BP-11150) oder in eine kurierte Zelle (cured cell) einer von Li23 abstammenden HCV-Replikon-replizierenden Zelle oder in eine kurierte Zelle einer von Li23 abstammenden Zelle, die HCV-RNA voller Länge repliziert, wobei die HCV-Replikonsequenz eine Basensequenz umfasst, die eine wie in SEQ ID NO:2 gezeigte Aminosäuresequenz codiert, die die Aminosäuresubstitutionen Q1112R, K1609E und S2200R oder Q1112R, P1115L und S2200R enthält.

2. Verfahren nach Anspruch 1, wobei die HCV-Replikonsequenz wie in SEQ ID NO:3 oder 5 gezeigt ist.

3. Verfahren zum Herstellen einer Zelle, die HCV-RNA voller Länge repliziert, umfassend einen Schritt des Einführens von RNA, die eine HCV-Genomsequenz voller Länge und einen selektiven Marker enthält, in eine kurierte Zelle einer von Li23 (FERM-BP-11150) abstammenden HCV-Replikon-replizierenden Zelle oder in eine kurierte Zelle einer von Li23 (FERM-BP-11150) abstammenden Zelle, die HCV-RNA voller Länge repliziert;
wobei die von Li23 abstammende HCV-Replikon-replizierende Zelle durch ein Verfahren hergestellt wird, umfassend
einen Schritt des Einführens von RNA, die eine HCV-Replikonsequenz und eine selektierbare Marker-Gensequenz enthält, in eine Li23-Zelle (FERM-BP-11150), in eine kurierte Zelle einer von Li23 abstammenden HCV-Replikon-replizierenden Zelle oder in eine kurierte Zelle einer von Li23 abstammenden Zelle, die HCV-RNA voller Länge repliziert,
wobei die HCV-Replikonsequenz eine Basensequenz umfasst, die eine wie in SEQ ID NO:2 gezeigte Aminosäuresequenz codiert, die die Aminosäuresubstitutionen Q1112R, K1609E und S2200R oder Q1112R, P1115L und S2200R enthält.

4. Verfahren nach Anspruch 3, wobei die HCV-Genomsequenz voller Länge eine Basensequenz umfasst, die eine wie in SEQ ID NO:2 gezeigte Aminosäuresequenz codiert, die die Aminosäuresubstitutionen Q1112R, K1609E und S2200R oder Q1112R, P1115L und S2200R enthält.

5. Verfahren nach Anspruch 4, wobei die Basensequenz wie in SEQ ID NO:7 oder 9 gezeigt ist.

6. Verfahren nach Anspruch 4, wobei die HCV-Genomsequenz voller Länge eine wie in SEQ ID NO:11 oder 13 gezeigte Basensequenz enthält.

7. Verfahren nach einem der Ansprüche 3 bis 6, wobei die RNA des Weiteren eine Reportergensequenz enthält.

8. Verfahren nach einem der Ansprüche 3 bis 6, wobei die RNA des Weiteren eine exogene interne ribosomale Eintrittsstellen (internal ribosomal entry site; IRES)-Sequenz enthält.

9. Verfahren zum Screenen einer Substanz, die eine anti-HCV-Wirkung hat, umfassend einen Schritt des Inkubierens einer Zelle mit einem Kandidaten-Agens, wobei die Zelle durch das Verfahren nach einem der Ansprüche 1 bis 6 hergestellt wurde; und
einen Schritt des Messens des Spiegels eines HCV-Genprodukts.

10. Kit zum Screenen einer Substanz, die eine anti-HCV-Wirkung hat, umfassend eine Zelle, die durch das Verfahren nach einem der Ansprüche 1 bis 6 hergestellt wurde; und ein Reagens zum Messen des Spiegels eines HCV-Genprodukts.

11. Verfahren zum Screenen einer Substanz, die eine anti-HCV-Wirkung hat, umfassend einen Schritt des Inkubierens einer Zelle mit einem Kandidaten-Agens, wobei die Zelle durch das Verfahren nach Anspruch 7 hergestellt wurde; und
einen Schritt des Messens des Spiegels eines Reportergenprodukts.

12. Kit zum Screenen einer Substanz, die eine anti-HCV-Wirkung hat, umfassend eine Zelle, die durch das Verfahren nach Anspruch 7 hergestellt wurde; und
ein Reagens zum Messen des Spiegels eines Reportergenprodukts.

13. Verfahren zur Herstellung einer kurierten Zelle, die von einer Li23-Zelle abstammt, umfassend einen Schritt des Züchtens der Zelle in einem Medium, das einen pharmazeutischen Wirkstoff enthält, das eine anti-virale Wirkung hat, wobei die Zelle durch das Verfahren nach einem der Ansprüche 1 bis 8 hergestellt wurde.

14. Verfahren zur Herstellung eines infektiösen HCV-Partikels, umfassend einen Schritt des Inkubierens der kurierten Zelle mit infektiöser HCV-RNA, wobei die Zelle durch das Verfahren nach Anspruch 13 hergestellt wurde.

## Revendications

1. Procédé de production d'une cellule répliquant un réplicon du VHC, comprenant une étape d'introduction d'ARN contenant une séquence de réplicon du VHC et une séquence de gène marqueur sélectionnable dans une cellule Li23 (FERM-BP-11150) ou une cellule guérie d'une cellule répliquant un réplicon du VHC dérivée d'une cellule Li23 ou une cellule guérie d'une cellule répliquant l'ARN du VHC pleine longueur dérivée d'une cellule Li23, la séquence du réplicon du VHC comprenant une séquence de bases codant pour une séquence d'acides aminés telle que représentée par SEQ ID NO : 2 contenant des substitutions d'acides aminés au niveau de Q1112R, K1609E et S2200R ou au niveau de Q1112R, P1115L et S2200R.

2. Procédé selon la revendication 1, dans lequel la séquence du réplicon du VHC est telle que représentée par SEQ ID NO : 3 ou 5.

3. Procédé de production d'une cellule répliquant l'ARN du VHC pleine longueur, comprenant une étape d'introduction d'ARN contenant une séquence du génome du VHC pleine longueur et un marqueur sélectif dans une cellule guérie d'une cellule répliquant un réplicon du VHC dérivée d'une cellule Li23 (FERM-BP-11150) ou une cellule guérie d'une cellule répliquant l'ARN du VHC pleine longueur dérivée d'une cellule Li23 (FERM-BP-11150) ;
la cellule répliquant un réplicon du VHC dérivée d'une cellule Li23 étant produite par un procédé comprenant
une étape d'introduction d'ARN contenant une séquence de réplicon du VHC et une séquence de gène de marqueur sélectionnable dans une cellule Li23 (FERM-BP-11150), une cellule guérie d'une cellule répliquant un réplicon du VHC dérivée d'une cellule Li23, ou une cellule guérie d'une cellule répliquant l'ARN du VHC pleine longueur dérivée d'une cellule Li23,
la séquence du réplicon du VHC comprenant une séquence de bases codant pour une séquence d'acides aminés telle que représentée par SEQ ID NO : 2 contenant des substitutions d'acides aminés au niveau de Q1112R, K1609E et S2200R ou au niveau de Q1112R, P1115L et S2200R.

4. Procédé selon la revendication 3, dans lequel la séquence du génome du VHC pleine longueur comprend une séquence de bases codant pour une séquence d'acides aminés telle que représentée par SEQ ID NO : 2 contenant des substitutions d'acides aminés au niveau de Q1112R, K1609E et S2200R ou au niveau de Q1112R, P1115L et S2200R.

5. Procédé selon la revendication 4, dans lequel la séquence de bases est telle que représentée par SEQ ID NO : 7 ou 9.

6. Procédé selon la revendication 4, dans lequel la séquence du génome du VHC pleine longueur contient une séquence de bases telle que représentée par SEQ ID NO : 11 ou 13.

7. Procédé selon l'une quelconque des revendications 3 à 6, dans lequel l'ARN contient en outre une séquence de gène rapporteur.

8. Procédé selon l'une quelconque des revendications 3 à 6, dans lequel l'ARN contient en outre une séquence de site d'entrée interne des ribosomes (IRES) exogène.

9. Procédé de criblage d'une substance dotée d'une action anti-VHC, comprenant une étape d'incubation d'une cellule préparée par le procédé selon l'une quelconque des revendications 1 à 6 avec un agent candidat ; et
une étape de mesure du taux d'un produit de gène du VHC.

10. Kit pour le criblage d'une substance dotée d'une action anti-VHC, comprenant une cellule préparée par le procédé selon l'une quelconque des revendications 1 à 6 ; et
un réactif pour la mesure du taux d'un produit de gène du VHC.

11. Procédé de criblage d'une substance dotée d'une action anti-VHC, comprenant une étape d'incubation d'une cellule préparée par le procédé selon la revendication 7 avec un agent candidat ; et
une étape de mesure du taux d'un produit de gène rapporteur.

12. Kit pour le criblage d'une substance dotée d'une action anti-VHC, comprenant une cellule préparée par le procédé selon la revendication 7 ; et
un réactif pour la mesure du taux d'un produit de gène rapporteur.

13. Procédé de production d'une cellule guérie dérivée d'une cellule Li23, comprenant une étape de culture de la cellule préparée par le procédé selon l'une quelconque des revendications 1 à 8 dans un milieu contenant un agent pharmaceutique doté d'une action antivirale.

14. Procédé de production d'une particule de VHC infectieux, comprenant une étape d'incubation de la cellule guérie préparée par le procédé selon la revendication 13 avec de l'ARN de VHC infectieux.
